# EUROPEAN PATENT SPECIFICATION

(11) **EP 2 847 230 B1**
(45) Date of publication and mention of the grant of the patent: **12.08.2020**
(21) Application number: 13788302.1
(22) Date of filing: 10.05.2013
(51) Int. Cl.: C07K 16/46, A61K 39/395, A61P 35/00, A61P 37/02, C07K 16/12, C07K 16/24, C07K 16/28, C07K 16/30

(54) **HETEROMULTIMER CONSTRUCTS OF IMMUNOGLOBULIN HEAVY CHAINS WITH MUTATIONS IN THE FC DOMAIN**
HETEROMULTIMERKONSTRUKTE AUS SCHWEREN IMMUNOGLOBULINKETTEN MIT MUTATIONEN IN DER FC-DOMÄNE
CONSTRUCTIONS HÉTÉROMULTIMÈRES DE CHAÎNES LOURDES D'IMMUNOGLOBULINES COMPRENANT DES MUTATIONS DANS LE DOMAINE FC

(30) Priority: 10.05.2012 US 201261645555 P
(43) Date of publication of application: 18.03.2015
(73) Proprietor: Zymeworks Inc., Vancouver, British Columbia V6H 3V9 (CA)
(72) Inventor: SPRETER VON KREUDENSTEIN, Thomas, Vancouver British Columbia V6H 9V9 (CA); ESCOBAR-CABRERA, Eric, Burnaby British Columbia V6H 9V9 (CA); NG, Gordon YiuKon, Vancouver British Columbia V6P 2N8 (CA); DIXIT, Surjit Bhimarao, Vancouver British Columbia V6H 9V9 (CA); LARIO, Paula Irene, Vancouver British Columbia V5X 2Y2 (CA); POON, David Kai Yuen, Vancouver British Columbia V6H 9V9 (CA)
(74) Representative: Elkington and Fife LLP
(86) International application number: PCT/CA2013/000471
(87) International publication number: WO 2013/166594

(56) References cited:
- WO-A1-2006/064136
- WO-A1-2009/089004
- WO-A1-2011/063348
- WO-A1-2012/058768
- WO-A1-2013/002362
- WO-A1-2013/063702
- US-A1- 2011 008 345
- DEMAREST S J ET AL: "Optimization of the Antibody CH3 Domain by Residue Frequency Analysis of IgG Sequences", JOURNAL OF MOLECULAR BIOLOGY, ACADEMIC PRESS, UNITED KINGDOM, vol. 335, no. 1, 2 January 2004 (2004-01-02), pages 41-48, XP004476444, ISSN: 0022-2836, DOI: 10.1016/J.JMB.2003.10.040
- ALTINTAS ISIL ET AL: "Targeting epidermal growth factor receptor in tumors: From conventional monoclonal antibodies via heavy chain-only antibodies to nanobodies", EUROPEAN JOURNAL OF PHARMACEUTICAL SCIENCES, vol. 45, no. 4, 28 October 2011 (2011-10-28), pages 399-407, XP028887665, ISSN: 0928-0987, DOI: 10.1016/J.EJPS.2011.10.015
- K. GUNASEKARAN ET AL: "Enhancing Antibody Fc Heterodimer Formation through Electrostatic Steering Effects: APPLICATIONS TO BISPECIFIC MOLECULES AND MONOVALENT IgG", JOURNAL OF BIOLOGICAL CHEMISTRY, vol. 285, no. 25, 18 June 2010 (2010-06-18), pages 19637-19646, XP55001947, ISSN: 0021-9258, DOI: 10.1074/jbc.M110.117382
- BELL, A. ET AL.: 'Differential tumor-targeting abilities of three single-domain antibodv formats' CANCER LETTERS vol. 289, no. 1, 28 August 2009, pages 81 - 90, XP026897079
- MERCHANT, A.M. ET AL.: 'An efficient route to human bispecific IgG' NATURE BIOTECHNOLOGY vol. 16, July 1998, pages 677 - 81, XP002141015

## Description

### Field of the Invention

The present disclosure generally provides polypeptide heterodimers, compositions thereof, and methods for making and using such polypeptide heterodimers. More specifically, provided herein are thermo-stable antibody constructs, said constructs comprising heterodimeric Fc domain, wherein said constructs are devoid of immunoglobulin light chains. In certain embodiments, the antibody constructs are multi-specific and/or multivalent. In certain embodiments, the antibody constructs are devoid of an immunoglobulin first constant (CH1) region.

### Background of the Invention

Bi-specific therapeutics are antibody-based molecules that can simultaneously bind two separate and distinct targets or different epitopes of the same antigen. Bi-specific antibodies are comprised of the immunoglobulin domain based entities and try to structurally and functionally mimic components of the antibody molecule. One use of bi-specific antibodies has been to redirect cytotoxic immune effector cells for enhanced killing of tumor cells, such as by antibody dependent cellular cytotoxicity (ADCC). In this context, one arm of the bi-specific antibody binds an antigen on the tumor cell, and the other binds a determinant expressed on effector cells. By cross-linking tumor and effector cells, the bi-specific antibody not only brings the effector cells within the proximity of the tumor cells but also simultaneously triggers their activation, leading to effective tumor cell-killing. Bi-specific antibodies have also been used to enrich chemo- or radiotherapeutic agents in tumor tissues to minimize detrimental effects to normal tissue. In this setting, one arm of the bi-specific antibody binds an antigen expressed on the cell targeted for destruction, and the other arm delivers a chemotherapeutic drug, radioisotope, or toxin. Going beyond bi-specifics, there is a need for protein therapeutics that achieve their efficacy by targeting multiple modalities concurrently. Such complex and novel biological effects can be obtained with protein therapeutics with multi-target binding and multi-functional aspects designed into the protein.

A robust scaffold that provides a framework to fuse other functional war-heads or target protein binding domains in order to design these multifunctional and multi-target binding therapeutics is required. Ideally, the scaffold should not only provide the framework but also makes available a number of other therapeutically relevant and valuable features to the designed therapeutic. A major obstacle in the general development of antibody based bi-specific and multifunctional therapeutics has been the difficulty of producing materials of sufficient quality and quantity for both preclinical and clinical studies.

Antigen-binding polypeptides that lack a light chain (i.e. comprising a single variable domains) are known in the art and include those derived from camelids or cartilaginous fish, for example. These types of antigen-binding polypeptides have been shown to have many advantages as antigen-binding fragments, for example, they are more thermostable, can penetrate tumors and cross the blood-brain-barrier, and they can bind to epitopes that other antigen-binding polypeptide fragments (such as Fabs and scFvs) cannot. Thus, monovalent or bi-specific antibodies that have this type of antigen-binding polypeptide fragment have been developed. However, existing technologies for preparing such monovalent or bi-specific antibodies are not ideal, and results in products that lack the purity and/or stability required to manufacture them in the amounts and quality necessary for therapeutic and clinical applications. There remains a need in the art for polypeptide constructs that comprise single variable domains as protein binding domains that are linked to a variant Fc region, said variant Fc comprising CH3 domains, which have been modified to select for heterodimers with an increased stability and purity, with Fc effector activities.

### SUMMARY OF THE INVENTION

Described herein is an isolated heteromultimer comprising: at least one immunoglobulin single domain antigen-binding construct attached to at least one monomer of a heterodimer Fc region; wherein the heterodimer Fc region comprises a variant CH3 domain comprising amino acid mutations that promote the formation of said heterodimer with stability comparable to a native homodimeric Fc; and wherein said isolated heteromultimer is devoid of immunoglobulin light chains.

The invention is an isolated heteromultimer comprising an immunoglobulin heterodimer Fc region comprising a first monomeric Fc polypeptide and a second monomeric Fc polypeptide and at least one single domain antigen-binding construct attached to one monomeric Fc polypeptide,
wherein the isolated heteromultimer is devoid of immunoglobulin light chains; wherein the heterodimer Fc region comprises a variant CH3 region comprising amino acid mutations that promote the formation of the heterodimer Fc region as compared to a homodimeric Fc region, the amino acid mutations comprising:(a) an amino acid mutation at position F405 and the amino acid mutations L351Y and Y407V in the first monomeric Fc polypeptide, and the amino acid mutation T394W in the second monomeric Fc polypeptide, wherein the amino acid mutation at position F405 is F405A, F405I, F405M, F405T, F405S, F405V or F405W, or(b) amino acid mutations F405A and Y407V in the first monomeric Fc polypeptide, and amino acid mutations T366L or T366I and T394W in the second monomeric Fc polypeptide, or(c) amino acid mutations L351Y, F405A and Y407V in the first monomeric Fc polypeptide, and an amino acid mutation at position T366 and the amino acid mutation T394W in the second monomeric Fc polypeptide, wherein the amino acid mutation at position T366 is T366A, T366I, T366L, T366M, T366Y, T366S, T366C, T366V or T366W; or(d) the amino acid mutation Y407A in the first monomeric Fc polypeptide, and the amino acid mutation K409F and one of the amino acid mutations T366A, T366C, T366L, T366M or T366S in the second monomeric Fc polypeptide;(e) the amino acid mutations L351Y and Y407A in the first monomeric Fc polypeptide, and the amino acid mutation K409F and one of the amino acid mutations T366A or T366V in the second monomeric Fc polypeptide; or(f) the amino acid mutations L35 IF and Y407A in the first monomeric Fc polypeptide, and the amino acid mutations T366A and K409F in the second monomeric Fc polypeptide;wherein the heterodimer Fc region is based on a human type G immunoglobulin (lgG);wherein the variant CH3 domain has a melting temperature (Tm) of 74°C or greater, and the heterodimer Fc region has a purity greater than about 90%;wherein the single domain antigen-binding construct is a single domain antibody (sdAb), a camelid immunoglobulin single variable domain (VhH), a cartilaginous fish antibody fragment (VNAR), an SH3-derived fynomer or a fibronectin-derived binding domain, and wherein the numbering of amino acids is according to the EU index as set forth in Kabat.

The invention is further defined by the claims. In certain embodiments is the isolated heteromultimer provided herein, comprising one single domain antigen-binding construct attached to one monomeric Fc polypeptide.

In some embodiments, is the isolated heteromultimer described herein comprising one single domain antigen-binding construct attached to one monomeric Fc polypeptide, and a second single domain antigen-binding construct attached to the other monomeric Fc polypeptide. In some embodiments is the isolated heteromultimer provided herein, wherein both single-domain antigen-binding constructs bind to the same epitope. In some embodiments is the isolated heteromultimer provided herein, wherein said one single domain antigen-binding construct binds to one epitope, and the second single domain antigen-binding construct binds to a different epitope. In some embodiments, the single domain antigen binding construct is selected from single domain antibodies (sdAb or VH), camelid nanobodies (VₕH), cartilaginous fish (V_{NAR}), SH3-derived fynomers, and fibronectin-derived binding domains. Provided in some embodiments herein is an isolated heteromultimer described herein, wherein the single domain antigen-binding construct is a camelid nanobody (VₕH). In certain embodiments, the single domain antigen-binding construct binds to one or more cytokines or chemokines selected from IL2, IFNa-2a/b, IFN-1a/b, IL-21, IL-17a, TNF, IL23, VEGF, and ANG2. In some embodiments, the single domain antigen-binding construct binds to one or more tumor associated antigens such as EpCam, EGFR, VEGFR, CEA, or GP100. In select embodiments, the single domain antigen-binding construct binds to one or more immunoregulatory antigens such as CD16, CD30, CD137, CD22, CD52, CD80, CD23, CD2, CD4, CD40, KIR, CD32b, CD25, LAG3, or B7-H3. In an embodiment, the single domain antigen-binding construct binds to one or more bacterial toxins such as Clostridium difficile toxin A, Clostridium difficile toxin B.

Provided herein are isolated heteromultimer described herein wherein the single domain antigen-binding construct binds to EGFR1. In an embodiment the single domain antigen-binding construct binds to the EGFR1 mutated variant EGFRvIII.

Provided herein is an isolated heteromultimer comprising: at least one single domain antigen-binding construct attached to at least one monomer of a heterodimer Fc region; wherein the heterodimer Fc region comprises a variant CH3 region comprising amino acid mutations that promote the formation of said heterodimer with stability comparable to a native homodimeric Fc; and wherein said isolated heteromultimer is devoid of immunoglobulin light chains.

Provided is an isolated heteromultimer comprising: at least one single domain antigen-binding construct attached to at least one monomer of a heterodimer Fc region; wherein the heterodimer Fc region comprises a variant constant domain comprising amino acid mutations that promote the formation of said heterodimer with stability comparable to a native homodimeric Fc; and wherein said isolated heteromultimer is devoid of immunoglobulin light chains.

Also provided is an isolated heteromultimer comprising: at least one single domain antigen-binding construct attached to at least one monomer of a heterodimer Fc region; wherein the heterodimer Fc region comprises a variant constant domain comprising amino acid mutations that promote the formation of said heterodimer with stability comparable to a native homodimeric Fc region; and wherein said isolated heteromultimer is devoid of immunoglobulin light chain and immunoglobulin first constant (CH1) region. Provided herein is the isolated heteromultimer described herein wherein said single domain antigen-binding construct is derived from a camelid or a cartilaginous fish. In an embodiment is the isolated heteromultimer described herein wherein said camelid is a llama. Also provided is the isolated heteromultimer described herein, wherein the heterodimer Fc region comprises a variant CH3 domain comprising amino acid mutations to promote heterodimer formation with increased stability wherein said amino acid mutations promote the formation of heterodimer Fc region with increased stability as compared to a CH3 domain that does not comprise amino acid mutations, and wherein the variant CH3 domain has a melting temperature (Tm) of about 70 °C or greater. In an embodiment is the isolated heteromultimer described herein, wherein the heterodimer Fc region does not comprise an additional disulfide bond in the CH3 domain relative to a wild type Fc region. In a further embodiment is the isolated heteromultimer described herein, wherein the heterodimer Fc region comprises an additional disulfide bond in the variant CH3 domain relative to a wild type Fc region, with the proviso that the melting temperature (Tm) of about 70 °C or greater for the CH3 domain is in the absence of the additional disulfide bond. In another embodiment is the isolated heteromultimer described herein, wherein the heterodimer Fc region has a purity greater than about 90%. In a further embodiment is the isolated heteromultimer described herein, wherein the heterodimer Fc region has a purity of about 98% or greater. In another embodiment is the isolated heteromultimer described herein, wherein the Tm is about 74 °C or greater. In an embodiment is the isolated hereomultimer, wherein a first Fc polypeptide comprises amino acid modification at positions F405 and Y407 and a second Fc polypeptide comprises amino acid modification at position T394. In another embodiment is the isolated hereomultimer wherein the first Fc polypeptide comprises one or more amino acid modifications selected from L351Y, F405A and Y407V, and the second Fc polypeptide comprises one or more amino acid modifications selected from T366L, T366I, K392L, K392M and T394W. In a further embodiment is the isolated heteromultimer described herein, wherein a first Fc polypeptide comprises amino acid modifications at positions D399 and Y407 and a second Fc polypeptide comprises amino acid modification at positions K409 and T411.

Provided herein is an isolated heteromultimer described herein, said heterodimer Fc region comprising: a first monomeric Fc polypeptide comprising a first modified CH3 domain comprising at least three amino acid modifications as compared to a wild-type CH3 domain polypeptide, and a second monomeric Fc polypeptide comprising a second modified CH3 domain comprising at least three amino acid modifications as compared to a wild-type CH3 domain polypeptide; wherein one of said first and second CH3 domain comprises an amino acid modification of K392J wherein J is selected from L, I, M or an amino acid with a side chain volume not substantially larger than the side chain volume of K; wherein said first and second modified CH3 domain polypeptides preferentially form a heterodimeric CH3 domain with a melting temperature (Tm) of at least about 74°C and a purity of at least 95%; and wherein at least one amino acid modification is not of an amino acid which is at the interface between said first and said second CH3 domain polypeptides.

In an embodiment is the isolated heteromultimer, comprising at least one T350X modification, wherein X is a natural or non-natural amino acid selected from valine, isoleucine, leucine, methionine, and derivatives or variants thereof. In a further embodiment is the isolated heteromultimer comprising at least one T350V modification. In some embodiments each of said first and second Fc polypeptides further comprises a T350V modification. In some embodiments the Fc heterodimer domain has a Tm of about 77°C or greater.

In some embodiments is the isolated heteromultimer described herein, wherein at least one monomeric Fc polypeptide comprises the modification S400Z, wherein Z is selected from a positively charged amino acid and a negatively charged amino acid. In an embodiment is the isolated heteromultimer, wherein said first Fc polypeptide comprises an amino acid modification selected from S400E, S400D, S400K and S400R. In another embodiment, one of said first and second Fc polypeptides comprises the amino acid modification selected form S400E and S400R, and the other Fc polypeptide comprises an amino acid modification at position N390. In a further embodiment is the isolated heteromultimer, comprising the modification N390Z, wherein Z is selected from a positively charged amino acid and a negatively charged amino acid. In an embodiment is the isolated hereomultimer described herein, said second Fc polypeptide comprising the amino acid modification N390R or N390K.

In an embodiment is provided an isolated heteromultimer described herein, wherein said first Fc polypeptide is a modified CH3 domain polypeptide comprising the amino acid modification S400E and said second Fc polypeptide is a modified CH3 domain polypeptide comprising the amino acid modification N390R. In an embodiment is the isolated heteromultimer described herein, one said Fc polypeptide comprising the amino acid modification Q347R and the other Fc polypeptide comprising the amino acid modification K360E.

Provided is an isolated heteromultimer described herein, wherein the Fc polypeptide comprises at least one amino acid modification selected from T366V, T366I, T366A, T366M, T366L, K409F, T411E and T411D, and the second Fc polypeptide comprises at least one amino acid modification selected from L351Y, Y407A, Y407I, Y407V, D399R and D399K. In an embodiment is the isolated heteromultimer wherein the heterodimer Fc region further comprises a variant CH2 domain comprising asymmetric amino acid modifications to promote selective binding of a Fcgamma receptor.

In an embodiment is the isolated heteromultimer described herein, wherein the variant CH2 domain selectively binds Fcgammallla receptor as compared to wild-type CH2 domain.

In some embodiments is the isolated heteromultimer provided herein comprising a Fc construct based on a type G immunoglobulin (IgG). In certain embodiments is the isolated heteromultimer, wherein said IgG is one of IgG1, IgG2 IgG3 and IgG4. In some embodiments is the isolated heteromultimer comprising a Fc construct based on Immunoglobulin M (IgM), Immunoglobulin A (IgA), Immunoglobulin D (IgD), or Immunoglobulin E (IgE).

Provided in some embodiments is the isolated heteromultimer described herein, wherein said heteromultimer is a bispecific antibody or a multispecific antibody.

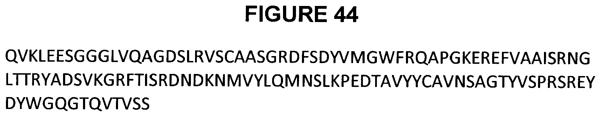

Provided in some embodiments is the isolated heteromultimer described herein, wherein at least one single domain antigen binding construct binds EGFR or EGFRvIII. In some embodiments is the isolated heteromultimer described herein wherein said EGFR or EGFRvIII binding construct is derived from an antibody or fragment thereof. In certain embodiments, said EGFR or EGFRvIII binding construct is a heavy chain antibody construct. In some embodiments said heavy chain antibody construct is a camelid construct. In an embodiments, said camelid construct comprises the sequence shown in Figure 44.

Provided is a composition comprising the isolated heteromultimer described herein and a pharmaceutically acceptable carrier.

Provided is a mammalian host cell comprising nucleic acid encoding the isolated heteromultimer described herein.

In an embodiment is the isolated heteromultimer described herein, wherein the single domain antigen-binding construct competes for binding with at least one therapeutic antibody. In an embodiment is the isolated heteromultimer, wherein said at least one therapeutic antibody is selected from the group consisting of abagovomab, adalimumab, alemtuzumab, aurograb, bapineuzumab, basiliximab, belimumab, bevacizumab, briakinumab, canakinumab, catumaxomab, certolizumab pegol, cetuximab, daclizumab, denosumab, efalizumab, galiximab, gemtuzumab ozogamicin, golimumab, ibritumomab tiuxetan, infliximab, ipilimumab, lumiliximab, mepolizumab, motavizumab, muromonab, mycograb, natalizumab, nimotuzumab, ocrelizumab, ofatumumab, omalizumab, palivizumab, panitumumab, pertuzumab, ranibizumab, reslizumab, rituximab, teplizumab, tocilizumab/atlizumab, tositumomab, trastuzumab, ProxiniumTM, RencarexTM, ustekinumab, and zalutumumab.

In an embodiment is a method of treating cancer in a patient having a cancer characterized by a cancer antigen, said method comprising administering to said patient a therapeutically effective amount of an isolated heteromultimer described herein. In an embodiment is the method of treating cancer, wherein said cancer is characterized by overexpression of EGFR or EFGRvIII.

Provided is a method of treating cancer cells expressing EGFR or EGFRvIII, comprising contacting said cells with an amount of a heteromultimer provided herein. I some embodiments is the said cancer cell is at least one of a breast cancer cell, a lung cancer cell, an anal cancer cell and a glioblastoma.

In some embodiments is the method of treating cancer described herein, comprising administration of said heteromultimer, in addition to another therapeutic molecule. In some embodiments, said therapeutic molecule is conjugated to the heteromultimer.

Provided is a method of treating immune disorders in a patient having an immune disorder characterized by an immune antigen, said method comprising administering to said patient a therapeutically effective amount of an isolated heteromultimer described herein.

In an aspect described herein is an isolated heteromultimer comprising: at least one immunoglobulin single domain antigen-binding construct attached to at least one monomer of a heterodimer Fc region; wherein the heterodimer Fc region comprises a variant CH3 domain comprising amino acid mutations that promote the formation of said heterodimer with stability comparable to a native homodimeric Fc; and wherein said isolated heteromultimer is devoid of immunoglobulin first constant (CH1) region and immunoglobulin light chains.

In an aspect described herein is an isolated heteromultimer comprising: at least one immunoglobulin single domain antigen-binding construct attached to at least one monomer of a heterodimer Fc region; wherein the heterodimer Fc region comprises a variant CH3 domain comprising amino acid mutations that promote the formation of said heterodimer with stability comparable to a native homodimeric Fc; and wherein said isolated heteromultimer is devoid of immunoglobulin first and second constant domains (CH1 & CH2) and immunoglobulin light chains.

In certain embodiments of the isolated heteromultimers provided herein, the variant CH3 domain has a melting temperature (Tm) of about 70 °C or greater. In certain embodiments, the variant CH3 domain has a melting temperature (Tm) of at least about 75 °C. In some embodiments, the variant CH3 domain has a melting temperature (Tm) of at least about 80 °C.

In some embodiments of the isolated heteromultimers provided herein, the heterodimer Fc region further comprises a variant CH2 domain comprising at least one asymmetric amino acid modification to promote selective binding to certain Fcgamma receptors. In one embodiment the variant CH2 domain selectively binds Fcgamma IIIa receptor as compared to wild-type CH2 domain.

In a particular embodiment of the invention, the heteromultimers described herein are the product of the expression in a prokaryotic or in a eukaryotic host cell, of a DNA or of a cDNA having the sequence of an immunoglobulin devoid of immunoglobulin first constant (CH1) region. In certain embodiments, the at least one immunoglobulin heavy chain variable region is from an immunoglobulin devoid of light chains, said immunoglobulin obtainable from lymphocytes or other cells of Camelids such as but not restricted to Dromedaries, Bactrian camels, llamas, alpacas, vicugnas, and guanacos. In a specific embodiment, the at least one immunoglobulin heavy chain variable region is from an immunoglobulin devoid of light chains, said immunoglobulin obtained from lymphocytes or other cells of a llama.

In a particular embodiment of the invention, the heteromultimers described herein are the product of the expression in a prokaryotic or in a eukaryotic host cell, of a DNA or of a cDNA. In certain embodiments, at least one immunoglobulin heavy chain variable region and/or Fc heterodimer is from an immunoglobulin devoid of light chains obtainable from cartilaginous fishes such as but not restricted to sharks, rays, skates, ghost sharks, ratfish, elephantfish and rabbitfish. In a specific embodiment, at least one immunoglobulin heavy chain variable region and/or Fc heterodimer is from an immunoglobulin devoid of light chains, said immunoglobulin obtained from a shark.

There is described in another aspect an isolated heteromultimer comprising at least one immunoglobulin single domain antigen-binding construct attached to at least one monomer of a heterodimer Fc region, wherein the heterodimer Fc region comprises a variant CH3 domain comprising amino acid mutations, wherein said isolated heteromultimer is devoid of immunoglobulin first constant (CH1) region, immunoglobulin light chains and optionally devoid of immunoglobulin second constant (CH2) region, wherein the variant CH3 domain has a melting temperature (Tm) of about 70 °C or greater, and wherein said variant CH3 domain results in the formation of heterodimer Fc region with stability comparable to a CH3 domain in native IgG1 antibody.

In one embodiment, the heterodimer Fc region does not comprise an additional disulfide bond in the CH3 domain relative to a wild type Fc region. In an alternative embodiment, the heterodimer Fc region comprises at least one additional disulfide bond in the variant CH3 domain relative to a wild type Fc region, with the proviso that the melting temperature (Tm) of about 70 °C or greater is in the absence of the additional disulfide bond. In another embodiment, the heterodimer Fc region comprises at least one additional disulfide bond in the variant CH3 domain relative to a wild type Fc region, and wherein the variant CH3 domain has a melting temperature (Tm) of about 77.5 °C or greater.

Also described herein is an isolated heteromultimer comprising at least one immunoglobulin single domain antigen-binding construct attached to at least one monomer of a heterodimer Fc region, wherein the heterodimer Fc region comprises a variant CH3 domain comprising amino acid mutations, wherein said isolated heteromultimer is devoid of immunoglobulin light chains and immunoglobulin first constant (CH1) domain and optionally devoid of the immunoglobulin second constant (CH2) domain, wherein the variant CH3 domain has a melting temperature (Tm) of about 70 °C or greater and the heterodimer Fc region is formed with a purity greater than about 90%, or the heterodimer Fc region is formed with a purity of about 95% or greater or the heterodimer Fc region is formed with a purity of about 98% or greater.

In certain embodiments, the immunoglobulins described herein, which are devoid of light chains, are such that the variable domains of their heavy chains (V_{H}) have properties differing from those of the V_{H} in four-chain immunoglobulin. In some embodiments, the variable domain of a heavy-chain immunoglobulin described herein has no interaction sites for V_{L} such as in the case of heavy chain immunoglobulins from cartilaginous fish. In some embodiments, the variable domain of a heavy-chain immunoglobulin described herein has no normal interaction sites with V_{L} or with C_{H 1} domain, neither of which exists in heavy chain immunoglobulins from Camelids and some cartilaginous fish.

In certain embodiments of the heteromultimer described herein, the at least one immunoglobulin heavy chain is attached to at least one monomer of the heterodimer Fc region by means of a linker. In certain embodiments, the at least one single domain antigen-binding construct is attached to at least one monomer of the heterodimer Fc region by means of a hinge region. Linkers and/or hinge regions of variable lengths are utilized in the heteromultimers provided herein. One of skill in the art can appreciate that the length of the linker and/or hinge region will participate to the determination of the distance separating the antigen binding sites. In certain embodiments provided herein, the hinge region comprises from 0 to 50 amino acids. In certain embodiments, the sequence of hinge region is as follows:
GTNEVCKCPKCP
In an embodiment, the sequence of the hinge region is:
EPKIPQPQPKPQPQPQPQPKPQPKPEPECTCPKCP

In certain embodiments, at least one single domain antigen-binding construct and/or modified Fc region utilized in the heteromultimer constructs described herein comprises type G immunoglobulins for instance immunoglobulins which are defined as immunoglobulins of class 2 (IgG2) or immunoglobulins of class 3 (IgG3). In some embodiments, at least one single domain antigen-binding construct and/or modified Fc region utilized in the heteromultimer constructs described herein comprises immunoglobulin M, or IgM. In some embodiments, at least one single domain antigen-binding construct and/or modified Fc region utilized in the heteromultimer constructs described herein comprises immunoglobulin A, or IgA. In some embodiments, at least one single domain antigen-binding construct and/or modified Fc region utilized in the heteromultimer constructs described herein comprises immunoglobulin D, or IgD. In some embodiments, at least one single domain antigen-binding construct and/or modified Fc region utilized in the heteromultimer constructs described herein comprises Immunoglobulin E, or IgE.

In certain embodiments, the heteromultimers described herein comprise an Fc portion that is derived from IgG (e.g. an IgG1, IgG2, IgG3 or IgG4) or IgA (or IgM, IgD). According to one specific aspect certain embodiments comprise a modified "IgE-derived Fc portion" (i.e. an Fc portion that is derived from IgE).

In certain embodiments, the hinge connecting the variable domain of the heavy chain and the Fc portion of the heavy chain is derived from the hinge sequence of an IgG isotype (e.g. an IgG1, IgG2, IgG3 or IgG4) or IgA (or IgM, IgD, IgE).

In certain embodiments described herein, the variable heavy chains may be a domain antibody (or an amino acid sequence that is suitable for use as a domain antibody), a single domain antibody (or an amino acid sequence that is suitable for use as a single domain antibody), a "dAb" (or an amino acid sequence that is suitable for use as a dAb) or a Nanobody (as defined herein, and including but not limited to a V_{HH} sequence); other single variable domains, or any suitable fragment of any one thereof. For a general description of (single) domain antibodies, reference is also made to the prior art cited above, as well as to EP 0 368 684. For the term "dAb's", reference is for example made to Ward et al. (Nature 1989 Oct. 12; 341 (6242): 544-6), to Holt et al. (Trends Biotechnol., 2003, 21(11):484-490); as well as to for example WO 04/068820, WO 06/030220, WO 06/003388 and other published patent applications of Domantis Ltd. In some embodiments, the variable heavy chains comprise single domain antibodies or single variable domains can be derived from certain species of shark (for example, the so-called "IgNAR domains", see for example WO 05/18629).

Provided herein are isolated heteromultimers according to the claims comprising: at least one single domain antigen-binding construct attached to at least one monomer of a heterodimer Fc region; wherein the heterodimer Fc region comprises a variant constant domain comprising amino acid mutations that promote the formation of said heterodimer with stability comparable to a native homodimeric Fc; and wherein said isolated heteromultimer is devoid of immunoglobulin light chains.

Further described herein is an isolated heteromultimer comprising: at least one single domain antigen-binding construct attached to at least one monomer of a heterodimer Fc region; wherein the heterodimer Fc region comprises a variant constant domain comprising amino acid mutations that promote the formation of said heterodimer with stability comparable to a native homodimeric Fc region; and wherein said isolated heteromultimer is devoid of immunoglobulin light chain and immunoglobulin first constant (CH1) region.

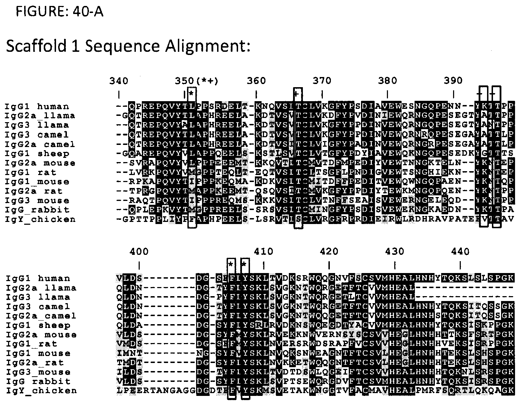

In certain embodiments, the heteromultimers described herein comprise an Fc portion or constant domain that is derived from antibodies from any animal origin (e.g. human, murine, donkey, sheep, rabbit, goat, guinea pig, camel, horse, or chicken) which maintain sequence similarity of the constant domains (Figures 40A-40B). In a specific embodiment the constant domain is derived from cartilaginous fishes such as but not restricted to sharks, rays, skates, ghost sharks, ratfish, elephantfish and rabbitfish. In specific embodiments, the heteromultimers described herein comprise a constant domain that is derived from antibodies from camels such as but not restricted to Dromedaries, Bactrian camels, llamas, alpacas, vicugnas, and guanacos.

Single domain antigen-binding constructs according to certain embodiments provided herein are obtainable by purification from serum of camelids such as but not restricted to llamas using processes of purification well known in the art.

In certain embodiments provided herein, the variable region of immunoglobulins of the heteromultimers comprises frameworks (FW) and complementarity determining regions (CDR), especially 4 frameworks and 3 complementarity regions. It is distinguished from the four-chain immunoglobulins especially by the fact that this variable region can itself contain an antigen binding site or several, without contribution of the variable region of a light chain which is absent.

Also provided in certain embodiments is the isolated heteromultimer comprising a heterodimer Fc region, wherein the heterodimer Fc region comprises a variant CH3 domain comprising one or more amino acid mutations that result in the formation of heterodimer Fc region with increased stability, wherein the variant CH3 domain has a melting temperature (Tm) of about 70 °C or greater or the Tm is about 71°C or greater or the Tm is about 74°C or greater. In another embodiment, the heterodimer Fc region is formed in solution with a purity of about 98% or greater, and Tm about 73°C or wherein the heterodimer Fc region is formed with a purity of about 90% or greater, and Tm about 75°C.

Provided in certain embodiments is the isolated heteromultimer comprising at least one single domain antigen-binding construct attached to at least one monomer of a heterodimer Fc region, wherein the heterodimer Fc region comprises a first and a second CH3 domain polypeptides, wherein at least one of said first and second CH3 domain polypeptides comprises amino acid modification T350V. Provided in certain embodiments is an isolated heteromultimer comprising at least one single domain antigen-binding construct attached to at least one monomer of a heterodimer Fc region, wherein the heterodimer Fc region comprises a first CH3 domain polypeptide comprising amino acid modification T350V and a second CH3 domain polypeptide also comprising amino acid modification T350V. Provided in certain embodiments is an isolated heteromultimer comprising at least one single domain antigen-binding construct attached to at least one monomer of a heterodimer Fc region, wherein the heterodimer Fc region comprises a first CH3 domain polypeptide comprising amino acid modification at positions F405 and Y407 and a second CH3 domain polypeptide comprising amino acid modification at position T394. In certain embodiments, a first CH3 domain polypeptide comprises amino acid modifications at positions D399 and Y407 and a second CH3 domain polypeptide comprises amino acid modification at positions K409 and T411. Provided in certain embodiments is an isolated heteromultimer comprising at least one single domain antigen-binding construct attached to at least one monomer of a heterodimer Fc region, wherein the heterodimer Fc region comprises a first CH3 domain polypeptide comprising amino acid modifications L351Y and Y407A and a second CH3 domain polypeptide comprising amino acid modifications T366A and K409F. In one aspect, the first CH3 domain polypeptide or the second CH3 domain polypeptide comprises a further amino acid modification at position T411, D399, S400, F405, N390, or K392. The amino acid modification at position T411 is selected from T411N, T411R, T411Q, T411K, T411D, T411E or T411W. The amino acid modification at position D399 is selected from D399R, D399W, D399Y or D399K.The amino acid modification at position S400 is selected from S400E, S400D, S400R, or S400K. The amino acid modification at position F405 is selected from F4051, F405M, F405T, F405S, F405V or F405W. The amino acid modification at position N390 is selected from N390R, N390K or N390D. The amino acid modification at position K392 is selected from K392V, K392M, K392R, K392L, K392F or K392E.

In certain embodiments is provided an isolated heteromultimer comprising a heterodimer Fc region, wherein the heterodimer Fc region comprises a first CH3 domain polypeptide comprising amino acid modifications T350V and L351Y and a second CH3 domain polypeptide also comprising amino acid modifications T350V and L351Y.

In another embodiment is provided the isolated heteromultimer comprising at least one single domain antigen-binding construct attached to at least one monomer of a heterodimer Fc region, wherein the heterodimer Fc region comprises a first CH3 domain polypeptide comprising amino acid modification Y407A and a second CH3 domain polypeptide comprising amino acid modifications T366A and K409F. In one aspect the first CH3 domain polypeptide or the second CH3 domain polypeptide comprises further amino acid modifications K392E, T411E, D399R and S400R. In another aspect, the first CH3 domain polypeptide comprises amino acid modification D399R, S400R and Y407A and the second CH3 domain polypeptide comprises amino acid modification T366A, K409F, K392E and T411E. In a further embodiment the variant CH3 domain has a melting temperature (Tm) of about 74°C or greater and the heterodimer has a purity of about 95% or greater.

Provided in another embodiment is the isolated heteromultimer comprising at least one single domain antigen-binding construct attached to at least one monomer of a heterodimer Fc region, wherein the heterodimer Fc region comprises a first CH3 domain polypeptide comprising an amino acid modification at positions L351 and amino acid modification Y407A and a second CH3 domain polypeptide comprises an amino acid modification at position T366 and amino acid modification K409F. In one aspect the amino acid modification at position L351 is selected from L351Y, L351I, L351D, L351R or L351F. In another aspect, the amino acid modification at position Y407 is selected from Y407A, Y407V or Y407S. In yet another aspect the amino acid modification at position T366 is selected from T366A, T366I, T366L, T366M, T366Y, T366S, T366C, T366V or T366W. In one embodiment the variant CH3 domain has a melting temperature (Tm) of about 75°C or greater and the heterodimer has a purity of about 90% or greater.

Provided in another embodiment is the isolated heteromultimer comprising at least one single domain antigen-binding construct attached to at least one monomer of a heterodimer Fc region, wherein the heterodimer Fc region comprises a first CH3 domain polypeptide comprising an amino acid modification at position F405 and amino acid modifications L351Y and Y407V and a second CH3 domain polypeptide comprises amino acid modification T394W. In one aspect the first CH3 domain polypeptide or the second CH3 domain polypeptide comprise an amino acid modification at positions K392, T411, T366, L368 or S400. The amino acid modification at position F405 is F405A, F405I, F405M, F405T, F405S, F405V or F405W. The amino acid modification at position K392 is K392V, K392M, K392R, K392L, K392F or K392E. The amino acid modification at position T411 is T411N, T411R, T411Q, T411K, T411D, T411E or T411W. The amino acid modification at position S400 is S400E, S400D, S400R or S400K. The amino acid modification at position T366 is T366A, T366I, T366L, T366M, T366Y, T366S, T366C, T366V or T366W. The amino acid modification at position L368 is L368D, L368R, L368T, L368M, L368V, L368F, L368S and L368A.

In another embodiment is provided the isolated heteromultimer comprising at least one single domain antigen-binding construct attached to at least one monomer of a heterodimer Fc region, wherein the heterodimer Fc region comprises a first CH3 domain polypeptide comprising an amino acid modifications L351Y, F405A and Y407V and a second CH3 domain polypeptide comprises amino acid modification T394W. In one aspect, the second CH3 domain polypeptide comprises amino acid modification T366L or T366I.

In yet another embodiment is provided the isolated heteromultimer comprising at least one single domain antigen-binding construct attached to at least one monomer of a heterodimer Fc region, wherein the heterodimer Fc region comprises a first CH3 domain polypeptide comprising at least one of amino acid modifications Y349C, F405A and Y407V and a second CH3 domain polypeptide comprises amino acid modifications T366I, K392M and T394W.

In certain embodiments is provided the isolated heteromultimer comprising a heterodimer Fc region, wherein the heterodimer Fc region comprises a first CH3 domain polypeptide comprising amino acid modifications L351Y, F405A and Y407V and a second CH3 domain polypeptide comprises amino acid modifications K392M and T394W, and one of T366L and T3661.

In another embodiment is provided the isolated heteromultimer comprising at least one single domain antigen-binding construct attached to at least one monomer of a heterodimer Fc region, wherein the heterodimer Fc region comprises a first CH3 domain polypeptide comprising amino acid modifications F405A and Y407V and a second CH3 domain polypeptide comprises amino acid modifications T366L and T394W.

In another embodiment is provided the isolated heteromultimer comprising at least one single domain antigen-binding construct attached to at least one monomer of a heterodimer Fc region, wherein the heterodimer Fc region comprises a first CH3 domain polypeptide comprising amino acid modifications F405A and Y407V and a second CH3 domain polypeptide comprises amino acid modifications T366I and T394W. In certain embodiments of the heteromultimer is provided bi-specific antibody or a multispecific antibody.

In another embodiment is provided a composition comprising a heteromultimer of the invention and a pharmaceutically acceptable carrier.

In another embodiment is provided a host cell comprising nucleic acid encoding the heteromultimer of the invention.

In certain embodiments is provided the heteromultimer, wherein target binding by the heteromultimer is competitive to at least one other therapeutic antibody. In one aspect the therapeutic antibody is selected from the group consisting of abagovomab, adalimumab, alemtuzumab, aurograb, bapineuzumab, basiliximab, belimumab, bevacizumab, briakinumab, canakinumab, catumaxomab, certolizumab pegol, cetuximab, daclizumab, denosumab, efalizumab, galiximab, gemtuzumab ozogamicin, golimumab, ibritumomab tiuxetan, infliximab, ipilimumab, lumiliximab, mepolizumab, motavizumab, muromonab, mycograb, natalizumab, nimotuzumab, ocrelizumab, ofatumumab, omalizumab, palivizumab, panitumumab, pertuzumab, ranibizumab, reslizumab, rituximab, teplizumab, tocilizumab/atlizumab, tositumomab, trastuzumab, Proxinium, Rencarex, ustekinumab, and zalutumumab.

In another embodiment of the heteromultimer of the invention is provided a method of treating cancer in a patient having a cancer characterized by a cancer antigen, said method comprising administering to said patient a therapeutically effective amount of a heteromultimer.

In another embodiment of the heteromultimer of the invention is provided a method of treating immune disorders in a patient having an immune disorder characterized by an immune antigen, said method comprising administering to said patient a therapeutically effective amount of a heteromultimer.

In yet another embodiment is provided the isolated heteromultimer comprising at least one single domain antigen-binding construct attached to at least one monomer of a heterodimer Fc region, wherein the heterodimer Fc region comprises a variant CH3 domain comprising amino acid mutations to promote heterodimer formation with increased stability and wherein the variant CH3 domains are selected from the variants listed in Table 1, Table 6 or Table 7.

In certain embodiments are provided heteromultimers that comprise modified anti-idiotypes antibodies belonging to the heavy chain immunoglobulin classes. Such anti-idiotypes can be produced against human or animal idiotypes. A property of these anti-idiotypes is that they can be used as idiotypic vaccines, in particular for vaccination against glycoproteins or glycolipids and where the carbohydrate determines the epitope.

Provided herein are cells or organisms in which heteromultimers described herein have been cloned, said heteromultimers comprising at least one single domain antigen-binding construct attached to at least one monomer of a heterodimer Fc region. Such cells or organisms can be used for the purpose of producing heteromultimers having a desired preselected specificity, or corresponding to a particular repertoire. They can also be produced for the purpose of modifying the metabolism of the cell which expresses them. In an embodiment, the heteromultimers comprising at least one single domain antigen-binding construct attached to at least one monomer of a heterodimer Fc region is produced in plant cells, especially in transgenics plants.

### BRIEF DESCRIPTION OF THE FIGURES

**Figure 1** is a graphical 3-D structure of a wild type antibody showing the CH3 (top), CH2 (middle) and receptor regions. The dotted line rectangle on the left hand side is expanded to the right hand side showing two regions, Region 1 and Region 2, of the target area of CH3;
**Figure 2** is a graphical 3-D representation of showing the wild type residue at position 368;

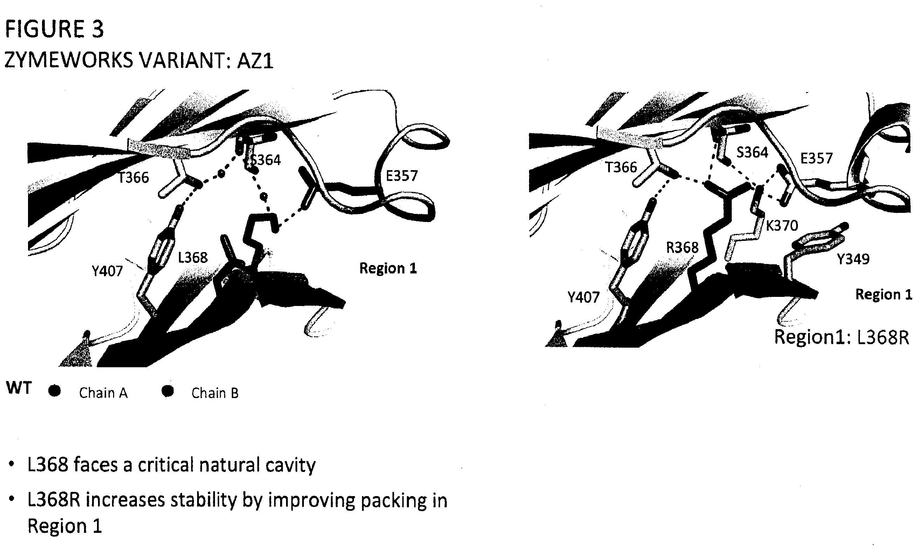

**Figure 3** is a graphical 3-D representation of Region 1 showing mutated position 368;
**Figure 4** is a graphical 3-D representation of additional mutations in Region 2;

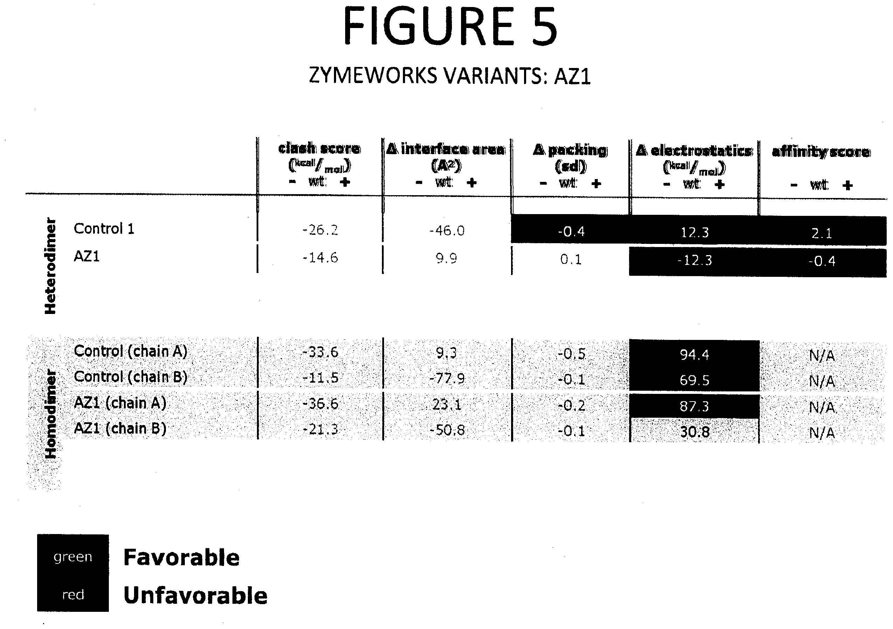

**Figure 5** is a table of in silico calculations for clash score, interface area difference, packing different, electrostatic energy difference and overall "affinity score" for the first three variants AZ1, AZ2 and AZ3;

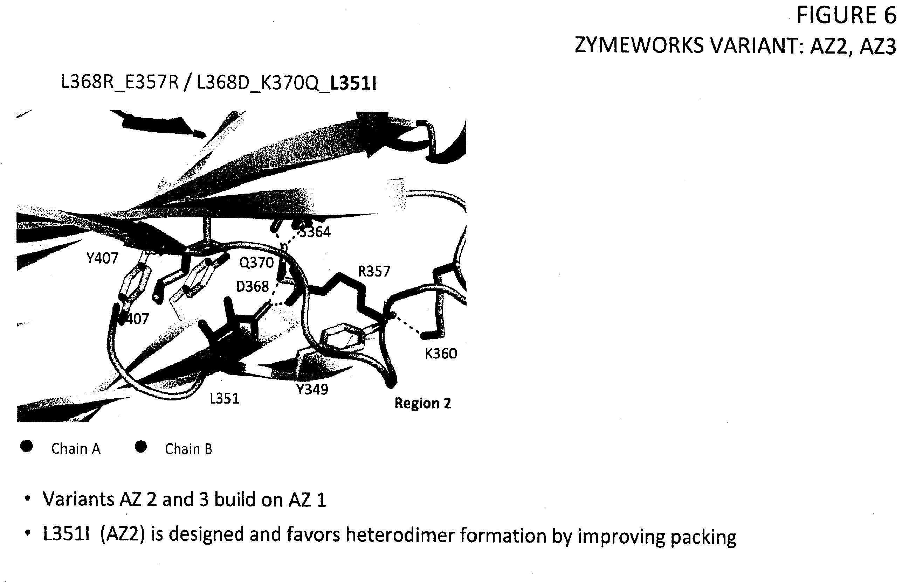

**Figure 6** shows a graphical 3-D image showing variants AZ2 and AZ3, which are "built onto" variant AZ1;

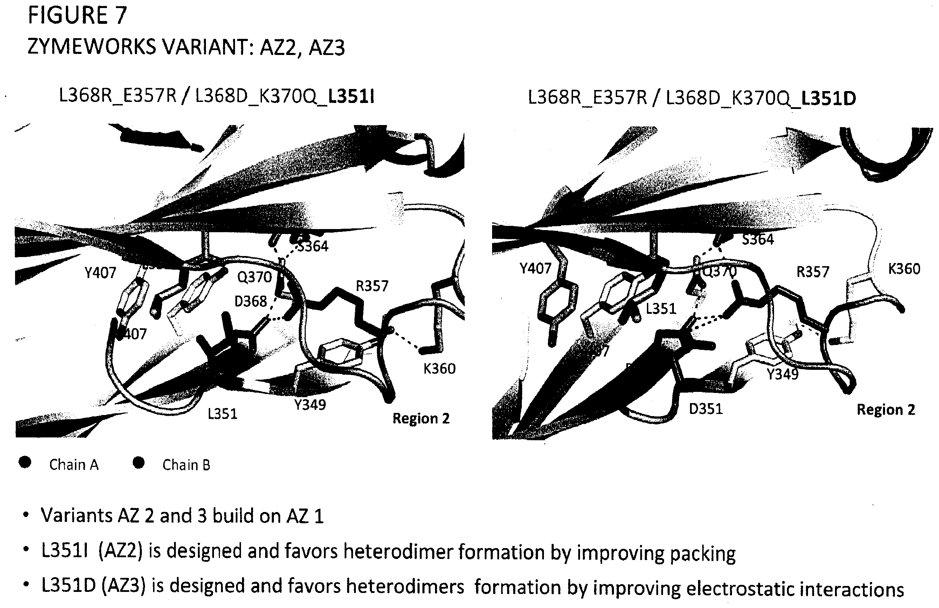

**Figure 7** show graphical 3-D representations of AZ2 and AZ3 variants;

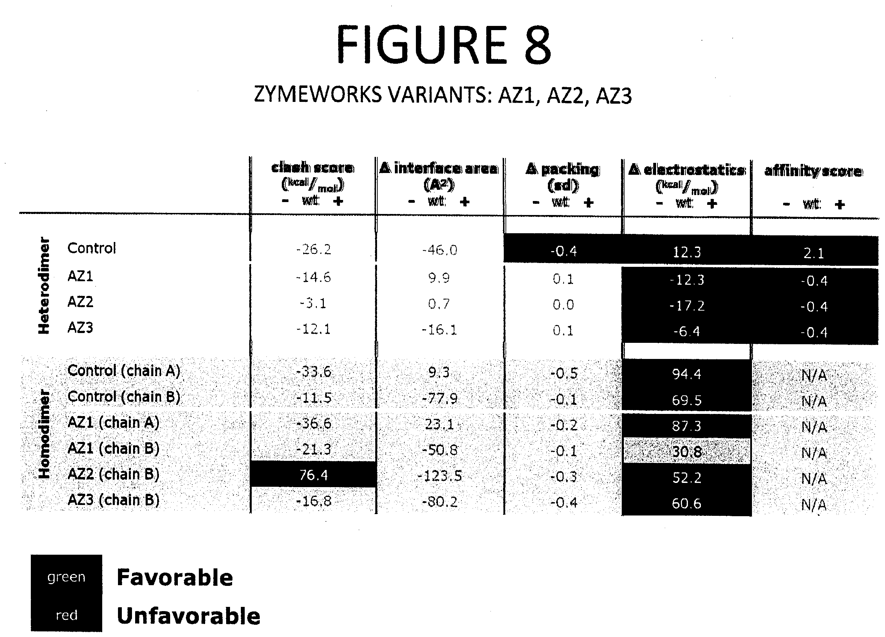

**Figure 8** shows a table as in Figure 5 but for AZ1, AZ2 and AZ3 heterodimers, and potential homodimers. Affinity score is not shown for homodimers, at it is not relavant;

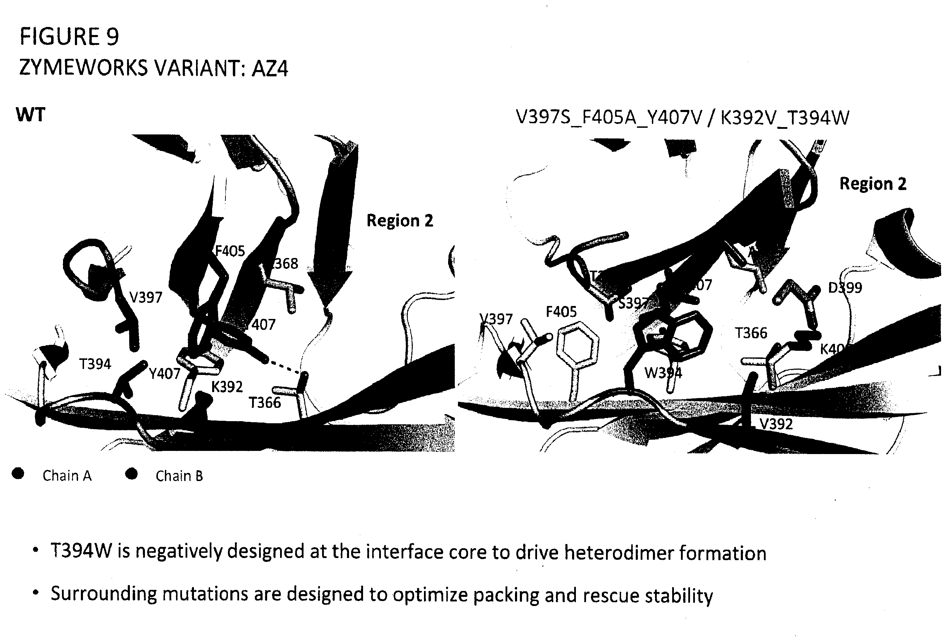

**Figure 9** is a graphical representation of a 3-D representation of wild type (left) and mutated AZ4 (right);

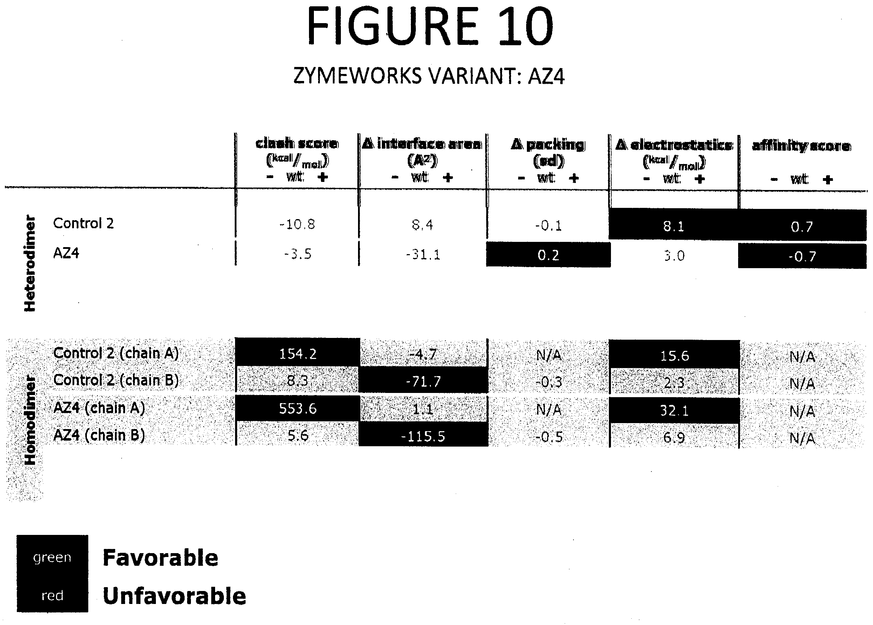

**Figure 10** is a table as Figure 5 showing in silico calculations for AZ4 heterodimer and potential homodimers;

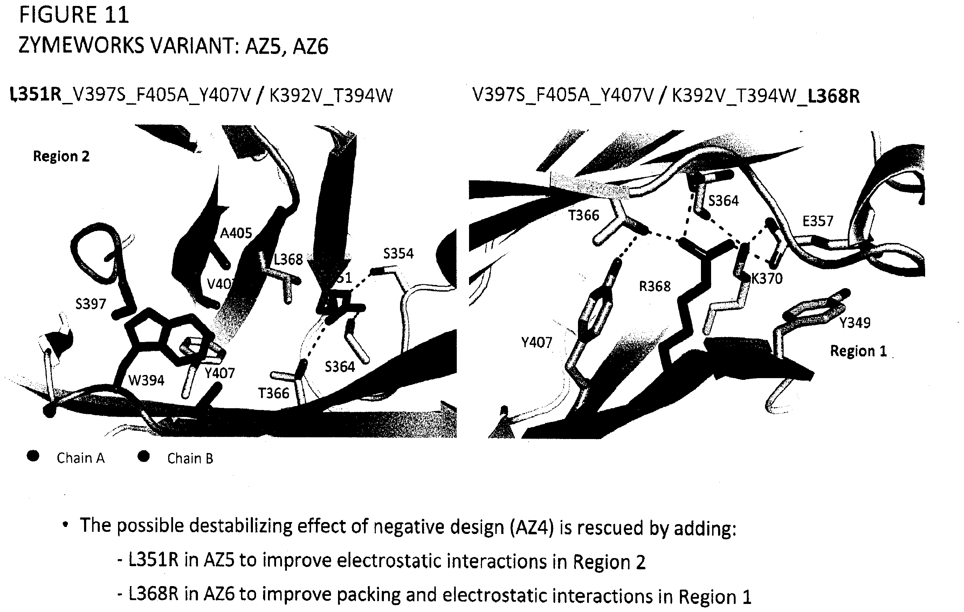

**Figure 11** is a graphical representation of CH3 variants AZ5 (left) and AZ6 (right);

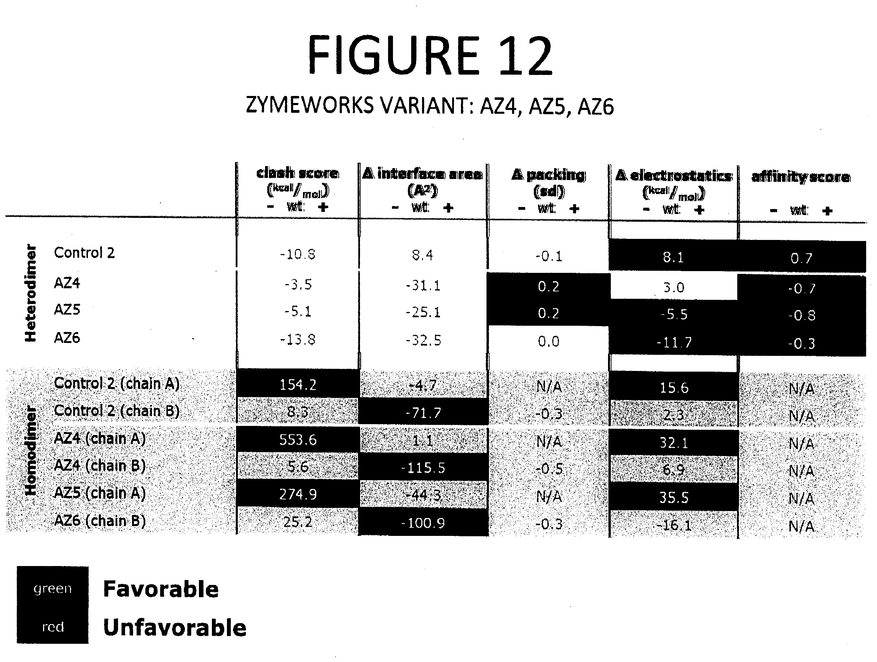

**Figure 12** is a table as described for Figure 5 showing in silico data for AZ4, AZ5 and AZ6;

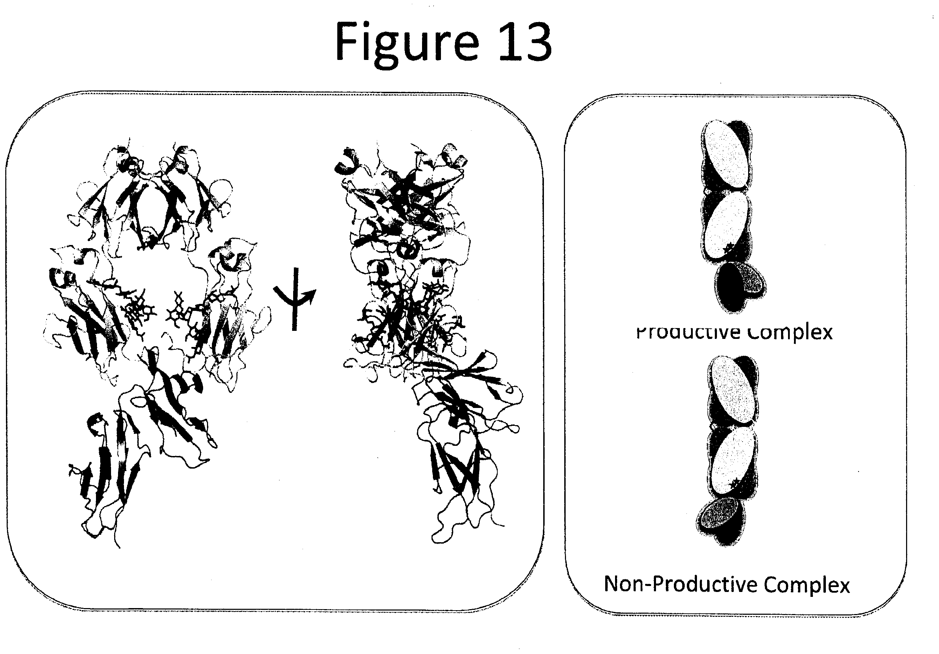

**Figure 13** is a graphical 3-D representation of an antibody on the left, with a drawing of the possibilities of binding characteristics at the receptor region using a heterodimeric approach;

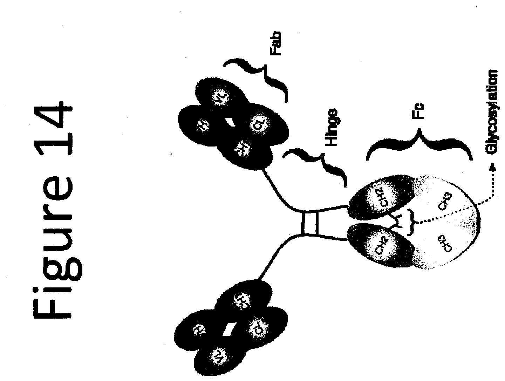

**Figure 14** is a schematic representation of the IgG molecule;

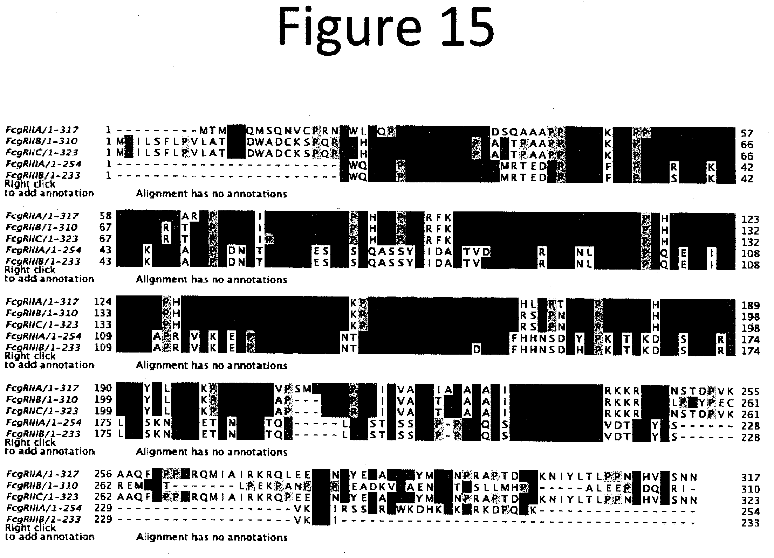

**Figure 15** shows multiple sequence alignment of Fcγ receptors. Genebank/Uniprot Sequence ID's: FcγRIIA (sp P12318), FcγRIIB (sp P31994), FcγRIIC (gi 126116592), FcγRIIIA (sp P08637), FcγRIIIB (sp 075015);
**Figure 16** is a schematic of the crystal structure of Fc-FcγRlllb Complex [PDB ID: 1T83, Radaev & Sun]. A 1:1 complex of the Fc and Fcγ receptor is observed with an asymmetric contact between the two chains of Fc and the FcγR;

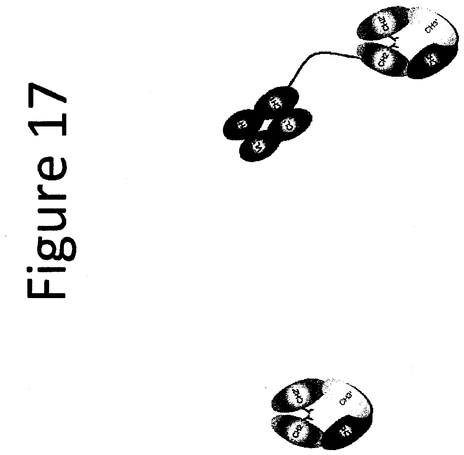

**Figure 17** shows a schematic of multifunctional molecules based on the asymmetric Fc scaffold formed by heterodimeric variants described herein: Asymetric Fc Scaffold and Asymetric Fc- Monomeric IgG Arm;

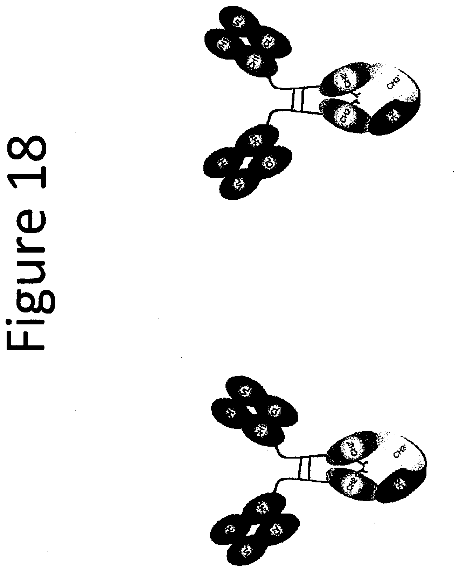

**Figure 18** shows a schematic of multifunctional molecules based on the asymmetric Fc scaffold formed by heterodimeric variants described herein: Asymmetric Fc-Monospecific IgG arms and Asymmetric Fc - Bi-specific IgG Arms (Common Light Chain);

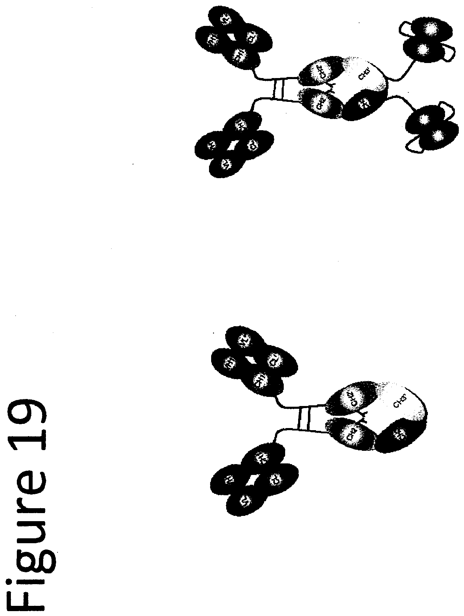

**Figure 19** shows an illustration of multifunctional molecules based on the the asymmetric Fc scaffold formed by heterodimeric variants described herein. Asymmetric Fc- Bi-specific IgG Arms and a functional molecule such as toxin;
**Figure 20** illustrates multifunctional molecules based on the asymmetric Fc scaffold formed by heterodimeric variants described herein: Asymmetric Fc- Single scFv arm and Asymmetric Fc- bi-specific scFv Arms.

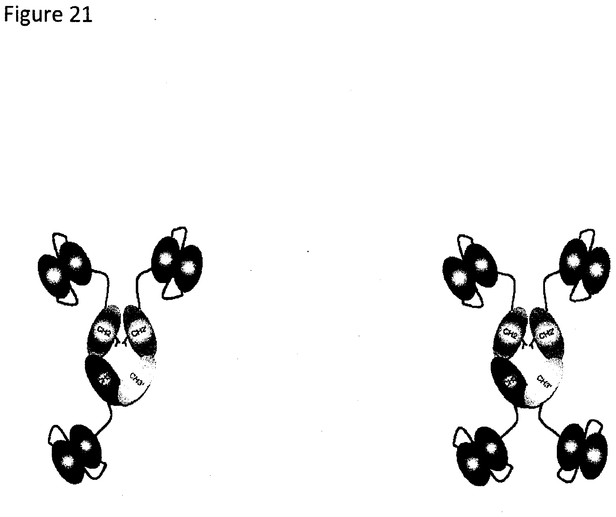

**Figure 21** illustrations of alternative multifunctional molecules based on the asymmetric Fc scaffold formed by the heterodimeric variants described herein: Asymmetric Fc- Trispecific scFv Arms and Asymmetric Fc-tetraspecific scFv arms.

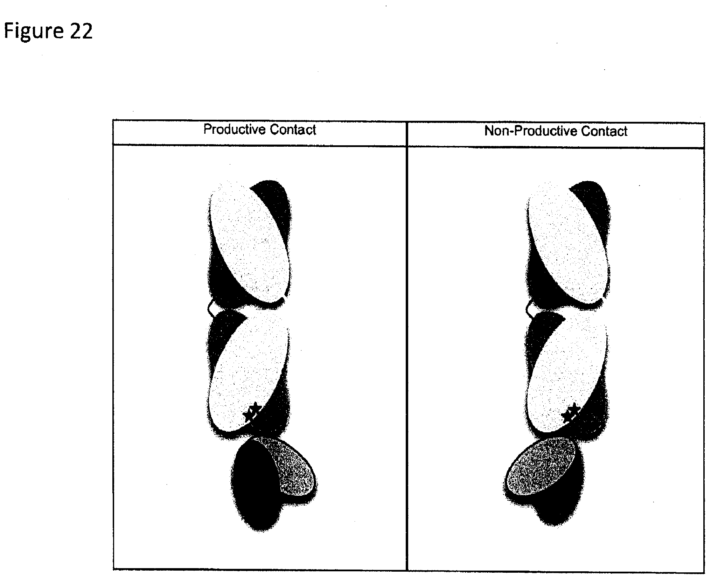

**Figure 22** displays asymmetric design of mutations on one face of the Fc for better FcγR selectivity introduces a productive side for FcγR interactions and a non-productive face with wild type like interactions. Mutations on the non-productive face of the Fc can be introduced to block interactions with FcR and bias polarity of the Fc so as to interact on the productive face only.

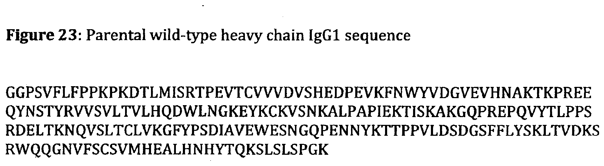

**Figure 23** shows the amino acid sequence for wild-type human IgG1.
**Figure 24**Shows the iterative process of the Fc heterodimer design, combining positive and negative design strategies as described in detail below.

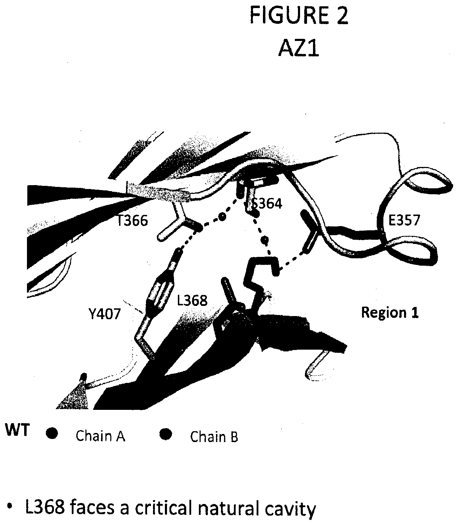

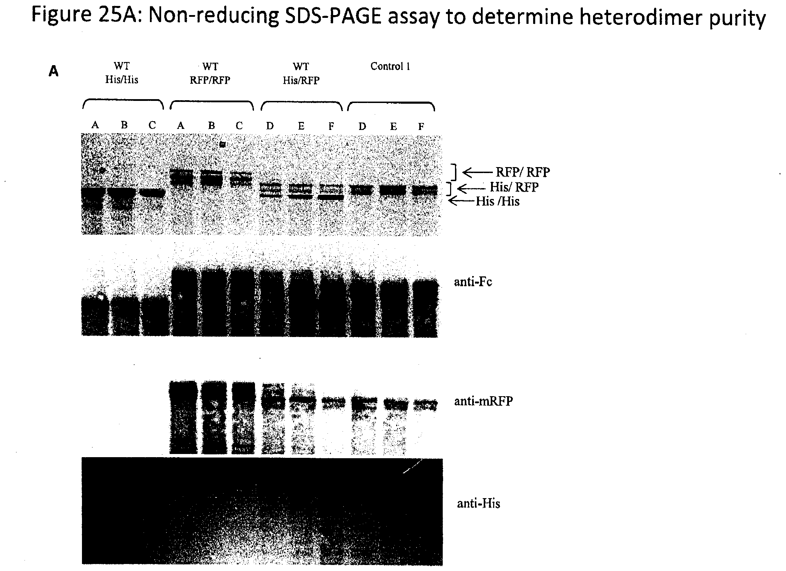

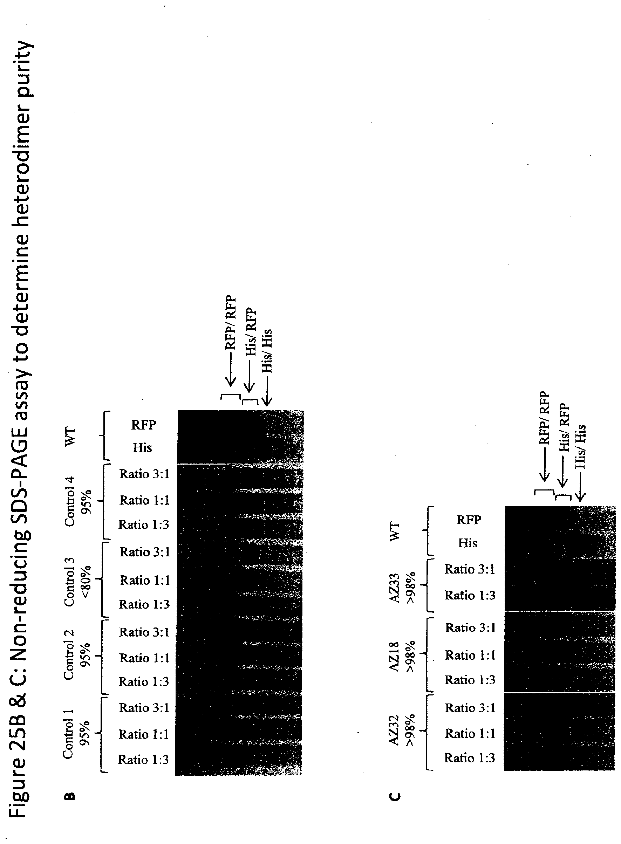

**Figures 25A-25** **C** show the in vitro assay used to determine heterodimer purity. The assay is based on a full length monospecific antibody scaffold with two Fc heavy chains of different molecular weight; Heavy chain A has a C-terminal HisTag (His) and heavy chain B a C-terminal, cleavable mRFP Tag (RFP). The two heavy chains A (His) and B (RFP) are expressed in different relative ratios together with a fixed amount of light chain, giving rise to 3 possible dimer species with different molecular weight: a) Homodimer Chain A(His)/ Chain A (His) (∼150kDa); b) Heterodimer Chain A (His)/ Chain B (RFP) (∼175kDa); c) Homodimer Chain B (RFP)/ Chain B (RFP) (∼200kDa). After expression, as described in Example 2, the ratio of heterodimer vs. the two homodimers was determined by non-reducing SDS-PAGE, which allows separation of the 3 dimer species by molecular weight. SDS-PAGE gels were stained with Coomassie Brilliant Blue. **Figure 25A****:** Variants tested were WT Chain A (His) only; WT chain B (RFP) only; WT chain A (His) plus chain B (RFP); Control 1 chain A (His) plus chain B (RFP), which has a reported heterodimer purity of >95%. The composition of the dimer bands was verified by Western Blot with antibodies directed against the IgG-Fc (anti-Fc), the mRFP Tag (anti-mRFP) and the HisTag (anti-His), as illustrated above. The SDS-PAGE shows a single band for the His/His homodimer, a double band for the His/RFP heterodimer and multiple bands for the RFP homodimer. The multiple bands are an artifact of the mRFP Tag and have been confirmed not to influence the physical properties of the Fc heterodimer. **Figure 25B****:** The SDS-PAGE assay was validated with the published Fc heterodimer variants Controls 1-4 as controls, See, Table A. The variants were expressed with different relative ratios of chain A (His) vs chain B (RFP): Specifically, Ratio 1:3 is equivalent to a LC,HC_His,HC_mRFP ratio of 25%,10%,65%; Ratio 1:1 of 25%,20%,55% and Ratio 3:1 of 25%, 40%,35% respectively (the apparent 1:1 expression of chain A (His) to chain B (RFP) has been determined to be close to 20%/55% (His/RFP) for WT Fc). **Figure 25C** shows a non-reducing SDS-PAGE assay to determine heterodimer purity of Scaffold 1 variants.The heteromultimers were expressed with different relative ratios of chain A (His) vs chain B (RFP) and analyzed by non-reducing SDS-PAGE as described in Figure 2. Specifically, Ratio 1:3 is equivalent to a LC,HC_His,HC_mRFP ratio of 25%,10%,65%; Ratio 1:1 of 25%,20%,55% and Ratio 3:1 of 25%, 40%,35% respectively (the apparent 1:1 expression of chain A (His) to chain B (RFP) has been determined to be close to 20%/55% (His/RFP) for WT Fc).

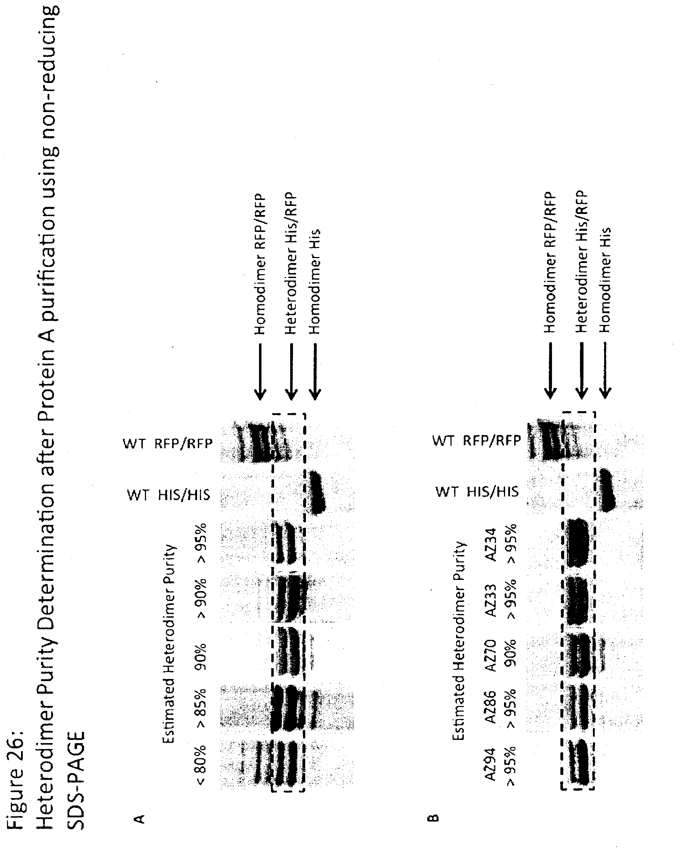

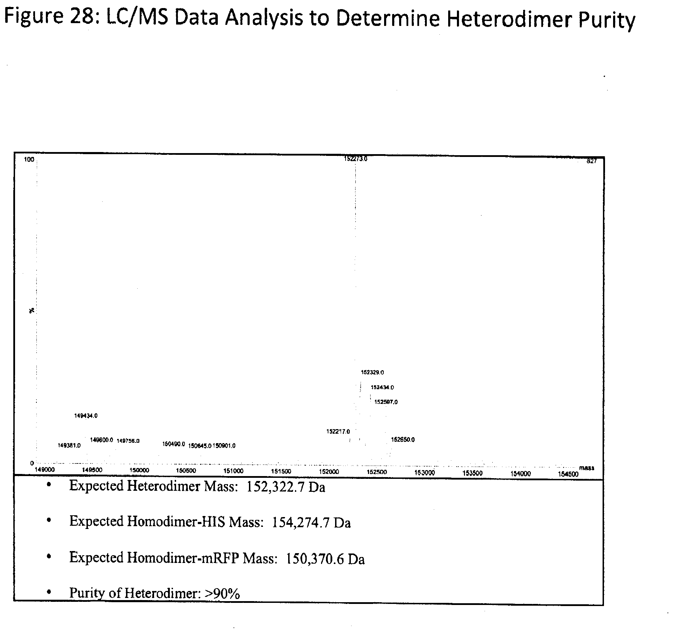

**Figures 26A-26B** show Fc Heterodimer variants expressed with a specific ratio of chain A (His) vs chain B (RFP) (See, Table 2), purified by Protein A affinity chromatography and analyzed by non-reducing SDS-PAGE as described in Figures 25A-25C. **Figure 26A** Illustrates classification of heterodimers based on purity as observed by visual inspection of the SDS-PAGE results. For comparison the equivalent amount of Protein A purified product was loaded on the gel. This definition of purity based on non-reducing SDS-PAGE has been confirmed by LC/MS on selected variants (see Figure 28). **Figure 26B** provides exemplary SDS-PAGE results of selected Protein A purified heterodimer variants (AZ94, AZ86, AZ70, AZ33 and AZ34).

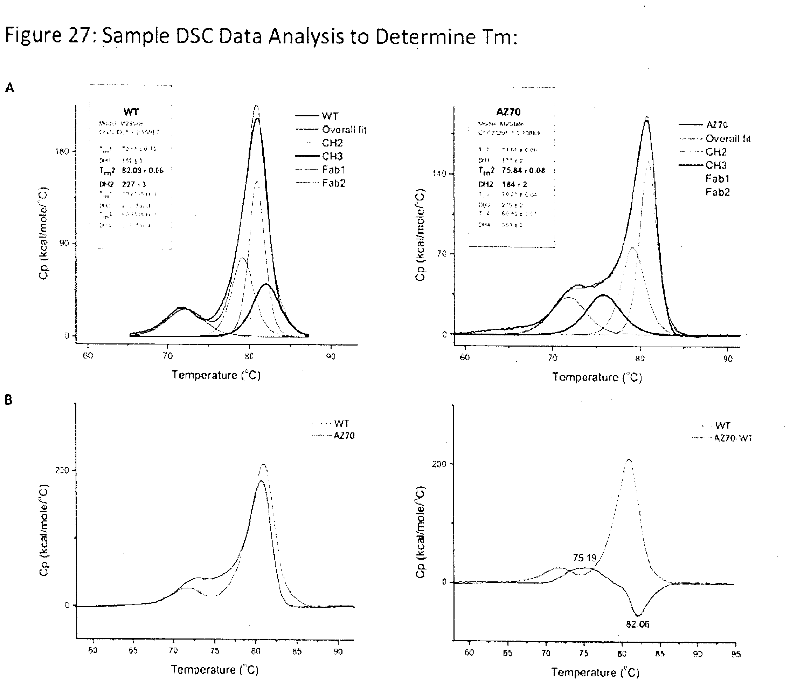

**Figures 27A-27B** illustrate DSC analyses to determine the melting temperature of the heterodimeric CH3-CH3 domain formed by the Heterodimer variants described herein. Two independent methods were used to determine the melting temperatures. **Figure 27A** **provides** thermograms fitted to 4 independent non-2-state-transitions and optimized to yield values for the CH2 and Fab transitions close to the reported literature values for Herceptin of ∼72°C (CH2) and ∼82°C (Fab). **Figure 27B** **shows** the normalized and baseline corrected thermograms for the heterodimer variants were substracted from the WT to yield a positive and negative difference peak for only the CH3 transition.
**Figure 28** Illustrates the LC/MS analysis of example variant AZ70 as described in the example 2. The expected (calculated average) masses for the glycosylated heterodimer and homodimers are indicated. The region consistent with the heterodimer mass contains major peaks corresponding to the loss of a glycine (-57 Da) and the addition of 1 or 2 hexoses (+162 Da and +324 Da, respectively).The Heterodimer purity is classified as >90% if there are no significant peaks corresponding to either of the homodimers.

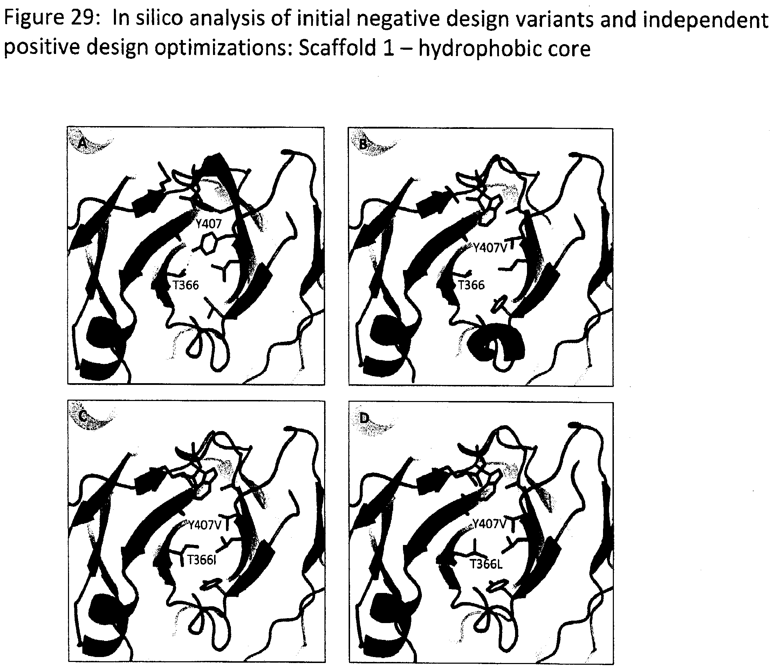

**Figures 29A-29D** shows the CH3 interface of Fig29**A**WT Fc; Fig29**B**AZ6; Fig29**C** AZ33;Fig29DAZ19. The comprehensive in silico analysis, as described in the detailed description section, and the comparison of the variants to the WT indicated that one of the reasons for the lower than WT stability of the initial AZ33 heterodimer is the loss of the core interaction/packing of Y407 and T366. The initial AZ33 shows non-optimal packing at this hydrophobic core as illustrated Fig29**B**, suggesting that optimization of this region, particularly at position T366 would improve the stability of AZ33. This is illustrated in Fig 29C and Fig29D with T366I and T366L. The experimental data correlates with this structural analysis and shows that T366L gives the greatest improvement in Tm.See, Example 5.
**Figure 30** Illustrates the utility and importance of the conformational dynamics analysis, exemplified at the initial Scaffold 1 variant AZ8. The structure after in silico mutagenesis (backbone conformation close to WT) is superimposed with a representative structure of a 50ns Molecular Dynamics simulation analysis. The figure highlights the large conformational difference in the loop region D399-S400 of AZ8 variant vs. WT, which in turn exposes the hydrophobic core to solvent and causes decreased stability of the AZ8 heterodimer.

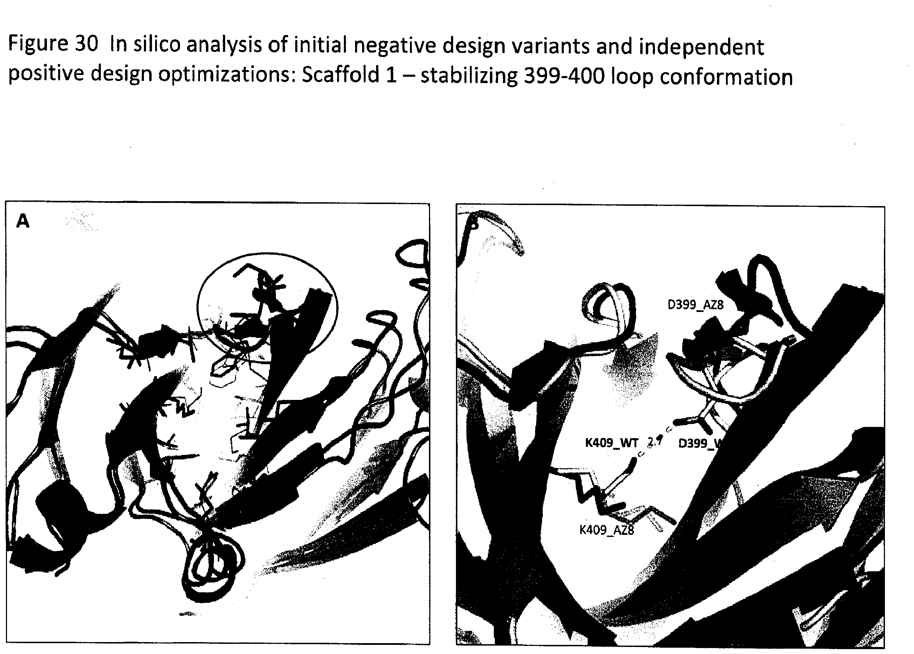

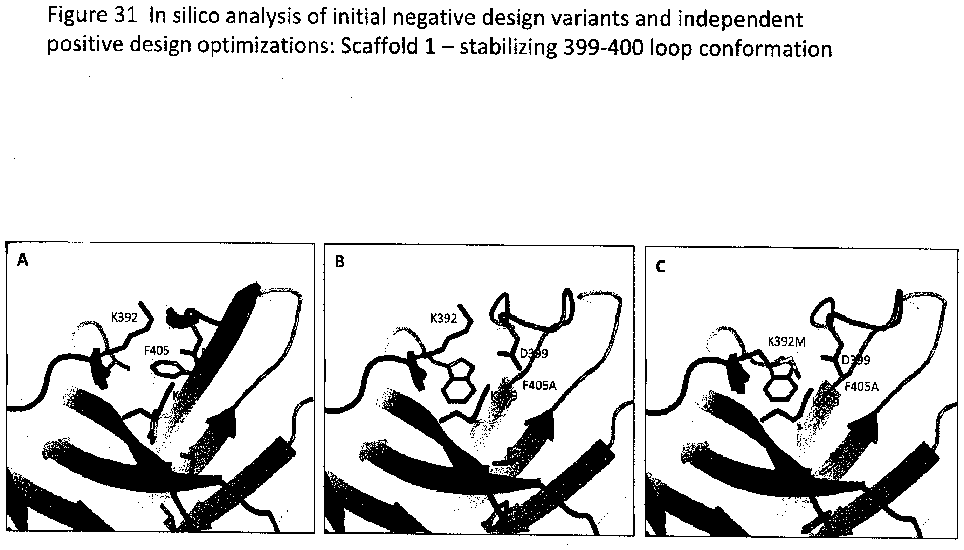

**Figures 31A-31** **C** illustrate how the information from the comprehensive in silico analysis and the MD simulation was used in the described positive design strategy. As illustrated in Figure 30, one of the reasons for the lower than WT stability of AZ8 is the weakened interaction of the loop 399-400 to 409, which is mainly due to the loss of the F405 packing interactions (see comparison of **Fig31A** (WT) vs **Fig31B** (AZ8)). One of the positive design strategies was optimization of the hydrophobic packing of area, to stabilize the 399-400 loop conformation. This was achieved by the K392M mutation that is illustrated in Fig 31C. **Fig 31C** represents the heterodimer AZ33, which has a Tm of 74° vs. 68° of the initial negative design variant AZ8.

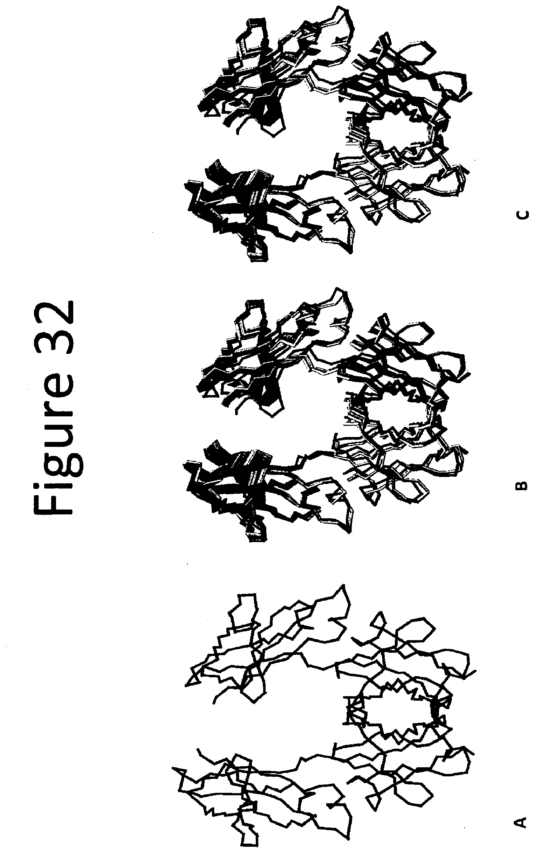

**Figures 32A-32B** Illustrate the dynamics of the Fc molecule observed using principal component analysis of a molecular dynamics trajectory. **Fig 32A**shows a backbone trace of the Fc structure as reference. **Fig 32B** and C represent an overlay of dynamics observed along the top 2 principal modes of motion in the Fc structure. The CH2 domains of chain A and B exhibits significant opening /closing motion relative to each other while the CH3 domains are relatively rigid. Mutations at the CH3 interface impact the relative flexibility and dynamics of this open/close motion in the CH2 domains.

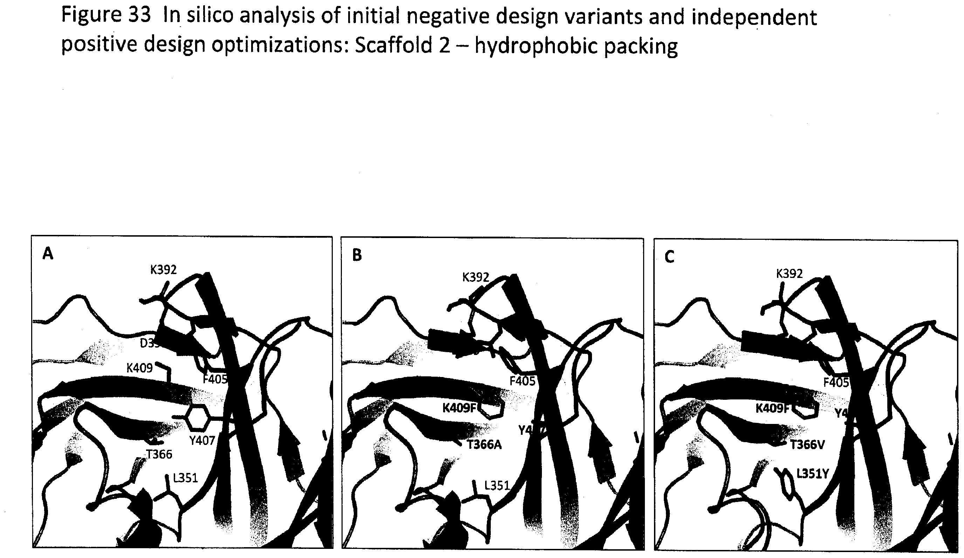

**Figures 33A-33C** illustrate the hydrophobic core packing of two Scaffold-2 variants vs. WT. **Fig 33A** WT Fc; **Fig 33B** AZ63; and **Fig 33C**AZ70. The comprehensive in-silico analysis of the initial Scaffold-2 variant suggested that loss of the core WT interactions of Y407-T366 is one of the reasons for the lower than WT stability for the initial Scaffold-2 variants. The loss of Y407-T366 is partially compensated by the mutations K409F, but as illustrated in Fig 33B, particularly the T366A mutation leaves a cavity in the hydrophobic core, which destabilizes the variant vs. WT. Targeting this hydrophobic core by additional mutations T366V_L351Y, as shown by AZ70 in **Fig33C****,** proved to be successful; AZ70 has an experimentally determined Tm of 75.5°C.See, Table 4 and Example 6.

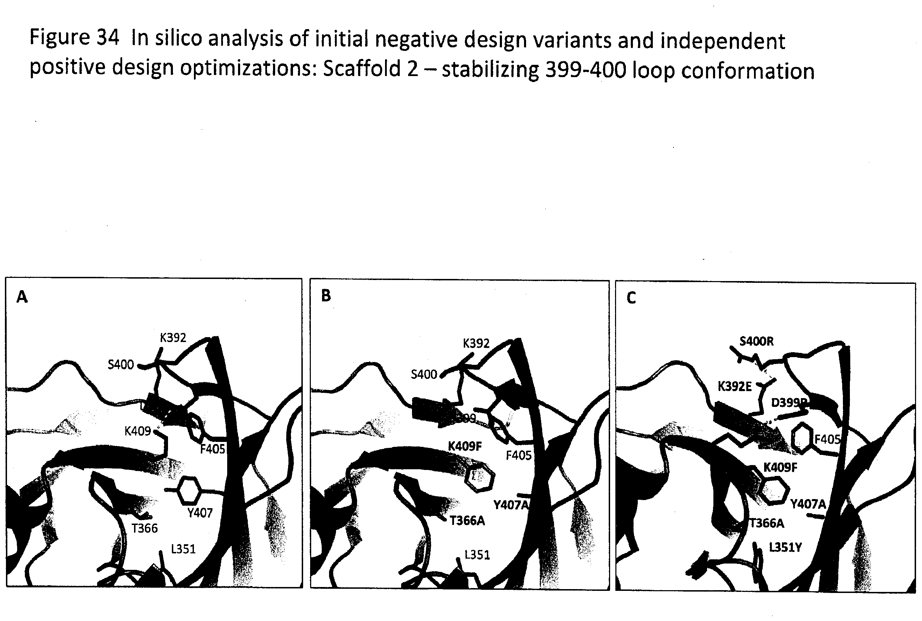

**Figures 34A-34C** illustrate the interactions of the loop 399-400 of two Scaffold-2 variants vs. the WT: **Fig 34A** WT Fc; **Fig 34B** AZ63; and **Fig 34C**AZ94. The comprehensive in-silico analysis of the initial Scaffold-2 variant suggested that loss of the WT salt-bridge K409-D399 (**Fig 34A**) due to the mutation K409F and the hence unsatisfied D399 (**Fig34B**) causes a more 'open' conformation of the 399-400 loop. This leads furthermore to a greater solvent exposure of the hydrophobic core and a further destabilization of the variant vs WT. One of the strategies employed to stabilize the 399-400 loop and compensate for the loss of the K409-D399 interaction was the design of additional salt bridges D399R-T411 E and S400R-K392E as illustrated in **Fig34C** for variant AZ94. Experimental data showed a purity of >95% and Tm of 74°C.*See*, Table 4 and Example 6. Further, although AZ94 has a considerably higher purity and stability compared to the initial Scaffold-2 variant (purity <90%, Tm 71°C), the hydrophobic core mutations of AZ94 are less preferred than the 'best' hydrophobic core mutations identified in variant AZ70 (Figure 33). Since the mutations at the hydrophobic core in AZ70 (T366V_L351Y) are distal from the salt-bridge mutations of AZ94 at the loop 399-400, the combination of AZ70 amino acid mutations and the additional AZ94 mutations,is expected to have a higher melting temperature then AZ70 or AZ94. This combination can be tested as described in Examples 1-4.
**Figure 35** Illustrates the association constant (Ka(M⁻¹)) of homodimeric IgG1 Fc, the heterodimeric variants het1 (Control 1): A:Y349C_T366S_L368A_Y407V/B:S354C_T366W and het2(Control 4): A:K409D_K392D/B:D399K_D356K binding to the six Fcgamma receptors. The heterodimers tend to show slightly altered binding to the Fcgamma receptors compared to the wild type IgG1 Fc. See, Example 7

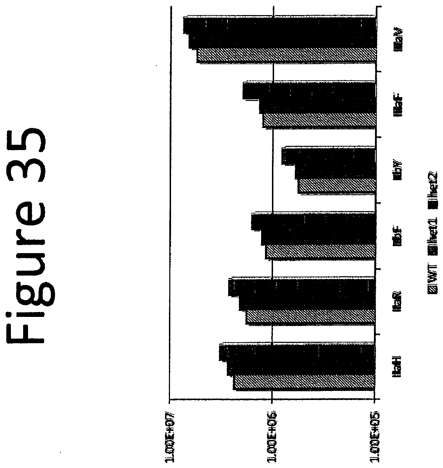

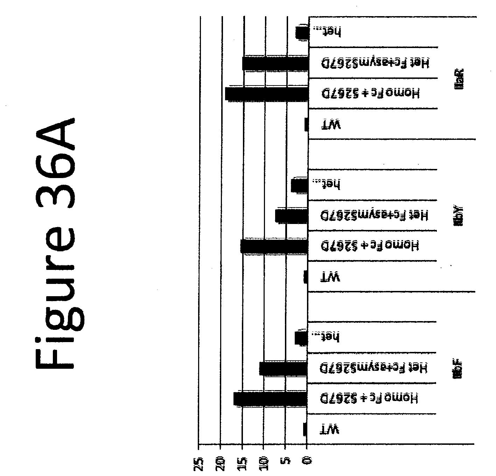

**Figure 36A** Shows the relative binding strength of a wild type IgG1 Fc and its various homodimeric and asymmetric mutant forms to the IIbF, IIBY and IIaR receptors, based on the wild type binding strength as reference. (Homo Fc + S267D) refers to the binding strength of a homodimeric Fc with the S267D mutation on both chains. (Het Fc + asym S267D) refers to the binding strength of a heterodimeric Fc with the S267D mutation introduced in one of the two chains in Fc. The average of the binding strength obtained by introducing the mutation on either of the two Fc chains is reported. Introduction of this mutation on one chain reduced the binding strength to roughly half the strength observed for the same mutation in a homodimeric manner. The (Het Fc + asym S267D + asym E269K) refers to the binding strength of a heterodimeric Fc with both the S267D and E269K mutations introduced in an asymmetric manner on one of the two Fc chains. The E269K mutation blocks the interaction of the FcgR to one of the faces of the Fc and is able to bring down the binding strength by roughly half of what was observed for the asymmetric S267D variant (Het Fc+S267D) by itself. The Het Fc here is comprised of CH3 mutations as indicated for the variant het2 (Control 4) in Figure 35.

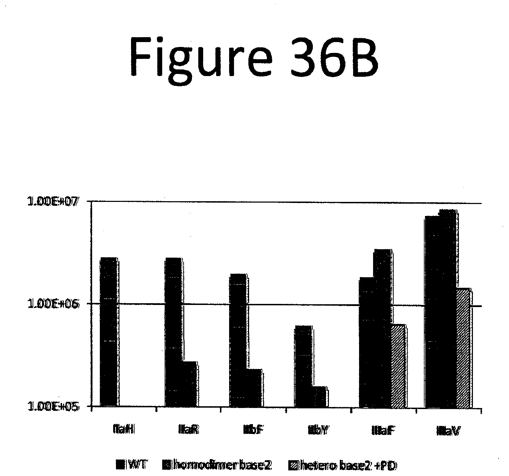

**Figure 36B** Shows the association constant (Ka(M⁻¹)) of various Fc's and its variants with a number of FcgRlla, FcgRllb and FcgRllla allotypes. The Ka of wild type IgG1 Fc to various Fcg receptors is represented as columns with horizontal shade. The bars with vertical shades (homodimer base2) represent the Ka of homodimeric Fc with the mutations S239D/D265S/I332E/S298A. The columns with the slanted shade represent the Ka of heterodimeric Fc with asymmetric mutations A:S239D/D265S/1332E/E269K and B:S239D/D265S/S298A in the CH2 domain. The introduction of asymmetric mutations is able to achieve increased selectivity between the IIIa and IIa/IIb receptors. The Heterodimeric Fc here is comprised of CH3 mutations as indicated for the variant het2 (Control 4) in Figure 35.

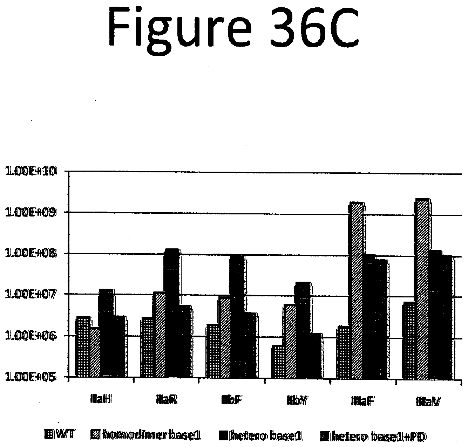

**Figure 36C** Shows the association constant (Ka (M⁻¹)) for wild type IgG1 and three other constructs involving homodimeric or asymmetric mutations in the CH2 domain of the Fc region. The Ka of wild type Fc is represented in the column shaded with grids. The Ka of Fc variant with the base mutation S239D/K326E/A330L/I332E/S298A introduced in a homodimeric manner (homodimer base1) on both the chains of Fc is shown with the slanted patterned column. Introduction of related mutations in an asymmetric manner in chains A and B of a heterodimeric Fc (hetero base1) is shown with the horizontal lines. The column with vertical shaded lines represents the asymmetric variant including the E269K mutation (hetero base 1+PD). The Heterodimeric Fc here is comprised of CH3 mutations as indicated for the variant het2 (Control 4) in Figure 35.

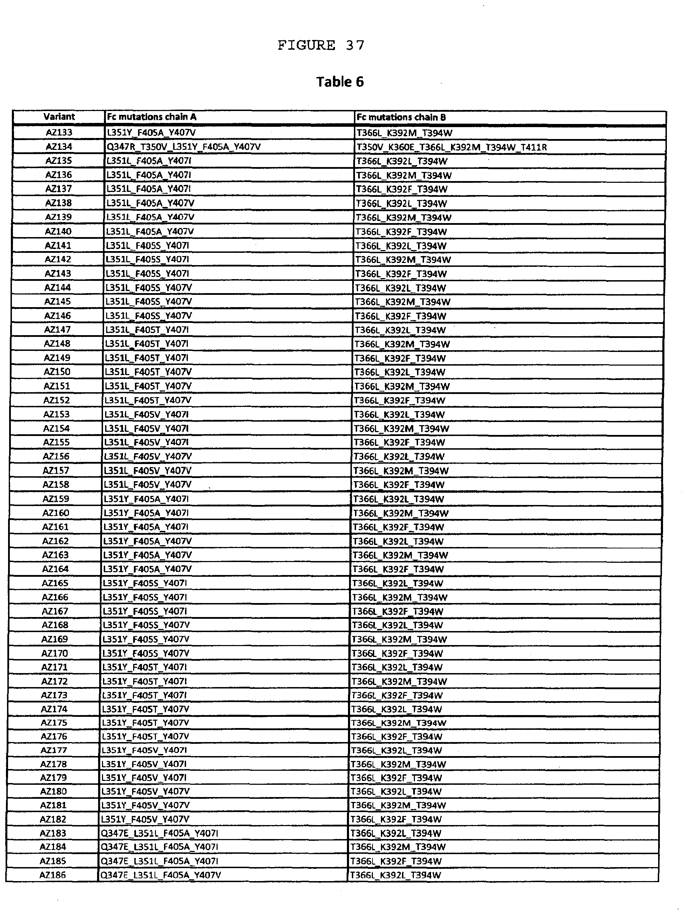

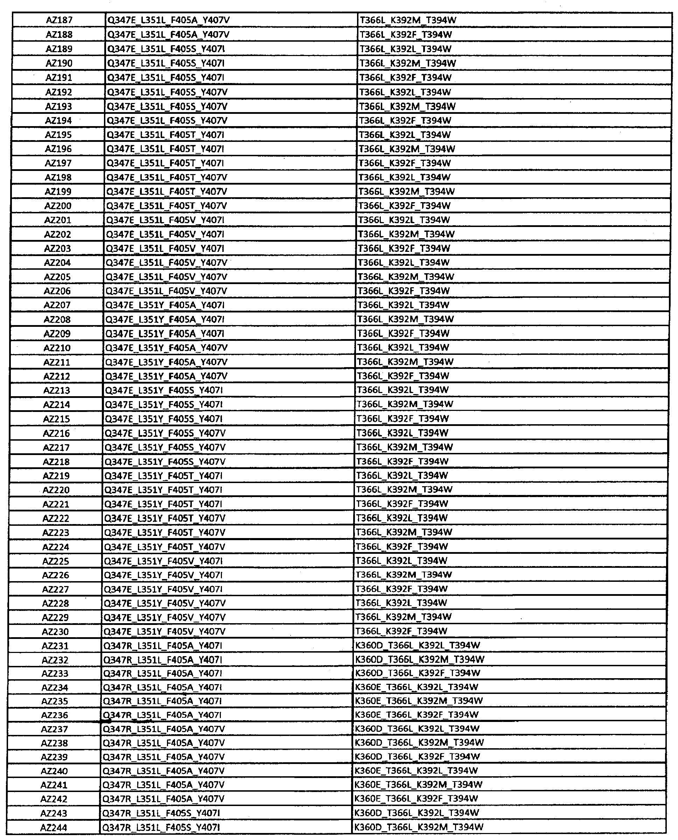

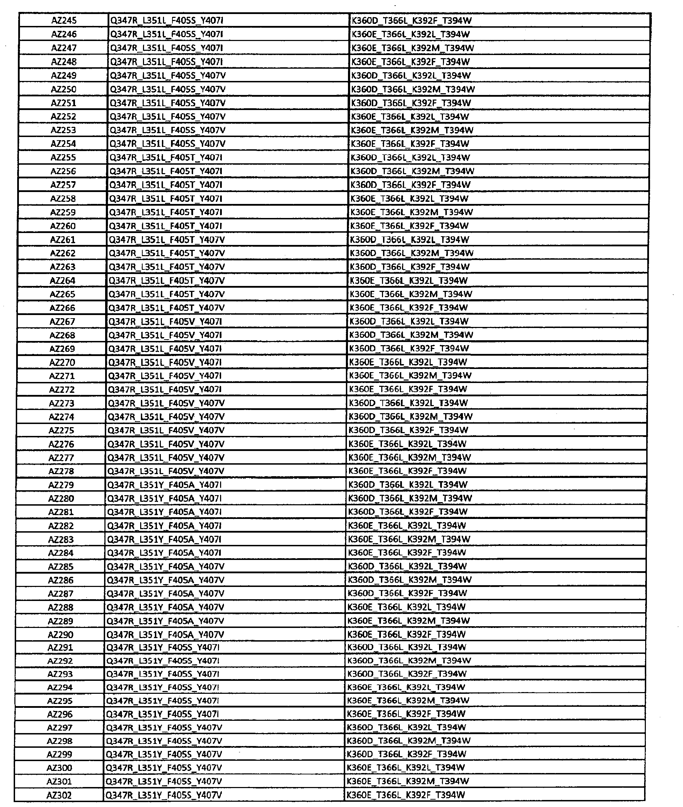

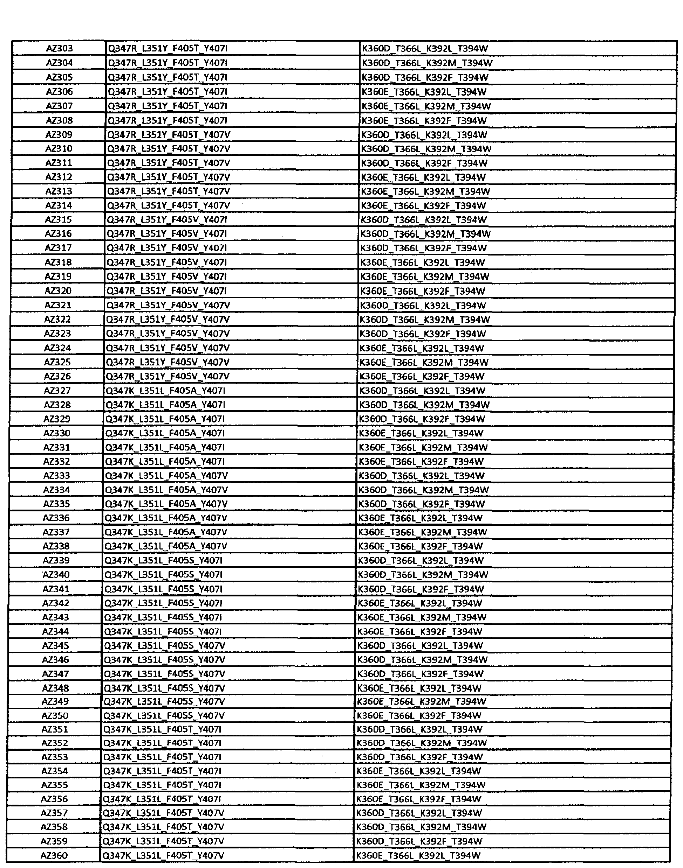

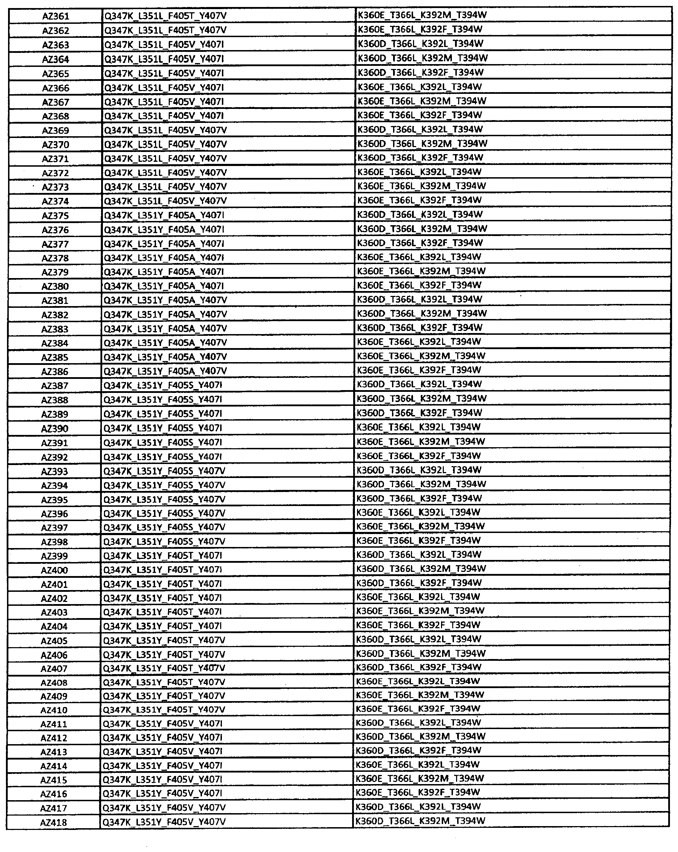

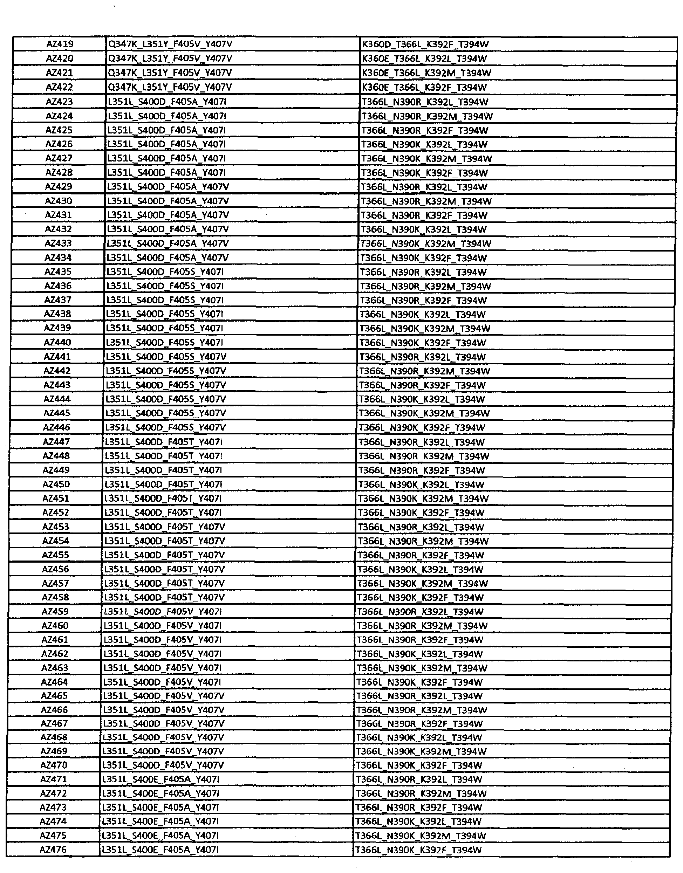

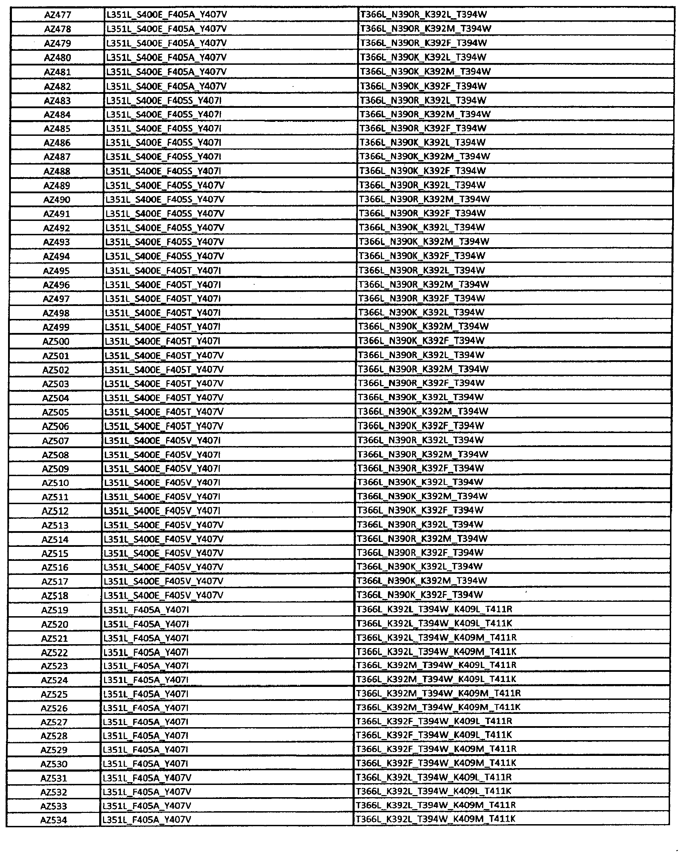

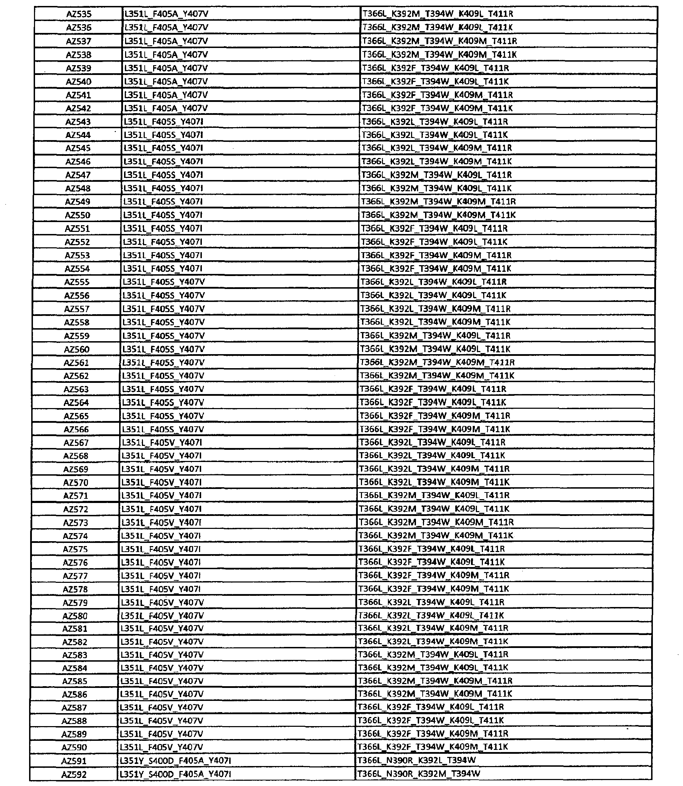

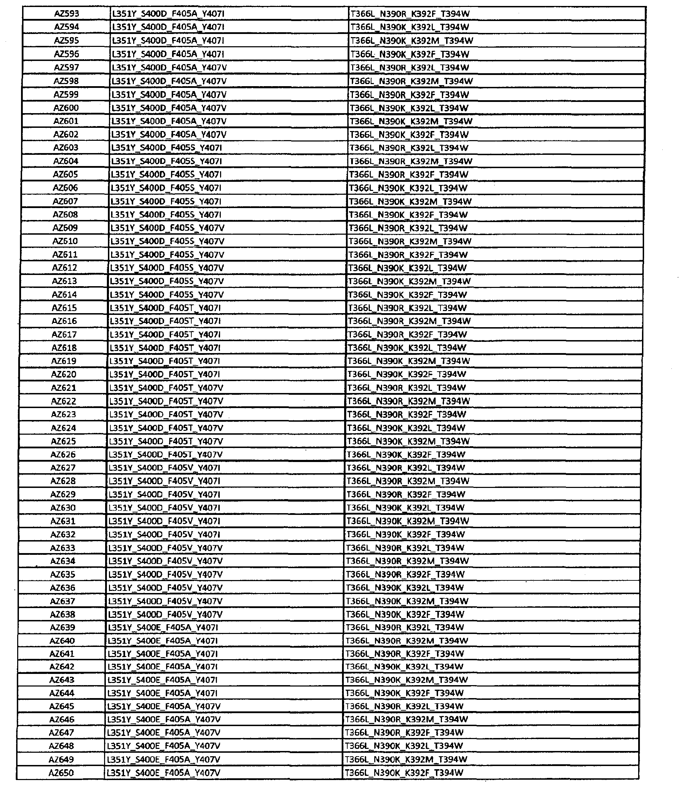

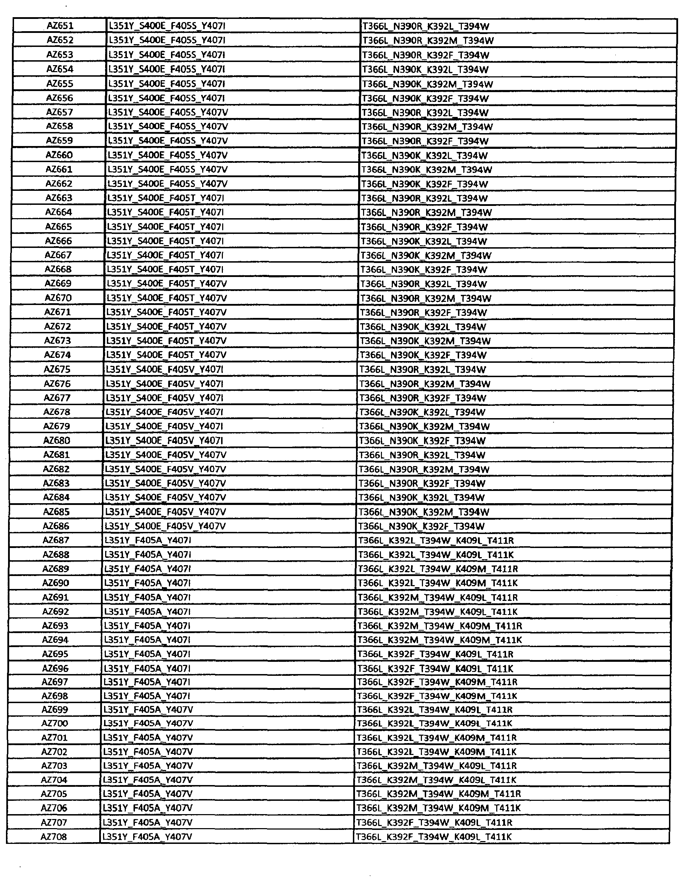

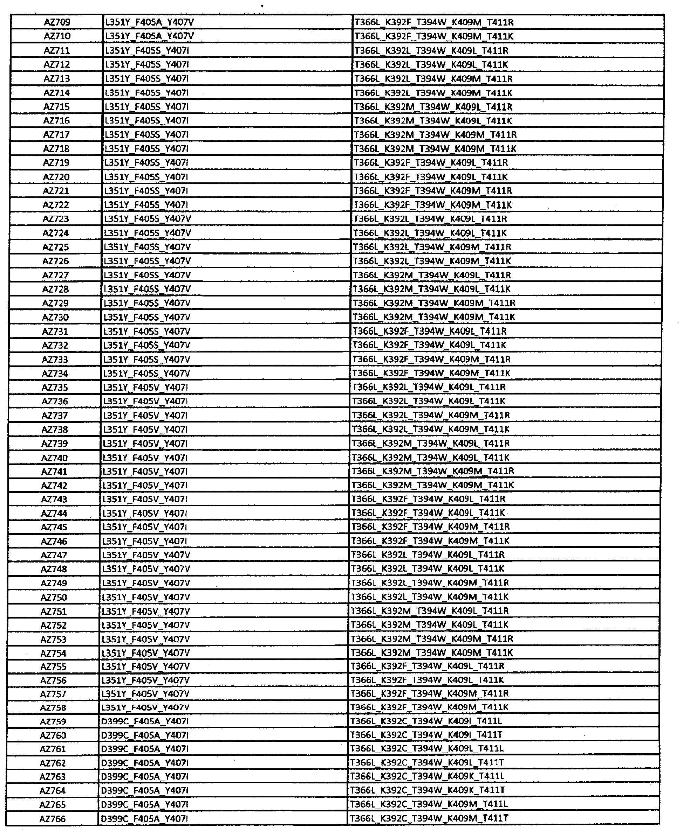

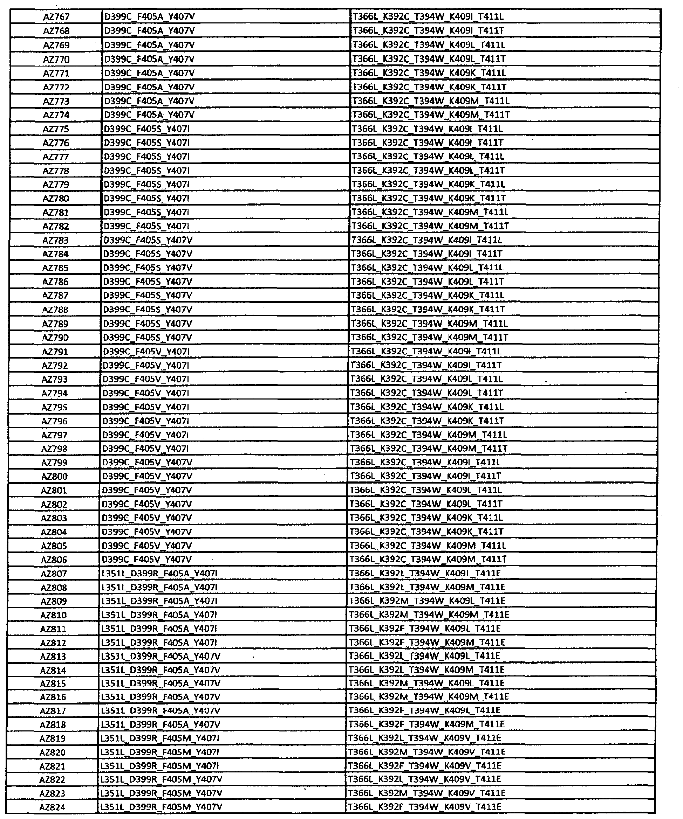

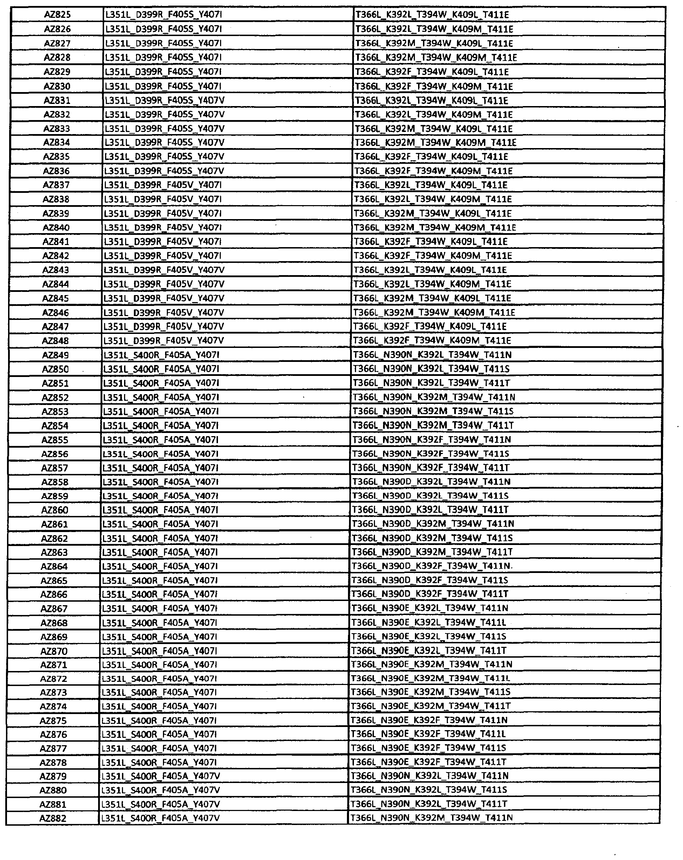

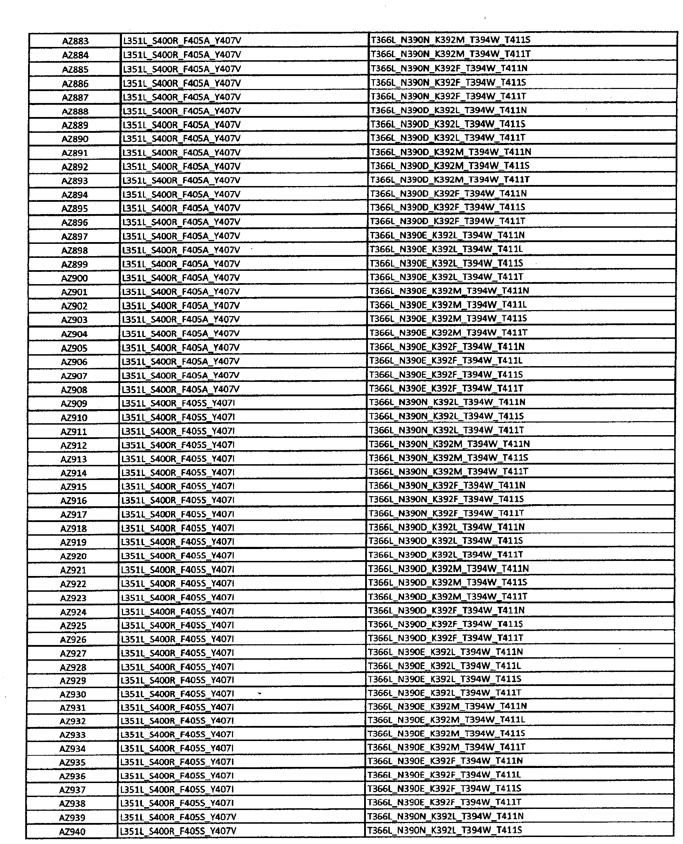

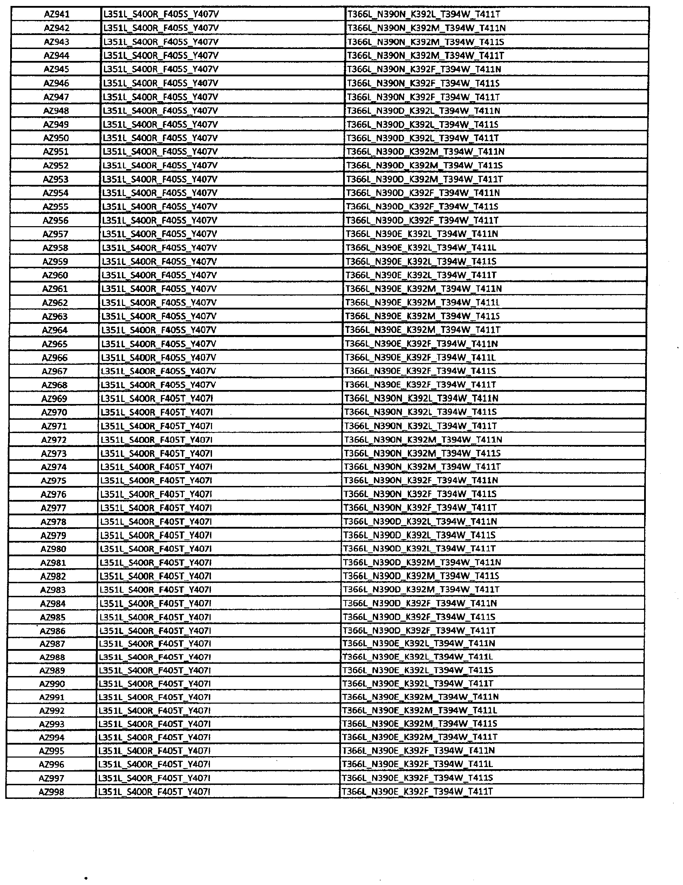

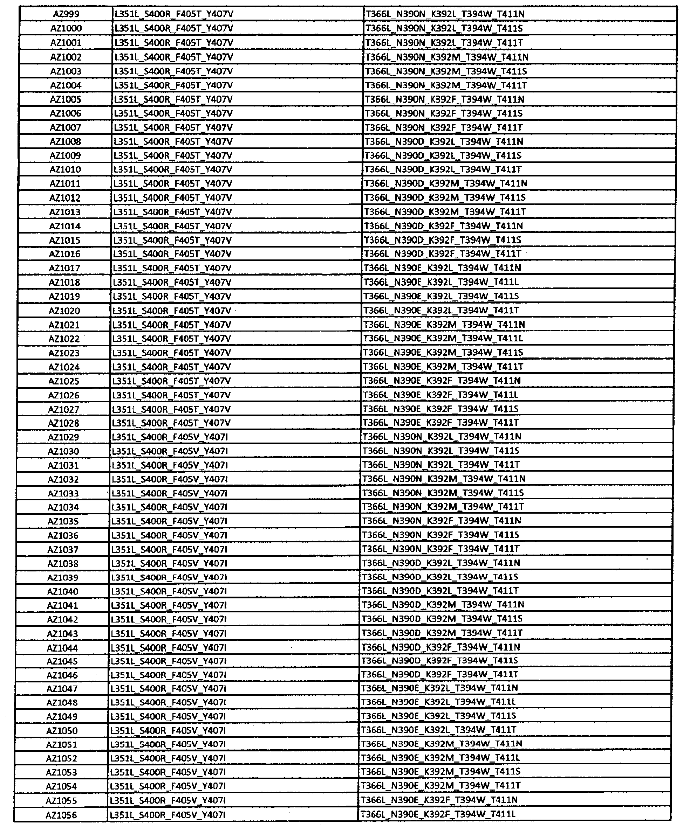

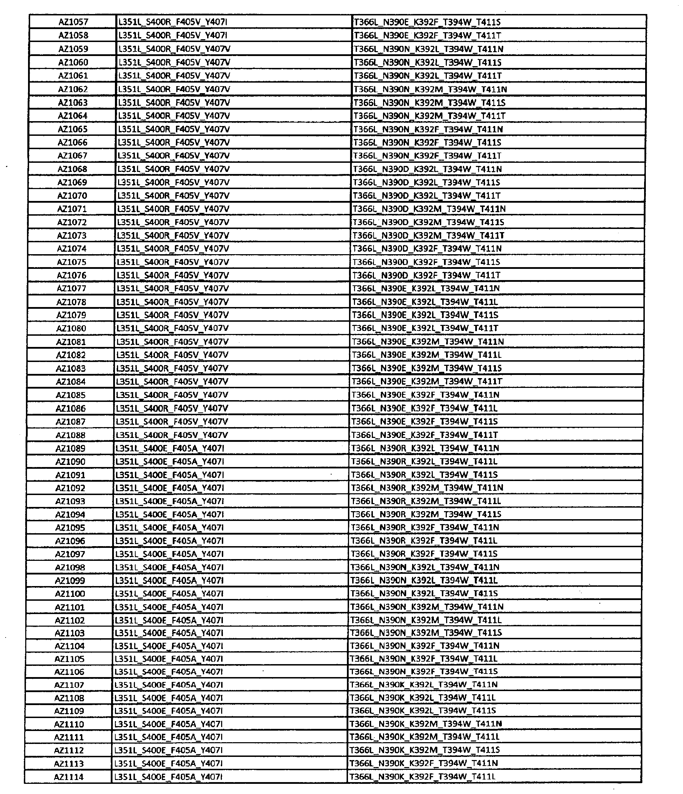

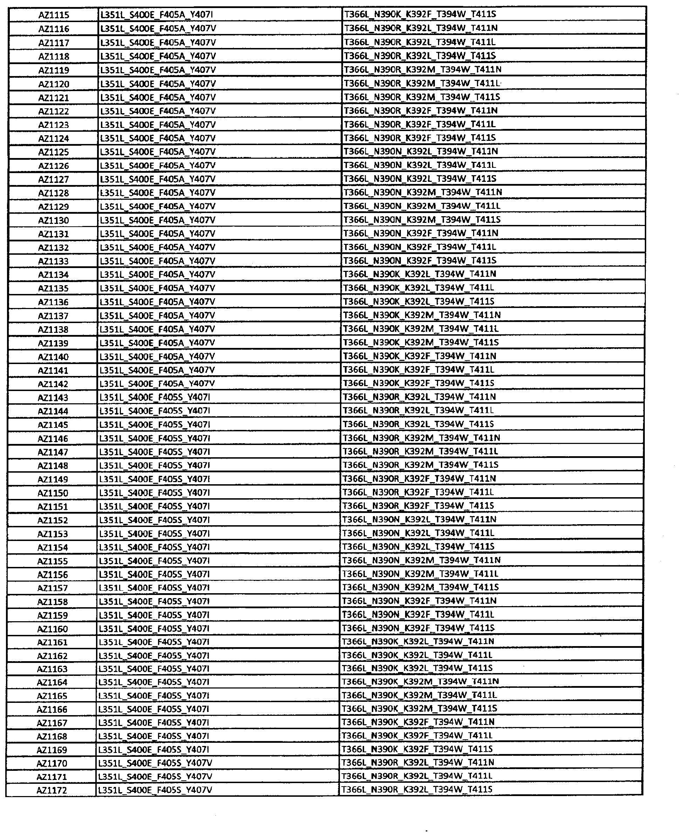

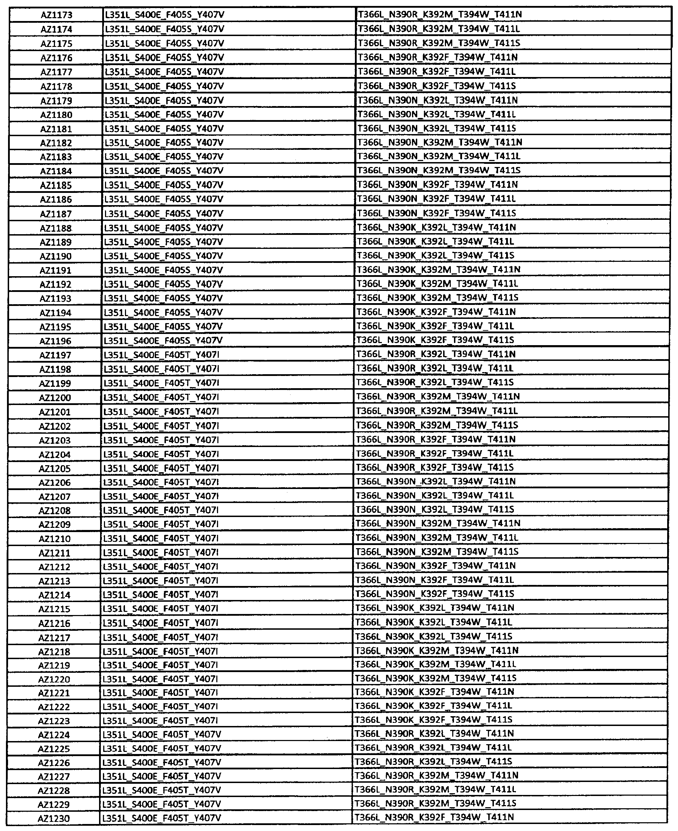

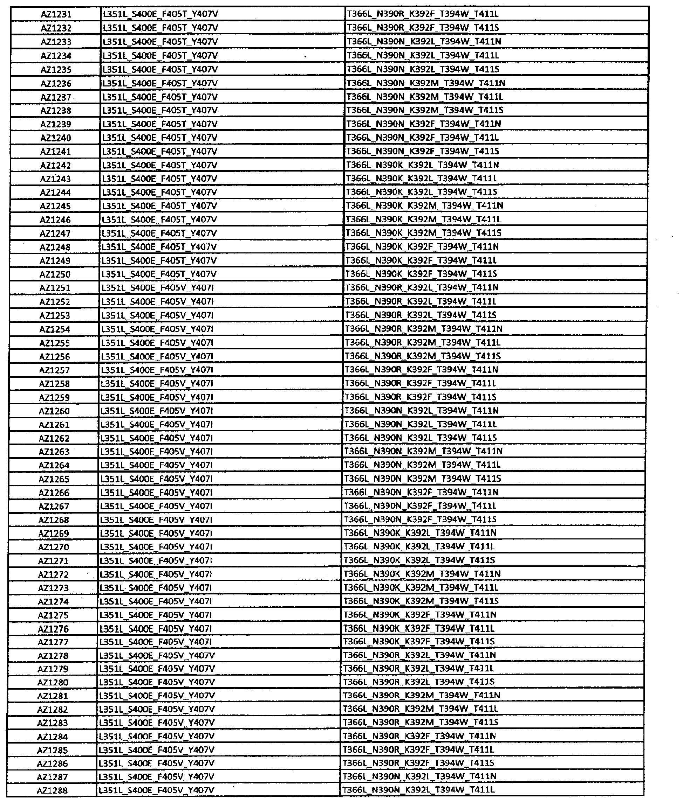

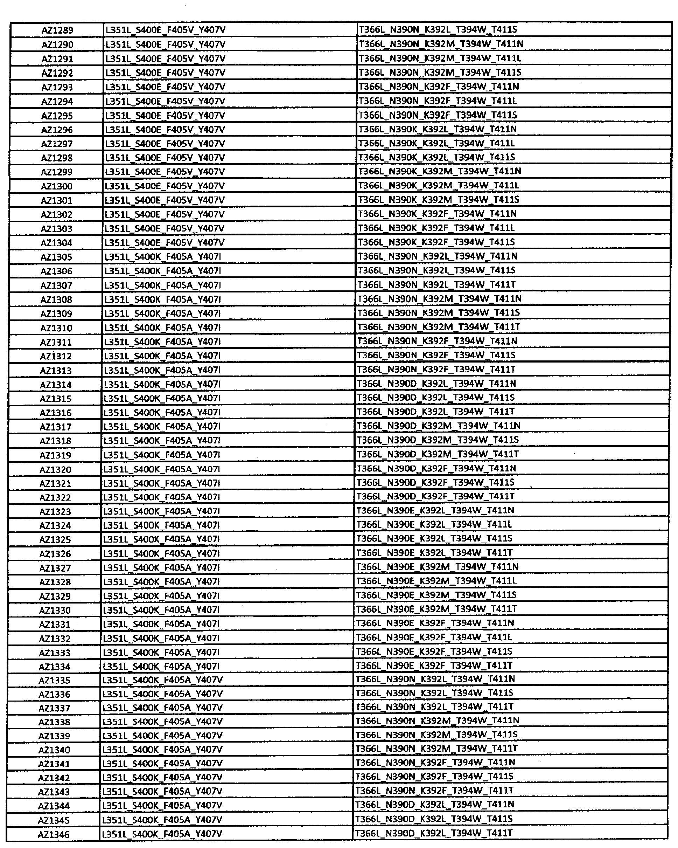

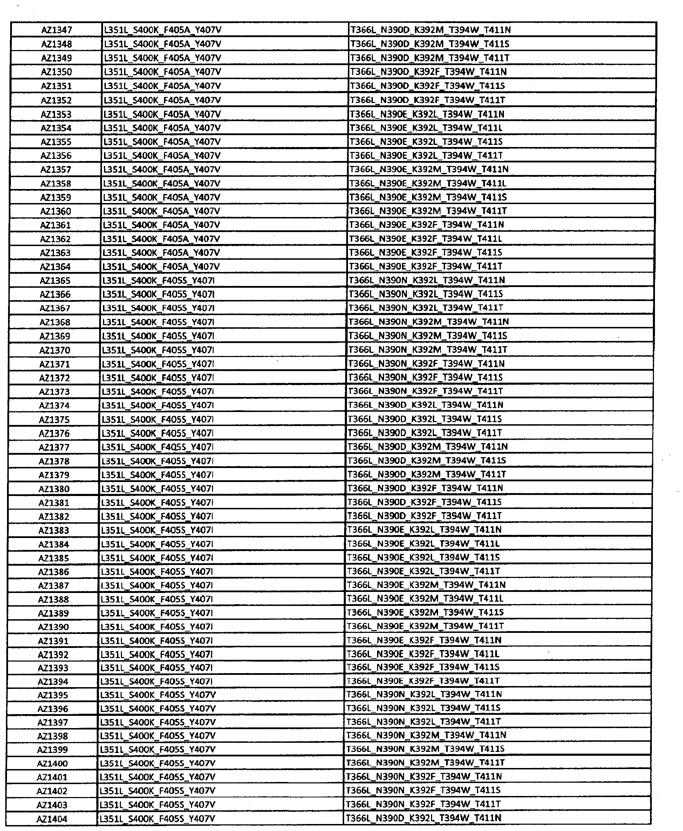

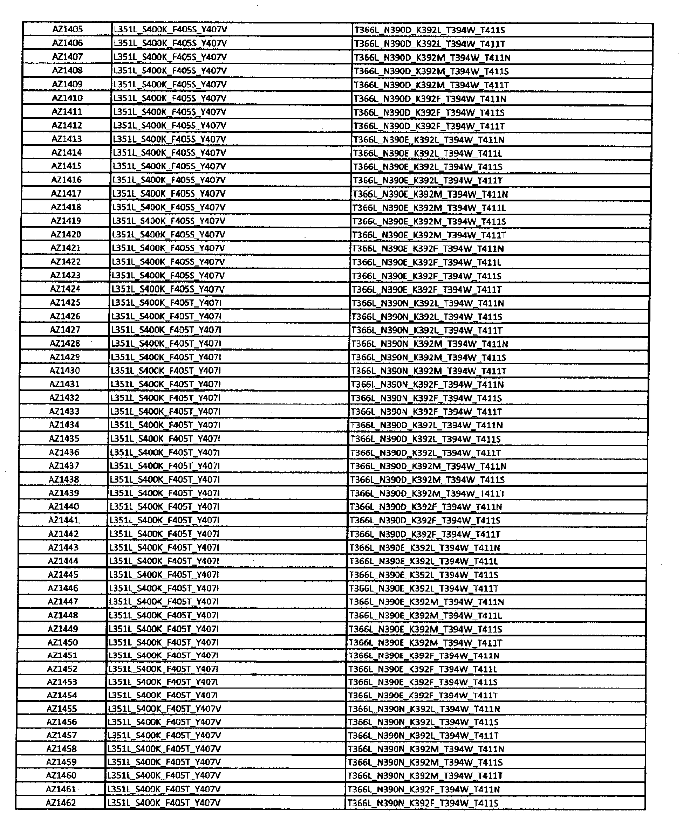

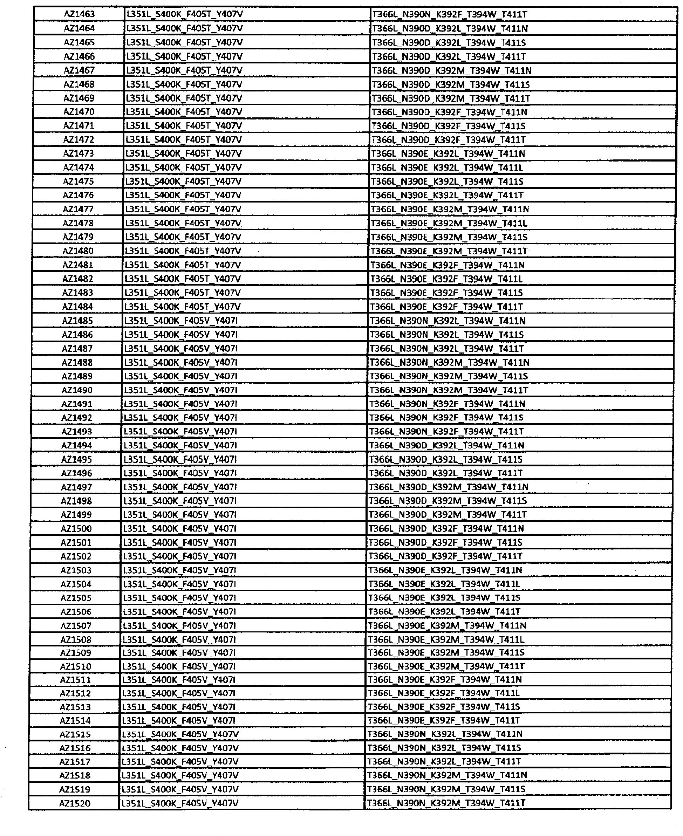

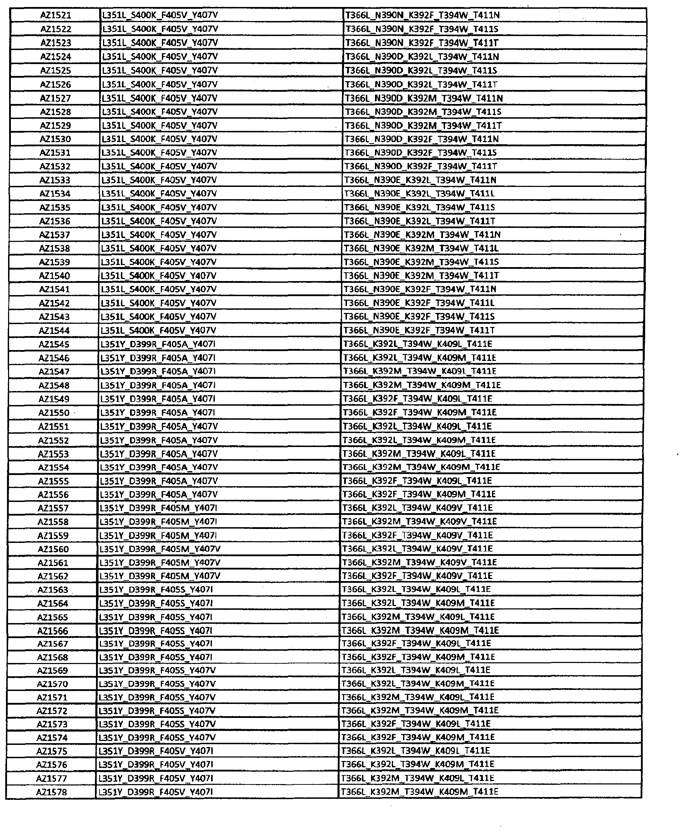

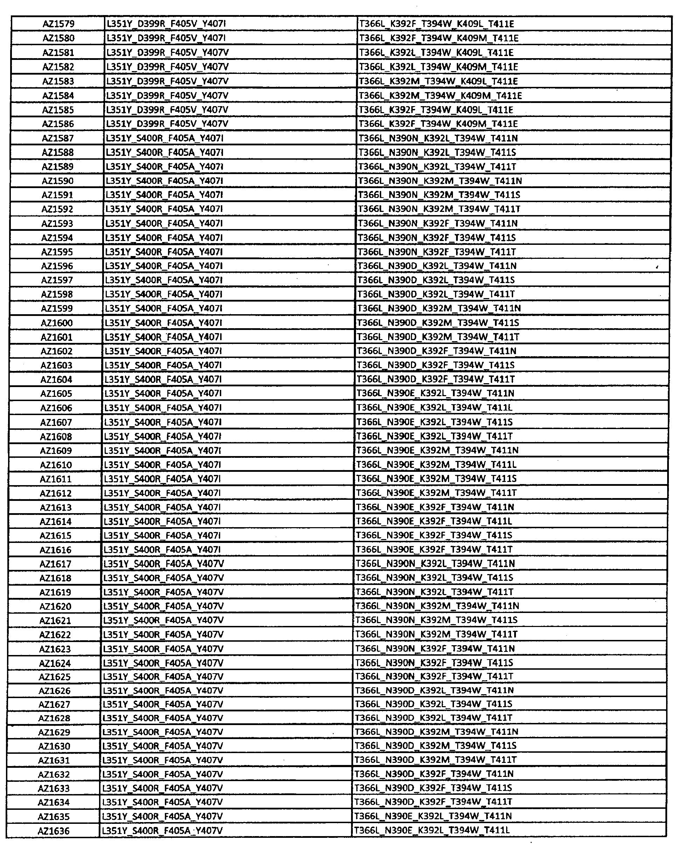

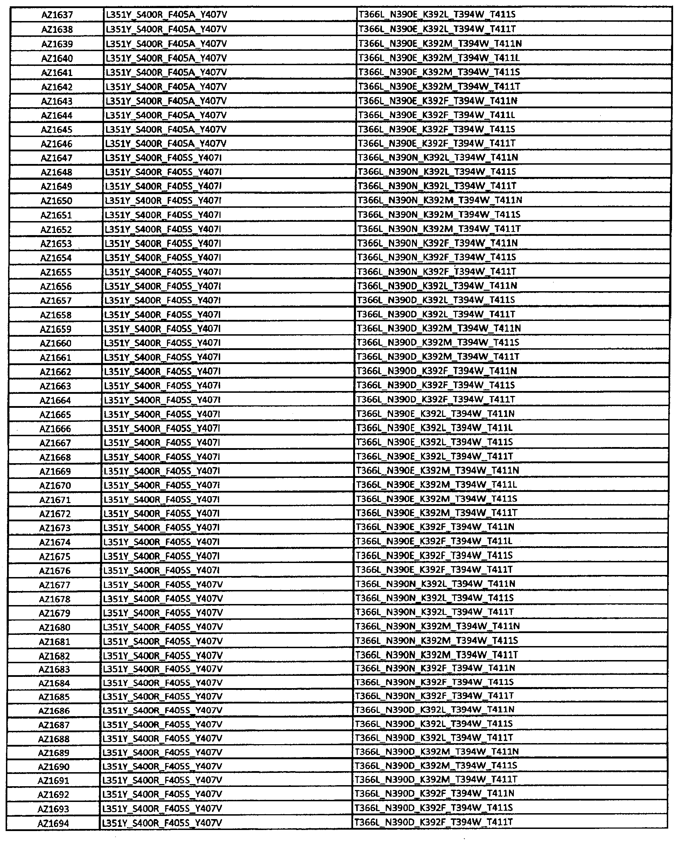

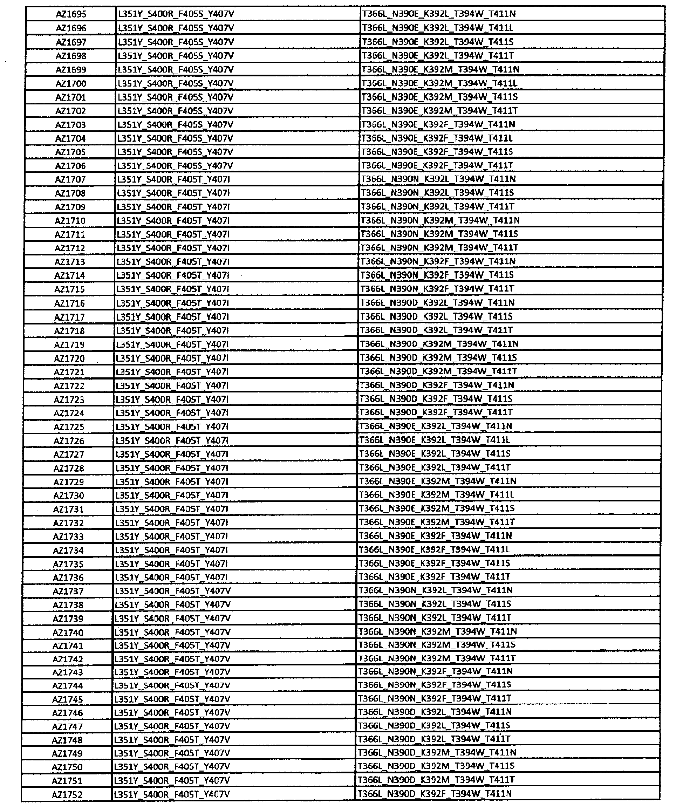

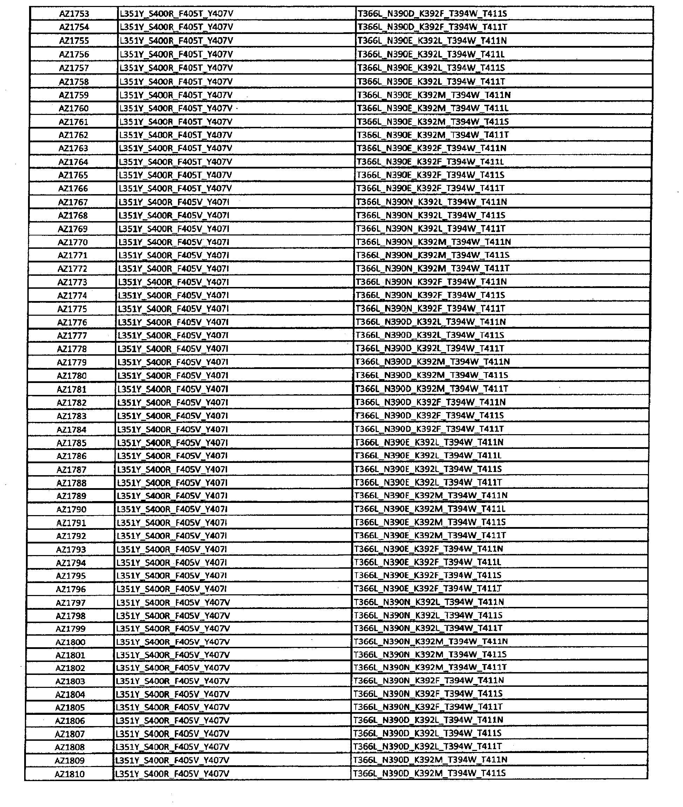

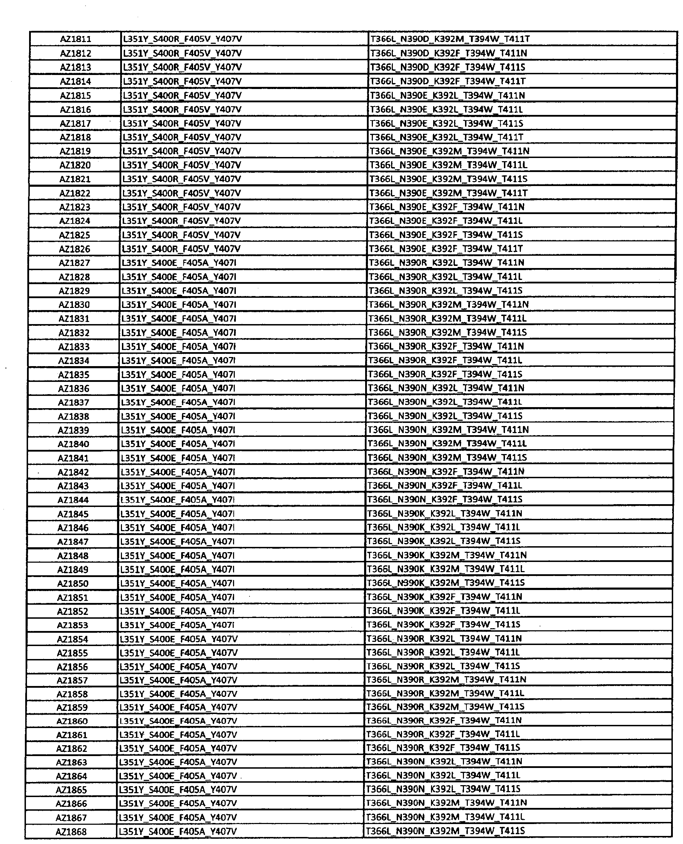

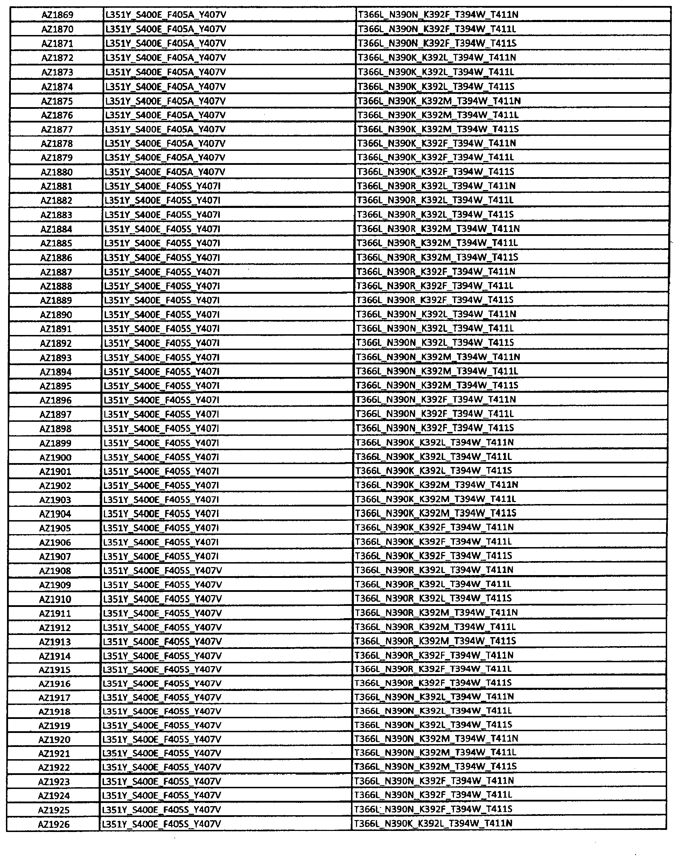

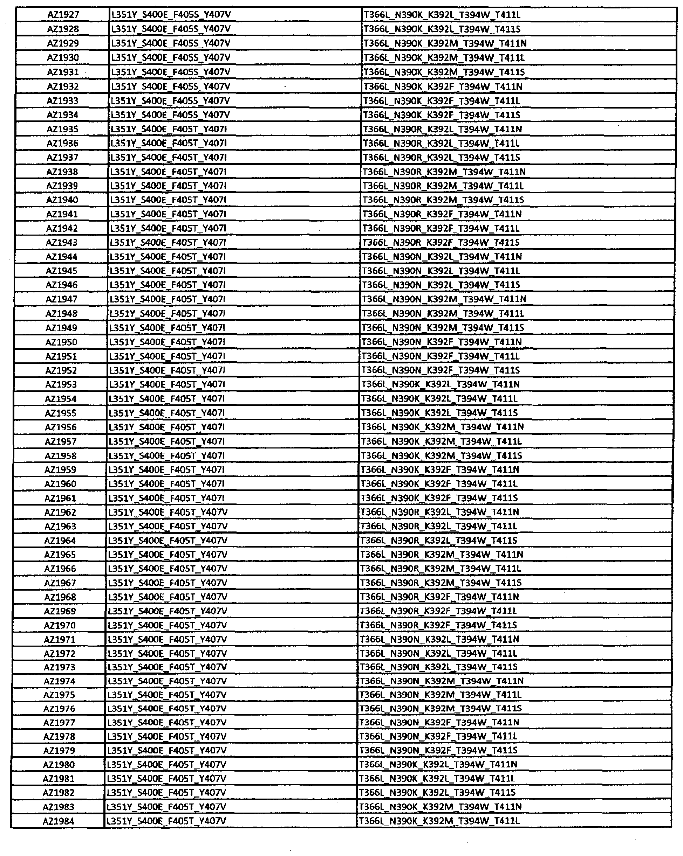

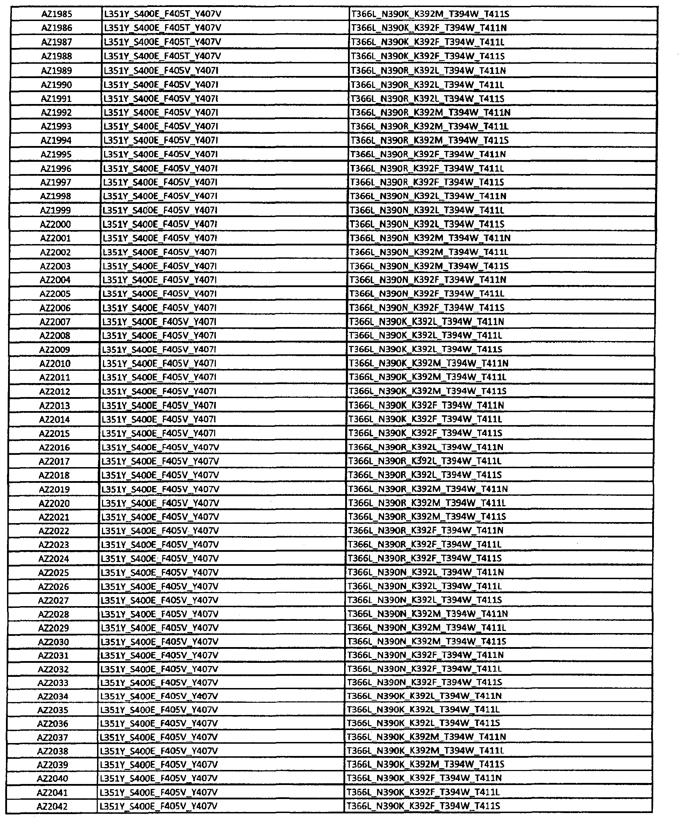

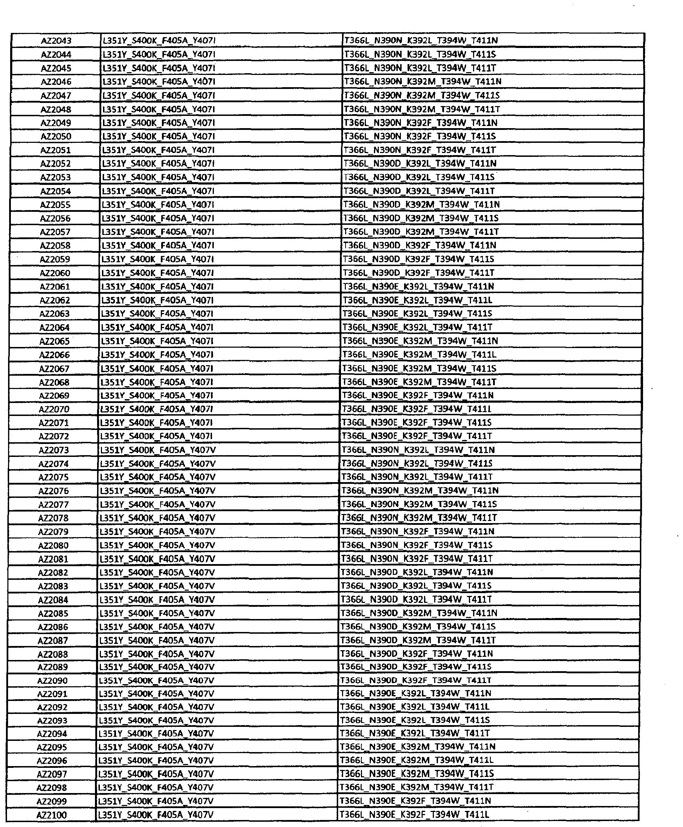

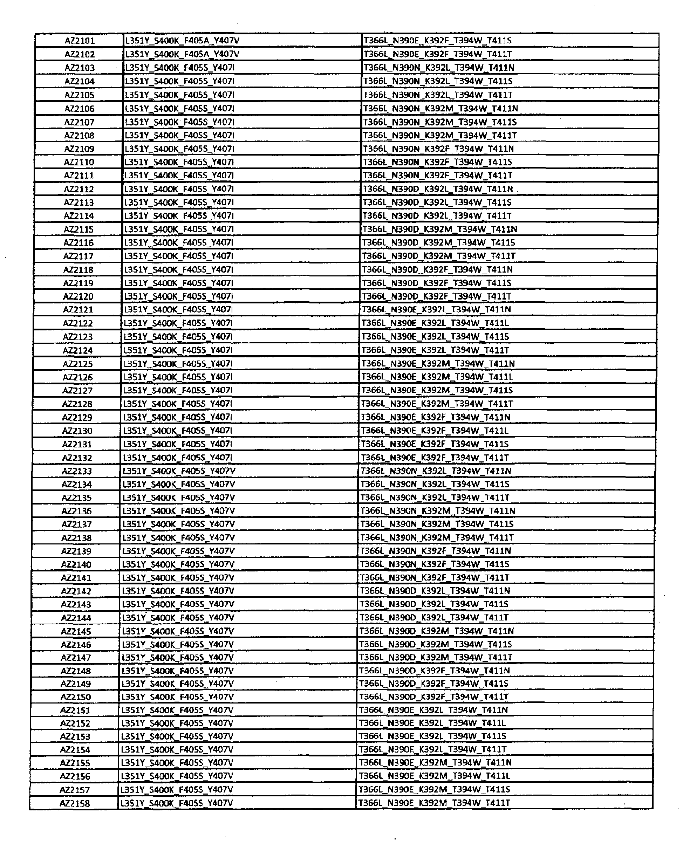

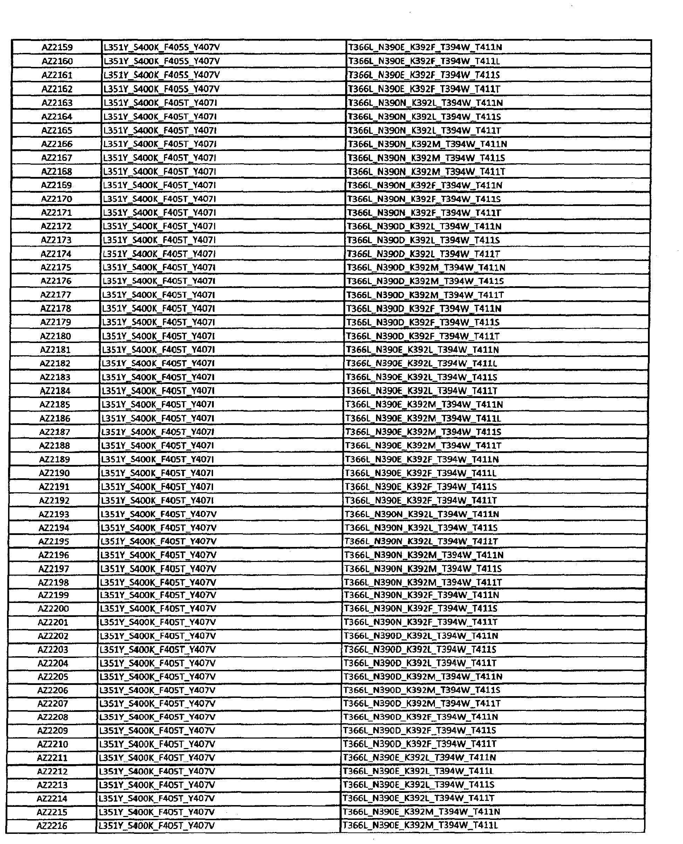

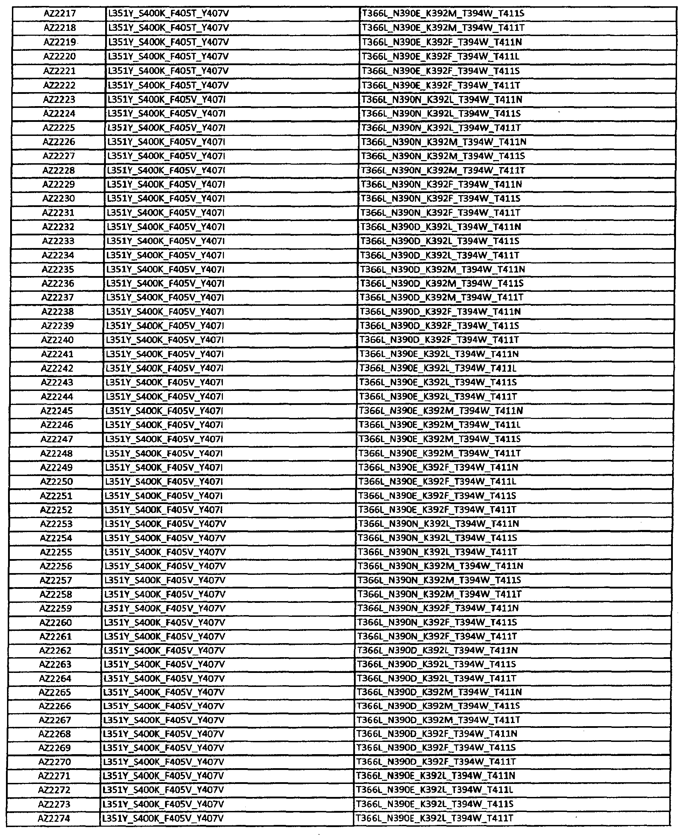

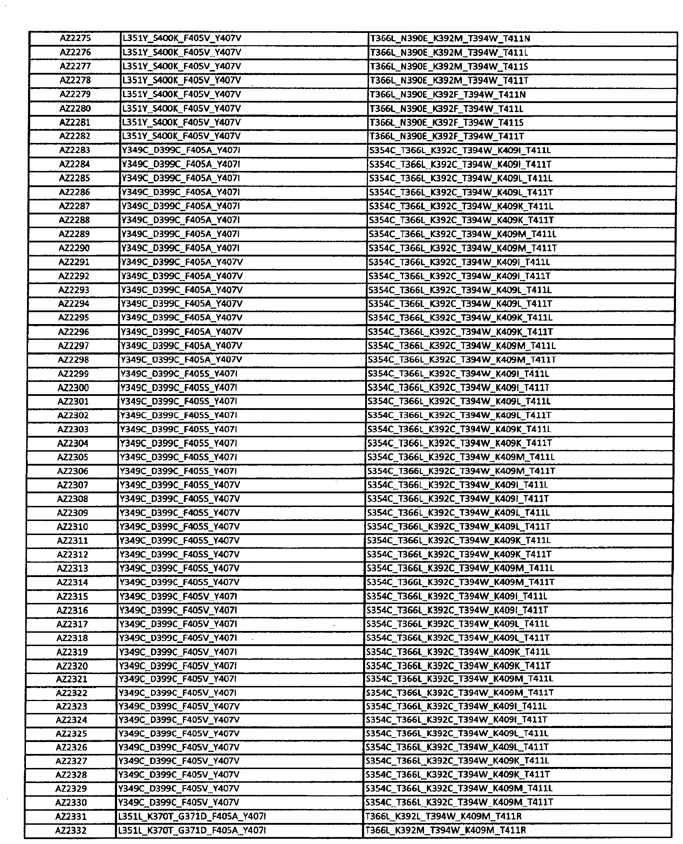

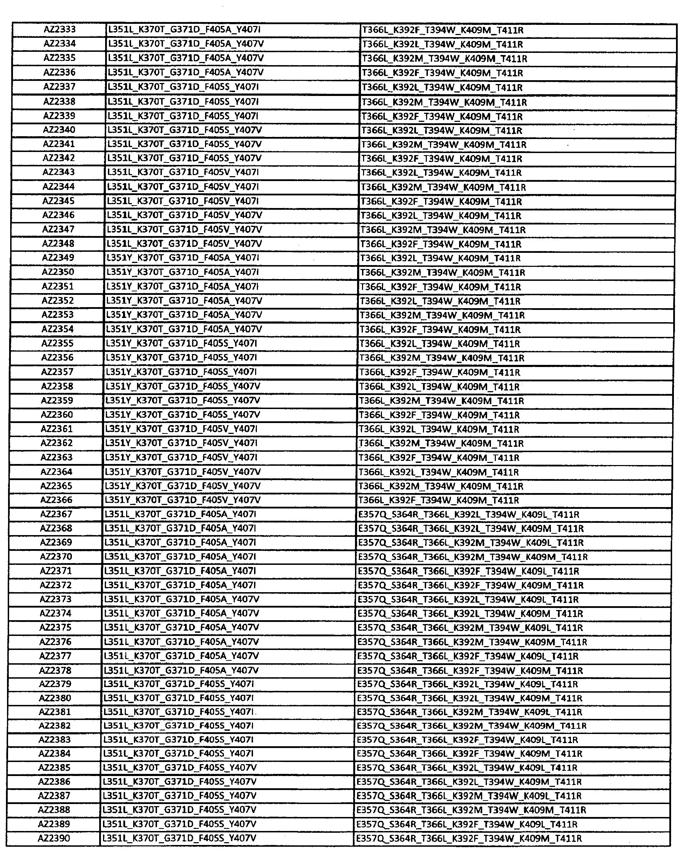

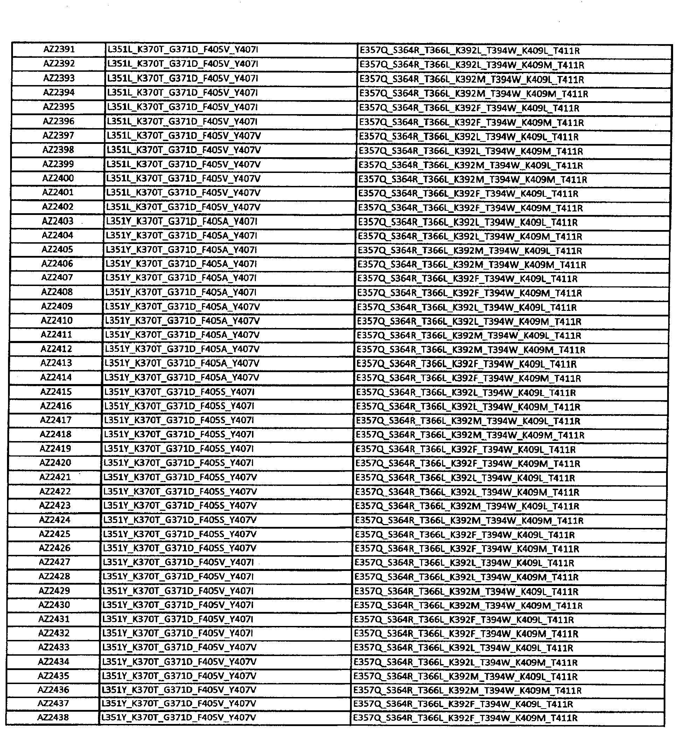

**Figure 37** - **Table 6** is a list of variants CH3 domains based on the third design phase as described in Example 5 for Scaffold 1.

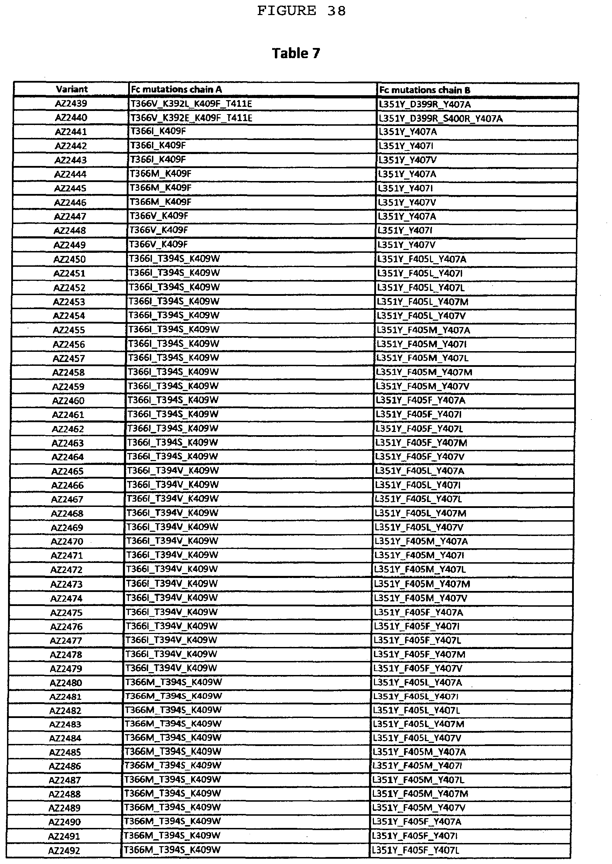

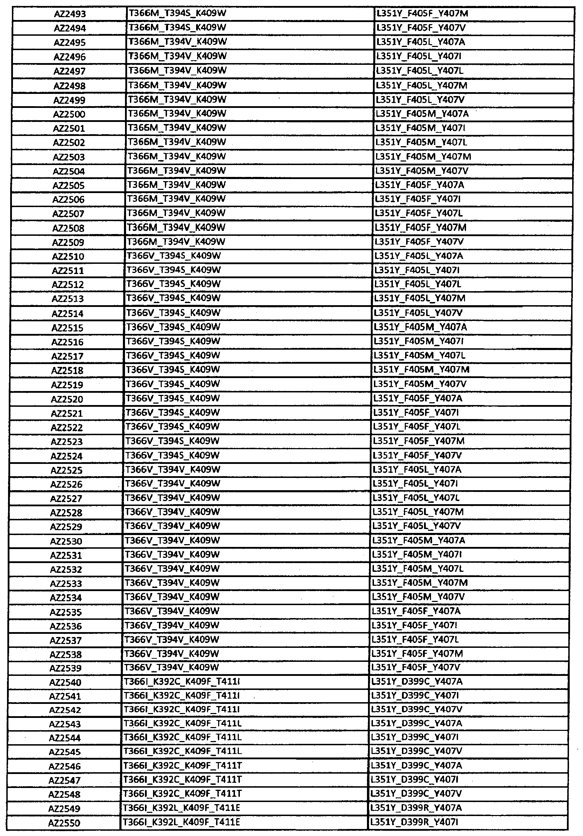

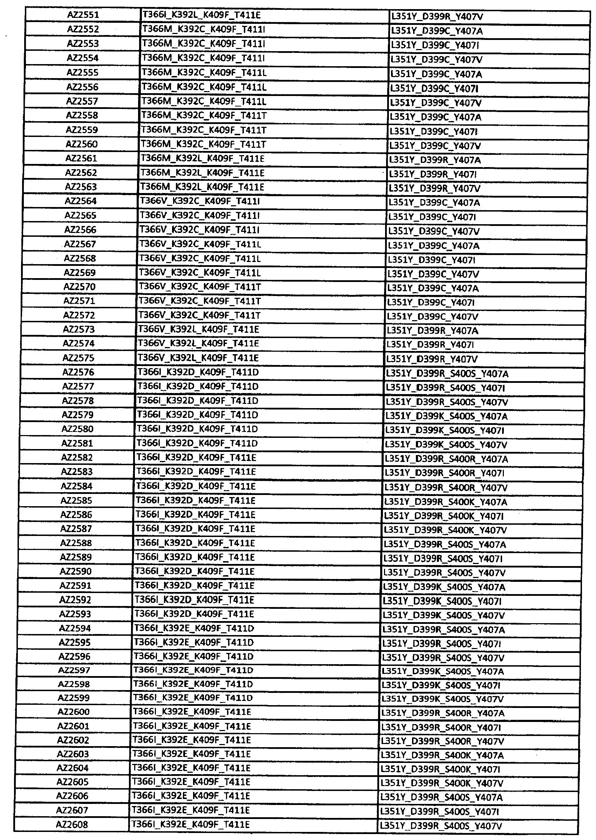

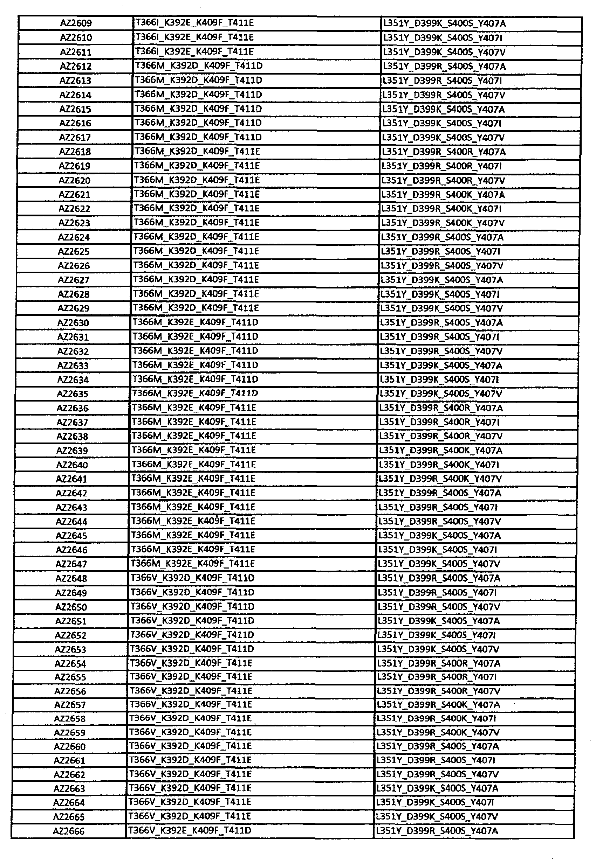

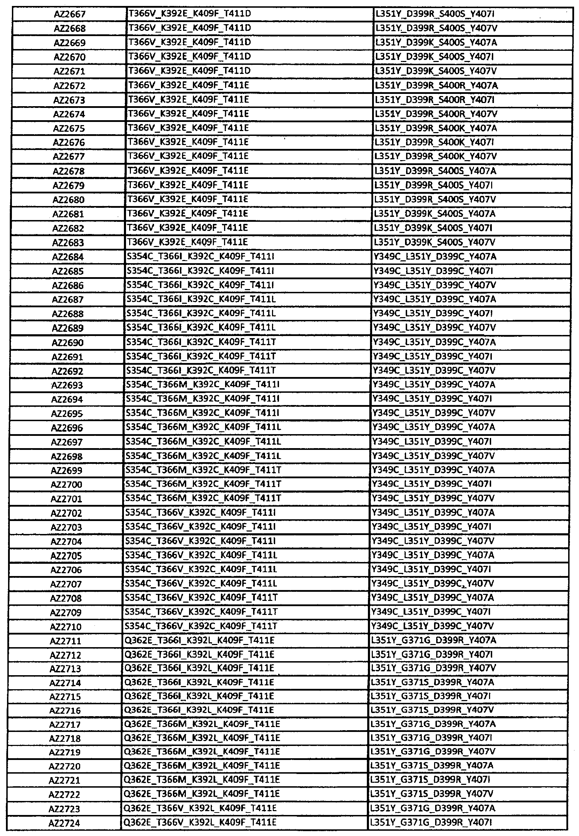

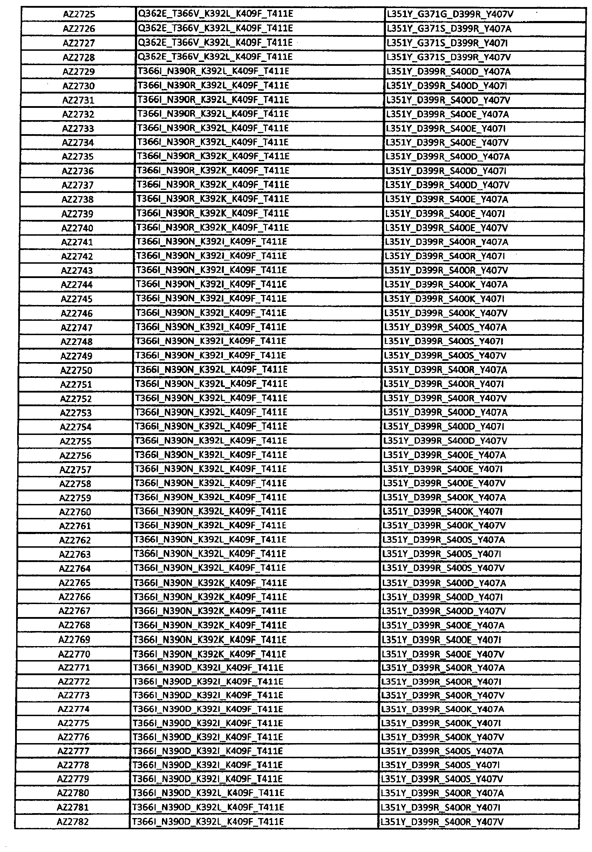

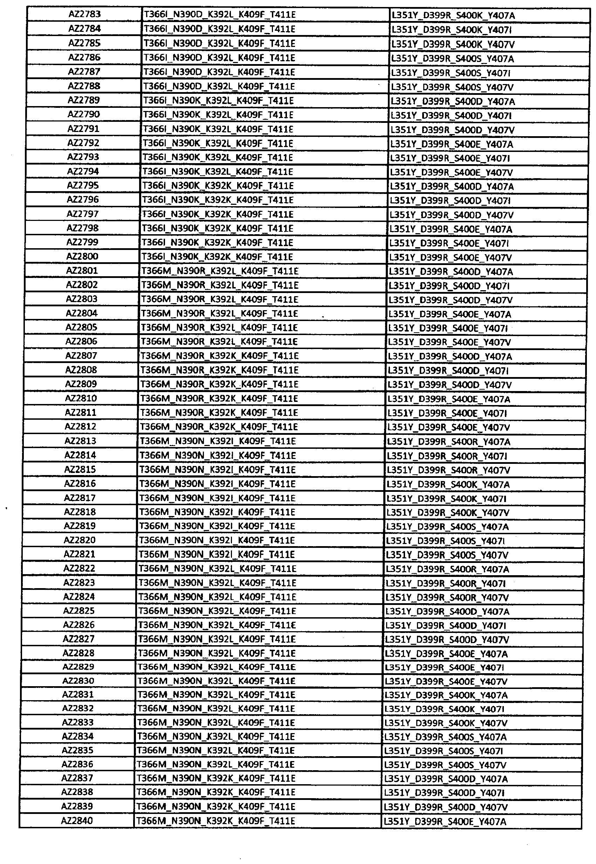

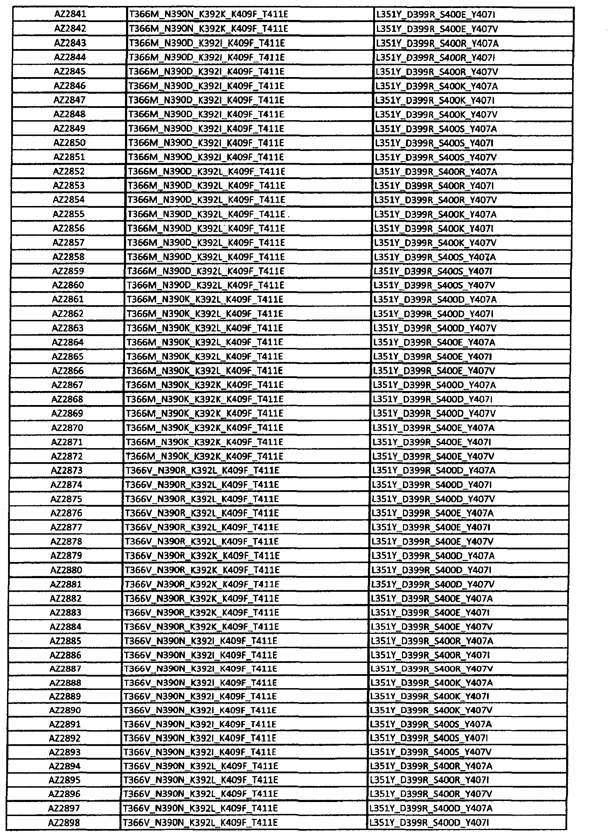

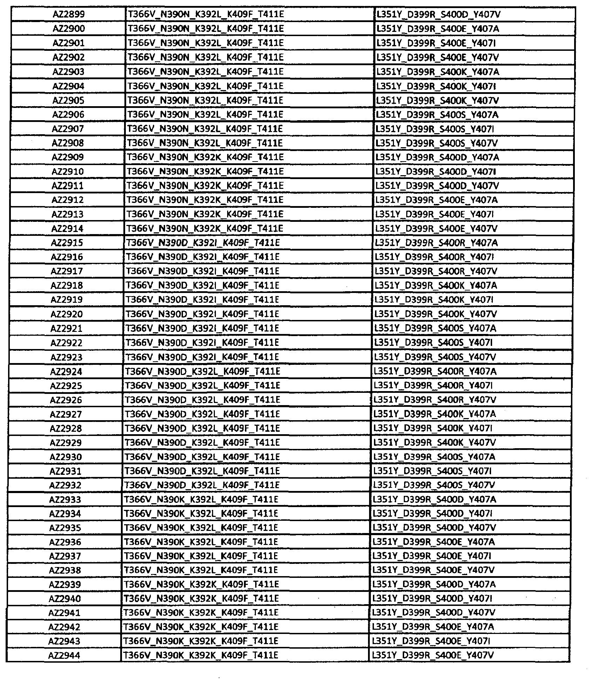

**Figure 38** - **Table 7** is a list of variant CH3 domains based on the third design phase as described in Example 6 for scaffold 2.

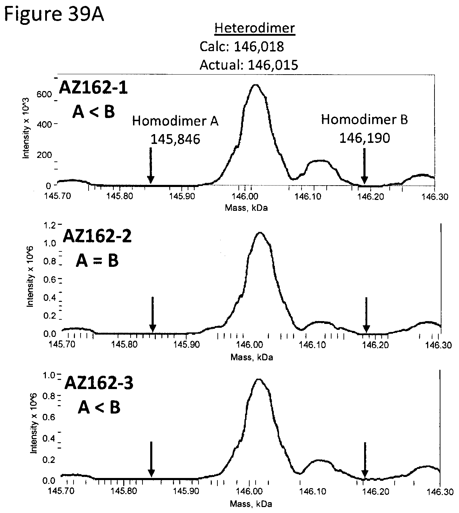

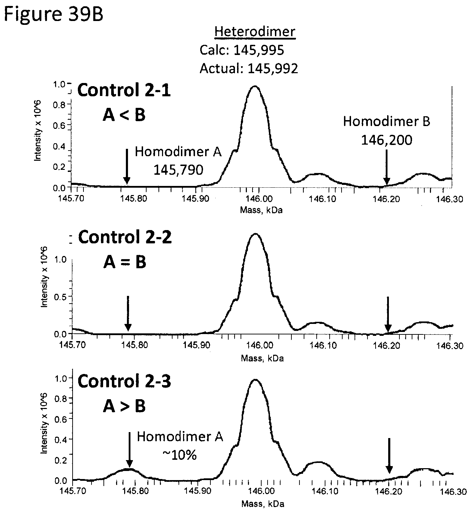

**Figure 39A-39B** illustrate Purity determination of variants without any C-terminal Tags using LC/MS. **Fig 39A** shows the LC/MS sprectra of one representative variant (AZ162: L351Y_F405A_Y407V/T366L_K392L_T394W). The variant was expressed by transient co-expression as described in the Examples using 3 different HeavyChain-A to HeavyChain-B ratios of 1:1.5 (AZ133-1), 1:1 (AZ133-2) and 1.5:1 (AZ133-3). The samples were purified and deglycosylated with Endo S for 1 hr at 37°C. Prior to MS analysis the samples were injected onto a Poros R2 column and eluted in a gradient with 20-90% ACN, 0.2% FA in 3 minutes. The peak of the LC column was analyzed with a LTQ-Orbitrap XL mass spectrometer (Cone Voltage: 50 V' Tube lens: 215 V; FT Resolution: 7,500) and integrated with the software Promass to generate molecular weight profiles. **Fig 39B** shows the LC/MS sprectra of the Control 2 sample, which represents the Knobs-into-Holes variant. The variant was expressed by transient co-expression as described in the Examples using 3 different HeavyCain-A to HeavyChain-B ratios of 1:1.5 (Control 2-1), 1:1 (Control 2-2) and 1.5:1 (Control 2-3). The samples were purified and deglycosylated with Endo S for 1 hr at 37°C. Prior to MS analysis the samples were injected onto a Poros R2 column and eluted in a gradient with 20-90% ACN, 0.2% FA in 3 minutes. The peak of the LC column was analyzed with a LTQ-Orbitrap XL mass spectrometer (Cone Voltage: 50 V' Tube lens: 215 V; FT Resolution: 7,500) and integrated with the software Promass to generate molecular weight profiles.

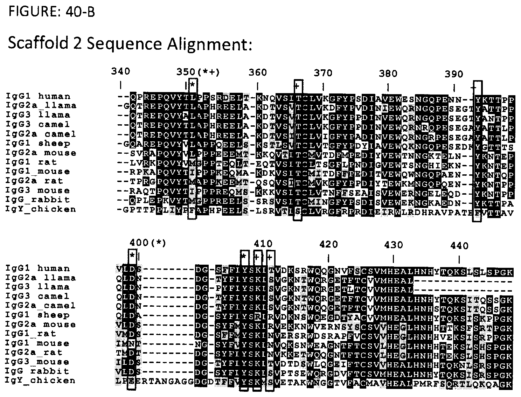

**Figure 40** provides multiple sequence alignment of CH3 domain sequences. The CH3 sequences of the different species are numbered according to the human IgG1 reference and the Eu numbering scheme. The residues mutated to achieve heterodimer formation are indicated by * for Chain_A and + for Chain_B. The mutations for Scaffold 1 include: Chain_A: (350V)_351Y_405A_407V and Chain_B: (350V)_366L_392L_394W. The corresponding positions for each of the species, as illustrated by gray and black boxes in the sequence alignment, can be mutated to form heterodimers in an IgG1 like manner with high purity. Species legend: IgG1_human (Homo sapiens); IgG2a_camel Camelus dromedarius (Arabian camel); IgG3_camel Camelus dromedarius (Arabian camel); IgG2a_llama Lama glama (Llama); IgG3_llama Lama glama (Llama); IgG2a_mouse Mus musculus (house mouse); IgG1_mouse Mus musculus (house mouse); IgG3_mouse Mus musculus (house mouse); IgG_rabbit Oryctolagus cuniculus (rabbit); IgG1_sheep Ovis aries (sheep); IgG2a_rat Rattus norvegicus (Norway rat); IgG1_rat Rattus norvegicus (Norway rat); IgY_chicken Gallus gallus (Chicken).
**Figure 41** depicts SDS-PAGE analysis of an exemplary heteromultimer
**Figure 42** depicts UPLC analysis of an exemplary heteromultimer.
**Figure 43** depicts the ability of an exemplary heteromultimer to bind to EGFR.
**Figure 44** depicts the amino acid sequence of an sdab that binds EGFR

### DETAILED DESCRIPTION

Described herein are isolated heteromultimers that comprise at least one single domain antigen-binding construct attached to at least one monomer of a heterodimer FC region that comprises modified CH3 domain comprising specific amino acid modifications to promote heteromultimer formation, wherein said isolated heteromultimers are devoid of immunoglobulin light chains, and optionally devoid of immunoglobulin first constant (CH1) region and immunoglobulin second constant (CH2) region. In some embodiments, the modified CH3 domains comprise specific amino acid modifications to promote heterodimer formation (See, for example Tables 1.1-1.3). In another embodiment the modified CH3 domains comprise specific amino acid modifications to promote heterodimer formation with increased stability (See, for example Table 4, Table 6 and Table 7). Stability is measured as the melting temperature (Tm) of the CH3 domain and an increased stability refers to a Tm of about 70°C or greater. The CH3 domains form part of the Fc region of a heteromultimeric, multispecific antibody. Provided herein in one embodiment are heteromultimers comprising a heterodimer Fc region, wherein the heterodimer Fc region comprises a modified or variant CH3 domain comprising amino acid mutations to promote heterodimer formation wherein the variant CH3 domains are selected from the variants listed in Table 1. In a second embodiment, provided are heteromultimers of the invention comprising a heterodimer Fc region, wherein the heterodimer Fc region comprises a variant CH3 domain comprising amino acid mutations to promote heterodimer formation with increased stability, wherein the variant CH3 domain has a melting temperature (Tm) of about 70°C or greater.

Amino acid modifications utilized to generate a modified CH3 domain include, but are not limited to, amino acid insertions, deletions, substitutions, and rearrangements. The modifications of the CH3 domain and the modified CH3 domains are referred to herein collectively as "CH3 modifications", "modified CH3 domains", "variant CH3 domains" or "CH3 variants". In certain embodiments, the modified CH3 domains are incorporated into a molecule of choice. Accordingly, in one embodiment are provided molecules, for instance polypeptides, such as immunoglobulins (e.g., antibodies) and other binding proteins, comprising an Fc region (as used herein "Fc region" and similar terms encompass any heavy chain constant region domain comprising at least a portion of the CH3 domain) incorporating a modified CH3 domain. Molecules comprising Fc regions comprising a modified CH3 domain (e.g., a CH3 domain comprising one or more amino acid insertions, deletions, substitutions, or rearrangements) are referred to herein as "Fc variants", "heterodimers" or "heteromultimers". The present Fc variants comprise a CH3 domain that has been asymmetrically modified to generate heterodimer Fc variants or regions. The heteromultimer is comprised of two heavy chain polypeptides - Chain A and Chain B, which can be used interchangeably provided that each Fc region comprises one Chain A and one Chain B polypeptide, and provided that at least one of Chain A and Chain B comprises a heavy chain variable region. The amino acid modifications are introduced into the CH3 in an asymmetric fashion resulting in a heterodimer when two modified CH3 domains form an Fc variant (See, e.g., Table 1). As used herein, asymmetric amino acid modifications are any modification wherein an amino acid at a specific position on one polypeptide (e.g., "Chain A") is different from the amino acid on the second polypeptide (e.g., "Chain B") at the same position of the heterodimer or Fc variant. This can be a result of modification of only one of the two amino acids or modification of both amino acids to two different amino acids from Chain A and Chain B. It is understood that the variant CH3 domains comprise one or more asymmetric amino acid modifications.

### Definitions

In the present description, any concentration range, percentage range, ratio range, or integer range is to be understood to include the value of any integer within the recited range and, when appropriate, fractions thereof (such as one tenth and one hundredth of an integer), unless otherwise indicated. As used herein, "about" means ± 10% of the indicated range, value, sequence, or structure, unless otherwise indicated. It should be understood that the terms "a" and "an" as used herein refer to "one or more" of the enumerated components unless otherwise indicated or dictated by its context. The use of the alternative (e.g., "or") should be understood to mean either one, both, or any combination thereof of the alternatives. As used herein, the terms "include" and "comprise" are used synonymously. In addition, it should be understood that the individual single chain polypeptides or heterodimers derived from various combinations of the structures and substituents (e.g., variant CH3 domains) described herein are disclosed by the present application to the same extent as if each single chain polypeptide or heterodimer were set forth individually. Thus, selection of particular components to form individual single chain polypeptides or heterodimers is within the scope of the present disclosure.

The "first polypeptide" is any polypeptide that is to be associated with a second polypeptide, also referred to herein as "Chain A". The first and second polypeptide meet at an "interface". The "second polypeptide" is any polypeptide that is to be associated with the first polypeptide via an "interface", also referred to herein as "Chain B". At least one of said first and second polypeptides comprise at least one heavy chain variable domain. In certain embodiments, the at least one heavy chain variable domain is obtained from a heavy chain antibody. In some embodiments, the heavy chain antibody is obtained from a cartilaginous fish such as a shark or a camelid such as a llama. The "interface" comprises those "contact" amino acid residues in the first polypeptide that interact with one or more "contact" amino acid residues in the interface of the second polypeptide. As used herein, the interface comprises the CH3 domain of an Fc region. In some embodiments, the Fc region is derived from an IgG antibody such as, but not restricted to a human IgG₁ antibody. In certain embodiments, the at least one heavy chain variable domain is connected to the CH3 domain by means of a linker.

As used herein, "isolated" heteromultimer means a heteromultimer that has been identified and separated and/or recovered from a component of its natural cell culture environment. Contaminant components of its natural environment are materials that would interfere with diagnostic or therapeutic uses for the heteromultimer, and may include enzymes, hormones, and other proteinaceous or non-proteinaceous solutes.

As used herein, "stability compareable to a native Fc homodimer" means that the Fc heterodimer has a stability analogous to that of the native homodimer. In certain embodiments, the stability of the heterodimer is within ±5°C of the corresponding native homodimeric Fc. In some embodiments, the stability of the heterodimer is within ±2°C of the corresponding native homodimeric Fc.

The heteromultimers described herein are generally purified to substantial homogeneity. The phrases "substantially homogeneous", "substantially homogeneous form" and "substantial homogeneity" are used to indicate that the product is substantially devoid of by-products originated from undesired polypeptide combinations (e.g. homodimers). Expressed in terms of purity, substantial homogeneity means that the amount of by-products does not exceed 10%, and preferably is below 5%, more preferably below 1%, most preferably below 0.5%, wherein the percentages are by weight.

Terms understood by those in the art of antibody technology are each given the meaning acquired in the art, unless expressly defined differently herein. Antibodies are known to have variable regions, a hinge region, and constant domains. Immunoglobulin structure and function are reviewed, for example, in Harlow et al, Eds., Antibodies: A Laboratory Manual, Chapter 14 (Cold Spring Harbor Laboratory, Cold Spring Harbor, 1988).

The "Fab fragment" of an antibody (also referred to as fragment antigen binding) contains the constant domain (CL) of the light chain and the first constant domain (CH1) of the heavy chain along with the variable domains VL and VH on the light and heavy chains respectively. The variable domains comprise the complementarity determining loops (CDR, also referred to as hypervariable region) that are involved in antigen binding. Fab' fragments differ from Fab fragments by the addition of a few residues at the carboxy terminus of the heavy chain CH1 domain including one or more cysteines from the antibody hinge region.

"Single-chain Fv" or "scFv" antibody fragments comprise the VH and VL domains of an antibody, wherein these domains are present in a single polypeptide chain. In one embodiment, the Fv polypeptide further comprises a polypeptide linker between the VH and VL domains which enables the scFv to form the desired structure for antigen binding.

For a review of scFv see Pluckthun in The Pharmacology of Monoclonal Antibodies, vol. 113, Rosenburg and Moore eds., Springer-Verlag, New York, pp. 269-315 (1994). HER2 antibody scFv fragments are described in WO93/16185; U.S. Pat. No. 5,571,894; and U.S. Pat. No. 5,587,458.

"Humanized" forms of non-human (e.g., rodent) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

### Heteromultimers

The present invention provides heteromultimers comprising at least one single domain antigen-binding (sdAg) construct and an immunoglobulin heterodimer Fc region, said immunoglobulin heterodimer Fc region comprising two monomeric Fc polypeptides, wherein the single domain antigen-binding construct is attached to one monomeric Fc polypeptide attached to an immunoglobulin heterodimer Fc region that comprises amino acid modifications that promote the formation of a heterodimeric Fc region with stability comparable to that of a native immunoglobulin homodimeric Fc region, and are devoid of IgG light chains and IgG CH1 regions. The heteromultimers can be monovalent and monospecific, bivalent and monospecific, or bivalent and bi-specific. The heteromultimers described herein, comprising the heterodimer Fc region described herein, have an intrinsic stability comparable to wild-type IgG1, and are formed with a purity of greater than about 85%.

In one embodiment the heteromultimer is a heterodimer comprising two heavy chains.

The heteromultimers described herein allow for easier construction and manufacturability of multi-functional, bi-specific antibodies compared to scFv or Fab comprising antibody formats. Since the heteromultimers described here are devoid of IgG light chains, the "light chain scrambling" problem inherent to making a bi-specific antibody comprising two light chains is avoided.

Heteromultimers as described herein also demonstrate
a) high affinity binding of antigen by the single domain antigen binding construct;
b) good heteromultimer quality and biophysical stability (lack of aggregation) of the constituent single domain antigen-binding construct, and
c) high antibody titre in Chinese hamster ovary (CHO) cell expression systems and manufacturability compared to other heteromultimer constructs comprising scFv and Fab formats.

These properties allow the heteromultimers described here to be used in a variety of applications including the development of bi-specific and multifunctional therapeutic antibodies and diagnostic or targeting reagents.

In one aspect described herein, the isolated heteromultimer comprises at least one single domain antigen-binding construct and an immunoglobulin heterodimer Fc region, said immunoglobulin heterodimer Fc region comprising two monomeric Fc polypeptides, wherein the single domain antigen-binding construct is attached to one monomeric Fc polypeptide, wherein the heterodimer Fc region comprises a variant CH3 domain comprising amino acid mutations that promote the formation of said heterodimer Fc region with stability comparable to a native homodimeric Fc region; and wherein said isolated heteromultimer is devoid of immunoglobulin light chain and immunoglobulin first constant (CH1) region.

It is contemplated that the heteromultimer described herein may have only one single domain antigen-binding construct attached to the heterodimer Fc region. Thus, in one embodiment, the isolated heteromultimer comprises one single domain antigen-binding construct and an immunoglobulin heterodimer Fc region, said immunoglobulin heterodimer Fc region comprising two monomeric Fc polypeptides, wherein the single domain antigen-binding construct is attached to one monomeric Fc polypeptide.

It is further contemplated that the isolated heteromultimer described herein may comprise two single domain antigen-binding constructs attached to the heteromultimer Fc region. In another embodiment, the isolated heteromultimer comprises one single domain antigen-binding construct and an immunoglobulin heterodimer Fc region, said immunoglobulin heterodimer Fc region comprising two monomeric Fc polypeptides, wherein the one single domain antigen-binding construct is attached to one monomeric Fc polypeptide and a second single domain antigen-binding construct is attached to the second monomeric Fc polypeptide.

### Single domain antigen binding constructs

As indicated above, the heteromultimers described herein comprise at least one single domain antigen-binding construct and an immunoglobulin heterodimer Fc region, said immunoglobulin heterodimer Fc region comprising two monomeric Fc polypeptides, wherein the single domain antigen-binding construct is attached to one monomeric Fc polypeptide.

Single domain antigen-binding constructs include binding polypeptides that can specifically or selectively bind target polypeptides. The term "specific binding" as used herein, refers to high-affinity binding of the antigen binding construct to the antigen as observed in the equilibrium dissociation constant K_{d}. K_{d} is the equilibrium dissociation constant and equal to k_{off}/kₒₙ. k_{off} describes the dissociation rate of antigen binding construct complexed to the antigen and kₒₙ describes the association rate of antigen binding construct to the antigen. The term "selective binding" as used herein, refers to the differing affinities with which a ligand binds to receptors or targets, such that a ligand shows higher affinity for one target over another; Selective ligands bind to a very limited types of receptors, whereas non-selective ligands bind to several types of receptors. As is known in the art, specific and selective binding to target receptor can empower a therapeutic comprising the single domain antigen binding construct to recognize and treat diseased cells that express the target.

Examples of suitable single domain antigen-binding constructs include those that are devoid of antibody light chains such as single domain antibodies (sdAb or V_{H}), camelid nanobodies (VₕH), shark V_{NAR}, SH3-derived fynomers, and fibronectin-derived binding domains such as adnectins and DARPins (designed ankyrin repeat proteins). These single domain antigen-binding constructs have been shown to exhibit properties such as the ability to bind to alternative or cryptic epitopes that may not be accessible by traditional Fabs due to their size and structural conformation.

In one embodiment, the single domain antigen-binding construct is an immunoglobulin heavy chain variable region or variable heavy chain selected from a domain antibody (or an amino acid sequence that is suitable for use as a domain antibody), a single domain antibody (or an amino acid sequence that is suitable for use as a single domain antibody), a "dAb" (or an amino acid sequence that is suitable for use as a dAb) or a Nanobody (as defined herein, and including but not limited to a V_{HH} sequence); other single variable domains, or any suitable fragment of any one thereof. For a general description of (single) domain antibodies, reference is also made to the art cited above, as well as to EP 0 368 684. For the term "dAb's", reference is for example made to Ward et al. (Nature 1989 Oct. 12; 341 (6242): 544-6), to Holt et al. (Trends Biotechnol., 2003, 21(11):484-490); as well as to for example WO 04/068820, WO 06/030220, WO 06/003388 and other published patent applications of Domantis Ltd. In some embodiments, the variable heavy chains comprise single domain antibodies or single variable domains can be derived from certain species of shark (for example, the so-called "IgNAR domains", see for example WO 05/18629).

Thus, in one embodiment, the isolated heteromultimer comprises a single domain antigen-binding construct selected from single domain antibodies, camelid nanobodies, shark V_{NAR}, SH3-derived fynomers, and fibronectin-derived binding domains such as adnectins and DARPins. In one embodiment, the isolated heteromultimer comprises a single domain antigen-binding construct selected from single domain antibodies, camelid nanobodies and cartilaginous fish V_{NAR}. In one embodiment, the isolated heteromultimer comprises a single domain antigen-binding construct that is an SH3-derived fynomer or a fibronectin-derived binding domain. In one embodiment, the fibronectin-derived binding domain is a DARPin or adnectin. SH3-derived fynomers, adnectins and DARPins are known in the art (see for example Grabulovski et al. J Biol Chem. (2007) 282(5):3196-204; Lipovšek Protein Engineering, Design and Selection (2011) 24(1-2): 3-9, and Tamaskovic, Methods in Enzymology (2012), 503, 101-134).

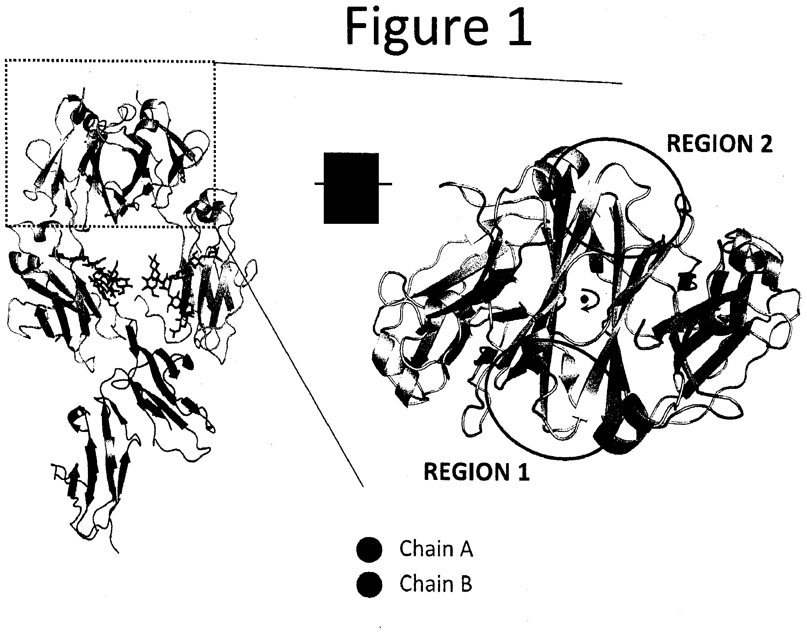

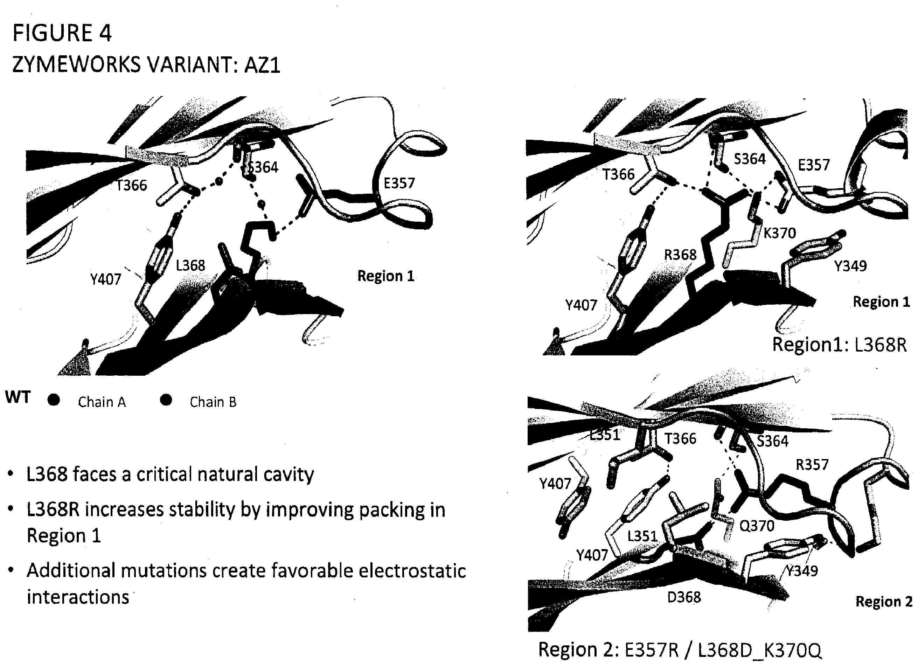

In one embodiment, the single domain antigen-binding construct is a single domain antibody. Single domain antibodies are antibody fragments that consist of a single monomeric variable antibody domain. Suitable examples of domain antibodies are known in the art and include, for example, (see for example Figure 4 of Wesolowski et al. Med Microbiol Immunol. (2009), 198(3): 157-174 and Figure 1A of Barthelemy et al. J. Biol. Chem. (2008) 138, 3639-54).

In one embodiment, the single domain antigen-binding construct is a camelid nanobody (VₕH). Camelid nanobodies are antibody fragments derived from heavy chain antibodies found in camelids that are devoid of light chain and heavy chain CH1 constant domain. In one embodiment, the single domain antigen-binding construct is a cartilaginous fish V_{NAR}. Such cartilaginous fish V_{NAR} include V_{NAR}s antibody fragments derived from heavy chain antibodies found in sharks. These antibody fragments are also devoid of light chains and heavy chain CH1 constant domain. Examples of known camelid and shark single domain antigen-binding constructs are identified in Table 1 of Wesolowski et al, Med Microbiol Immunol (2009) 198:157-174. For example, single domain antigen-binding constructs that target membrane proteins include ART2.2 from Immune llama; CD16 from Immune llama; EGFR from Immune camel, immune llama; CEA Cancer immunotherapy from Immune llama; MUC 1 Tumor targeting Immune from camel and llama, and CD105 (endoglin) from Immune camel. Single domain antigen-binding constructs that target secretory proteins include TNF from Immune llama and alpaca; PSA from Immune dromedary; von Willebrand factor from Immune llama; Amyloid A peptide from Immune dromedary and alpaca; Lysozyme from Immune dromedary; IgG from Immune llama; and Serum albumin from Immune llama. Single domain antigen-binding constructs that target intracellular proteins include Bax from Non-immune llama; HIF-1 from Non-immune llama, PABPN1 from Immune and non-immune llama.

Single domain antigen-binding constructs suitable for use in the heteromultimers described herein can be obtained from naturally occurring sources such as camelids (including camels, llamas, and alpacas, for example), and sharks. Single domain antigen-binding constructs may also be obtained by screening libraries such as phage-display libraries in order to select for single domain antigen-binding constructs that bind to a target of interest. Methods of screening such libraries in order to select target-specific single domain antigen-binding constructs are known in the art (see for example Groot et al, in Lab Invest (2006) 86:345-56, and Verheesen et al in Methods Mol Biol (2012) 911:81-104).

The nucleotide and/or amino acid sequences of specific single domain antigen-binding constructs are known in the art or are accessible in published sequence databases, for example, GenBank, SwissProt, or EMBL for example, thus facilitating the preparation of the heteromultimers comprising single domain antigen-binding constructs as described herein.

### Selection of targets

As indicated above, the single domain antigen-binding constructs are able to selectively and/or specifically bind to a target antigen. The target antigen is selected based on the intended use of the heteromultimer. In one embodiment, the target cell is a cell that is activated or amplified in a cancer, an infectious disease, an autoimmune disease, or in an inflammatory disease. In one embodiment, where the heteromultimer binds to EGFR1, the target cell is a cell that is activated or amplified in a cancer, an autoimmune disease, or in an inflammatory disease.

In another embodiment, the target cell is one that is activated or amplified when a subject is suffering from an infection with a pathogenic organism, such as bacteria or fungi.

In another embodiment, the heteromultimers are used to target a cell expressing a target antigen that is not typically accessible by traditional antigen-binding moieties such as Fabs. The nature of the single domain antigen-binding construct of the heteromultimer allows for binding to targets such as, for example, highly conserved residues such as CD4, for example, that are protected from the humoral immune system by conformational masking and steric occlusion, and to target antigen smaller epitopes.

### Specific targets

In one embodiment, the heteromultimers according to the invention target one or more cytokines or chemokines such as, for example, IL2, IFNa-2a/b, IFN-1a/b, IL-21, IL-17a, TNF, IL23, VEGF, or ANG2. In another embodiment, the heteromultimers according to the invention target one or more tumor associated antigens such as EpCam, EGFR, VEGFR, CEA, or GP100. In another embodiment, the heteromultimers according to the invention target immunoregulatory antigens such as CD16, CD30, CD137, CD22, CD52, CD80, CD23, CD2, CD4, CD40, KIR, CD32b, CD25, LAG3, or B7-H3.

In one embodiment, the heteromultimers provided herein target one or more bacterial toxins such as Clostridium difficile toxin A, Clostridium difficile toxin B.

In certain embodiments, the heteromultimers provided herein are useful to target one or more target antigen selected from EGFR, IGF1R, ICAM-1, Clostridium difficile toxin A, Clostridium difficile toxin B, ICAM-1, Bax-protein, CDC50A, and CD3 isoforms inclusive of the epsilon isoform. In certain embodiments are heteromultimers described herein, comprising at least one single domain antigen-binding construct that targets one or more of EGFR, IGF1R, ICAM-1, Clostridium difficile toxin A, Clostridium difficile toxin B, ICAM-1, Bax-protein, CDC50A, and CD3 isoforms inclusive of the epsilon isoform. In certain embodiments are heteromultimers described herein, comprising at least one single domain antigen-binding construct derived from a llama heavy chain antibody, wherein said varuable heavy chain targets one or more of EGFR, IGF1R, ICAM-1, Clostridium difficile toxin A, Clostridium difficile toxin B, ICAM-1, Bax-protein, CDC50A, and CD3 isoforms inclusive of the epsilon isoform. In certain embodiments are multispecific heteromultimers comprising single domain antigen-binding constructs that target one or more of EGFR, IGF1R, ICAM-1, Clostridium difficile toxin A, Clostridium difficile toxin B, ICAM-1, Bax-protein, CDC50A, and CD3 isoforms inclusive of the epsilon isoform. In certain embodiments are bi-specific heteromultimers described herein, wherein said heteromultimers comprise single domain antigen-binding constructs derived from camelid heavy chain antibodies that target one or more of EGFR, IGF1R, ICAM-1, Clostridium difficile toxin A, Clostridium difficile toxin B, ICAM-1, Bax-protein, CDC50A, and CD3 isoforms inclusive of the epsilon isoform. In certain embodiments are bi-specific heteromultimers described herein, wherein said heteromultimers comprise single domain antigen-binding constructs derived from llama heavy chain antibodies that target one or more of EGFR, IGF1R, ICAM-1, Clostridium difficile toxin A, Clostridium difficile toxin B, ICAM-1, Bax-protein, CDC50A, and CD3 isoforms inclusive of the epsilon isoform.

In one embodiment, the single domain antigen-binding construct is one that has a neutralizing activity on the target antigen. The term "neutralizing activity," as used herein in the context of a single domain antigen-binding construct, refers the ability of the single domain antigen-binding construct to block binding of a cognate ligand to the target antigen. In another embodiment, the single domain antigen-binding construct is one that does not have neutralizing activity on the target antigen. In one embodiment, the heteromultimer comprises an EFGR single domain antigen-binding construct that is non-neutralizing. In another embodiment, the heteromultimer comprises an EFGR single domain antigen-binding construct that is neutralizing. Examples of neutralizing EFGR single domain antigen-binding constructs are found in Omidfar *et al.* (2012) 31:1015-1026.

In one embodiment where the heteromultimer according to the invention comprises two single domain antigen-binding constructs, both single domain antigen-binding constructs bind to the same antigen. In another embodiment, where the heteromultimer according to the invention comprises two single domain antigen-binding constructs, both single domain antigen-binding constructs bind to the same epitope. In another embodiment, where the heteromultimer according to the invention comprises two single domain antigen-binding constructs, one single domain antigen-binding construct binds to one target and the second single domain antigen-binding construct binds to a different target. In still another embodiment, where the heteromultimer according to the invention comprises two single domain antigen-binding constructs, one single domain antigen-binding construct binds to one epitope and the second single domain antigen-binding construct binds to a different epitope.

### Immunoglobulin heterodimer Fc region

As indicated above, the heteromultimers described herein comprise at least one single domain antigen-binding (sdAg) construct and an immunoglobulin heterodimer Fc region, said immunoglobulin heterodimer Fc region comprising two monomeric Fc polypeptides, wherein the single domain antigen-binding construct is attached to one monomeric Fc polypeptide attached to an immunoglobulin heterodimer Fc region that comprises amino acid modifications that promote the formation of a heterodimeric Fc region with stability comparable to that of a native immunoglobulin homodimeric Fc region, and are devoid of IgG light chains and IgG CH1 regions.

Immunoglobulin heterodimer Fc regions are further described as follows. As indicated elsewhere herein, in one embodiment are provided molecules, for instance polypeptides, such as immunoglobulins (e.g., antibodies) and other binding proteins, comprising an Fc region (as used herein "Fc region" and similar terms encompass any heavy chain constant region domain comprising at least a portion of the CH3 domain) incorporating a modified CH3 domain. Molecules comprising Fc regions comprising a modified CH3 domain (e.g., a CH3 domain comprising one or more amino acid insertions, deletions, substitutions, or rearrangements) are referred to herein as "Fc variants", "heterodimers," "variant Fc heterodimers" or "heteromultimers". The present Fc variants comprise a CH3 domain that has been asymmetrically modified to generate heterodimer Fc variants or regions. The heteromultimer is comprised of two heavy chain polypeptides, or two monomeric Fc polypeptides - Chain A and Chain B, which can be used interchangeably provided that each Fc region comprises one Chain A and one Chain B polypeptide, and provided that at least one of Chain A and Chain B comprises a heavy chain variable region.

The design of variant Fc heterodimers from wildtype homodimers is illustrated by the concept of positive and negative design in the context of protein engineering by balancing stability vs. specificity, wherein mutations are introduced with the goal of driving heterodimer formation over homodimer formation when the polypeptides are expressed in cell culture conditions. Negative design strategies maximize unfavorable interactions for the formation of homodimers, by either introducing bulky sidechains on one chain and small sidechains on the opposite, for example the knobs-into-holes strategy developed by Genentech (Ridgway JB, Presta LG, Carter P. 'Knobs-into-holes' engineering of antibody CH3 domains for heavy chain heterodimerization. Protein Eng. 1996 Jul;9(7):617-21; Atwell S, Ridgway JB, Wells JA, Carter P. Stable heterodimers from remodeling the domain interface of a homodimer using a phage display library. J Mol Biol. 270(1):26-35 (1997))), or by electrostatic engineering that leads to repulsion of homodimer formation, for example the electrostatic steering strategy developed by Amgen (Gunaskekaran K, et al. Enhancing antibody Fc heterodimer formation through electrostatic steering effects: applications to bi-specific molecules and monovalent IgG. JBC 285 (25): 19637-19646 (2010)). In these two examples, negative design asymmetric point mutations were introduced into the wild-type CH3 domain to drive heterodimer formation. To date, only negative design strategies have been used to develop Fc heterodimers. Published results show that heterodimers designed using only a negative design approach leads to high specificity with >95% heterodimers, but destabilizes the complex considerably (*Supra*). These negative design heterodimers posses a melting temperature, of the modified CH3 domain, of 69°C or less, absent additional disulfide bonds as compared to the wild type. See, Table A below.

**Table A: Published Fc Heterodimer Antibodies.**

| | **Chains** | **Engineering Approach** | **Source** | **Purity** | **Tm °C** |
|---|---|---|---|---|---|
| **Wild-Type** | - | | | | 81-83 |
| | - | | | | |
| Control 4 | Y349C_T366S_ L368A_Y407V | Knobs-into-holes plus disulfide | Genentech (Merchant et al.) | 95% | >77** |
| | S354C_T366W | | | | |
| Control 3 | K409D_K392D | Electrostatic steering | Amgen (Gunaskekaran et al.) | <80% | NP |
| | D399K | | | | |
| Control 2 | T366S_L368A_ Y407V | Knobs-into-holes | Genentech (Atwell et al.) | 95% | 69 |
| | T366W | | | | |
| Control 1 | K409D_K392D | Electrostatic steering | Amgen (Gunaskekaran et al.) | 100%* | 67 |
| | D399K_E356K | | | | |
| Control 5 | IgG-IgA chimera | Strand Exchange | EMD Serono (Muda et al.) | >90% | 68 |

| | | | | | |
|---|---|---|---|---|---|
| * We observed a purity of >90% for Control 1 in our assay system, but not 100% as previously reported in the literature. ** We observed a Tm greater than 77°C for control 4 in our assay system; the Tm for this variant has not been published in the literature. NP - The Tm for Control 3 has not been published and it was not tested in our assays systems. | | | | | |

The melting temperature for wild-type IgG1 is shown as a range from 81-83 as the values in the literature vary depending on the assay system used, we report a value of 81.5°C in our assay system.

In contrast to negative design, a general concept used to engineer proteins is positive design. In this instance amino acid modifications are introduced into polypeptides to maximize favorable interactions within or between proteins. This strategy assumes that when introducing multiple mutations that specifically stabilize the desired heterodimer while neglecting the effect on the homodimers, the net effect will be better specificity for the desired heterodimer interactions over the homodimers and hence a greater heterodimer specificity. It is understood in the context of protein engineering that positive design strategies optimize the stability of the desired protein interactions, but rarely achieve >90% specificity (Havranek JJ & Harbury PB. Automated design of specificity in molecular recognition.Nat Struct Biol. 10(1):45-52 (2003); Bolon DN, Grant RA, Baker TA, Sauer RT. Specificity versus stability in computational protein design. Proc Natl Acad Sci U S A. 6;102(36):12724-9 (2005); Huang PS, Love JJ, Mayo SL. A de novo designed protein protein interface Protein Sci. 16(12):2770-4 (2007)). Prior to this disclosure positive design strategies have not been used to design Fc heterodimers as more attention was devoted to specificity as compared to stability for therapeutic antibody manufacturing and development. In addition, beneficial positive design mutations can be hard to predict. Other methodologies for improving stability, such as additional disulfide bonds,have been tried to improve stability in Fc heterodimers with limited success on improvements to the molecule.(See, Table A) This may be because all engineered Fc CH3 domain disulfide bonds are solvent exposed, which results in a short lifetime of the disulfide bond and therefore a significant impact on the long-term stability of the heterodimer - especially when the engineered CH3 domain has a Tm of less than 70°C without the additional disulfide bond (as in Control 4 which has a Tm of 69°C without the disulfide (see Control 2).It is contemplated that other methodologies to improve stability, such as disulfide bonds, can also be used with the present Fc variants, provided the intrinsic stability (measured as melting temperature) of the CH3 domain is 70°C or greater without the disulfide bond, in particular when the intrinsic stability (measured as melting temperature) of the CH3 domain is 72°C or greater without the disulfide bond.

Therefore, we herein disclose a novel method for designing Fc heterodimers that results in both stable and highly specific heterodimer formation. This design method combines both negative and positive design strategies along with structural and computational modeling guided protein engineering techniques. This powerful method has allowed us to design novel combinations of mutations in the IgG1 CH3 domain wherein using only standard cell culture conditions heterodimers were formed with more than 90% purity compared to homodimers and the resulting heterodimers had a melting temperature of 70°C or greater. In exemplary embodiments, the Fc variant heterodimers have a melting temperature of 73°C or greater and a purity of greater than 98%. In other exemplary embodiments, the Fc variant heterodimers have a melting temperature of 75°C or greater and a purity of greater than 90%. In certain embodiments of the heterodimer Fc variants described herein, the Fc variant heterodimers have a melting temperature of 77°C or greater and a purity of greater than 98%. In some embodiments of the heterodimer Fc variants described herein, the Fc variant heterodimers have a melting temperature of 78°C or greater and a purity of greater than 98%. In certain embodiments of the heterodimer Fc variants described herein, the Fc variant heterodimers have a melting temperature of 79°C or greater and a purity of greater than 98%. In certain embodiments of the heterodimer Fc variants described herein, the Fc variant heterodimers have a melting temperature of 80°C or greater and a purity of greater than 98%. In certain embodiments of the heterodimer Fc variants described herein, the Fc variant heterodimers have a melting temperature of 81°C or greater and a purity of greater than 98%.

In certain embodiments, an isolated heteromultimer comprising at least one heavy chain variable domain and a heterodimer Fc region is provided wherein the heterodimer Fc region comprises a variant CH3 domain comprising amino acid mutations to promote heterodimer formation with increased stability, wherein the variant CH3 domain has a melting temperature (Tm) of 70°C or greater. As used herein "increased stability" or "stable heterodimer", refers to a variant CH3 domain, in heterodimer formation, with a melting temperature of about 70°C or greater. In certain embodiments, "increased stability" or "stable heterodimer", refers to a variant CH3 domain, in heterodimer formation, with a melting temperature of about 72°C or greater. In certain embodiments, "increased stability" or "stable heterodimer", refers to a variant CH3 domain, in heterodimer formation, with a melting temperature of about 74°C or greater. In certain embodiments, "increased stability" or "stable heterodimer", refers to a variant CH3 domain, in heterodimer formation, with a melting temperature of about 75°C or greater. In certain embodiments, "increased stability" or "stable heterodimer", refers to a variant CH3 domain, in heterodimer formation, with a melting temperature of about 76°C or greater. In certain embodiments, "increased stability" or "stable heterodimer", refers to a variant CH3 domain, in heterodimer formation, with a melting temperature of about 78°C or greater. In certain embodiments, "increased stability" or "stable heterodimer", refers to a variant CH3 domain, in heterodimer formation, with a melting temperature of about 79°C or greater. In certain embodiments, "increased stability" or "stable heterodimer", refers to a variant CH3 domain, in heterodimer formation, with a melting temperature of about 80°C or greater. In certain embodiments, "increased stability" or "stable heterodimer", refers to a variant CH3 domain, in heterodimer formation, with a melting temperature of about 81°C or greater. In addition, it is understood that the term "to promote heterodimer formation" refers herein to the amino acid mutations in the CH3 domain that result in greater than 90% heterodimer formation compared to homodimer formation.

In a further embodiment, this increased stability is in the absence of an additional disulfide bond. Specifically, the increased stability is in the absence of an additional disulfide bond in the CH3 domain. In one embodiment, the variant CH3 domain does not comprise an additional disulfide bond as compared to wild-type CH3 domain. In an alternative embodiment, the variant CH3 comprises at least one disulfide bond as compared to wild-type CH3 domain, provided that the variant CH3 has a melting temperature of 70°C or greater in the absence of the disulfide bond. In one embodiments, the variant CH3 domain comprises at least one disulfide bond as compared to wild-type CH3 domain, and the variant CH3 domain has a melting temperature (Tm) of about 77.5 °C or greater. In an embodiment, the variant CH3 domain comprises at least one disulfide bond as compared to wild-type CH3 domain, and the variant CH3 domain has a melting temperature (Tm) of about 78°C or greater. In another embodiment, the variant CH3 domain comprises at least one disulfide bond as compared to wild-type CH3 domain, and the variant CH3 domain has a melting temperature (Tm) of greater than about 78°C, or greater than about 78.5°C, or greater than about 79°C, or greater than about 79.5°C, or greater than about 80°C, or greater than about 80.5°C, or greater than about 81°C, or greater than about 81.5°C, or greater than about 82°C, or greater than about 82.5°C, or greater than about 83°C.

In one embodiment, the variant CH3 domain has a melting temperature of greater than about 70°C, or greater than about 70.5°C, or greater than about 71°C, or greater than about 71.5°C, or greater than about 72°C, or greater than about 72.5°C, or greater than about 73°C, or greater than about 73.5°C, or greater than about 74°C, or greater than about 74.5°C, or greater than about 75°C, or greater than about 75.5°C, or greater than about 76°C, or greater than about 76.5°C, or greater than about 77°C, or greater than about 77.5°C, or greater than about 78°C, or greater than about 78.5°C, or greater than about 79°C, or greater than about 79.5°C, or greater than about 80°C, or greater than about 80.5°C, or greater than about 81°C, or greater than about 81.5°C, or greater than about 82°C, or greater than about 82.5°C, or greater than about 83°C. In another embodiment, the variant CH3 domain has a melting temperature of about 70°C,or about 70.5°C, or about 71°C, or about 71.5°C, or about 72°C, or about 72.5°C, or about 73°C, or about 73.5°C, or about 74°C, or about 74.5°C, or about 75°C, or about 75.5°C, or about 76°C, or about 76.5°C, or about 77°C, or about 77.5°C, or about 78°C, or about 78.5°C, or about 79°C, or about 79.5°C, or about 80°C, or about 80.5°C, or about 81°C. In yet another embodiment, the variant CH3 domain has a melting temperature of about 70°C to about 81°C,or about 70.5°C to about 81°C, or about 71°C to about 81°C, or about 71.5°C to about 81°C, or about 72°C to about 81°C, or about 72.5°C to about 81°C, or about 73°C to about 81°C, or about 73.5°C to about 81°C, or about 74°C to about 81°C, or about 74.5°C to about 81°C, or about 75°C to about 81°C, or about 75.5°C to about 81°C, or 76°C to about 81°C, or about 76.5°C to about 81°C, or about 77°C to about 81°C, or about 77.5°C to about 81°C, or about 78°C to about 81°C, or about 78.5°C to about 82°C, or about 79°C to about 81°C. In yet another embodiment, the variant CH3 domain has a melting temperature of about 71°C to about 76°C, or about 72°C to about 76°C, or about 73°C to about 76°C, or about 74°C to about 76°C.

In addition to improved stability, the heterodimer Fc region comprises a variant CH3 domain comprising amino acid mutations to promote heterodimer formation. It is understood that these amino acid mutations to promote heterodimer formation are as compared to homodimer formation. This heterodimer formation as compared to homodimer formation is referred jointly herein as "purity" or "specificity" or "heterodimer purity" or "heterodimer specificity". It is understood that the heterodimer purity refers to the percentage of desired heterodimer formed as compared to homodimer species formed in solution under standard cell culture conditions prior to selective purification of the heterodimer species. For instance, a heterodimer purity of 90% indicates that 90% of the dimer species in solution is the desired heterodimer. In one embodiment, the Fc variant heterodimers have a purity of greater than about 90%, or greater than about 91 %, or greater than about 92%, or greater than about 93%, or greater than about 94%, or greater than about 95%, or greater than about 96%, or greater than about 97%, or greater than about 98%, or greater than about 99%. In another embodiment, the Fc variant heterodimers have a purity of about 90%, or about 91 %, or about 92%, or about 93%, or about 94%, or about 95%, or about 96%, or about 97%, or about 98%, or about 99%, or about 100%.

In a specific embodiment, is provided an isolated heteromultimer comprising at least one heavy chain variable domain and a heterodimer Fc region, wherein the heterodimer Fc region comprises a variant CH3 domain comprising amino acid mutations to promote heterodimer formation with increased stability, wherein the variant CH3 domain has a melting temperature (Tm) of 70 °C or greater and the resulting heterodimer has a purity greater than 90%. In one aspect, the resulting heterodimer has a purity greater than 98% and the variant CH3 domain has a melting temperature of greater than about 70°C, or greater than about 71 °C, or greater than about 72°C, or greater than about 73°C, or greater than about 74°C, or greater than about 75°C, or greater than about 76°C, or greater than about 77°C, or greater than about 78°C, or greater than about 79°C, or greater than about 80°C or greater than about 81°C. In a further aspect, the variant CH3 domain has a melting temperature of 70°C or greater and the resulting Fc variant heterodimer has a purity greater than about 90%, or greater than about 91%, or greater than about 92%, or greater than about 93%, or greater than about 94%, or greater than about 95%, or greater than about 96%, or greater than about 97%, or greater than about 98%, or greater than about 99%.

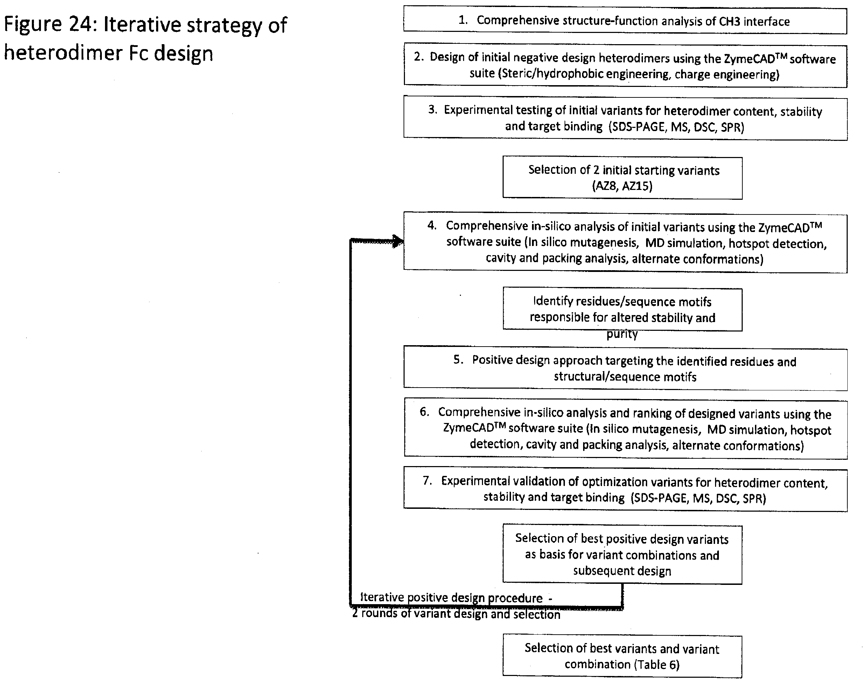

In order to design these heteromultimers comprising Fc variants with improved stability and purity we employed an iterative process of computational design and experimental screening to select the most successful combinations of positive and negative design strategies (See, Figure 24).

Specifically, in the initial design phase different negative design Fc variant heterodimers were made and tested for expression and stability as described in Examples 1-3. The initial design phase included Fc variant heterodimers AZ1-AZ16 (See, Table 1).From this initial set of negative design Fc variant heterodimers, which were expected to have low stability (e.g., a Tm of less than 71°C), the Fc variant heterodimers with greater than 90% purity and a melting temperature of about 68°C or greater were selected for further development. This included Fc variant heterodimers AZ6, AZ8 and AZ15. In the second design phase, those selected Fc variant heterodimers were further modifiedto drive both stability and purity using positive design strategies following a detailed computational and structural analysis. The selected Fc variant heterodimers (AZ6, AZ8, and AZ15) were each analyzed with computational methods and comprehensive structure function analysis to identify the structural reasons these Fc variants had a lower stability than the wild-type Fc homodimer, which is 81°C for IgG1. See, Table 4 for the list of Fc variant heterodimers and the Tm values.

In certain embodiments, the variant CH3 domain is selected from AZ1, or AZ2, or AZ3, or AZ4, or AZ5, or AZ6, or AZ7, or AZ8, or AZ9, or AZ10, or AZ11, or AZ12, or AZ13, or AZ14, or AZ15, or AZ16. In selected embodiments, the variant CH3 domain is AZ6, or AZ8 or AZ15.

The computational tools and structure-function analysis included, but were not limited to molecular dynamic analysis (MD), sidechain/backbone re-packing, Knowledge Base Potential (KBP), cavity and (hydrophobic) packing analysis (LJ, CCSD, SASA, dSASA(carbon/all-atom)), electrostatic-GB calculations, and coupling analysis. (See, Figure 24 for an overview of the computational strategy)

An aspect of the protein engineering approach relied on combining structural information of the Fc IgG protein derived from X-ray crystallography with computational modeling and simulation of the wild type and variant forms of the CH3 domain. This allowed us to gain novel structural and physico-chemical insights about the potential role of individual amino acids and their cooperative action. These structural and physico-chemical insights, obtained from multiple variant CH3 domains,along with the resulting empirical data pertaining to their stability and purity helped us develop anunderstanding for the relationship between purity and stability of the heterodimer. In order to execute our simulations we started by building complete and realistic models and refining the quality of the wild type Fc structure of an IgG1 antibody. Protein structures derived from X-ray crystallography are lacking in detail regarding certain features of the protein in aqueous medium under physiological condition and our refinement procedures addressed these limitations. These include building missing regions of the protein structure, often flexible portions of the protein such as loops and some residue side chains, evaluating and defining the protonation states of the neutral and charged residues and placement of potential functionally relevant water molecules associated with the protein.

Molecular dynamics (MD) algorithms are one tool we used, by simulating the protein structure, to evaluate the intrinsic dynamic nature of the Fc homodimer and the variant CH3 domainsin an aqueous environment. Molecular dynamics simulations track the dynamic trajectory of a molecule resulting from motions arising out of interactions and forces acting between all the atomic entities in the protein and its local environment, in this case the atoms constituting the Fc and its surrounding water molecules. Following molecular dynamics simulations, various aspects of the trajectories were analyzed to gain insight into the structural and dynamic characteristics of the Fc homodimer and variant Fc heterodimer, which we used to identify specific amino acid mutations to improve both purity and stability of the molecule.

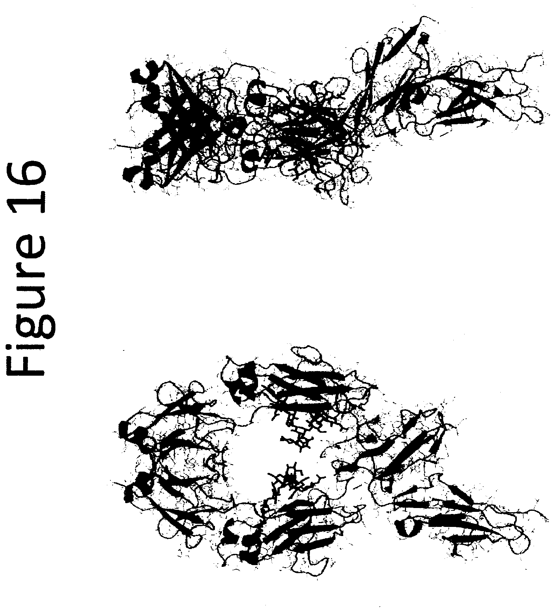

Therefore, the generated MD trajectories were studied using methods such as the principal component analysis to reveal the intrinsic low frequency modes of motion in the Fc structure. This provides insight into the potential conformational sub-states of the protein (See, Figure 32). While the critical protein-protein interactions between chain A and B in the Fc region occur at the interface of the CH3 domains, our simulations indicated that this interface acts as a hinge in a motion that involves the "opening" and "closing" of the N-terminal ends of the CH2 domains relative to each other. The CH2 domain interacts with FcgR's at this end as seen in figure 16. Thus, while not wishing to be bound by a theory, it appears that introduction of amino acid mutations at the CH3 interface impacts the magnitude and nature of the open/close motion at the N-terminal end of the Fc and therefore how the Fc interacts with the FcgR's.See, example 4 and Table 5.

The generated MD trajectories werealso studied to determine the mutability of specific amino acid residue positions in the Fc structure based on profiling their flexibility and analysis of their environment. This algorithm allowed us to identify residues that could affect protein structure and function, providing unique insight into residue characteristics and mutability for subsequent design phases of the variant CH3 domains. This analysis also enabled us to compare multiple simulations, and assess mutability based on outliers following profiling.

The generated MD trajectories werealso studied to determine correlated residue motions in the protein and the formation of networks of residues as a result of coupling between them. Finding dynamic correlations and networks of residues within the Fc structure is a critical step in understanding the protein as a dynamic entity and for developing insight into the effects of mutations at distal sites. See, e.g. Example 6

Thus, we studied in detail the impact of mutations on the local environment of the site of mutation. The formation of a well packed core at the CH3 interface between chain A and B is critical for the spontaneous pairing of the two chains in a stable Fc structure. Good packing is the result of strong structural complementarity between interacting molecular partners coupled with favorable interactions between the contacting groups. The favorable interactions result from either buried hydrophobic contacts well removed from solvent exposure and/or from the formation of complementary electrostatic contacts between hydrophilic polar groups. These hydrophobic and hydrophilic contacts have entropic and enthalpic contributions to the free energy of dimer formation at the CH3 interface. We employ a variety of algorithms to accurately model the packing at the CH3 interface between chain A and chain B and subsequently evaluate the thermodynamic properties of the interface by scoring a number of relevant physicochemical properties.

We employed a number of protein packing methods including flexible backbones to optimize and prepare model structures for the large number of variants we computationally screened. Following packing we evaluated a number of terms including contact density, clash score, hydrogen bonds, hydrophobicity and electrostatics. The use of the solvation models allowed us to more accurately address the effect of solvent environment and contrast the free energy differences following mutation of specific positions in the protein to alternate residue types. Contact density and clash score provide a measure of complementarity, a critical aspect of effective protein packing. These screening procedures are based on the application of knowledge-based potentials or coupling analysis schemes relying on pair-wise residue interaction energy and entropy computations.

This comprehensive in-silico analysis provided a detailed understanding of the differences of each Fc variant compared to wild-type with respect to interface hotspots, sites of asymmetry, cavities and poorly packed regions, structural dynamics of individual sites and sites of local unfolding. These combined results of the described computational analysis identified specific residues, sequence/structural motifs and cavities that were not optimized and in combination responsible for the lower stability (e.g., Tm of 68°C) and/or lower specificity of <90% purity. In the second design phase we used targeted positive design to specifically address these hypothesis by additional point-mutations and tested these by in-silico engineering using the above described methodology and analysis (See, Figure 24). TheFc variant heterodimers designed to improve stability and purity for each targeted design in phase two (Fc variant heterodimers AZ17-AZ101) were validated experimentally for expression and stability as described in Examples 1-4.

In certain embodiments, provided herein are isolated heteromultimers comprising a heterodimer Fc region, wherein the heterodimer Fc region comprises a variant CH3 domain comprising amino acid mutations to promote heterodimer formation with increased stability, wherein the variant CH3 domain is AZ17, or AZ18, or AZ19, or AZ20, or AZ21, or AZ22, or AZ23, or AZ24, or AZ25, or AZ26, or AZ27, or AZ28, or AZ29, or AZ30, or AZ21, or AZ32, or AZ33, or AZ34, or AZ35, or AZ36, or AZ37, or AZ38, or AZ39, or AZ40, or AZ41, or AZ42, or AZ43, or AZ44, or AZ45, or AZ46, or AZ47, or AZ48, or AZ49, or AZ50, or AZ51, or AZ52, or AZ53, or AZ54, or AZ55, or AZ56 or AZ57, or AZ58, or AZ59, or AZ60, or AZ61, or AZ62, or AZ63, or AZ64, or AZ65, or AZ66, orAZ67, or AZ68, or AZ69, or AZ70, or AZ71, or AZ72, orAZ73, or AZ74, or AZ75, or AZ76, or AZ77, or AZ78, or AZ79, or AZ80, or AZ81, or AZ82, or AZ83, or AZ84, or AZ85, or AZ86, or AZ87, or AZ88, or AZ89, or AZ90, or AZ91, or AZ92, or AZ93, or AZ94, or AZ95, or AZ96, or AZ97, or AZ98, or AZ99, or AZ100 or AZ101. In an exemplary embodiment, the variant CH3 domain is AZ17, or AZ18, or AZ19, or AZ20, or AZ21, or AZ22, or AZ23, or AZ24, or AZ25, or AZ26, or AZ27, or AZ28, or AZ29, or AZ30, or AZ21, or AZ32, or AZ33, or AZ34, or AZ38, or AZ42, or AZ43, or AZ 44, or AZ45, or AZ46, or AZ47, or AZ48, or AZ49, or AZ50, or AZ52, or AZ53, or AZ54, or AZ58, or AZ59, or AZ60, or AZ61, or AZ62, or AZ63, or AZ64, or AZ65, or AZ66, orAZ67, or AZ68, or AZ69, or AZ70, or AZ71, or AZ72, orAZ73, or AZ74, or AZ75, or AZ76, or AZ77, or AZ78, or AZ79, or AZ81, or AZ82, or AZ83, or AZ84, or AZ85, or AZ86, or AZ87, or AZ88, or AZ89, or AZ91, or AZ92, or AZ93, or AZ94, or AZ95, or AZ98, or AZ99, or AZ100 or AZ101. In a specific embodiment, the variant CH3 domain is AZ33 or AZ34. In another embodiment, the variant CH3 domain is AZ70 or AZ90.

In an exemplary embodiment, the heteromultimer comprises a first and a second polypeptide, wherein the first polypeptide comprises amino acid modifications L351Y, F405A, and Y407V and wherein the second polypeptide comprises amino acid modifications T366I, K392M and T394W. In another embodiment, a first polypeptide comprises amino acid modifications L351Y, S400E, F405A and Y407V and the second polypeptide comprises amino acid modifications T366I, N390R, K392M and T394W.

This iterative process of computational structure-function analysis, targeted engineering and experimental validation was used to design the remaining Fc variants listed in Table 1 in subsequent design phases and resulting in heteromultimers with a purity greater than 90% and an increased stability with a CH3 domain melting temperature greater than 70°C. In certain embodiments, the Fc variants comprise amino acid mutations selected from AZ1 to AZ 136. In further embodiments, the Fc variants comprise amino acid mutations selected from the Fc variants listed in Table 4.

From the first and second design phases two core scaffolds were identified, Scaffold 1 and Scaffold 2, wherein additional amino acid modificationswere introduced into these scaffolds to fine tune the purity and stability of the Fc variant heterodimers. See Example 5 for a detailed description of the development of Scaffold 1 including AZ8, AZ17-62and the variants listed in Table 6. See Example 6 for a detailed description of the development of Scaffold 2 including AZ15 and AZ63-101 and the variants listed in Table 7.

The core mutations of Scaffold 1 comprise L351Y_F405A_Y407V/T394W. Scaffold 1a comprises the amino acid mutations T366I_K392M_T394W/F405A_Y407V and Scaffold 1b comprises the amino acid mutations T366L_K392M_T394W/F405A_Y407V. See, Example 5.

In certain embodiments, the heteromultimer comprises a first and second polypeptide (also referred to herein as Chain A and Chain B) wherein the first polypeptide comprises amino acid modificationsL351Y, F405A and Y407V and the second polypeptide comprises amino acid modification T394W. In one aspect the heteromultimer further comprises point mutations at positions F405 and/or K392. These mutations at position K392 include, but are not limited to, K392V, K392M, K392R, K392L, K392F or K392E. These mutations at position F405 include, but are not limited to, F405I, F405M, F405S, F405S, F405V or F405W. In another aspect, the heteromultimer further comprises point mutations at positions T411 and/or S400. These mutations at position T411 include, but are not limited to, T411N, T411R, T411Q, T411K, T411D, T411E or T411W. These mutations at position S400 include, but are not limited to, S400E, S400D, S400R or S400K. In yet another embodiment, the heteromultimer comprises a first and second polypeptide wherein the first polypeptide comprises amino acid modifications L351Y, F405A and Y407V and the second polypeptide comprises amino acid modification T394W, wherein the first and/or second polypeptide comprises further amino acid modifications at positions T366 and/or L368. These mutations at position T366 include, but are not limited to, T366A, T366I, T366L, T366M, T366Y, T366S, T366C, T366V or T366W. In an exemplary embodiment, the amino acid mutation at position T366 is T366I. In another exemplary embodiment, the amino acid mutation at position T366 is T366L. The mutations at position L368 include, but are not limited to, L368D, L368R, L368T, L368M, L368V, L368F, L368S and L368A.

In certain embodiments, the heteromultimer comprises a first and second polypeptide (also referred to herein as Chain A and Chain B) wherein the first polypeptide comprises amino acid modifications L351Y, F405A and Y407V and the second polypeptide comprises amino acid modifications T366L and T394W. In another embodiment, the heteromultimer comprises a first and second polypeptide wherein the first polypeptide comprises amino acid modifications L351Y, F405A and Y407V and the second polypeptide comprises amino acid modifications T366I and T394W.

In certain other embodiments, the heteromultimer comprises a first and second polypeptide (also referred to herein as Chain A and Chain B) wherein the first polypeptide comprises amino acid modifications L351Y, F405A and Y407V and the second polypeptide comprises amino acid modifications T366L, K392M and T394W. In another embodiment, the heteromultimer comprises a first and second polypeptide wherein the first polypeptide comprises amino acid modifications L351Y, F405A and Y407V and the second polypeptide comprises amino acid modifications T366I, K392M and T394W.

In yet another embodiment, the heteromultimer comprises a first and second polypeptide (also referred to herein as Chain A and Chain B) wherein the first polypeptide comprises amino acid modifications F405A and Y407V and the second polypeptide comprises amino acid modifications T366L, K392M and T394W. In another embodiment, the heteromultimer comprises a first and second polypeptide wherein the first polypeptide comprises amino acid modifications F405A and Y407V and the second polypeptide comprises amino acid modifications T366I, K392M and T394W.

In certain embodiments, the heteromultimer comprises a first and second polypeptide (also referred to herein as Chain A and Chain B) wherein the first polypeptide comprises amino acid modifications F405A and Y407V and the second polypeptide comprises amino acid modifications T366L and T394W. In another embodiment, the heteromultimer comprises a first and second polypeptide wherein the first polypeptide comprises amino acid modifications F405A and Y407V and the second polypeptide comprises amino acid modifications T366I and T394W.

In an exemplary embodiment, described herein are isolated heteromultimers comprising a heterodimer Fc region, wherein the heterodimer Fc region comprises a heteromultimer comprising amino acid mutations to promote heterodimer formation with increased stability, wherein the heteromultimer has a melting temperature (Tm) of about 74°C or greater. In another embodiment, described herein are isolated heteromultimers comprising a heterodimer Fc region, wherein the heteromultimer comprises at least one heavy chain variable domain and a variant CH3 region comprising amino acid mutations to promote heterodimer formation with increased stability, wherein the variant CH3 domain has a melting temperature (Tm) of about 74°C or greater and the heterodimer has a purity of about 98% or greater.

In certain embodiments described herein, the isolated heteromultimer comprising a heterodimer Fc region, wherein the heterodimer Fc region comprises a variant CH3 domain comprising amino acid mutations to promote heterodimer formation with increased stability, wherein the variant CH3 domain has a melting temperature (Tm) greater than 70 °C and the variant CH3 domains are selected from Table 6.

The core mutations of Scaffold 2 comprise L351Y_Y407A / T366A_K409F. Scaffold 2a comprises the amino acid mutations L351Y_Y407A/T366V_K409F and Scaffold 2b comprises the amino acid mutations Y407A / T366A_K409F. See, Example 6.

In certain embodiments, the heteromultimer comprises a first and second polypeptide (also referred to herein as Chain A and Chain B) wherein the first polypeptide comprises amino acid modifications L351Y and Y407A and the second polypeptide comprises amino acid modificationsT366A and K409F. In one aspect the heteromultimer further comprises point mutations at positions T366, L351, and Y407. These mutations at position T366 include, but are not limited to, T366I, T366L, T366M, T366Y, T366S, T366C, T366V or T366W. In a specific embodiment, the mutation at position T366 is T366V. The mutations at position L351 include, but are not limited to, L351I, L351D, L351R or L351F. The mutations at position Y407 include, but are not limited to, Y407V or Y407S. See, CH3 variants AZ63-AZ70 in Table 1 and Table 4 and Example 6.

In an exemplary embodiment, the heteromultimer comprises a first and second polypeptide (also referred to herein as Chain A and Chain B) wherein the first polypeptide comprises amino acid modifications L351Y and Y407A and the second polypeptide comprises amino acid modification T366V and K409F.

In an exemplary embodiment, described herein are isolated heteromultimers comprising at least one single domain antigen-binding construct, and a heterodimer Fc region, wherein the heterodimer Fc region comprises a variant CH3 domain comprising amino acid mutations to promote heterodimer formation with increased stability, wherein the variant CH3 domain has a melting temperature (Tm) of about 75.5°C or greater; and wherein the heteromultimer is devoid of immunoglobulin light chains, immunoglobulin CH1 and optionally devoid of immunoglobulin CH2 regions. In another embodiment, described herein are isolated heteromultimers comprising at least one heavy chain variable region and a heterodimer Fc region, wherein the heterodimer Fc region comprises a variant CH3 domain comprising amino acid mutations to promote heterodimer formation with increased stability, wherein the variant CH3 domain has a melting temperature (Tm) of about 75°C or greater and the heterodimer has a purity of about 90% or greater.
In other certain embodiments, the heteromultimer comprises a first and second polypeptide (also referred to herein as Chain A and Chain B) wherein the first polypeptide comprises amino acid modifications L351Y and Y407A and the second polypeptide comprises amino acid modification T366A and K409F, wherein the variant CH3 domain comprises one or more amino acid modifications at positions T411, D399, S400, F405, N390, and/or K392. These mutations at position D399 include, but are not limited to, D399R, D399W, D399Y or D399K. The mutations at position T411 includes, but are not limited to, T411N, T411R, T411Q, T411K, T411D, T411E or T411W. The mutations at position S400 includes, but are not limited to, S400E, S400D, S400R, or S400K. The mutations at position F405 includes, but are not limited to, F405I, F405M, F405S, F405S, F405V or F405W. The mutations at position N390 include, but are not limited to, N390R, N390K or N390D. The mutations at position K392 include, but are not limited to, K392V, K392M, K392R, K392L, K392F or K392E. See, CH3 variants AZ71-101 in Table 1 and Table 4 and Example 6.

In an exemplary embodiment, the heteromultimer is a heterodimer that comprises a first and second polypeptide (also referred to herein as Chain A and Chain B) wherein the first polypeptide comprises amino acid modification Y407A and the second polypeptide comprises amino acid modification T366A and K409F. In one aspect, this heterodimer further comprises the amino acid modifications K392E, T411E, D399R and S400R. In a further embodiment, the heteromultimer comprises a first and second polypeptide wherein the first polypeptide comprises amino acid modification D399R, S400R and Y407A and the second polypeptide comprises amino acid modification T366A, K409F, K392E and T411E. In an exemplary embodiment, provided herein are isolated heteromultimers comprising at least one heavy chain variable region and a heterodimer Fc region, wherein the heterodimer Fc region comprises a variant CH3 domain comprising amino acid mutations to promote heterodimer formation with increased stability, wherein the variant CH3 domain has a melting temperature (Tm) of about 74°C or greater, and wherein the heteromultimer is devoid of immunoglobulin light chains and CH₁ domains. In another embodiment, provided herein are isolated heteromultimers comprising a heterodimer Fc region, wherein the heterodimer Fc region comprises a variant CH3 domain comprising amino acid mutations to promote heterodimer formation with increased stability, wherein the variant CH3 domain has a melting temperature (Tm) of about 74°C or greater and the heterodimer has a purity of about 95% or greater, and wherein the heteromultimer is devoid of immunoglobulin light chains and CH₁ domains.

In certain embodiments, described herein are isolated heteromultimers comprising a heterodimer Fc region, wherein the heterodimer Fc region comprises a variant CH3 domain comprising amino acid mutations to promote heterodimer formation with increased stability, wherein the variant CH3 domain has a melting temperature (Tm) greater than 70 °C and the variant CH3 domains are selected from Table 7, and wherein the heteromultimer is devoid of immunoglobulin light chains and CH₁ domains.

Furthermore, this new method of designing heteromultimers comprising at least one single domain antigen-binding construct and Fc variant heterodimers with improved stability and purity can be applied to other classes and isotypes of Fc regions. In certain embodiments, the Fc region is a human IgG Fc region. In further embodiments, the human IgG Fc region is a human IgGI, IgG2, IgG3, or IgG4 Fc region. In some embodiments the Fc regions is from an immunoglobulin selected from the group consisting of IgG, IgA, IgD, IgE and IgM. In some embodiments, the IgG is of subtype selected from the group consisting of IgG1, IgG2a, IgG2b, IgG3 and IgG4.

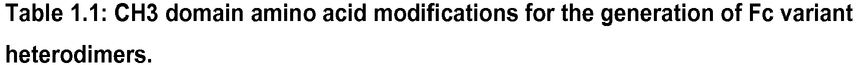

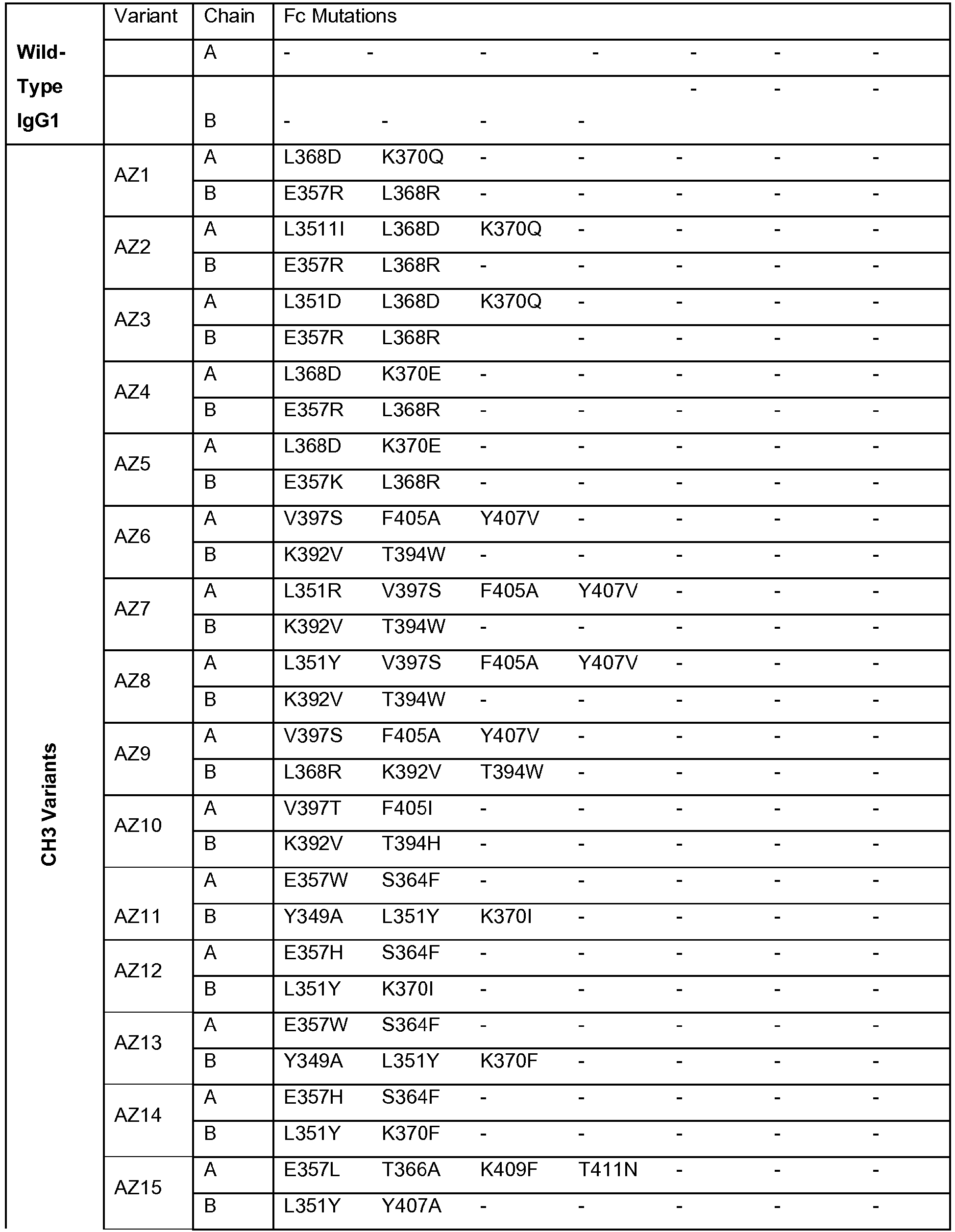

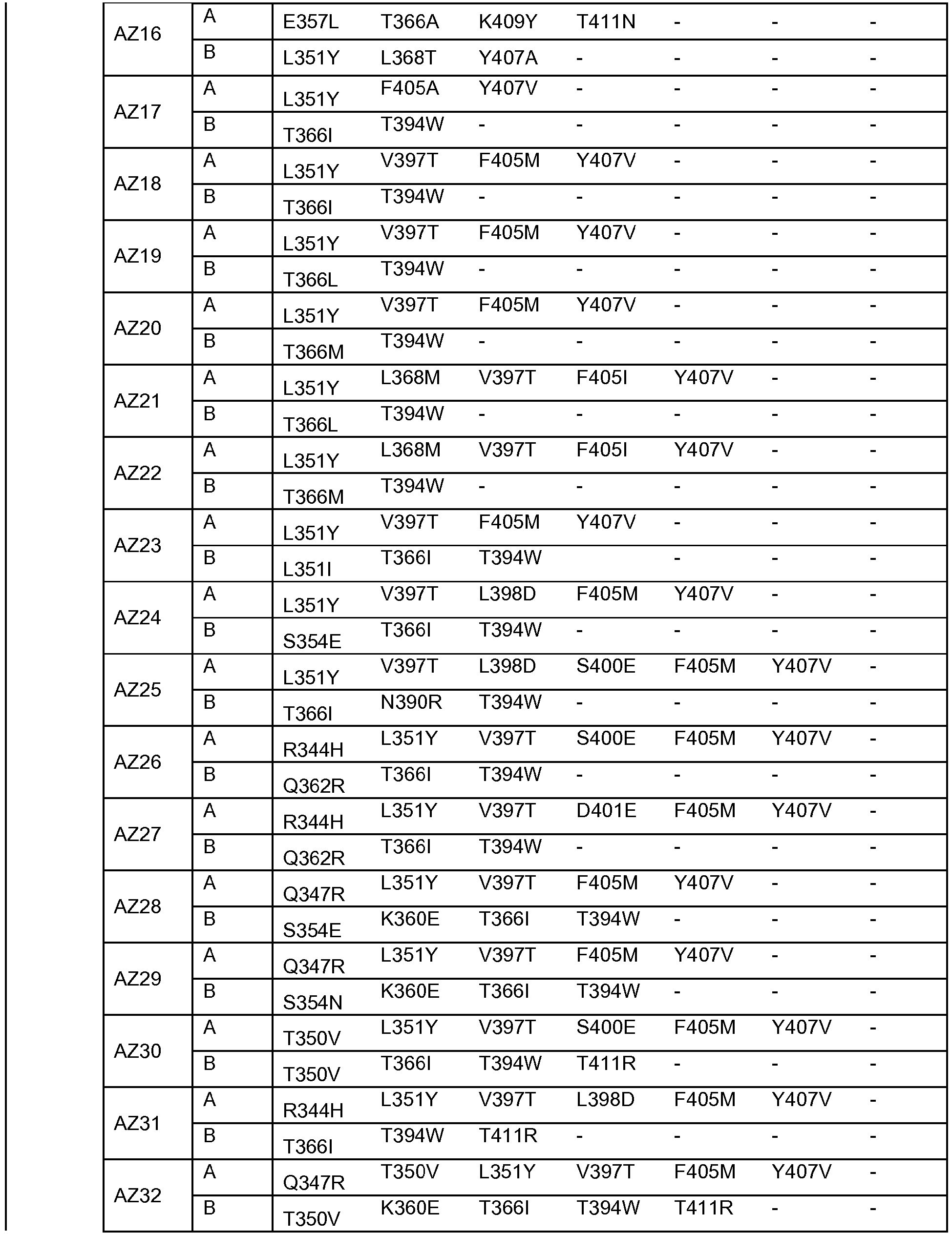

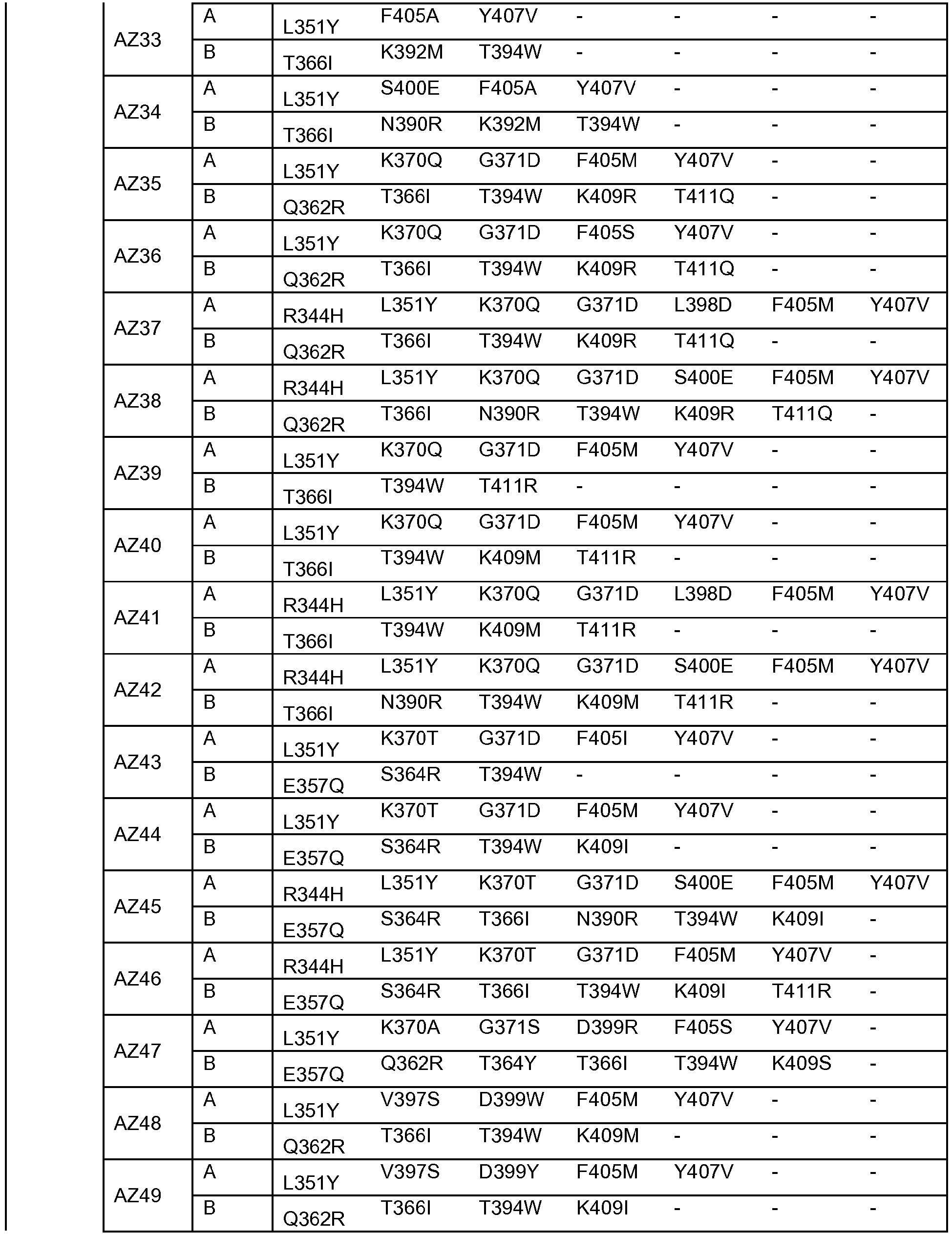

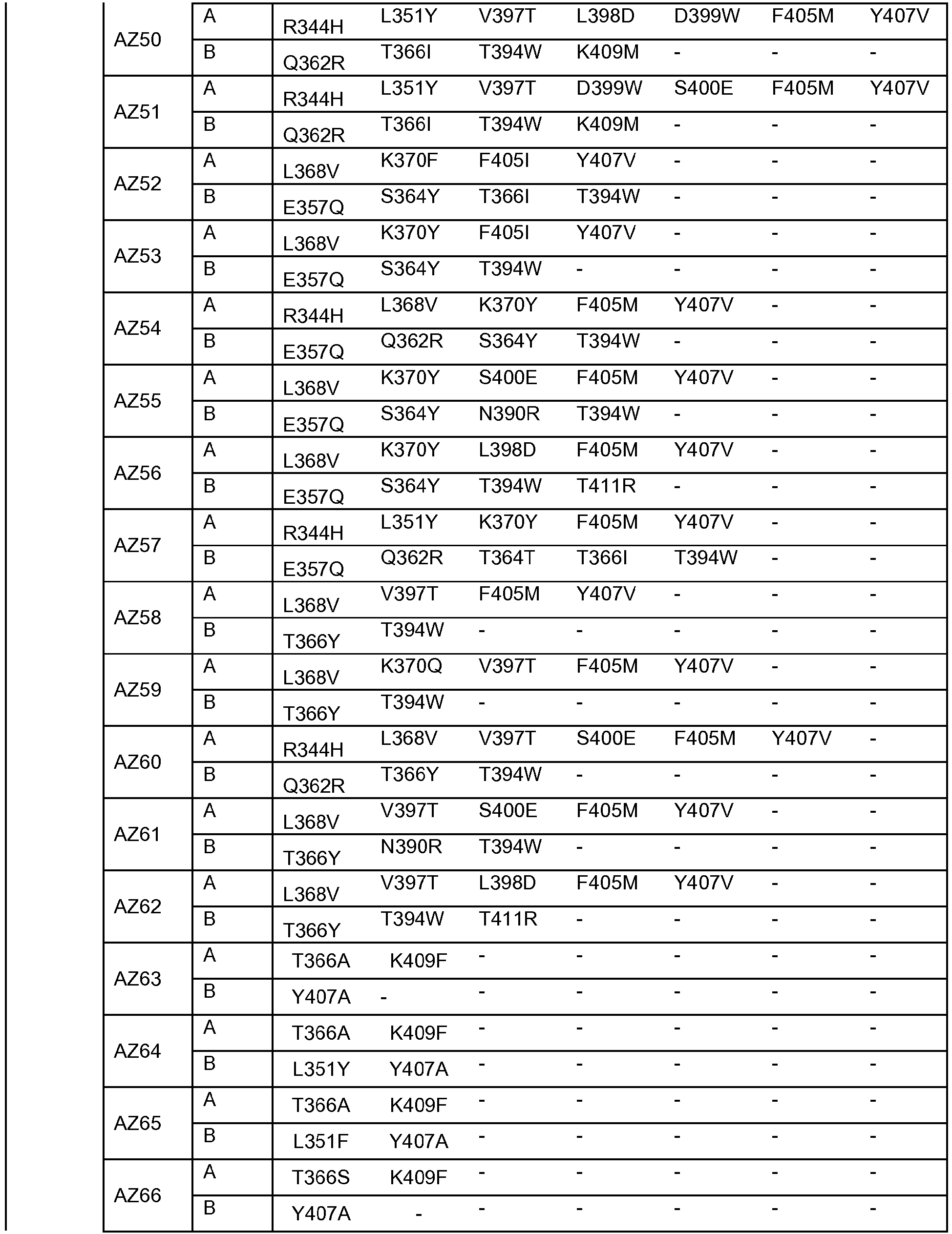

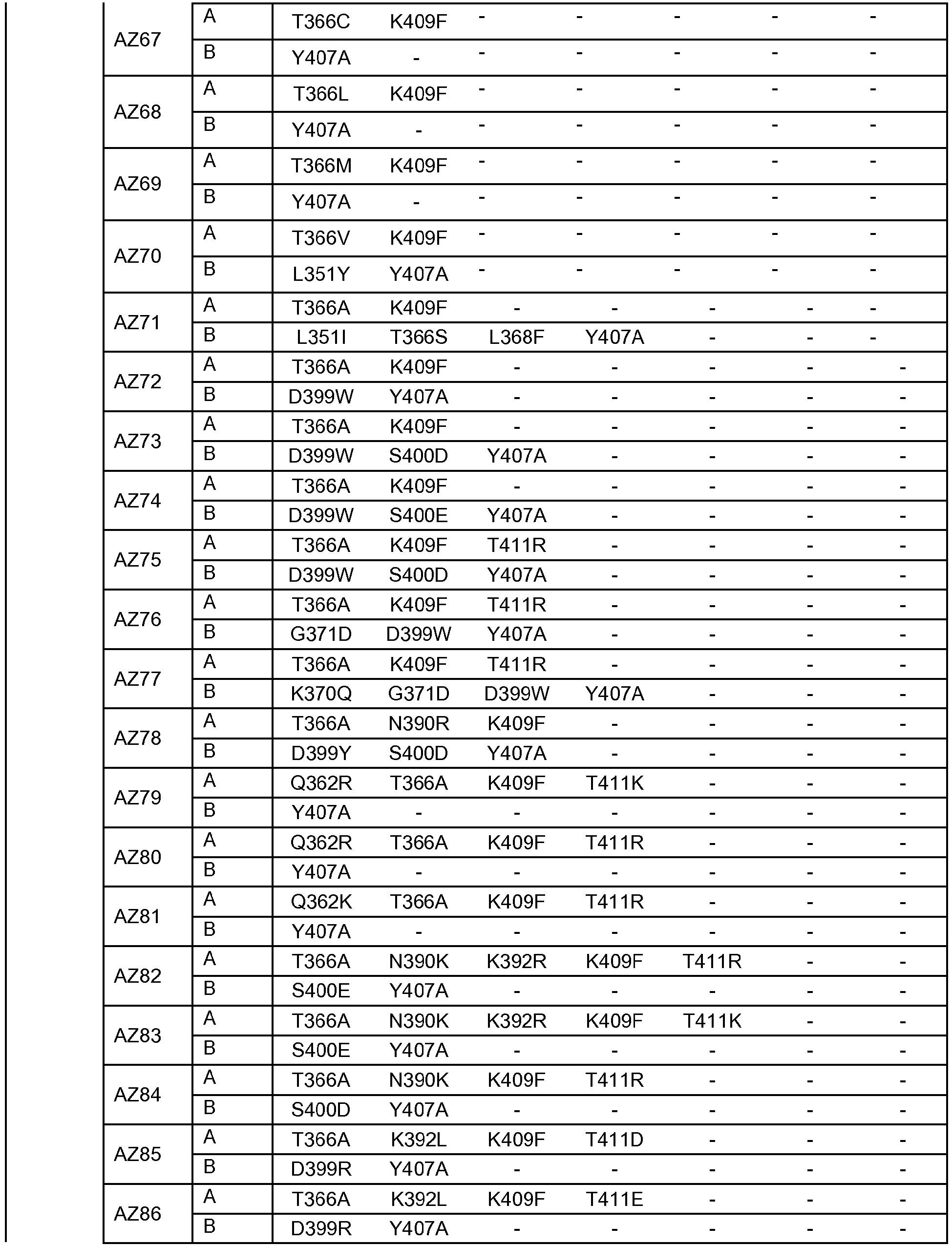

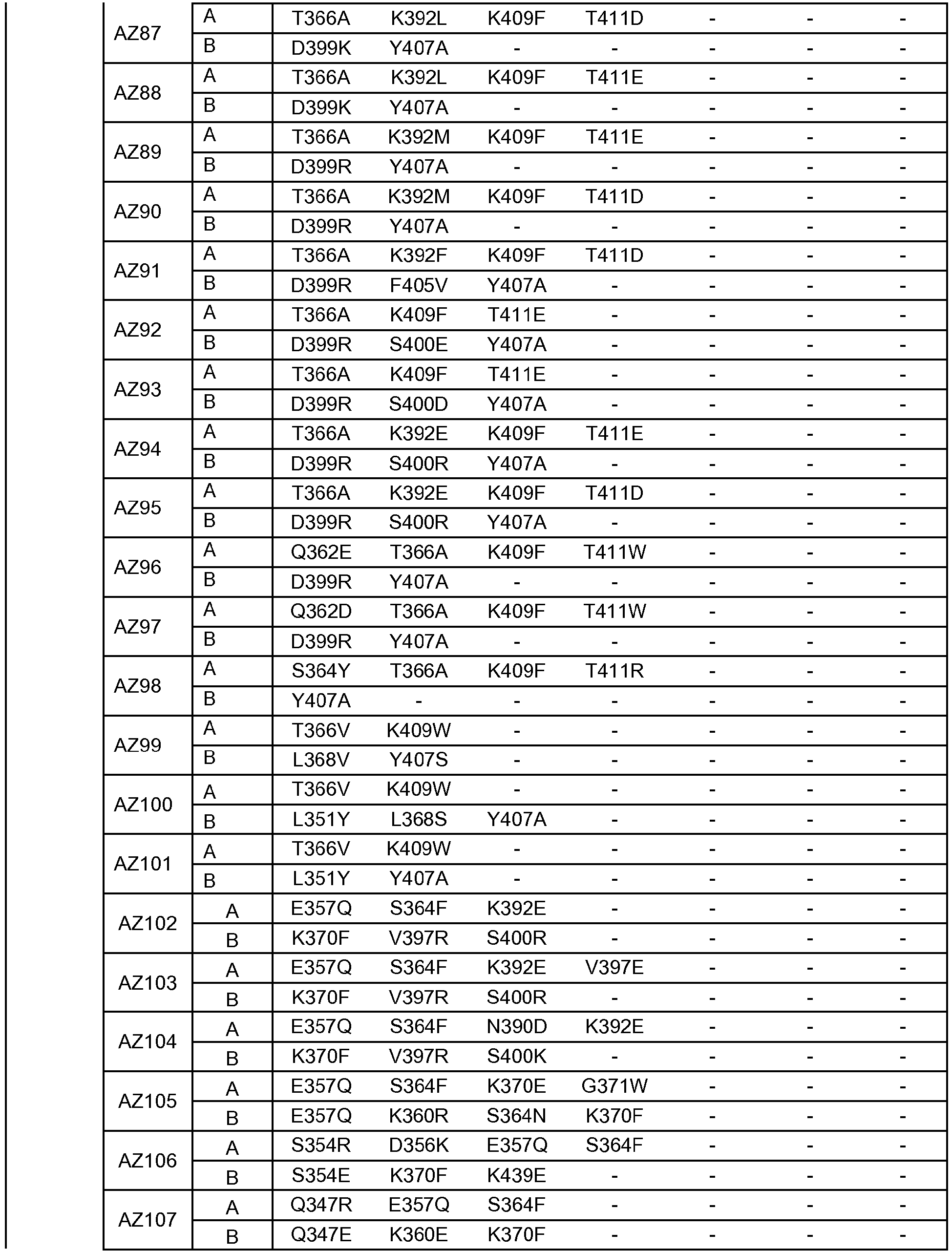

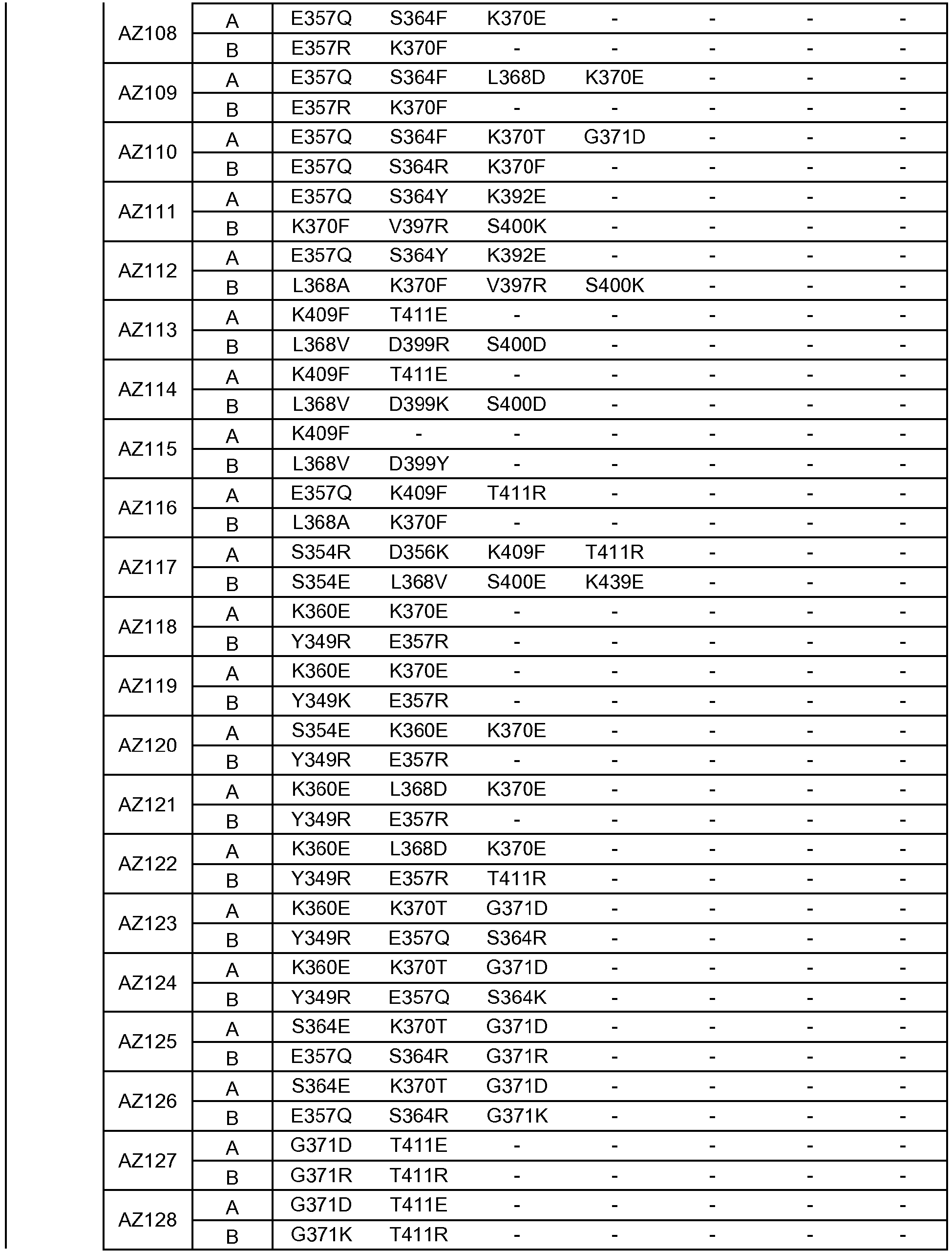

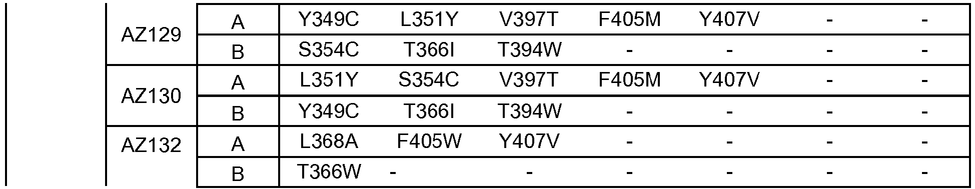

**Table 1.2: CH3 domain amino acid modifications for the generation of Fc variant heterodimers. The DSC melting temperature of the CH3 domain was estimated as shown in Figures 29A-29B and described in the Examples.**

| **Heterodimer Purity (%)** | **CH3 Tm (°C)** | **Mutations (Chain A)** | **Mutations (Chain B)** |
|---|---|---|---|
| >98 | 70.5 | F405A_Y407V | T366L_T394W |
| >98 | 73.5 | F405A_Y407V | T366L_K392M_T394W |
| >98 | 76.5 | T350V_F405A_Y407V | T350V_T366L_K392M_T394W |
| >98 | 78.7 | L351Y_F405A_Y407V | T366L_K392M_T394W |
| >98 | 79.5 | T350V_L351Y_F405A_Y407V | T350V_T366L_K392M_T394W |
| >98 | 81.8 | T350V_L351Y_F405A_Y407V | T350V_T366L_K392L_T394W |
| >98 | 81 | T350V_L351Y_S400R_F405A_Y407V | T350V_T366L_K392M_T394W |
| >98 | 79.5 | T350V_L351Y_S400E_F405A_Y407V | T350V_T366L_N390R_K392M_T394W |
| >98 | 77.5 | T350V_L351Y_S400E_F405V_Y407V | T350V_T366L_N390R_K392M_T394W |
| >98 | 77 | T350V_L351Y_S400E_F405T_Y407V | T350V_T366L_N390R_K392M_T394W |
| >98 | 78 | T350V_L351Y_S400E_F405S_Y407V | T350V_T366L_N390R_K392M_T394W |
| >98 | 76.5 | T350V_S400E_F405A_Y407V | T350V_T366L_N390R_K392M_T394W |
| >98 | 76.5 | T350V_L351Y_S400E_F405A_Y407V | T350V_L351Y_T366L_N390R_K392M_T394W |
| >98 | 81.5 | Q347R_T350V_L351Y_S400E_F405A_Y407V | T350V_K360E_T366L_N390R_K392M_T394W |
| >98 | 80.5 | T350V_L351Y_S400R_F405A_Y407V | T350V_T366L_N390D_K392M_T394W |
| >98 | 79.5 | T350V_L351Y_S400R_F405A_Y407V | T350V_T366L_N390E_K392M_T394W |
| >98 | 81.5 | T350V_L351Y_S400E_F405A_Y407V | T350V_T366L_N390R_K392L_T394W |
| >98 | 76.5 | T350V_L351Y_S400E_F405A_Y407V | T350V_T366L_N390R_K392F_T394W |
| >98 | 73.5 | Y349C_F405A_Y407V | S354C_T366L_T394W |
| >98 | 78 | Y349C_D399C_F405A_Y407V | S354C_T366L_K392C_T394W |
| >98 | 82 | Y349C_T350V_L351Y_S400E_F405A_Y407V | T350V_S354C_T366L_N390R_K392M_T394W |
| >98 | 82 | Y349C_T350V_S400E_F405A_Y407V | T350V_S354C_T366L_N390R_K392M_T394W |
| >98 | 76 | L351Y_F405A_Y407V | T366I_K392M_T394W |
| >98 | 81.5 | Y349C_T350V_F405A_Y407V | T350V_S354C_T366L_K392M_T394W |

**Table 1.3: CH3 domain amino acid modifications for the generation of Fc variant heterodimers. The Kd in the table above were determined as described in the Examples and Figure 35**

| **CD16a(F158) Kd [M]** | **CD32b(Y163) Kd[M]** | **Mutations(Chain-A)** | **Mutations(Chain-B)** |
|---|---|---|---|
| 4.4E-07 | 1.7E-06 | Herceptin WT | |
| 4.5E-07 | 9.0E-07 | T350V_L351Y_S400E_F405A_Y407V | T350V_T366L_N390R_K392M_T394W |
| 3.7E-07 | 7.0E-07 | T350V_L351Y_S400E_F405V_Y407V | T350V_T366L_N390R_K392M_T394W |
| 3.9E-07 | 6.7E-07 | T350V_L351Y_S400E_F405T_Y407V | T350V_T366L_N390R_K392M_T394W |
| 4.2E-07 | 8.3E-07 | T350V_L351Y_S400E_F405S_Y407V | T350V_T366L_N390R_K392M_T394W |
| 4.5E-07 | 1.0E-06 | T350V_S400E_F405A_Y407V | T350V_T366L_N390R_K392M_T394W |
| 3.7E-07 | 7.1E-07 | T350V_L351Y_S400E_F405A_Y407V | T350V_L351Y_T366L_N390R_K392M_T394W |
| 4.2E-07 | 9.2E-07 | Q347R_T350V_L351Y_S400E_F405A_Y407V | T350V_K360E_T366L_N390R_K392M_T394W |
| 4.3E-07 | 8.9E-07 | T350V_L351Y_S400R_F405A_Y407V | T350V_T366L_K392M_T394W |
| 4.3E-07 | 9.4E-07 | T350V_L351Y_S400R_F405A_Y407V | T350V_T366L_N390D_K392M_T394W |
| 4.2E-07 | 8.9E-07 | T350V_L351Y_S400R_F405A_Y407V | T350V_T366L_N390E_K392M_T394W |
| 4.4E-07 | 9.1E-07 | T350V_L351Y_S400E_F405A_Y407V | T350V_T366L_N390R_K392L_T394W |
| 3.6E-07 | 7.1E-07 | T350V_L351Y_S400E_F405A_Y407V | T350V_T366L_N390R_K392F_T394W |
| 4.6E-07 | 1.1E-06 | F405A_Y407V | T366L_K392M_T394W |
| 4.3E-07 | 1.0E-06 | T350V_F405A_Y407V | T350V_T366L_K392M_T394W |
| 4.8E-07 | 1.1E-06 | F405A_Y407V | T366L_T394W |
| 5.1E-07 | 1.2E-06 | D399C_F405A_Y407V | T366L_K392C_{_}T394W |
| 5.8E-07 | 1.2E-06 | Y349C_F405A_Y407V | S354C_T366L_T394W |
| 6.3E-07 | 1.3E-06 | Y349C_D399C_F405A_Y407V | S354C_T366L_K392C_T394W |
| 4.2E-07 | 9.5E-07 | Y349C_T350V_L351Y_S400E_F405A_Y407V | T350V_S354C_T366L_N390R_K392M_T394W |
| 4.4E-07 | 1.1E-06 | Y349C_T350V_S400E_F405A_Y407V | T350V_S354C_T366L_N390R_K392M_T394W |
| 4.2E-07 | 1.2E-06 | L351Y_F405A_Y407V | T366I_K392M_T394W |
| 4.2E-07 | 1.3E-06 | L351Y_F405A_Y407V | T366L_K392M_T394W |
| 4.6E-07 | 1.2E-06 | T350V_L351Y_F405A_Y407V | T350V_T366L_K392M_T394W |
| 4.6E-07 | 1.3E-06 | Y349C_T350V_F405A_Y407V | T350V_S354C_T366L_K392M_T394W |
| 4.2E-07 | 1.1E-06 | T350V_L351Y_S400E_F405A_Y407V | T350V_T366L_N390R_K392M_T394W |
| 3.6E-07 | 9.9E-07 | T350V_L351Y_F405A_Y407V | T350V_T366L_K392L_T394W |

### Fc region definition

The Fc region as defined herein comprises a CH3 domain or fragment thereof, and may additionally comprise one or more addition constant region domains, or fragments thereof, including hinge, CH1, or CH2. It will be understood that the numbering of the Fc amino acid residues is that of the EU index as in Kabat et al., 1991, NIH Publication 91-3242, National Technical Information Service, Springfield, Va. The "EU index as set forth in Kabat" refers to the EU index numbering of the human IgG1 Kabat antibody. For convenience, **Table B** provides the amino acids numbered according to the EU index as set forth in Kabat of the CH2 and CH3 domain from human IgG1.

**Table B**

| CH2 Domain | | | | | | | CH3 Domain | | | | | |
|---|---|---|---|---|---|---|---|---|---|---|---|---|
| EU No. | Amino Acid | EU No. | Amino Acid | EU No. | Amino Acid | | EU No. | Amino Acid | EU No. | Amino Acid | EU No. | Amino Acid |
| 231 | A | 271 | P | 311 | Q | | 341 | G | 381 | W | 421 | N |
| 232 | P | 272 | E | 312 | D | | 342 | Q | 382 | E | 422 | V |
| 233 | E | 273 | V | 313 | W | | 343 | P | 383 | S | 423 | F |
| 234 | L | 274 | K | 314 | L | | 344 | R | 384 | N | 424 | S |
| 235 | L | 275 | F | 315 | N | | 345 | E | 385 | G | 425 | C |
| 236 | G | 276 | N | 316 | G | | 346 | P | 386 | Q | 426 | S |
| 237 | G | 277 | W | 317 | K | | 347 | Q | 387 | P | 427 | V |
| 238 | P | 278 | Y | 318 | E | | 348 | V | 388 | E | 428 | M |
| 239 | S | 279 | V | 319 | Y | | 349 | Y | 389 | N | 429 | H |
| 240 | V | 280 | D | 320 | K | | 350 | T | 390 | N | 430 | E |
| 241 | F | 281 | G | 321 | C | | 351 | L | 391 | Y | 431 | A |
| 242 | L | 282 | V | 322 | K | | 352 | P | 392 | K | 432 | L |
| 243 | F | 283 | E | 323 | V | | 353 | P | 393 | T | 433 | H |
| 244 | P | 284 | V | 324 | S | | 354 | S | 394 | T | 434 | N |
| 245 | P | 285 | H | 325 | N | | 355 | R | 395 | P | 435 | H |
| 246 | K | 286 | N | 326 | K | | 356 | D | 396 | P | 436 | Y |
| 247 | P | 287 | A | 327 | A | | 357 | E | 397 | V | 437 | T |
| 248 | K | 288 | K | 328 | L | | 358 | L | 398 | L | 438 | Q |
| 249 | D | 289 | T | 329 | P | | 359 | T | 399 | D | 439 | K |
| 250 | T | 290 | K | 330 | A | | 360 | K | 400 | S | 440 | S |
| 251 | L | 291 | P | 331 | P | | 361 | N | 401 | D | 441 | L |
| 252 | M | 292 | R | 332 | I | | 362 | Q | 402 | G | 442 | S |
| 253 | I | 293 | E | 333 | E | | 363 | V | 403 | S | 443 | L |
| 254 | S | 294 | E | 334 | K | | 364 | S | 404 | F | 444 | S |
| 255 | R | 295 | Q | 335 | T | | 365 | L | 405 | F | 445 | P |
| 256 | T | 296 | Y | 336 | I | | 366 | T | 406 | L | 446 | G |
| 257 | P | 297 | N | 337 | S | | 367 | C | 407 | Y | 447 | K |
| 258 | E | 298 | S | 338 | K | | 368 | L | 408 | S | | |
| 259 | V | 299 | T | 339 | A | | 369 | V | 409 | K | | |
| 260 | T | 300 | Y | 340 | K | | 370 | K | 410 | L | | |
| 261 | C | 301 | R | | | | 371 | G | 411 | T | | |
| 262 | V | 302 | V | | | | 372 | F | 412 | V | | |
| 263 | V | 303 | V | | | | 373 | Y | 413 | D | | |
| 264 | V | 304 | S | | | | 374 | P | 414 | K | | |
| 265 | D | 305 | V | | | | 375 | S | 415 | S | | |
| 266 | V | 306 | L | | | | 376 | D | 416 | R | | |
| 267 | S | 307 | T | | | | 377 | I | 417 | W | | |
| 268 | H | 308 | V | | | | 378 | A | 418 | Q | | |
| 269 | E | 309 | L | | | | 379 | V | 419 | Q | | |
| 270 | D | 310 | H | | | | 380 | E | 420 | G | | |

In certain embodiments, the heteromultimers comprise an Fc region that comprises a CH2 domain. In some embodiments, the CH2 domain is a variant CH2 domain. In some embodiments, the variant CH2 domains comprise asymmetric amino acid substitutions in the first and/or second polypeptide chain. In some embodiments, the heteromultimer comprises asymmetric amino acid substitutions in the CH2 domain such that one chain of said heteromultimer selectively binds an Fc receptor.

### FcγR selectivity

In one aspect, this application describes a molecular design for achieving exquisite FcγR selectivity profiles via the design of an asymmetric scaffold built on a heterodimeric Fc. This scaffold allows for asymmetric mutations in the CH2 domain to achieve a variety of novel selectivity profiles. Further, the scaffold has inherent features for the engineering of multifunctional (bi, tri, tetra or penta functional) therapeutic molecules. In certain embodiments, the asymmetric scaffold is optimized for pH dependent binding properties to the neonatal Fc receptor (FcRn) to enable better recycling of the molecule and enhance its half life and related pharmacokinetic properties.

The asymmetric scaffold can be optimized for binding to the functionally relevant FcyRI receptor allotypes. FcγRI is a prominent marker on macrophages that are involved in chronic inflammatory disorders such as Rheumatoid Arthritis, Atopic Dermatitis, Psoriasis and a number of pulmonary diseases.

The asymmetric scaffold can be optimized for protein A binding. Protein A binding is often employed for separation and purification of antibody molecules. Mutations can be introduced in the asymmetric scaffold to avoid aggregation of the therapeutic during storage.

Therefore, it is specifically contemplated that the heteromultimers comprising heavy chain variable region and Fc variants of the invention may contain inter alia one or more additional amino acid residue substitutions, mutations and/or modifications which result in an antibody with preferred characteristics including but not limited to: increased serum half life, increase binding affinity, reduced immunogenicity, increased production, enhanced or reduced ADCC or CDC activity, altered glycosylation and/or disulfide bonds and modified binding specificity.

### In vivo half life

It is contemplated that the heteromultimers described herein may have other altered characteristics including increased in vivo half-lives (e.g., serum half-lives) in a mammal; in particular a human, increased stability in vivo (e.g., serum half-lives) and/or in vitro (e.g., shelf-life) and/or increased melting temperature (Tm), relative to a comparable molecule. In one embodiment, a heteromultimer of the invention has an in vivo half-life of greater then 15 days, greater than 20 days, greater than 25 days, greater than 30 days, greater than 35 days, greater than 40 days, greater than 45 days, greater than 2 months, greater than 3 months, greater than 4 months, or greater than 5 months. In another embodiment, a heteromultimer of the invention has an in vitro half-live (e.g, liquid or powder formulation) of greater then 15 days, greater than 30 days, greater than 2 months, greater than 3 months, greater than 6 months, or greater than 12 months, or greater than 24 months, or greater than 36 months, or greater than 60 months.

It will also be appreciated by one skilled in the art that the heteromultimers of the invention may have altered immunogenicity when administered to a subject. Accordingly, it is contemplated that the variant CH3 domain, which minimize the immunogenicity of the Fc variant are generally more desirable for therapeutic applications.

### Altered effector function

The heteromultimers of the present invention may be combined with other modifications, including but not limited to modifications that alter effector function. The invention encompasses combining a heteromultimer of the invention with other Fc modifications to provide additive, synergistic, or novel properties in antibodies or Fc fusion proteins. Such modifications may be in the hinge, or CH2, (or CH3 provided it does not negatively alter the stability and purity properties of the present variant CH3 domains) domains or a combination thereof. It is contemplated that the heteromultimers of the invention enhance the property of the modification with which they are combined.

### FcγR binding

As part of the characterization of the heteromultimers they were tested for their binding affinity to FcyRIIIA (CD16a) and FcyRIIB(CD32b) reported as a ratio in comparison to wild-type IgG1. (See, Example 4 and Table 5)ln this instance it was possible to evaluate the impact of the CH3 domain mutations on binding to these activating and inhibitory Fc receptors. In one embodiment, provided herein are isolated heteromultimers comprising a heterodimer Fc region, wherein the heterodimer Fc region comprises a variant CH3 domain comprising amino acid mutations to promote heterodimer formation with increased stability, wherein the variant CH3 domain has a melting temperature (Tm) greater than 70 °C, wherein the heterodimer binding to CD16a is about the same as compared to wild-type homodimer. In certain embodiments the heterodimer binding to CD16a is increased as compared to wild-type homodimer. In an alternative embodiment, the heterodimer binding to CD16a is reduced as compared to wild-type homodimer.

In certain embodiments, provided herein are isolated heteromultimers comprising at least one single domain antigen-binding construct and a heterodimer Fc region, wherein the heterodimer Fc region comprises a variant CH3 domain comprising amino acid mutations to promote heterodimer formation with increased stability, wherein the variant CH3 domain has a melting temperature (Tm) greater than 70 °C, wherein the heterodimer binding to CD32b is about the same as compared to wild-type homodimer, and wherein the heteromultimer is devoid of immunoglobulin light chains and immunoglobulin CH1 and optionally devoid of immunoglobulin CH2 regions. In certain embodiments the heterodimer binding to CD32b is increased as compared to wild-type homodimer. In an alternative embodiment, the heterodimer binding to CD32b is reduced as compared to wild-type homodimer.

One of skill in the art will understand that instead of reporting the K_{D} of binding CD16a and CD32b as a ratio Fc variant to wild-type homodimer, the K_{D} could be reported as a ratio of Fc variant binding to CD16a to Fc variant binding to CD32b (data not shown). This ratio would provide an indication of the variant CH3 domain mutation on ADCC, either unchanged, increased to decreased compared to wild-type, described below in more detail.

The affinities and binding properties of the heteromultimers of the invention for an FcγR are initially determined using in vitro assays (biochemical or immunological based assays) known in the art for determining Fc-FcyR interactions, i.e., specific binding of an Fc region to an FcγR including but not limited to ELISA assay, surface plasmon resonance assay, immunoprecipitation assays (See section entitled "Characterization and Functional Assays" infra) and other methods such as indirect binding assays, competitive inhibition assays, fluorescence resonance energy transfer (FRET), gel electrophoresis and chromatography (e.g., gel filtration). These and other methods may utilize a label on one or more of the components being examined and/or employ a variety of detection methods including but not limited to chromogenic, fluorescent, luminescent, or isotopic labels. A detailed description of binding affinities and kinetics can be found in Paul, W. E., ed., Fundamental Immunology, 4th Ed., Lippincott-Raven, Philadelphia (1999), which focuses on antibody-immunogen interactions.

It is contemplated that the binding properties of the molecules of the invention are also characterized by in vitro functional assays for determining one or more FcγR mediator effector cell functions (See section entitled "Characterization and Functional Assays" infra). In certain embodiments, the molecules of the invention have similar binding properties in *in vivo* models (such as those described and disclosed herein) as those in in vitro based assays. However, the present invention does not exclude molecules of the invention that do not exhibit the desired phenotype in *in vitro* based assays but do exhibit the desired phenotype *in vivo.*

The invention encompasses heteromutlimers comprising Fc variants that bind FcγRIIIA (CD16a) with increased affinity, relative to a comparable molecule. In a specific embodiment, the Fc variants of the invention bind FcγRIIIA with increased affinity and bind FcyRIIB (CD32b) with a binding affinity that is either unchanged or reduced, relative to a comparable molecule. In yet another embodiment, the Fc variants of the invention have a ratio of FcγRIIIA/FcγRIIB equilibrium dissociation constants (K_{D}) that is decreased relative to a comparable molecule.

Also encompassed by the present invention are heteromultimers comprising Fc variants that bind FcγRIIIA (CD16a) with decreased affinity, relative to a comparable molecule. In a specific embodiment, the Fc variants of the invention bind FcγRIIIA with decreased affinity, relative to a comparable molecule and bind FcγRIIB with a binding affinity that is unchanged or increased, relative to a comparable molecule.

In one embodiment, the heteromultimers comprise Fc variants bind with increased affinity to FcγRIIIA. In a specific embodiment, said Fc variants have affinity for FcγRIIIA that is at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold greater than that of a comparable molecule. In other embodiments, the Fc variants have an affinity for FcγRIIIA that is increased by at least 10%, or at least 20%, or at least 30%, or at least 40%, or at least 50%, or at least 60%, or at least 70%, or at least S0%, or at least 90%, or at least 100%, or at least 150%, or at least 200%, relative to a comparable molecule.

In another embodiment, the Fc variant has an equilibrium dissociation constant (K_{D}) for an Fc ligand (e.g., FcyR, C1q) that is decreased between about 2 fold and 10 fold, or between about 5 fold and 50 fold, or between about 25 fold and 250 fold, or between about 100 fold and 500 fold, or between about 250 fold and 1000 fold relative to a comparable molecule.

In a another embodiment, said Fc variants have an equilibrium dissociation constant (K_{D}) for FcγRIIIA that is reduced by at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold, or at least 400 fold, or at least 600 fold, relative to a comparable molecule. In another embodiment, the Fc variants have an equilibrium dissociation constant (K_{D}) for FcγRIIIA that is reduced by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule.

In one embodiment, the Fc variant binds to FcγRIIB with an affinity that is unchanged or reduced. In a specific embodiment, said Fc variants have affinity for FcγRIIB that is unchanged or reduced by at least 1 fold, or by at least 3 fold, or by at least 5 fold, or by at least 10 fold, or by at least 20 fold, or by at least 50 fold, or by at least 100 fold, relative to a comparable molecule. In other embodiments, the Fc variants have an affinity for FcyRIIB that is unchanged or reduced by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule.

In another embodiment, the Fc variants have an equilibrium dissociation constant (K_{D}) for FcγRIIB that is unchanged or increased by at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least S0 fold, or at least 90 fold, or at least 100 fold, or at least 200 fold relative to a comparable molecule. In another specific embodiment, the Fc variants have an equilibrium dissociation constant (K_{D}) for FcγRIIB that is unchanged or increased by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule.

In still another embodiment, the Fc variants bind FcγRIIIA with increased affinity, relative to a comparable molecule and bind FcyRIIB with an affinity that is unchanged or reduced, relative to a comparable molecule. In a specific embodiment, the Fc variants have affinity for FcγRIIIA that is increased by at least 1 fold, or by at least 3 fold, or by at least 5 fold, or by at least 10 fold, or by at least 20 fold, or by at least 50 fold, or by at least 100 fold, relative to a comparable molecule. In another specific embodiment, the Fc variants have affinity for FcγRIIB that is either unchanged or is reduced by at least 2 fold, or at least 3 fold, or at least 5 fold, or at least 7 fold, or a least 10 fold, or at least 20 fold, or at least 50 fold, or at least 100 fold, relative to a comparable molecule. In other embodiments, the Fc variants have an affinity for FcγRIIIA that is increased by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule and the Fc variants have an affinity for FcγRIIB that is either unchanged or is increased by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule.

In yet another embodiment, the Fc variants have a ratio of FcγRIIIA/FcγRIIB equilibrium dissociation constants (K_{D}) that is decreased relative to a comparable molecule. In a specific embodiment, the Fc variants have a ratio of FcγRIIIA/FcγRIIB equilibrium dissociation constants (K_{D}) that is decreased by at least 1 fold, or by at least 3 fold, or by at least 5 fold, or by at least 10 fold, or by at least 20 fold, or by at least 50 fold, or by at least 100 fold, relative to a comparable molecule. In another specific embodiment, the Fc variants have a ratio of FcγRIIIA/FcγRIIB equilibrium dissociation constants (K_{D}) that is decreased by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule.

In another embodiment, the Fc variants bind FcγRIIIA with a decreased affinity, relative to a comparable molecule. In a specific embodiment, said Fc variants have affinity for FcγRIIIA that is reduced by at least 1 fold, or by at least 3 fold, or by at least 5 fold, or by at least 10 fold, or by at least 20 fold, or by at least 50 fold, or by at least 100 fold, relative to a comparable molecule. In other embodiments, the Fc variants have an affinity for FcγRIIIA that is decreased by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule.

In still another embodiment, the Fc variants bind FcγRIIIA with decreased affinity and bind FcyRIIB with an affinity that is either unchanged or increased, relative to a comparable molecule. In a specific embodiment, the Fc variants have affinity for FcγRIIIA that is reduced by at least 1 fold, or by at least 3 fold, or by at least 5 fold, or by at least 10 fold, or by at least 20 fold, or by at least 50 fold, or by at least 100 fold relative to a comparable molecule. In another specific embodiment, the Fc variants have affinity for FcγRIIB that is at least 2 fold, or at least 3 fold, or at least 5 fold, or at least 7 fold, or a least 10 fold, or at least 20 fold, or at least 50 fold, or at least 100 fold, greater than that of a comparable molecule. In other embodiments, the Fc variants have an affinity for FcγRIIIA that is decreased by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule and the Fc variants have an affinity for FcγRIIB that is increased by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule.

In still another embodiment, the Fc variants have an equilibrium dissociation constant (K_{D}) for FcγRIIIA that are increased by at least 1 fold, or by at least 3 fold, or by at least 5 fold or by at least 10 or by at least 20 fold, or by at least 50 fold when compared to that of a comparable molecule. In a specific embodiment, said Fc variants have equilibrium dissociation constant (K_{D}) for FcyRIIB that are decreased at least 2 fold, or at least 3 fold, or at least 5 fold, or at least 7 fold, or a least 10 fold, or at least 20 fold, or at least 50 fold or at least 100 fold, relative to a comparable molecule.

### CH2 variations for fcyR selectivity

The Fc-FcγR protein-protein interaction in this complex indicates that the two chains in the heteromultimer interact with two distinct sites on the FcγR molecule. Although there is symmetry in the two heavy chains in the natural Fc molecules, the local FcγR environment around residues on one chain is different from the FcγR residues surrounding the same residue position on the opposite Fc chain. The two symmetry related positions interact with different selection of FcγR residues.

Given the asymmetry in the association of Fc to FcγR, concurrent mutations in chain A and B of the Fc molecule do not impact the interactions with FcγR in a symmetric manner. When introducing mutations to optimize interactions on one chain of the Fc with its local FcγR environment, in a homodimeric Fc structure, the corresponding mutation in the second chain may be favorable, unfavorable or non-contributing to the required FcγR binding and selectivity profile.

Using a structure and computation guided approach, asymmetric mutations are engineered in the two chains of the Fc to overcome these limitations of traditional Fc engineering strategies, which introduce the same mutations on both the chains of Fc. One can achieve better binding selectivity between the receptors if the two chains of Fc are optimized independently for enhanced binding to their corresponding face of the receptor molecule.

For instance, mutations at a particular position on one chain of the Fc can be designed to enhance selectivity to a particular residue, a positive design effort, while the same residue position can be mutated to unfavorably interact with its local environment in an alternate Fcγ receptor type, a negative design effort, hence achieving better selectivity between the two receptors. In certain embodiments, is provided a method for designing asymmetric amino acid modifications in the CH2 domain that selectively bind one Fc gamma receptor as compared to a different Fc gamma receptor (e.g., selectively bind FcgRllla instead of FcgRllb). In other certain embodiments, is provided a method for the design of asymmetric amino acid modifications in the CH2 domain of a variant Fc heterodimercomprising amino acid modifications in the CH3 domain to promote heterodimer formation. In another embodiment, is provided a method to design selectivity for the different Fc gamma receptors based on a variant Fc heterodimer comprising asymmetric amino acid modifications in the CH2 domain. In yet another embodiment, is provided a method for designingasymmetric amino acid modifications that bias binding of the Fc gamma receptors to one face of the Fc molecule. In other certain embodiments, is provided a method for designing polarity drivers that bias the Fcgamma receptors to interact with only one face of the variant Fc heterodimer comprising asymmetric amino acid modifications in theCH2 domain.

The asymmetric design of mutations in the CH2 domain can be tailored to recognize the FcγR on one face of the Fc molecule. This constitutes the productive face of the asymmetric Fc scaffold while the opposite face presents wild type like interaction propensity without the designed selectivity profile and can be considered a non-productive face. A negative design strategy can be employed to introduce mutations on the non-productive face to block FcγR interactions to this side of the asymmetric Fc scaffold, there by forcing the desired interaction tendencies to the Fcγ receptors.

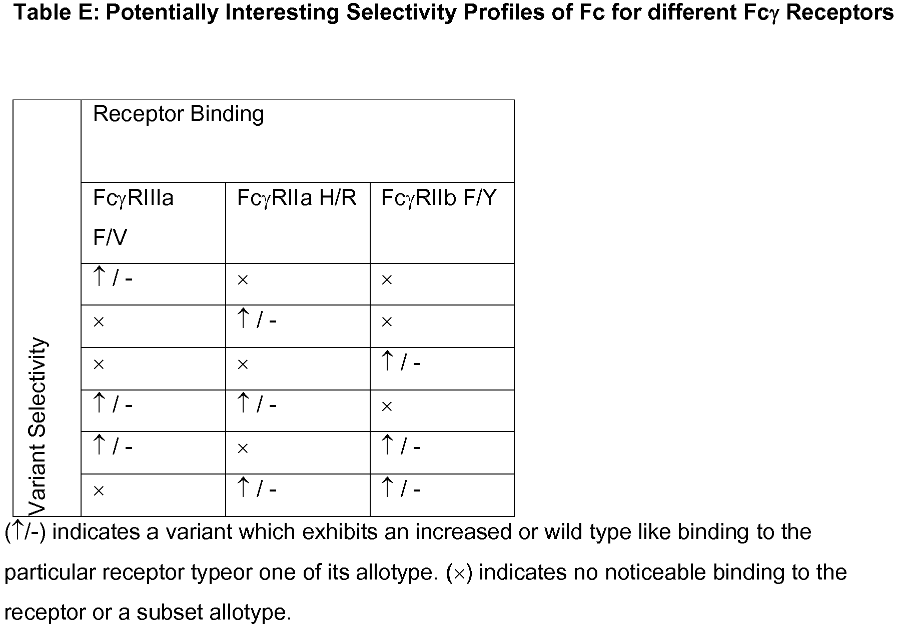

Certain embodiments provided herein relate to fusion polypeptides comprising a binding domain fused to an Fc region, wherein the Fc region comprising a variant CH3 domain, comprising amino acid mutations to promote heterodimer formation with increased stability, wherein the variant CH3 domain has a melting temperature (Tm) greater than 70 °C. It is specifically contemplated that molecules comprising a heterodimer comprising a variant CH3 domain may be generated by methods well known to one skilled in the art. Briefly, such methods include but are not limited to, combining a variable region or binding domain with the desired specificity (e.g., a variable region isolated from a phage display or expression library or derived from a human or non-human antibody or a binding domain of a receptor) with a variant Fc heterodimers. Alternatively, one skilled in the art may generate a variant Fc heterodimer by modifying the CH3 domain in the Fc region of a molecule comprising an Fc region (e.g., an antibody).

"Antibody-dependent cell-mediated cytotoxicity" or "ADCC" refers to a form of cytotoxicity in which secreted antibody bound onto Fc receptors (FcRs) present on certain cytotoxic cells (e.g., Natural Killer (NK) cells, neutrophils, and macrophages) enables these cytotoxic effector cells to bind specifically to an antigen-healing target cell and subsequently kill the target cell with cytotoxins. Specific high-affinity IgG antibodies directed to the surface of target cells "arm" the cytotoxic cells and are absolutely required for such killing. Lysis of the target cell is extracellular, requires direct cell-to-cell contact, and does not involve complement.

The ability of any particular antibody to mediate lysis of the target cell by ADCC can be assayed. To assess ADCC activity an antibody of interest is added to target cells in combination with immune effector cells, which may be activated by the antigen antibody complexes resulting in cytolysis of the target cell. Cytolysis is generally detected by the release of label (e.g. radioactive substrates, fluorescent dyes or natural intracellular proteins) from the lysed cells. Useful effector cells for such assays include peripheral blood mononuclear cells (PBMC) and Natural Killer (NK) cells. Specific examples of in vitro ADCC assays are described in Wisecarver et al., 1985, 79:277; Bruggemann et al., 1987, J Exp Med 166:1351; Wilkinson et al., 2001, J Immunol Methods 258:183; Patel et al., 1995 J Immunol Methods 184:29 and herein (see section entitled "Characterization and Functional Assays" infra). Alternatively, or additionally, ADCC activity of the antibody of interest may be assessed in vivo, e.g., in an animal model such as that disclosed in Clynes et al., 1998, PNAS USA 95:652.

The present invention further provides heteromultimers comprising Fc variants with enhanced CDC function. In one embodiment, the Fc variants have increased CDC activity. In one embodiment, the Fc variants have CDC activity that is at least 2 fold, or at least 3 fold, or at least 5 fold, or at least 10 fold, or at least 50 fold, or at least 100 fold greater than that of a comparable molecule. In another embodiment, the Fc variants bind C1q with an affinity that is at least 2 fold, or at least 3 fold, or at least 5 fold, or at least 7 fold, or a least 10 fold, or at least 20 fold, or at least 50 fold, or at least 100 fold, greater than that of a comparable molecule. In yet another embodiment, the Fc variants have CDC activity that is increased by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule. In a specific embodiment, the Fc variants of the invention bind C1q with increased affinity; have enhanced CDC activity and specifically bind to at least one antigen.

The present invention also provides heteromultimers comprising Fc variants with reduced CDC function. In one embodiment, the Fc variants have reduced CDC activity. In one embodiment, the Fc variants have CDC activity that is at least 2 fold, or at least 3 fold, or at least 5 fold or at least 10 fold or at least 50 fold or at least 100 fold less than that of a comparable molecule. In another embodiment, an Fc variant binds C1q with an affinity that is reduced by at least 1 fold, or by at least 3 fold, or by at least 5 fold, or by at least 10 fold, or by at least 20 fold, or by at least 50 fold, or by at least 100 fold, relative to a comparable molecule. In another embodiment, the Fc variants have CDC activity that is decreased by at least 10%, or at least 20%, or at least 30%, or at least 40%, or at least 50%, or at least 60%, or at least 70%, or at least 80%, or at least 90%, or at least 100%, or at least 150%, or at least 200%, relative to a comparable molecule. In a specific embodiment, Fc variants bind to C1q with decreased affinity have reduced CDC activity and specifically bind to at least one antigen.

In some embodiments, the Fc variants comprise one or more engineered glycoforms, i.e., a carbohydrate composition that is covalently attached to a molecule comprising an Fc region. Engineered glycoforms may be useful for a variety of purposes, including but not limited to enhancing or reducing effector function. Engineered glycoforms may be generated by any method known to one skilled in the art, for example by using engineered or variant expression strains, by co-expression with one or more enzymes, for example β(1,4)-N-acetylglucosaminyltransferase III (GnTI11), by expressing a molecule comprising an Fc region in various organisms or cell lines from various organisms, or by modifying carbohydrate(s) after the molecule comprising Fc region has been expressed. Methods for generating engineered glycoforms are known in the art, and include but are not limited to those described in Umana et al, 1999, Nat. Biotechnol 17:176-180; Davies et al., 20017 Biotechnol Bioeng 74:288-294; Shields et al, 2002, J Biol Chem 277:26733-26740; Shinkawa et al., 2003, J Biol Chem 278:3466-3473) U.S. Pat. No. 6,602,684; U.S. Ser. No. 10/277,370; U.S. Ser. No. 10/113,929; PCT WO 00/61739A1; PCT WO 01/292246A1; PCT WO 02/311140A1; PCT WO 02/30954A1; Potillegent™ technology (Biowa, Inc. Princeton, N.J.); GlycoMAb™ glycosylation engineering technology (GLYCART biotechnology AG, Zurich, Switzerland). See, e.g., WO 00061739; EA01229125; US 20030115614; Okazaki et al., 2004, JMB, 336: 1239-49.

It is contemplated that Fc variants include antibodies comprising a variable region and aheterodimer Fc region, wherein the heterodimer Fc region comprises a variant CH3 domain comprising amino acid mutations to promote heterodimer formation with increased stability, wherein the variant CH3 domain has a melting temperature (Tm) greater than 70 °C. The Fc variants which are antibodies may be produced "de novo" by combing a variable domain, of fragment thereof, that specifically binds at least one antigen with a heterodimer Fc region comprising a variant CH3 domain. Alternatively, heterodimer Fc variants may be produced by modifying the CH3 domain of an Fc region containing antibody that binds an antigen.

Heteromultimers of the invention may be monospecific, bi-specific, trispecific or have greater multispecificity. Multispecific antibodies may specifically bind to different epitopes of desired target molecule or may specifically bind to both the target molecule as well as a heterologous epitope, such as a heterologous polypeptide or solid support material. See, e.g., International Publication Nos. WO 94/04690; WO 93/17715; WO 92/08802; WO 91/00360; and WO 92/05793; Tutt, et al., 1991, J. Immunol. 147:60-69; U.S. Pat.Nos.; 4,474,893; 4,714,681; 4,925,648; 5,573,920 and 5,601,819 and Kostelny et al., 1992, J. Immunol.148: 1547).

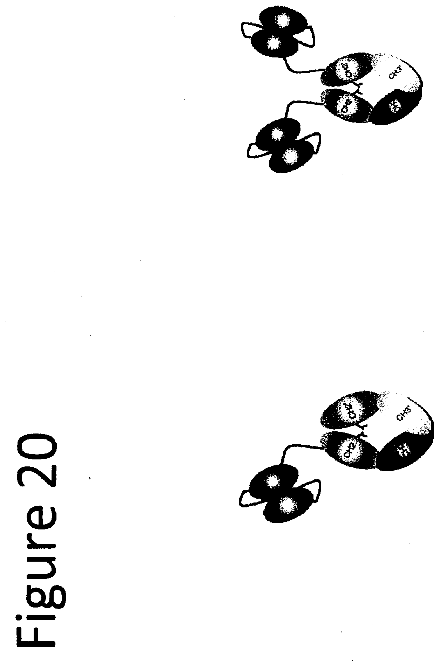

Various embodiments of multifunctional targeting molecules can be designed on the basis of this asymmetric scaffold as shown in Figure 20.

### Bi-specific or multi-specific antibodies

Multispecific heteromultimers are based on antibodies that have binding specificities for at least two different antigens. While such molecules normally will only bind two antigens (i.e. bi-specific antibodies, BsAbs), antibodies with additional specificities such as trispecific antibodies are encompassed by the instant invention. Examples of BsAbs include without limitation those with one arm directed against a tumor cell antigen and the other arm directed against a cytotoxic molecule, or both arms are directed again two different tumor cell antigens, or both arms are directed against two different soluable ligands, or one arm is directed against a soluable ligand and the other arm is directed against a cell surface receptor, or both arms are directed against two different cell surface receptors. Methods for making bi-specific antibodies are known in the art.

According to a different approach, antibody variable domains with the desired binding specificities (antibody-antigen combining sites) are fused to immunoglobulin constant domain sequences. The fusion may be with an immunoglobulin heavy chain constant domain, comprising at least part of the hinge, CH2, and CH3 regions. DNAs encoding the immunoglobulin heavy chain fusions, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. See, Example 1 and Table 2. It is, however, possible to insert the coding sequences for two or all three polypeptide chains in one expression vector when, the expression of at least two polypeptide chains in equal ratios results in high yields or when the ratios are of no particular significance.

Bi-specific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Pat. No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Pat. No. 4,676,980, along with a number of cross-linking techniques.

Antibodies with more than two valencies incorporating variant CH3 domains and resulting Fc heterodimers of the invention are contemplated. For example, trispecific antibodies can be prepared. See, e.g., Tutt et al. J. Immunol. 147: 60 (1991).

### Serum half-life

Antibodies of the present invention also encompass those that have half-lives (e.g., serum half-lives) in a mammal, (e.g., a human), of greater than 15 days, greater than 20 days, greater than 25 days, greater than 30 days, greater than 35 days, greater than 40 days, greater than 45 days, greater than 2 months, greater than 3 months, greater than 4 months, or greater than 5 months. The increased half-lives of the antibodies of the present invention in a mammal, (e.g., a human), results in a higher serum titer of said antibodies or antibody fragments in the mammal, and thus, reduces the frequency of the administration of said antibodies or antibody fragments and/or reduces the concentration of said antibodies or antibody fragments to be administered. Antibodies having increased in vitro half-lives can be generated by techniques known to those of skill in the art. For example, antibodies with increased in vivo half-lives can be generated by modifying (e.g., substituting, deleting or adding) amino acid residues identified as involved in the interaction between the Fc domain and the FcRn receptor (see, e.g., International Publication Nos. WO 97/34631; WO 04/029207; U.S. Pat. No. 6,737,056 and U.S. Patent Publication No. 2003/0190311).

### Therapeutic antibodies

In a specific embodiment the variant Fc heterodimer comprising at least an immunoglobulin heavy chain variable region and a variant CH3 domain is a multi-specific antibody, wherein the heteromultimer is devoid of immunoglobulin light chains and immunoglobulin CH1 region and optionally devoid of immunoglobulin CH2 region (referred to herein as an antibody of the invention), the antibody of the invention specifically binds an antigen of interest. In particular the antibody of the invention is a bi-specific antibody. In one embodiment, an antibody of the invention specifically binds a polypeptide antigen. In another embodiment, an antibody of the invention specifically binds a nonpolypeptide antigen. In yet another embodiment, administration of an antibody of the invention to a mammal suffering from a disease or disorder can result in a therapeutic benefit in that mammal.

Also provided are antibodies of the invention that specifically bind cancer antigens including, but not limited to, ALK receptor (pleiotrophin receptor), pleiotrophin, KS 1/4 pan-carcinoma antigen; ovarian carcinoma antigen (CA125); prostatic acid phosphate; prostate specific antigen (PSA); melanoma-associated antigen p97; melanoma antigen gp75; high molecular weight melanoma antigen (HMW-MAA); prostate specific membrane antigen; carcinoembryonic antigen (CEA); polymorphic epithelial mucin antigen; human milk fat globule antigen; colorectal tumor-associated antigens such as: CEA, TAG-72, CO17-1A, GICA 19-9, CTA-1 and LEA; Burkitt's lymphoma antigen-38.13; CD19; human B-lymphoma antigen-CD20; CD33; melanoma specific antigens such as ganglioside GD2, ganglioside GD3, ganglioside GM2 and ganglioside GM3; tumor-specific transplantation type cell-surface antigen (TSTA); virally-induced tumor antigens including T-antigen, DNA tumor viruses and Envelope antigens of RNA tumor viruses; oncofetal antigen-alpha-fetoprotein such as CEA of colon, 5T4 oncofetal trophoblast glycoprotein and bladder tumor oncofetal antigen; differentiation antigen such as human lung carcinoma antigens L6 and L20; antigens of fibrosarcoma; human leukemia T cell antigen-Gp37; neoglycoprotein; sphingolipids; breast cancer antigens such as EGFR (Epidermal growth factor receptor); NY-BR-16; NY-BR-16 and HER2 antigen (p185HER2); polymorphic epithelial mucin (PEM); malignant human lymphocyte antigen-APO-1; differentiation antigen such as I antigen found in fetal erythrocytes; primary endoderm I antigen found in adult erythrocytes; preimplantation embryos; I(Ma) found in gastric adenocarcinomas; M18, M39 found in breast epithelium; SSEA-1 found in myeloid cells; VEP8; VEP9; Myl; Va4-D5; D₁56-22 found in colorectal cancer; TRA-1-85 (blood group H); SCP-1 found in testis and ovarian cancer; C14 found in colonic adenocarcinoma; F3 found in lung adenocarcinoma; AH6 found in gastric cancer; Y hapten; Ley found in embryonal carcinoma cells; TL5 (blood group A); EGF receptor found in A431 cells; E₁ series (blood group B) found in pancreatic cancer; FC10.2 found in embryonal carcinoma cells; gastric adenocarcinoma antigen; CO-514 (blood group Lea) found in Adenocarcinoma; NS-10 found in adenocarcinomas; CO-43 (blood group Leb); G49 found in EGF receptor of A431 cells; MH2 (blood group ALeb/Ley) found in colonic adenocarcinoma; 19.9 found in colon cancer; gastric cancer mucins; T₅A₇ found in myeloid cells; R₂₄ found in melanoma; 4.2, G_{D3}, D1.1, OFA-1, G_{M2}, OFA-2, G_{D2}, and M1:22:25:8 found in embryonal carcinoma cells and SSEA-3 and SSEA-4 found in 4 to 8-cell stage embryos; Cutaneous Tcell Lymphoma antigen; MART-1 antigen; Sialy Tn (STn) antigen; Colon cancer antigen NY-CO-45; Lung cancer antigen NY-LU-12 valiant A; Adenocarcinoma antigen ART1; Paraneoplastic associated brain-testis-cancer antigen (onconeuronal antigen MA2; paraneoplastic neuronal antigen); Neuro-oncological ventral antigen 2 (NOVA2); Hepatocellular carcinoma antigen gene 520; TUMOR-ASSOCIATED ANTIGEN CO-029; Tumor-associated antigens MAGE-C1 (cancer/testis antigen CT7), MAGE-B1 (MAGE-XP antigen), MAGE-B2 (DAM6), MAGE-2, MAGE-4-a, MAGE-4-b and MAGE-X2; Cancer-Testis Antigen (NY-EOS-1) and fragments of any of the above-listed polypeptides.

In certain embodiments, the heteromultimer described herein is competitive to at least one domain of at least one therapeutic antibody. In some embodiments, the therapeutic antibody binds a cancer target antigen. In an embodiment, the therapeutic antibody may be selected from the group consisting of abagovomab, adalimumab, alemtuzumab, aurograb, bapineuzumab, basiliximab, belimumab, bevacizumab, briakinumab, canakinumab, catumaxomab, certolizumab pegol, cetuximab, daclizumab, denosumab, efalizumab, galiximab, gemtuzumab ozogamicin, golimumab, ibritumomab tiuxetan, infliximab, ipilimumab, lumiliximab, mepolizumab, motavizumab, muromonab, mycograb, natalizumab, nimotuzumab, ocrelizumab, ofatumumab, omalizumab, palivizumab, panitumumab, pertuzumab, ranibizumab, reslizumab, rituximab, teplizumab, tocilizumab/atlizumab, tositumomab, trastuzumab, ProxiniumTM, RencarexTM, ustekinumab, zalutumumab, and any other antibodies.

### Antibody derivatives

Antibodies of the invention include derivatives that are modified (i.e., by the covalent attachment of any type of molecule to the antibody such that covalent attachment). For example, but not by way of limitation, the antibody derivatives include antibodies that have been modified, e.g., by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

Antibodies or fragments thereof with increased in vivo half-lives can be generated by attaching polymer molecules such as high molecular weight polyethyleneglycol (PEG)to the antibodies or antibody fragments. PEG can be attached to the antibodies or antibody fragments with or without a multifunctional linker either through site-specific conjugation of the PEG to the N- or C-terminus of said antibodies or antibody fragments or via epsilon-amino groups present on lysine residues. Linear or branched polymer derivatization that results in minimal loss of biological activity will be used. The degree of conjugation will be closely monitored by SDS-PAGE and mass spectrometry to ensure proper conjugation of PEG molecules to the antibodies. Unreacted PEG can be separated from antibody-PEG conjugates by, e.g., size exclusion or ion-exchange chromatography.

Further, antibodies can be conjugated to albumin in order to make the antibody or antibody fragment more stable in vivo or have a longer half life *in vivo.* The techniques are well known in the art, see e.g., International Publication Nos. WO 93/15199, WO 93/15200, and WO 01/77137; and European Patent No. EP 413,622. The present invention encompasses the use of antibodies or fragments thereof conjugated or fused to one or more moieties, including but not limited to, peptides, polypeptides, proteins, fusion proteins, nucleic acid molecules, small molecules, mimetic agents, synthetic drugs, inorganic molecules, and organic molecules.

The present invention encompasses the use of antibodies or fragments thereof recombinantly fused or chemically conjugated (including both covalent and non-covalent conjugations) to a heterologous protein or polypeptide (or fragment thereof, for example, to a polypeptide of at least 10, at least 20, at least 30, at least 40, at least 50, at least 60, at least 70, at least 80, at least 90 or at least 100 amino acids) to generate fusion proteins. The fusion does not necessarily need to be direct, but may occur through linker sequences. For example, antibodies may be used to target heterologous polypeptides to particular cell types, either in vitro or in vivo, by fusing or conjugating the antibodies to antibodies specific for particular cell surface receptors. Antibodies fused or conjugated to heterologous polypeptides may also be used in in vitro immunoassays and purification methods using methods known in the art. See e.g., International publication No. WO 93/21232; European Patent No. EP 439,095; Naramura et al., 1994, Immunol. Lett. 39:91-99; U.S. Pat.No. 5,474,981; Gillies et al., 1992, PNAS 89:1428-1432; and Fell et al., 1991, J. Immunol. 146:2446-2452.

The present invention further includes compositions comprising heterologous proteins, peptides or polypeptides fused or conjugated to antibody fragments. For example, the heterologous polypeptides may be fused or conjugated to a Fab fragment, Fd fragment, Fv fragment, F(ab)₂ fragment, a VH domain, a VL domain, a VH CDR, a VL CDR, or fragment thereof. Methods for fusing or conjugating polypeptides to antibody portions are well known in the art. See, e.g., U.S. Pat. Nos. 5,336,603; 5,622,929; 5,359,046; 5,349,053; 5,447,851 and 5,112,946; European Patent Nos. EP 307,434 and EP 367,166; International publication Nos. WO 96/04388 and WO 91/06570; Ashkenazi et al., 1991, Proc. Natl. Acad. Sci. USA 88: 10535-10539; Zheng et al., 1995, J. Immunol. 154:5590-5600; and Vil et al., 1992, Proc. Natl. Acad. Sci. USA 89:11337-11341.

Additional fusion proteins, e.g. of antibodies that specifically bind an antigen (e.g., supra), may be generated through the techniques of gene-shuffling, motif-shuffling, exon-shuffling, and/or codon-shuffling (collectively referred to as "DNA shuffling"). DNA shuffling may be employed to alter the activities of antibodies of the invention or fragments thereof (e.g., antibodies or fragments thereof with higher affinities and lower dissociation rates). See, generally, U.S. Pat. Nos. 5,605,793; 5,811,238; 5,830,721; 5,834,252 and 5,837,458, and Patten et al., 1997, Curr. Opinion Biotechnol. 8:724-33; Harayama, 1998, Trends Biotechnol. 16(2): 76-82; Hansson, et al., 1999, J. Mol. Biol. 287:265-76; and Lorenzo and Blasco, 1998, Biotechniques 24(2): 308-313. Antibodies or fragments thereof, or the encoded antibodies or fragments thereof, may be altered by being subjected to random mutagenesis by error-prone PCR, random nucleotide insertion or other methods prior to recombination. One or more portions of a polynucleotide encoding an antibody or antibody fragment, which portions specifically bind to an antigen may be recombined with one or more components, motifs, sections, parts, domains, fragments, etc. of one or more heterologous molecules.

### Drug conjugation

The present invention further encompasses uses of heteromultimers comprising variant Fc heterodimers or fragments thereof conjugated to a therapeutic agent or a cytotoxin.

An antibody or fragment thereof may be conjugated to a therapeutic moiety such as a cytotoxin, e.g., a cytostatic or cytocidal agent, a therapeutic agent or a radioactive metal ion, e.g., alpha-emitters. A cytotoxin or cytotoxic agent includes any agent that is detrimental to cells. Examples include ribonuclease, monomethylauristatin E and F, paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, epirubicin, and cyclophosphamide and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BCNU) and lomustine (CCNU), cyclophosphamide, busulfan, dibromomannitol, streptozotocin, mitomycin C, and cisdichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). A more extensive list of therapeutic moieties can be found in PCT publications WO 03/075957.

Methods for fusing or conjugating antibodies to polypeptide moieties are known in the art. See, e.g., U.S. Pat. Nos. 5,336,603; 5,622,929; 5,359,046; 5,349,053; 5,447,851 and 5,112,946; EP 307,434; EP 367,166; PCT Publications WO 96/04388 and WO 91/06570; Ashkenazi et al., 1991, PNAS USA 88:10535; Zheng et al., 1995, J Immunol 154:5590; and Vil et al., 1992, PNAS USA 89:11337. The fusion of an antibody to a moiety does not necessarily need to be direct, but may occur through linker sequences. Such linker molecules are commonly known in the art and described in Denardo et al., 1998, Clin Cancer Res 4:2483; Peterson et al., 1999, Bioconjug Chem 10:553; Zimmerman et al., 1999, Nucl Med Biol 26:943; Garnett, 2002, Adv Drug Deliv Rev 53:171.

### Recombinant expression

Recombinant expression of a heteromultimer, derivative, analog or fragment thereof, (e.g., an antibody or fusion protein of the invention), requires construction of an expression vector containing a polynucleotide that encodes the heteromultimer (e.g., antibody, or fusion protein). Once a polynucleotide encoding the heteromultimer (e.g., antibody, or fusion protein) has been obtained, the vector for the production of the heteromultimer (e.g., antibody, or fusion protein) may be produced by recombinant DNA technology using techniques well known in the art. Thus, methods for preparing a protein by expressing a polynucleotide containing a heteromultimer(e.g., antibody, or fusion protein) encoding nucleotide sequence are described herein. Methods that are well known to those skilled in the art can be used to construct expression vectors containing the heteromultimer (e.g., antibody, or fusion protein) coding sequences and appropriate transcriptional and translational control signals. These methods include, for example, in vitro recombinant DNA techniques, synthetic techniques, and in vivo genetic recombination. The invention, thus, provides replicable vectors comprising a nucleotide sequence encoding a heteromultimer of the invention, operably linked to a promoter. Such vectors may include the nucleotide sequence encoding the constant region of the antibody molecule (see, e.g., International Publication No. WO 86/05807; International Publication No. WO 89/01036; and U.S. Pat. No. 5,122,464 and the variable domain of the antibody, or a polypeptide for generating an Fc variant may be cloned into such a vector for expression of the full length antibody chain (e.g. heavy or light chain), or complete Fc variant comprising a fusion of a non-antibody derived polypeptide and an Fc region incorporating at least the variant CH3 domain.

The expression vector is transferred to a host cell by conventional techniques and the transfected cells are then cultured by conventional techniques to produce an Fc variant of the invention. Thus, the invention includes host cells containing a polynucleotide encoding a heteromultimer of the invention, operably linked to a heterologous promoter. In specific embodiments for the expression of heteromultimers comprising double-chained antibodies, vectors encoding both the heavy and light chains may be co-expressed in the host cell for expression of the entire immunoglobulin molecule, as detailed below.

A variety of host-expression vector systems may be utilized to express the heteromultimers of the invention (e.g., antibody or fusion protein molecules) (see, e.g., U.S. Pat. No. 5,807,715).Such host-expression systems represent vehicles by which the coding sequences of interest may be produced and subsequently purified, but also represent cells which may, when transformed or transfected with the appropriate nucleotide coding sequences, express an heteromultimer of the invention in situ. These include but are not limited to microorganisms such as bacteria (e.g., E. coli and B. subtilis) transformed with recombinant bacteriophage DNA, plasmid DNA or cosmid DNA expression vectors containing heteromultimer coding sequences; yeast (e.g., Saccharomyces Pichia) transformed with recombinant yeast expression vectors containing Fc variant coding sequences; insect cell systems infected with recombinant virus expression vectors (e.g., baculovirus) containing heteromultimer coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing Fc variant coding sequences; or mammalian cell systems (e.g., COS, CHO, BHK, 293, NS0, and 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter). In certain embodiments, bacterial cells such as Escherichia coli, or eukaryotic cells, are used for the expression of a heteromultimer, which is a recombinant antibody or fusion protein molecules. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, Bio/Technology 8:2). In a specific embodiment, the expression of nucleotide sequences encoding an Fc variant of the invention (e.g., antibody or fusion protein) is regulated by a constitutive promoter, inducible promoter or tissue specific promoter.

In bacterial systems, a number of expression vectors may be advantageously selected depending upon the use intended for the heteromultimer (e.g., antibody or fusion protein) being expressed. For example, when a large quantity of such a protein is to be produced, for the generation of pharmaceutical compositions of an Fc variant, vectors that direct the expression of high levels of fusion protein products that are readily purified may be desirable. Such vectors include, but are not limited to, the E. coli expression vector pUR278 (Ruther et al., 1983, EMBO 12:1791), in which the heteromultimer coding sequence may be ligated individually into the vector in frame with the lac Z coding region so that a lac Z-fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione agarose beads followed by elution in the presence of free glutathione. The pGEX vectors are designed to include thrombin or factor Xa protease cleavage sites so that the cloned target gene product can be released from the GST moiety.

In an insect system Autographa californica nuclear polyhedrosis virus (AcNPV) is used as a vector to express foreign genes. The virus grows in Spodoptera frugiperda cells. The heteromultimer (e.g., antibody or fusion protein) coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the heteromultimer (e.g., antibody or fusion protein) coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing the heteromultimer (e.g., antibody or fusion protein) in infected hosts (e.g., see Logan & Shenk, 1984, Proc. Natl. Acad. Sci. USA 81:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, e.g., Bittner et al., 1987, Methods in Enzymol. 153:516-544).

The expression of a heteromultimer (e.g., antibody or fusion protein) may be controlled by any promoter or enhancer element known in the art. Promoters which may be used to control the expression of the gene encoding a heteromultimer (e.g., antibody or fusion protein) include, but are not limited to, the SV40 early promoter region (Bernoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42), the tetracycline (Tet) promoter (Gossen et al., 1995, Proc. Nat. Acad. Sci. USA 89:5547-5551); prokaryotic expression vectors such as the β-lactamase promoter (Villa-Kamaroff et al, 1978, Proc. Natl. Acad. Sci. U.S.A. 75:3727-3731), or the tac promoter (DeBoer et al., 1983, Proc. Natl. Acad. Sci. U.S.A. 80:21-25; see also "Useful proteins from recombinant bacteria" in Scientific American, 1980, 242:74-94); plant expression vectors comprising the nopaline synthetase promoter region (Herrera-Estrella et al., Nature 303:209-213) or the cauliflower mosaic virus 35S RNA promoter (Gardner et al., 1981, Nucl. Acids Res. 9:2871), and the promoter of the photosynthetic enzyme ribulose biphosphate carboxylase (Herrera-Estrella et al., 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286); neuronal-specific enolase (NSE) which is active in neuronal cells (Morelli et al., 1999, Gen. Virol. 80:571-83); brain-derived neurotrophic factor (BDNF) gene control region which is active in neuronal cells (Tabuchi et al., 1998, Biochem. Biophysic. Res. Com. 253:818-823); glial fibrillary acidic protein (GFAP) promoter which is active in astrocytes (Gomes et al., 1999, Braz J Med Biol Res 32(5): 619-631; Morelli et al., 1999, Gen. Virol. 80:571-83) and gonadotropic releasing hormone gene control region which is active in the hypothalamus (Mason et al., 1986, Science 234:1372-1378).

Expression vectors containing inserts of a gene encoding a heteromultimer of the invention (e.g., antibody or fusion protein) can be identified by three general approaches: (a) nucleic acid hybridization, (b) presence or absence of "marker" gene functions, and (c) expression of inserted sequences. In the first approach, the presence of a gene encoding a peptide, polypeptide, protein or a fusion protein in an expression vector can be detected by nucleic acid hybridization using probes comprising sequences that are homologous to an inserted gene encoding the peptide, polypeptide, protein or the fusion protein, respectively. In the second approach, the recombinant vector/host system can be identified and selected based upon the presence or absence of certain "marker" gene functions (e.g., thymidine kinase activity, resistance to antibiotics, transformation phenotype, occlusion body formation in baculovirus, etc.) caused by the insertion of a nucleotide sequence encoding an antibody or fusion protein in the vector. For example, if the nucleotide sequence encoding the heteromultimer (e.g., antibody or fusion protein) is inserted within the marker gene sequence of the vector, recombinants containing the gene encoding the antibody or fusion protein insert can be identified by the absence of the marker gene function. In the third approach, recombinant expression vectors can be identified by assaying the gene product (e.g., antibody or fusion protein) expressed by the recombinant. Such assays can be based, for example, on the physical or functional properties of the fusion protein in in vitro assay systems, e.g., binding with anti-bioactive molecule antibody.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered fusion protein may be controlled. Furthermore, different host cells have characteristic and specific mechanisms for the translational and post-translational processing and modification (e.g., glycosylation, phosphorylation of proteins). Appropriate cell lines or host systems can be chosen to ensure the desired modification and processing of the foreign protein expressed. For example, expression in a bacterial system will produce an unglycosylated product and expression in yeast will produce a glycosylated product. Eukaryotic host cells that possess the cellular machinery for proper processing of the primary transcript (e.g., glycosylation, and phosphorylation) of the gene product may be used. Such mammalian host cells include, but are not limited to, CHO, VERY, BHK, Hela, COS, MDCK, 293, 3T3, WI38, NS0, and in particular, neuronal cell lines such as, for example, SK-N-AS, SK-N-FI, SK-N-DZ human neuroblastomas (Sugimoto et al., 1984, J. Natl. Cancer Inst. 73: 51-57), SK-N-SH human neuroblastoma (Biochim. Biophys. Acta, 1982, 704: 450-460), Daoy human cerebellar medulloblastoma (He et al., 1992, Cancer Res. 52: 1144-1148) DBTRG-05MG glioblastoma cells (Kruse et al., 1992, In Vitro Cell. Dev. Biol. 28A: 609-614), IMR-32 human neuroblastoma (Cancer Res., 1970, 30: 2110-2118), 1321N1 human astrocytoma (Proc. Natl. Acad. Sci. USA, 1977, 74: 4816), MOG-G-CCM human astrocytoma (Br. J. Cancer, 1984, 49: 269), U87MG human glioblastoma-astrocytoma (Acta Pathol. Microbiol. Scand., 1968, 74: 465-486), A172 human glioblastoma (Olopade et al., 1992, Cancer Res. 52: 2523-2529), C6 rat glioma cells (Benda et al., 1968, Science 161: 370-371), Neuro-2a mouse neuroblastoma (Proc. Natl. Acad. Sci. USA, 1970, 65: 129-136), NB41A3 mouse neuroblastoma (Proc. Natl. Acad. Sci. USA, 1962, 48: 1184-1190), SCP sheep choroid plexus (Bolin et al., 1994, J. Virol. Methods 48: 211-221), G355-5, PG-4 Cat normal astrocyte (Haapala et al., 1985, J. Virol. 53: 827-833), Mpf ferret brain (Trowbridge et al., 1982, In Vitro 18: 952-960), and normal cell lines such as, for example, CTX TNA2 rat normal cortex brain (Radany et al., 1992, Proc. Natl. Acad. Sci. USA 89: 6467-6471) such as, for example, CRL7030 and Hs578Bst. Furthermore, different vector/host expression systems may effect processing reactions to different extents.

For long-term, high-yield production of recombinant proteins, stable expression is often preferred. For example, cell lines that stably express a heteromultimer of the invention (e.g., antibody or fusion protein) may be engineered. Rather than using expression vectors that contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched medium, and then are switched to a selective medium. The selectable marker in the recombinant plasmid confers resistance to the selection and allows cells to stably integrate the plasmid into their chromosomes and grow to form foci that in turn can be cloned and expanded into cell lines. This method may advantageously be used to engineer cell lines that express a heteromultimer that specifically binds to an Antigen. Such engineered cell lines may be particularly useful in screening and evaluation of compounds that affect the activity of a heteromultimer (e.g., a polypeptide or a fusion protein) that specifically binds to an antigen.

A number of selection systems may be used, including but not limited to the herpes simplex virus thymidine kinase (Wigler et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy et al., 1980, Cell 22:817) genes can be employed in tk-, hgprt- or aprt-cells, respectively. Also, antimetabolite resistance can be used as the basis of selection for dhfr, which confers resistance to methotrexate (Wigler et al., 1980, Natl. Acad. Sci. USA 77:3567; O'Hare et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin et al., 1981, J. Mol. Biol. 150:1); and hygro, which confers resistance to hygromycin (Santerre et al., 1984, Gene 30:147) genes.

### Purification

Once a heteromultimer (e.g., antibody, or a fusion protein) of the invention has been produced by recombinant expression, it may be purified by any method known in the art for purification of a protein, for example, by chromatography (e.g., ion exchange, affinity, particularly by affinity for the specific antigen after Protein A, and sizing column chromatography), centrifugation, differential solubility, or by any other standard technique for the purification of proteins.

The heteromultimer is generally recovered from the culture medium as a secreted polypeptide, although it also may be recovered from host cell lysate when directly produced without a secretory signal. If the heteromultimer is membrane-bound, it can be released from the membrane using a suitable detergent solution (e.g. Triton-X 100).

When the heteromultimer is produced in a recombinant cell other than one of human origin, it is completely free of proteins or polypeptides of human origin. However, it is necessary to purify the heteromultimer from recombinant cell proteins or polypeptides to obtain preparations that are substantially homogeneous as to the heteromultimer. As a first step, the culture medium or lysate is normally centrifuged to remove particulate cell debris.

Heteromultimers having antibody constant domains can be conveniently purified by hydroxylapatite chromatography, gel electrophoresis, dialysis, or affinity chromatography, with affinity chromatography being the preferred purification technique. Other techniques for protein purification such as fractionation on an ion-exchange column, ethanol precipitation, reverse phase HPLC, chromatography on silica, chromatography on heparin Sepharose, chromatography on an anion or cation exchange resin (such as a polyaspartic acid column), chromatofocusing, SDS-PAGE, and ammonium sulfate precipitation are also available depending on the polypeptide to be recovered. The suitability of protein A as an affinity ligand depends on the species and isotype of the immunoglobulin Fc domain that is used. Protein A can be used to purify immunoglobulin Fc regions that are based on human y1, y2, or y4 heavy chains (Lindmark et al., J. Immunol. Meth. 62:1-13 (1983)). Protein G is recommended for all mouse isotypes and for human y3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. The conditions for binding an immunoadhesin to the protein A or G affinity column are dictated entirely by the characteristics of the Fc domain; that is, its species and isotype. Generally, when the proper ligand is chosen, efficient binding occurs directly from unconditioned culture fluid. Bound variant Fc heterodimers can be efficiently eluted either at acidic pH (at or above 3.0), or in a neutral pH buffer containing a mildly chaotropic salt. This affinity chromatography step can result in a variant Fc heterodimer preparation that is >95% pure.

The expression levels of a heteromultimer (e.g., antibody or fusion protein) can be increased by vector amplification (for a review, see Bebbington and Hentschel, The use of vectors based on gene amplification for the expression of cloned genes in mammalian cells in DNA cloning, Vol. 3. (Academic Press, New York, 1987)). For example, when a marker in the vector system expressing an antibody or fusion protein is amplifiable, increase in the level of inhibitor present in culture of host cell will increase the number of copies of the marker gene. Since the amplified region is associated with the antibody gene, production of the antibody or fusion protein will also increase (Crouse et al., 1983, Mol. Cell. Biol. 3:257).

### Characterization and Functional Assays

Fc variants (e.g., antibodies or fusion proteins) of the present invention may be characterized in a variety of ways. In one embodiment, purity of the variant Fc heterodimers is assessed using techniques well known in the art including, but not limited to, SDS-PAGE gels, western blots, densitometry or mass spectrometry. Protein stability can be characterized using an array of techniques, not limited to, size exclusion chromatography, UV Visible and CD spectroscopy, mass spectroscopy, differential light scattering, bench top stability assay, freeze thawing coupled with other characterization techniques, differential scanning calorimetry, differential scanning fluorimetry, hydrophobic interaction chromatorgraphy, isoelectric focusing, receptor binding assays or relative protein expression levels. In en exemplary embodiment, stability of the variant Fc heterodimers is assessed by melting temperature of the variant CH3 domain, as compared to wild-type CH3 domain, using techniques well known in the art such as Differential Scanning Calorimetryor differential scanning flourimetry.

Fc variants of the present invention may also be assayed for the ability to specifically bind to a ligand, (e.g., FcγRIIIA, FcγRIIB, C1q). Such an assay may be performed in solution (e.g., Houghten, Bio/Techniques, 13:412-421, 1992), on beads (Lam, Nature, 354:82-84, 1991, on chips (Fodor, Nature, 364:555-556, 1993), on bacteria (U.S. Pat. No. 5,223,409) on plasmids (Cull et al., Proc. Natl. Acad. Sci. USA, 89:1865-1869, 1992) or on phage (Scott and Smith, Science, 249:386-390, 1990; Devlin, Science, 249:404-406, 1990; Cwirla et al., Proc. Natl. Acad. Sci. USA, 87:6378-6382, 1990; and Felici, J. Mol. Biol., 222:301-310, 1991). Molecules that have been identified to specifically bind to a ligand, (e.g., FcγRIIIA, FcγRIIB, C1q or to an antigen) can then be assayed for their affinity for the ligand.

Fc variants of the invention may be assayed for specific binding to a molecule such as an antigen (e.g., cancer antigen and cross-reactivity with other antigens) or a ligand (e.g., FcyR) by any method known in the art. Immunoassays which can be used to analyze specific binding and cross-reactivity include, but are not limited to, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e.g., Ausubel et al., eds, 1994, Current Protocols in Molecular Biology, Vol. 1, John Wiley & Sons, Inc., New York).

The binding affinity of the Fc variants of the present invention to a molecule such as an antigen or a ligand, (e.g., FcyR) and the off-rate of the interaction can be determined by competitive binding assays. One example of a competitive binding assay is a radioimmunoassay comprising the incubation of labeled ligand, such as FcγR (e.g., 3H or 1251 with a molecule of interest (e.g., Fc variants of the present invention) in the presence of increasing amounts of unlabeled ligand, such as FcyR, and the detection of the molecule bound to the labeled ligand. The affinity of the molecule of the present invention for the ligand and the binding off-rates can be determined from the saturation data by scatchard analysis.

### Affinity maturation

As is known in the art, once a single domain antigen-binding construct has been identified, and its affinity for the target antigen measured, if necessary, the affinity of the single domain antigen-binding construct for its target antigen can be improved by affinity maturation according to methods known in the art. One exemplary method for affinity maturation of an antigen-binding domain where the crystal structure of the target antigen to the antibody is available is described as follows. Structures of the antigen:antibody complex are used for modeling. Molecular dynamics (MD) can be employed to evaluate the intrinsic dynamic nature of the WT complex in an aqueous environment. Mean field and dead-end elimination methods along with flexible backbones can be used to optimize and prepare model structures for the mutants to be screened. Following packing a number of features will be scored including contact density, clash score, hydrophobicity and electrostatics. Generalized Born method will allow accurate modeling of the effect of solvent environment and compute the free energy differences following mutation of specific positions in the protein to alternate residue types. Contact density and clash score will provide a measure of complementarity, a critical aspect of effective protein packing. The screening procedure employs knowledge-based potentials as well as coupling analysis schemes relying on pair-wise residue interaction energy and entropy computations.

The kinetic parameters of an Fc variant may also be determined using any surface plasmon resonance (SPR) based assays known in the art (e.g., BIAcore kinetic analysis). For a review of SPR-based technology see Mullet et al., 2000, Methods 22: 77-91; Dong et al., 2002, Review in Mol. Biotech., 82: 303-23; Fivash et al., 1998, Current Opinion in Biotechnology 9: 97-101; Rich et al., 2000, Current Opinion in Biotechnology 11: 54-61. Additionally, any of the SPR instruments and SPR based methods for measuring protein-protein interactions described in U.S. Pat. Nos. 6,373,577; 6,289,286; 5,322,798; 5,341,215; 6,268,125 are contemplated in the methods of the invention.

Fluorescence activated cell sorting (FACS), using any of the techniques known to those skilled in the art, can be used for characterizing the binding of Fc variants to a molecule expressed on the cell surface (e.g., FcγRIIIA, FcγRIIB). Flow sorters are capable of rapidly examining a large number of individual cells that contain library inserts (e.g., 10-100 million cells per hour) (Shapiro et al., Practical Flow, Cytometry, 1995). Flow cytometers for sorting and examining biological cells are well known in the art. Known flow cytometers are described, for example, in U.S. Pat. Nos. 4,347,935; 5,464,581; 5,483,469; 5,602,039; 5,643,796; and 6,211,477. Other known flow cytometers are the FACS Vantage™ system manufactured by Becton Dickinson and Company, and the COPAS™ system manufactured by Union Biometrica.

The Fc variants of the invention can be characterized by their ability to mediate FcyR-mediated effector cell function. Examples of effector cell functions that can be assayed include, but are not limited to, antibody-dependent cell mediated cytotoxicity (ADCC), phagocytosis, opsonization, opsonophagocytosis, C1q binding, and complement dependent cell mediated cytotoxicity (CDC). Any cell-based or cell free assay known to those skilled in the art for determining effector cell function activity can be used (For effector cell assays, see Perussia et al., 2000, Methods Mol. Biol. 121: 179-92; Baggiolini et al., 1998 Experientia, 44(10): 841-8; Lehmann et al., 2000 J. Immunol. Methods, 243(1-2): 229-42; Brown E J. 1994, Methods Cell Biol., 45: 147-64; Munn et al., 1990 J. Exp. Med., 172: 231-237, Abdul-Majid et al., 2002 Scand. J. Immunol. 55: 70-81; Ding et al., 1998, Immunity 8:403-411).

In particular, the Fc variants of the invention can be assayed for FcyR-mediated ADCC activity in effector cells, (e.g., natural killer cells) using any of the standard methods known to those skilled in the art (See e.g., Perussia et al., 2000, Methods Mol. Biol. 121: 179-92). An exemplary assay for determining ADCC activity of the molecules of the invention is based on a 51Cr release assay comprising of: labeling target cells with [51Cr]Na₂CrO₄(this cell-membrane permeable molecule is commonly used for labeling since it binds cytoplasmic proteins and although spontaneously released from the cells with slow kinetics, it is released massively following target cell necrosis); osponizing the target cells with the Fc variants of the invention; combining the opsonized radiolabeled target cells with effector cells in a microtitre plate at an appropriate ratio of target cells to effector cells; incubating the mixture of cells for 16-18 hours at 37° C.; collecting supernatants; and analyzing radioactivity. The cytotoxicity of the molecules of the invention can then be determined, for example using the following formula: % lysis=(experimental cpm-target leak cpm)/(detergent lysis cpm-target leak cpm)×100%. Alternatively, % lysis=(ADCC-AICC)/(maximum release-spontaneous release). Specific lysis can be calculated using the formula: specific lysis=% lysis with the molecules of the invention-% lysis in the absence of the molecules of the invention. A graph can be generated by varying either the target:effector cell ratio or antibody concentration.

Method to characterize the ability of the Fc variants to bind C1q and mediate complement dependent cytotoxicity (CDC) are well known in the art. For example, to determine C1q binding, a C1q binding ELISA may be performed. An exemplary assay may comprise the following: assay plates may be coated overnight at 4 C with polypeptide variant or starting polypeptide (control) in coating buffer. The plates may then be washed and blocked. Following washing, an aliquot of human C1q may be added to each well and incubated for 2 hrs at room temperature. Following a further wash, 100 uL of a sheep anti-complement C1q peroxidase conjugated antibody may be added to each well and incubated for 1 hour at room temperature. The plate may again be washed with wash buffer and 100 ul of substrate buffer containing OPD (O-phenylenediamine dihydrochloride (Sigma)) may be added to each well. The oxidation reaction, observed by the appearance of a yellow color, may be allowed to proceed for 30 minutes and stopped by the addition of 100 ul of 4.5 NH2 SO4. The absorbance may then read at (492-405) nm.

To assess complement activation, a complement dependent cytotoxicity (CDC) assay may be performed, (e.g. as described in Gazzano-Santoro et al., 1996, J. Immunol. Methods 202:163). Briefly, various concentrations of Fc variant and human complement may be diluted with buffer. Cells which express the antigen to which the Fc variant binds may be diluted to a density of about 1×106 cells/ml. Mixtures of the Fc variant, diluted human complement and cells expressing the antigen may be added to a flat bottom tissue culture 96 well plate and allowed to incubate for 2 hrs at 37C. and 5% CO2 to facilitate complement mediated cell lysis. 50 uL of alamar blue (Accumed International) may then be added to each well and incubated overnight at 37C. The absorbance is measured using a 96-well fluorometer with excitation at 530 nm n and emission at 590 nm. The results may be expressed in relative fluorescence units (RFU). The sample concentrations may be computed from a standard curve and the percent activity, relative to a comparable molecule (i.e., a molecule comprising an Fc region with an unmodified or wild type CH3 domain) is reported for the Fc variant of interest.

Complement assays may be performed with guinea pig, rabbit or human serum. Complement lysis of target cells may be detected by monitoring the release of intracellular enzymes such as lactate dehydrogenase (LDH), as described in Korzeniewski et al., 1983, Immunol. Methods 64(3): 313-20; and Decker et al., 1988, J. Immunol Methods 115(1): 61-9; or the release of an intracellular label such as europium, chromium 51 or indium 111 in which target cells are labeled.

### Pharmacokinetic stability

In certain embodiments, the heteromultimers provided herein exhibits pharmacokinetic (PK) or *in vivo* stability properties comparable with commercially available therapeutic antibodies. In one embodiment, the heteromultimers described herein exhibit PK properties similar to known therapeutic antibodies, with respect to serum concentration, t1/2, beta half-life, and/or CL.

In some embodiments, active transport processes such as uptake by neonatal Fc receptor (FcRn) also impact antibody biodistribution among other binding proteins. In one embodiment, the heteromultimers bind FcRn with similar affinity compared to commercially available therapeutic antibodies.

### Humanized single domain antigen-binding constructs

In some embodiments it may be necessary to humanize the single domain antigen-binding construct prior to use in the heteromultimer described herein, for example, if the single domain antigen-binding construct is obtained from a camelid or shark. "Humanized" forms of non-human antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

### Methods of treatment

The present invention encompasses administering one or more heteromultimer of the invention (e.g., antibodies) to an animal, in particular a mammal, specifically, a human, for preventing, treating, or ameliorating one or more symptoms associated with a disease, disorder, or infection.

In one embodiment, the heteromultimers described herein exert their therapeutic effects through Fc effector function activity. In one embodiment, the heteromultimers described herein are conjugated to a cytotoxic drug molecule, and exert their therapeutic effects through internalization of the heteromultimer and cytotoxic drug molecule into the target cell.

In one embodiment, the heteromultimers are used to treat infections of pathogenic organisms, such as bacteria or fungi.

The heteromultimers of the invention are particularly useful for the treatment or prevention of a disease or disorder where an altered efficacy of effector cell function (e.g., ADCC, CDC) is desired. The heteromultimers and compositions thereof are particularly useful for the treatment or prevention of primary or metastatic neoplastic disease (i.e., cancer), and infectious diseases. Molecules of the invention may be provided in pharmaceutically acceptable compositions as known in the art or as described herein. As detailed below, the molecules of the invention can be used in methods of treating or preventing cancer (particularly in passive immunotherapy), autoimmune disease, inflammatory disorders or infectious diseases.

The heteromultimers of the invention may also be advantageously utilized in combination with other therapeutic agents known in the art for the treatment or prevention of a cancer, autoimmune disease, inflammatory disorders or infectious diseases. In a specific embodiment, heteromultimers of the invention may be used in combination with monoclonal or chimeric antibodies, lymphokines, or hematopoietic growth factors (such as, e.g., IL-2, IL-3 and IL-7), which, for example, serve to increase the number or activity of effector cells which interact with the molecules and, increase immune response. The heteromultimers of the invention may also be advantageously utilized in combination with one or more drugs used to treat a disease, disorder, or infection such as, for example anti-cancer agents, anti-inflammatory agents or anti-viral agents.

Accordingly, the present invention provides methods for preventing, treating, or ameliorating one or more symptoms associated with cancer and related conditions by administering one or more heteromultimers of the invention. Although not intending to be bound by any mechanism of actions, a heteromultimer of the invention that binds FcγRIIIA and/or FcyRIIA with a greater affinity than a comparable molecule, and further binds FcyRIIB with a lower affinity than a comparable molecule, and/or said Heteromultimer has an enhanced effector function, e.g., ADCC, CDC, phagocytosis, opsonization, etc. will result in the selective targeting and efficient destruction of cancer cells.

The invention further encompasses administering one or more heteromultimers of the invention in combination with other therapies known to those skilled in the art for the treatment or prevention of cancer, including but not limited to, current standard and experimental chemotherapies, hormonal therapies, biological therapies, immunotherapies, radiation therapies, or surgery. In some embodiments, the molecules of the invention may be administered in combination with a therapeutically or prophylactically effective amount of one or more anti-cancer agents, therapeutic antibodies or other agents known to those skilled in the art for the treatment and/or prevention of cancer. Examples of dosing regimes and therapies which can be used in combination with the heteromultimers of the invention are well known in the art and have been described in detail elsewhere (see for example, PCT publications WO 02/070007 and WO 03/075957).

Cancers and related disorders that can be treated or prevented by methods and compositions of the present invention include, but are not limited to, the following: Leukemias, lymphomas, multiple myelomas, bone and connective tissue sarcomas, brain tumors, breast cancer, adrenal cancer, thyroid cancer, pancreatic cancer, pituitary cancers, eye cancers, vaginal cancers, vulvar cancer, cervical cancers, uterine cancers, ovarian cancers, esophageal cancers, stomach cancers, colon cancers, rectal cancers, liver cancers, gallbladder cancers, cholangiocarcinomas, lung cancers, testicular cancers, prostate cancers, penal cancers; oral cancers, salivary gland cancers pharynx cancers, skin cancers, kidney cancers, bladder cancers (for a review of such disorders, see Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America).

In one embodiment, where the heteromultimer comprises a single domain antigen-binding construct that bind to EGFR1, EGFR1 or the mutant EGFR variant III (EGFRvIII) expressing cells, the heteromultimer can be used to treat cancers that overexpress EGFR1 or cancer cells that are resistant to treatment by binding to EGFRvIII.

The invention further contemplates engineering any of the antibodies known in the art for the treatment and/or prevention of cancer and related disorders, so that the antibodies comprise an Fc region incorporating a variant CH3 domain of the invention.

In a specific embodiment, a molecule of the invention (e.g., an antibody comprising a variant Fc heterodimer inhibits or reduces the growth of primary tumor or metastasis of cancerous cells by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the growth of primary tumor or metastasis in the absence of said molecule of the invention.

The present invention encompasses the use of one or more heteromultimers of the invention for preventing, treating, or managing one or more symptoms associated with an inflammatory disorder in a subject. Although not intending to be bound by any mechanism of actions, heteromultimers with enhanced affinity for FcyRIIB will lead to a dampening of the activating receptors and thus a dampening of the immune response and have therapeutic efficacy for treating and/or preventing an autoimmune disorder. Furthermore, antibodies binding more than one target, such as bi-specific antibodies comprising a variant Fc heterodimer, associated with an inflammatory disorder may provide synergist effects over monovalent therapy.

The invention further encompasses administering the heteromultimers of the invention in combination with a therapeutically or prophylactically effective amount of one or more anti-inflammatory agents. The invention also provides methods for preventing, treating, or managing one or more symptoms associated with an autoimmune disease further comprising, administering to said subject a heteromultimer of the invention in combination with a therapeutically or prophylactically effective amount of one or more immunomodulatory agents. Examples of autoimmune disorders that may be treated by administering the heteromultimers of the invention include, but are not limited to, alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erthematosus, Meniere's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, Sjogren's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, and Wegener's granulomatosis. Examples of inflamatory disorders include, but are not limited to, asthma, encephilitis, inflammatory bowel disease, chronic obstructive pulmonary disease (COPD), allergic disorders, septic shock, pulmonary fibrosis, undifferentiated spondyloarthropathy, undifferentiated arthropathy, arthritis, inflammatory osteolysis, and chronic inflammation resulting from chronic viral or bacteria infections. Some autoimmune disorders are associated with an inflammatory condition, thus, there is overlap between what is considered an autoimmune disorder and an inflammatory disorder. Therefore, some autoimmune disorders may also be characterized as inflammatory disorders. Examples of inflammatory disorders which can be prevented, treated or managed in accordance with the methods of the invention include, but are not limited to, asthma, encephilitis, inflammatory bowel disease, chronic obstructive pulmonary disease (COPD), allergic disorders, septic shock, pulmonary fibrosis, undifferentiated spondyloarthropathy, undifferentiated arthropathy, arthritis, inflammatory osteolysis, and chronic inflammation resulting from chronic viral or bacteria infections.

Heteromultimers of the invention can also be used to reduce the inflammation experienced by animals, particularly mammals, with inflammatory disorders. In a specific embodiment, an Fc of the invention reduces the inflammation in an animal by at least 99%, at least 95%, at least 90%, at least 85%, at least 80%, at least 75%, at least 70%, at least 60%, at least 50%, at least 45%, at least 40%, at least 45%, at least 35%, at least 30%, at least 25%, at least 20%, or at least 10% relative to the inflammation in an animal, which is not administered the said molecule.

The invention further contemplates engineering any of the antibodies known in the art for the treatment and/or prevention of autoimmune disease or inflammatory disease, so that the antibodies comprisea variant Fc heterodimer of the invention.

The invention also encompasses methods for treating or preventing an infectious disease in a subject comprising administering a therapeutically or prophylactically effective amount of one or more heteromultimers of the invention. Infectious diseases that can be treated or prevented by the heteromultimers of the invention are caused by infectious agents including but not limited to viruses, bacteria, fungi, protozae, and viruses.

Viral diseases that can be treated or prevented using the heteromultimers of the invention in conjunction with the methods of the present invention include, but are not limited to, those caused by hepatitis type A, hepatitis type B, hepatitis type C, influenza, varicella, adenovirus, herpes simplex type I (HSV-I), herpes simplex type II (HSV-II), rinderpest, rhinovirus, echovirus, rotavirus, respiratory syncytial virus, papilloma virus, papova virus, cytomegalovirus, echinovirus, arbovirus, huntavirus, coxsackie virus, mumps virus, measles virus, rubella virus, polio virus, small pox, Epstein Barr virus, human immunodeficiency virus type I (HIV-I), human immunodeficiency virus type II (HIV-II), and agents of viral diseases such as viral meningitis, encephalitis, dengue or small pox.

Bacterial diseases that can be treated or prevented using the heteromultimers of the invention in conjunction with the methods of the present invention, that are caused by bacteria include, but are not limited to, mycobacteria rickettsia, mycoplasma, neisseria, S. pneumonia, Borrelia burgdorferi (Lyme disease), Bacillus antracis (anthrax), tetanus, streptococcus, staphylococcus, mycobacterium, tetanus, pertissus, cholera, plague, diptheria, chlamydia, S. aureus and legionella. Protozoal diseases that can be treated or prevented using the molecules of the invention in conjunction with the methods of the present invention, that are caused by protozoa include, but are not limited to, leishmania, kokzidioa, trypanosoma or malaria. Parasitic diseases that can be treated or prevented using the molecules of the invention in conjunction with the methods of the present invention, that are caused by parasites include, but are not limited to, chlamydia and rickettsia.

In some embodiments, the heteromultimers of the invention may be administered in combination with a therapeutically or prophylactically effective amount of one or additional therapeutic agents known to those skilled in the art for the treatment and/or prevention of an infectious disease. The invention contemplates the use of the molecules of the invention in combination with other molecules known to those skilled in the art for the treatment and or prevention of an infectious disease including, but not limited to, antibiotics, antifungal agents and anti-viral agents.

The invention provides methods and pharmaceutical compositions comprising heteromultimers of the invention (e.g., antibodies, polypeptides). The invention also provides methods of treatment, prophylaxis, and amelioration of one or more symptoms associated with a disease, disorder or infection by administering to a subject an effective amount of at least one Heteromultimer of the invention, or a pharmaceutical composition comprising at least one Heteromultimer of the invention. In a one aspect, the Heteromultimer, is substantially purified (i.e., substantially free from substances that limit its effect or produce undesired side-effects this includes homodimers and other cellular material). In a specific embodiment, the subject is an animal, such as a mammal including non-primates (e.g., cows, pigs, horses, cats, dogs, rats etc.) and primates (e.g., monkey such as, a cynomolgous monkey and a human). In a specific embodiment, the subject is a human. In yet another specific embodiment, the antibody of the invention is from the same species as the subject.

The route of administration of the composition depends on the condition to be treated. For example, intravenous injection may be preferred for treatment of a systemic disorder such as a lymphatic cancer or a tumor that has metastasized. The dosage of the compositions to be administered can be determined by the skilled artisan without undue experimentation in conjunction with standard dose-response studies. Relevant circumstances to be considered in making those determinations include the condition or conditions to be treated, the choice of composition to be administered, the age, weight, and response of the individual patient, and the severity of the patient's symptoms. Depending on the condition, the composition can be administered orally, parenterally, intranasally, vaginally, rectally, lingually, sublingually, buccally, intrabuccally and/or transdermally to the patient.

Accordingly, compositions designed for oral, lingual, sublingual, buccal and intrabuccal administration can be made without undue experimentation by means well known in the art, for example, with an inert diluent or with an edible carrier. The composition may be enclosed in gelatin capsules or compressed into tablets. For the purpose of oral therapeutic administration, the pharmaceutical compositions of the present invention may be incorporated with excipients and used in the form of tablets, troches, capsules, elixirs, suspensions, syrups, wafers, chewing gums, and the like.

Tablets, pills, capsules, troches and the like may also contain binders, recipients, disintegrating agent, lubricants, sweetening agents, and/or flavoring agents. Some examples of binders include microcrystalline cellulose, gum tragacanth and gelatin. Examples of excipients include starch and lactose. Some examples of disintegrating agents include alginic acid, cornstarch, and the like. Examples of lubricants include magnesium stearate and potassium stearate. An example of a glidant is colloidal silicon dioxide. Some examples of sweetening agents include sucrose, saccharin, and the like. Examples of flavoring agents include peppermint, methyl salicylate, orange flavoring, and the like. Materials used in preparing these various compositions should be pharmaceutically pure and non-toxic in the amounts used.

The pharmaceutical compositions of the present invention can be administered parenterally, such as, for example, by intravenous, intramuscular, intrathecal and/or subcutaneous injection. Parenteral administration can be accomplished by incorporating the compositions of the present invention into a solution or suspension. Such solutions or suspensions may also include sterile diluents, such as water for injection, saline solution, fixed oils, polyethylene glycols, glycerine, propylene glycol and/or other synthetic solvents. Parenteral formulations may also include antibacterial agents, such as, for example, benzyl alcohol and/or methyl parabens, antioxidants, such as, for example, ascorbic acid and/or sodium bisulfite, and chelating agents, such as EDTA. Buffers, such as acetates, citrates and phosphates, and agents for the adjustment of tonicity, such as sodium chloride and dextrose, may also be added. The parenteral preparation can be enclosed in ampules, disposable syringes and/or multiple dose vials made of glass or plastic. Rectal administration includes administering the composition into the rectum and/or large intestine. This can be accomplished using suppositories and/or enemas. Suppository formulations can be made by methods known in the art. Transdermal administration includes percutaneous absorption of the composition through the skin. Transdermal formulations include patches, ointments, creams, gels, salves, and the like. The compositions of the present invention can be administered nasally to a patient. As used herein, nasally administering or nasal administration includes administering the compositions to the mucous membranes of the nasal passage and/or nasal cavity of the patient.

The pharmaceutical compositions of the invention may be used in accordance with the methods of the invention for preventing, treating, or ameliorating one or more symptoms associated with a disease, disorder, or infection. It is contemplated that the pharmaceutical compositions of the invention are sterile and in suitable form for administration to a subject.

In one embodiment the compositions of the invention are pyrogen-free formulations that are substantially free of endotoxins and/or related pyrogenic substances. Endotoxins include toxins that are confined inside a microorganism and are released when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, it is advantageous to remove even low amounts of endotoxins from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight, as can be the case with monoclonal antibodies, it is advantageous to remove even trace amounts of endotoxin. In a specific embodiment, endotoxin and pyrogen levels in the composition are less then 10 EU/mg, or less then 5 EU/mg, or less then 1 EU/mg, or less then 0.1 EU/mg, or less then 0.01 EU/mg, or less then 0.001 EU/mg.

The invention provides methods for preventing, treating, or ameliorating one or more symptoms associated with a disease, disorder, or infection, said method comprising: (a) administering to a subject in need thereof a dose of a prophylactically or therapeutically effective amount of a composition comprising one or more heteromultimers and (b) administering one or more subsequent doses of said heteromultimers, to maintain a plasma concentration of the Heteromultimer at a desirable level (e.g., about 0.1 to about 100 µg/ml), which continuously binds to an antigen. In a specific embodiment, the plasma concentration of the Heteromultimer is maintained at 10 µg/ml, 15 µg/ml, 20 µg/ml, 25 µg/ml, 30 µg/ml, 35 µg/ml, 40 µg/ml, 45 µg/ml or 50 µg/ml. In a specific embodiment, said effective amount of Heteromultimer to be administered is between at least 1 mg/kg and 8 mg/kg per dose. In another specific embodiment, said effective amount of Heteromultimer to be administered is between at least 4 mg/kg and 5 mg/kg per dose. In yet another specific embodiment, said effective amount of Heteromultimer to be administered is between 50 mg and 250 mg per dose. In still another specific embodiment, said effective amount of Fc valiant to be administered is between 100 mg and 200 mg per dose.

The present invention also encompasses protocols for preventing, treating, or ameliorating one or more symptoms associated with a disease, disorder, or infection which a heteromultimer is used in combination with a therapy (e.g., prophylactic or therapeutic agent) other than a heteromultimer and/or variant fusion protein. The invention is based, in part, on the recognition that the heteromultimers of the invention potentiate and synergize with, enhance the effectiveness of, improve the tolerance of, and/or reduce the side effects caused by, other cancer therapies, including current standard and experimental chemotherapies. The combination therapies of the invention have additive potency, an additive therapeutic effect or a synergistic effect. The combination therapies of the invention enable lower dosages of the therapy (e.g., prophylactic or therapeutic agents) utilized in conjunction with heteromultimers for preventing, treating, or ameliorating one or more symptoms associated with a disease, disorder, or infection and/or less frequent administration of such prophylactic or therapeutic agents to a subject with a disease disorder, or infection to improve the quality of life of said subject and/or to achieve a prophylactic or therapeutic effect. Further, the combination therapies of the invention reduce or avoid unwanted or adverse side effects associated with the administration of current single agent therapies and/or existing combination therapies, which in turn improves patient compliance with the treatment protocol. Numerous molecules which can be utilized in combination with the heteromultimers of the invention are well known in the art. See for example, PCT publications WO 02/070007; WO 03/075957 and U.S. Patent Publication 2005/064514.

### Industrial Uses

In view of the biophysical stability of both the single domain antigen-binding construct and the heterodimeric Fc region of the heteromultimer, it is contemplated that the heteromultimers described herein can also be used in industrial applications in which single domain antigen-binding construct fragments (such as isolated VₕH) themselves have utility (see de Marco (2011) Microbial Cell factories 10:44). Thus, in one embodiment, the heteromultimer according to the invention may be used to identify and detoxify toxins, as reagents for immunodetection, purification and bioseparation, as crystallography chaperones, or as tools for studying protein aggregation and activity regulation.

### Kits

The present invention provides kits comprising one or more heteromultimers with altered binding affinity to FcγRs and/or C1q and altered ADCC and/or CDC activity that specifically bind to an antigen conjugated or fused to a detectable agent, therapeutic agent or drug, in one or more containers, for use in monitoring, diagnosis, preventing, treating, or ameliorating one or more symptoms associated with a disease, disorder, or infection.

### EXAMPLES

The examples below are given so as to illustrate the practice of this invention. They are not intended to limit or define the entire scope of this invention.

### Example 1: Generation of bivalent monospecific Antibodies with Heterodimer Fc domains.

The genes encoding the antibody heavy chains were constructed via gene synthesis using codons optimized for human/mammalian expression. The sequences were generated from a known Her2/neu binding Ab (Carter P. et al. (1992) Humanization of an anti P185 Her2 antibody for human cancer therapy. Proc Natl Acad Sci 89, 4285.)and the Fc was an IgG1 isotype (SEQ ID NO:1). The final gene products were sub-cloned into the mammalian expression vector pTT5 (NRC-BRI, Canada) (Durocher, Y., Perret, S. & Kamen, A. High-level and high-throughput recombinant protein production by transient transfection of suspension-growing human HEK293-EBNA1 cells. Nucleic acids research 30, E9 (2002)).The mutations in the CH3 domain were introduced via site-directed mutagenesis of the pTT5 template vectors. See *Table 1* and Table 6 and Table 7for a list of the variant CH3 domain mutations made.

In order to estimate the formation of heterodimers and determine the ratio of homodimers vs. heterodimers the two heterodimer heavy chains were designed with C-terminal extensions of different size (specifically, chain A with C-terminal HisTag and chain B with C-terminal mRFP plus StrepTagll). This difference in molecular weight allows differentiation of homodimers vs. heterodimer in non-reducing SDS-PAGE as illustrated in FIGURE 25A.

The HEK293 cells were transfected in exponential growth phase (1.5 to 2 million cells/mL) with aqueous 1mg/mL 25kDa polyethylenimine (PEI, Polysciences) at a PEI:DNA ratio of 2.5:1.(Raymond C. et al. A simplified polyethylenimine-mediated transfection process for large-scale and high-throughput applications. Methods. 55(1):44-51 (2011)). In order to determine the optimal concentration range for forming heterodimers, the DNA was transfected in three separate ratios of the two heavy chains. For example, this was done in 2ml culture volume and transfection DNA, comprised of 5% GFP, 45% salmon sperm DNA, 25% light chain and 25% total heavy chains, where the heavy chain A plasmid (with C-terminal His-Tag) and the heavy chain B plasmid (with C-terminal StrepTagll plus RFP)at 65%/55%/35% or 10%/20%/40%)were sampled at 3 different relative ratios (chain_A(His)/chain_B(mRFP)) of 10%/65%; 20%/55%; 40%/35% (the apparent 1:1 expression ratio of a WT_His/WT_mRFP heterodimer was determined to be close to the DNA ratio 20%/55%). At 4 to 48 hours after transfection in F17 serum-free media (Gibco), TN1 peptone is added to a final concentration of 0.5%. Expressed antibody was analyzed by SDS-PAGE to determine the best ratio of heavy to light chain for optimal heterodimer formation (See figure 25B and C).

A selected DNA ratio, for example 50% light chain plasmid, 25% heavy chain A plasmid, 25% heavy chain B of AZ33 and AZ34, with 5% GFP, and 45% salmon sperm DNA was used to transfect 150mL of cell culture as described above. Transfected cells were harvested after 5-6 days with the culture medium collected after centrifugation at 4000rpm and clarified using a 0.45µm filter.See *Table 2* below, for a list of the percentage of light and heavy chain A and B plasmids used in the scale up transfection assays for each of the antibodies with CH3 mutations generated for further analysis, includingdetermination of purity and melting temperature.

**Table 2:**

| **Variant** | **LC/HCA/HC B** | **Varian t** | **LC/HCA/HC B** | **Varian t** | **LC/HCA/HC B** | **Varian t** | **LC/HCA/HC B** |
|---|---|---|---|---|---|---|---|
| **Wild-Type** | 50%,50% | AZ47 | 50%,25%,25 % | AZ77 | 40%,20%,40 % | AZ98 | 50%,20%,30 % |
| AZ12 | 50%,25%,25 % | AZ48 | 40%,25%,35 % | AZ78 | 50%,20%,30 % | AZ100 | 50%,20%,30 % |
| AZ14 | 50%,25%,25 % | AZ49 | 50%,25%,25 % | AZ79 | 25%,35%,40 % | AZ101 | 50%,20%,30 % |
| AZ15 | 50%,25%,25 % | AZ63 | 50%,20%,30 % | AZ81 | 25%,35%,40 % | AZ106 | 25%,35%,40 % |
| AZ17 | 50%,25%,25 % | AZ64 | 50%,20%,30 % | AZ82 | 50%,20%,30 % | AZ114 | 25%,20%,55 % |
| AZ19 | 50%,25%,25 % | AZ65 | 50%,20%,30 % | AZ83 | 50%,20%,30 % | AZ115 | 25%,20%,55 % |
| AZ20 | 50%,25%,25 % | AZ66 | 50%,20%,30 % | AZ84 | 50%,20%,30 % | AZ122 | 25%,20%,55 % |
| AZ21 | 50%,25%,25 % | AZ67 | 50%,20%,30 % | AZ85 | 50%,25%,25 % | AZ123 | 40%,20%,40 % |
| AZ25 | 50%,25%,25 % | AZ68 | 50%,20%,30 % | AZ86 | 40%,15%,45 % | AZ124 | 40%,20%,40 % |
| AZ29 | 50%,25%,25 % | AZ69 | 50%,20%,30 % | AZ87 | 50%,25%,25 % | AZ129 | 40%,30%,30 % |
| AZ30 | 50%,25%,25 % | AZ70 | 50%,20%,30 % | AZ88 | 50%,25%,25 % | AZ130 | 40%,30%,30 % |
| AZ32 | 50%,25%,25 % | AZ71 | 40%,20%,40 % | AZ89 | 40%,15%,45 % | | |
| AZ33 | 50%,25%,25 % | AZ72 | 40%,20%,40 % | AZ91 | 50%,25%,25 % | | |
| AZ34 | 50%,25%,25 % | AZ73 | 40%,20%,40 % | AZ92 | 40%,20%,40 % | | |
| AZ42 | 50%,25%,25 % | AZ74 | 40%,20%,40 % | AZ93 | 40%,20%,40 % | | |
| AZ44 | 50%,25%,25 % | AZ75 | 40%,20%,40 % | AZ94 | 50%,25%,25 % | | |
| AZ46 | 50%,25%,25 % | AZ76 | 40%,20%,40 % | AZ95 | 50%,20%,30 % | | |

### Example 2: Purification of bivalent monospecific Antibodies with Heterodimer Fc domains.

The clarified culture medium was loaded onto a MabSelect SuRe (GE Healthcare) protein-A column and washed with 10 column volumes of PBS buffer at pH 7.2. The antibody was eluted with 10 column volumes of citrate buffer at pH 3.6 with the pooled fractions containing the antibody neutralized with TRIS at pH 11. The protein was finally desalted using an Econo-Pac 10DG column (Bio-Rad).The C-terminal mRFP tag on the heavy chain B was removed by incubating the antibody with enterokinase (NEB) at a ratio of 1:10,000 overnight in PBS at 25oC. The antibody was purified from the mixture by gel filtration.For gel filtration, 3.5mg of the antibody mixture was concentrated to 1.5mL and loaded onto a Sephadex 200 HiLoad 16/600 200 pg column (GE Healthcare) via an AKTA Express FPLC at a flow-rate of 1mL/min. PBS buffer at pH 7.4 was used at a flow-rate of 1mL/min. Fractions corresponding to the purified antibody were collected, concentrated to ∼1mg/mL and stored at -80°C.

Formation of heterodimers, as compared to homodimers, was assayed using non-reducing SDS-PAGE and mass spectrometry. Protein A purified antibody was run on a 4-12% gradient SDS-PAGE, non-reducing gel to determine the percentage of heterodimers formedprior to enterokinase (EK) treatment (*See,* Figure 26). For mass spectrometry, all Trap LC/MS (ESI-TOF)experiments were performed on an Agilent 1100 HPLC system interfaced with a Waters Q-TOF2 mass spectrometer. Five µg ofgel filtration purified antibody was injected into a Protein MicroTrap (1.0 by 8.0 mm), washed with 1% acetonitrile for 8 minutes, a gradient from 1 to 20% acetonitrile/0.1% formic acid for 2 minutes, then eluted with a 20 to 60% acetonitrile/0.1% formic acid gradient for 20 minutes.Eluate (30-50µL/min) was directed to the spectrometer with spectrum acquired every second (m/z 800 to 4,000).(*See,* Figure 28) Variants having greater than 90% heterodimers were selected for further analysis, with the exception of AZ12 and AZ14 which each had greater than 85% heterodimer formation.

### Example 3: Stability determination of bivalent monospecific antibodies with Heterodimer Fc domains using Differential Scanning Calorimetry (DSC).

All DSC experiments were carried out using a GE VP-Capillary instrument. The proteins were buffer-exchanged into PBS (pH 7.4) and diluted to 0.4 to 0.5mg/mL with 0.137mL loaded into the sample cell and measured with a scan rate of 1°C/min from 20 to 100°C. Data was analyzed using the Origin software (GE Healthcare) with the PBS buffer background subtracted.(*See,* Figure27). See Table 3 for a list of variants tested and a melting temperature determined. See Table 4 for a list of the variants with a melting temperature of 70°C and above and the specific Tm for each variant.

**Table 3: Melting temperature measurements of variant CH3 domains in an IgG1 antibody having 90% or more heterodimer formation compared to homodimer formation**

| **Variant** | **Tm °C** | **Variant** | **Tm °C** | **Variant** | **Tm °C** | **Variant** | **Tm °C** |
|---|---|---|---|---|---|---|---|
| **Wild-Type** | 81 | AZ29 | 70 | AZ63 | 71.5 | AZ87 | 71 |
| Control 1 | 69 | AZ30 | 71 | AZ64 | 74 | AZ88 | 72 |
| Control 2 | 69 | AZ31 | 68 | AZ65 | 73 | AZ89 | 72.5 |
| AZ3 | 65 | AZ32 | 71.5 | AZ66 | 72.5 | AZ91 | 71.5 |
| AZ6 | 68 | AZ33 | 74 | AZ67 | 72 | AZ92 | 71.5 |
| AZ8 | 68 | AZ34 | 73.5 | AZ68 | 72 | AZ93 | 71.5 |
| AZ12 | 77 | AZ38 | 69 | AZ69 | 71 | AZ94 | 73.5 |
| AZ14 | 77 | AZ42 | 70 | AZ70 | 75.5 | AZ95 | 72 |
| AZ15 | 71.5 | AZ43 | 67 | AZ71 | 71 | AZ98 | 70 |
| AZ16 | 68.5 | AZ44 | 71.5 | AZ72 | 70.5 | AZ99 | 69 |
| AZ17 | 71 | AZ46 | 70.5 | AZ73 | 71 | AZ100 | 71.5 |
| AZ18 | 69.5 | AZ47 | 70.5 | AZ74 | 71 | AZ101 | 74 |
| AZ19 | 70.5 | AZ48 | 70.5 | AZ75 | 70 | AZ106 | 74 |
| AZ20 | 70 | AZ49 | 71 | AZ76 | 71.5 | AZ114 | 71 |
| AZ21 | 70 | AZ50 | 69 | AZ77 | 71 | AZ115 | 70 |
| AZ22 | 69 | AZ52 | 68 | AZ78 | 70 | AZ117 | 69.5 |
| AZ23 | 69 | AZ53 | 68 | AZ79 | 70 | AZ122 | 71 |
| AZ24 | 69.5 | AZ54 | 67 | AZ81 | 70.5 | AZ123 | 70 |
| AZ25 | 70.5 | AZ58 | 69 | AZ82 | 71 | AZ124 | 70 |
| AZ26 | 69 | AZ59 | 69 | AZ83 | 71 | AZ125 | 69 |
| AZ27 | 68 | AZ60 | 67 | AZ84 | 71.5 | AZ126 | 69 |
| AZ28 | 69.5 | AZ61 | 69 | AZ85 | 71.5 | AZ129 | 70.5 |
| | | AZ62 | 68 | AZ86 | 72.5 | AZ130 | 71 |

**Table 4: Melting temperature measurements of select variant CH3 domains in an IgG1 antibody**

| **Variant** | **Tm °C** | **Variant** | **Tm °C** | **Variant** | **Tm °C** | **Variant** | **Tm °C** |
|---|---|---|---|---|---|---|---|
| **Wild-Type** | 81.5 | AZ42 | 70 | AZ73 | 71 | AZ91 | 71.5 |
| Control 1 | 69 | AZ44 | 71.5 | AZ74 | 71 | AZ92 | 71.5 |
| Control 2 | 69 | AZ46 | 70.5 | AZ75 | 70 | AZ93 | 71.5 |
| AZ12 | >77 | AZ47 | 70.5 | AZ76 | 71.5 | AZ94 | 73.5 |
| AZ14 | >77 | AZ48 | 70.5 | AZ77 | 71 | AZ95 | 72 |
| AZ15 | 71.5 | AZ49 | 71 | AZ78 | 70 | AZ98 | 70 |
| AZ17 | 71 | AZ63 | 71.5 | AZ79 | 70 | AZ100 | 71.5 |
| AZ19 | 70.5 | AZ64 | 74 | AZ81 | 70.5 | AZ101 | 74 |
| AZ20 | 70 | AZ65 | 73 | AZ82 | 71 | AZ106 | 74 |
| AZ21 | 70 | AZ66 | 72.5 | AZ83 | 71 | AZ114 | 71 |
| AZ25 | 70.5 | AZ67 | 72 | AZ84 | 71.5 | AZ115 | 70 |
| AZ29 | 70 | AZ68 | 72 | AZ85 | 71.5 | AZ122 | 71 |
| AZ30 | 71 | AZ69 | 71 | AZ86 | 72.5 | AZ123 | 70 |
| AZ32 | 71.5 | AZ70 | 75.5 | AZ87 | 71 | AZ124 | 70 |
| AZ33 | 74 | AZ71 | 71 | AZ88 | 72 | AZ129 | 70.5 |
| AZ34 | 73.5 | AZ72 | 70.5 | AZ89 | 72.5 | AZ130 | 71 |

### Example 4: Evaluation of FcgammaR Binding using Surface Plasmon Resonance

All binding experiments were carried out using a BioRad ProteOn XPR36 instrument at 25°C with 10mM HEPES, 150mM NaCl, 3.4mM EDTA, and 0.05% Tween 20 at pH 7.4.Recombinant HER-2/neu(p185, ErbB-2 (eBiosciences, Inc.))was captured on the activated GLM sensorchip by injecting 4.0µg/mL in 10mM NaOAc (pH 4.5) at 25µL/min until approx. 3000 resonance units (RUs) were immobilized with the remaining active groups quenched.40µg/mL of purified anti-HER-2/neu antibodies comprising the variant CH3 domains were indirectly captured on the sensorchip by binding the Her-2/neu protein when injected at 25µL/min for 240s (resulting in approx. 500RUs) following a buffer injection to establish a stable baseline. FcgammaR (CD16a(f allotype) and CD32b) concentrations (6000, 2000, 667, 222, and 74.0nM) were injected at 60µL/min for 120s with a 180s dissociation phase to obtain a set of binding sensograms. Resultant K_{D} values were determined from binding isotherms using the Equilibrium Fit model with reported values as the mean of three independent runs.Comparisons were made with the wild-type IgG1 Fc domain and binding is expressed as a ratio of the WT kD to the variant kD (See, Table 5).

**Table 5: Ratio of kD wild-type IgG1 to variant CH3 domain antibody binding independently to CD16a and CD32b**

| **Variant** | **CD16a Ratio WT/Variant** | **CD32b Ratio WT/Variant** | **Variant** | **CD16a Ratio WT/Variant** | **CD32b Ratio WT/Variant** |
|---|---|---|---|---|---|
| Control 1 | 1.28 | 1.68 | AZ64 | 0.95 | 0.9 |
| Control 2 | 1.1 | 1.13 | AZ65 | 0.93 | 0.9 |
| AZ3 | 1.75 | 1.87 | AZ66 | 1.26 | 1.19 |
| AZ6 | 1.38 | 1 | AZ67 | 1.21 | 1.13 |
| AZ8 | 1.75 | 1.64 | AZ68 | 1.02 | 1.1 |
| AZ12 | N/A | N/A | AZ69 | 0.96 | 1.05 |
| AZ14 | N/A | N/A | AZ70 | 1.06 | 1.11 |
| AZ15 | 0.72 | 0.59 | AZ71 | 0.89 | 0.95 |
| AZ16 | 0.95 | 0.64 | AZ72 | 1.04 | 1.02 |
| AZ17 | 2.28 | 2.37 | AZ73 | 1.09 | 1.07 |
| AZ18 | 1.53 | 1.7 | AZ74 | 1.25 | 1.17 |
| AZ19 | 1.55 | 1.89 | AZ75 | 1.34 | 1.22 |
| AZ20 | 2.56 | 1.93 | AZ76 | 0.99 | 1 |
| AZ21 | 2.41 | 3.28 | AZ77 | 1 | 1.08 |
| AZ22 | 2.02 | 2.37 | AZ78 | 0.9 | 1 |
| AZ23 | 1 | 2.16 | AZ79 | 1.01 | 0.8 |
| AZ24 | 1.79 | 2.26 | AZ81 | 1.01 | 0.84 |
| AZ25 | 2.02 | 2.37 | AZ82 | 0.97 | 0.94 |
| AZ26 | 2.38 | 2.59 | AZ83 | 0.94 | 0.94 |
| AZ27 | 2.27 | 2.38 | AZ84 | 0.93 | 1 |
| AZ28 | 1.45 | 2.15 | AZ85 | 1.01 | 1.14 |
| AZ29 | 1.62 | 2.13 | AZ86 | 1.22 | 1.18 |
| AZ30 | 1.61 | 2.38 | AZ87 | 1.03 | 1.1 |
| AZ31 | 1.63 | 2.29 | AZ88 | 1.11 | 1.15 |
| AZ32 | 1.82 | 2.48 | AZ89 | 1.12 | 1.24 |
| AZ33 | 1.91 | 1.89 | AZ91 | 1.11 | 1.11 |
| AZ34 | 1.88 | 1.88 | AZ92 | 1.21 | 1.24 |
| AZ38 | 1.78 | 1.44 | AZ93 | 1.21 | 1.18 |
| AZ42 | 1.28 | 1.09 | AZ94 | 1.17 | 1.19 |
| AZ43 | 1.63 | 1.73 | AZ95 | 0.86 | 0.96 |
| AZ44 | 2.76 | 3.07 | AZ98 | 0.79 | 0.82 |
| AZ46 | 2.16 | 2.66 | AZ99 | 1.16 | 1.15 |
| AZ47 | 1.76 | 2.12 | AZ100 | 1.13 | 1.12 |
| AZ48 | 2.02 | 1.59 | AZ101 | 1.24 | 1.23 |
| AZ49 | 2.09 | 2.9 | AZ106 | 0.76 | 0.64 |
| AZ50 | 2.33 | 1.86 | AZ114 | 1.3 | 0.84 |
| AZ52 | 1.55 | 1.5 | AZ115 | 1.13 | 0.82 |
| AZ53 | 1.87 | 1.27 | AZ117 | 0.89 | 1 |
| AZ54 | 1.36 | 1.64 | AZ122 | 0.89 | 0.92 |
| AZ58 | 2.33 | 1.48 | AZ123 | 0.85 | 0.92 |
| AZ59 | 1.18 | 1.57 | AZ124 | 0.99 | 1.09 |
| AZ60 | 1.51 | 1.23 | AZ125 | 1 | 1 |
| AZ61 | 1.41 | 1.75 | AZ126 | 0.86 | 0.9 |
| AZ62 | 1.53 | 1.88 | AZ129 | 1.91 | 2.57 |
| AZ63 | 0.9 | 0.95 | AZ130 | 1.91 | 2.54 |

### Example 5: Rational design of heteromultimers using Fc_CH3 engineering - Scaffold 1 (1a and 1b) and the development of AZ17-62 and AZ133-AZ2438

To improve the initial AZ8 for stability and purity, the structural and computational strategiesdescribed above were employed. (*See,* Figure 24) For example, the in depth structure-function analysis of AZ8 provided a detailed understanding for each of the introduced mutations of AZ8,L351Y_V397S_F405A_Y407V / K392V_T394Wcompared to wild-type human IgG1 and indicated that the important core heterodimer mutations were L351Y_F405A_Y407V / T394W,while V397S, K392V were not relevant for heterodimer formation. The core mutations (L351Y_F405A_Y407V / T394W) are herein referred to as "Scaffold 1" mutations. The analysis furthermore revealed that the important interface hotspots that are lost with respect to wild-type (WT) homodimer formation are the interactions of WT-F405-K409, Y407-T366 and the packing of Y407-Y407 and -F405 (See, Figure 29). This was reflected in the packing, cavity and MD analysis, which showed a large conformational difference in the loop region D399-S400-D401 (*See,* Figure 30) and the associated β-sheets at K370. This resulted in the loss of the interchain interactions K409-D399 (See, Figure 30) and weakening of the strong K370 hydrogen bond to E357 (K370 is no longer in direct contact with S364 and E357, but is entirely solvent exposed). In the WT IgG1 CH3 domain theseregions tether the interface at the rim protects the core interactions from bulk solvent competition and increases the dynamic occurrence of favorable hydrophobic van der Waals interactions. The consequence was a lower buried surface areaof AZ8 compared to WT and a higher solvent accessibility of the hydrophobic core.This indicated the most important factors for the lower stability of AZ8 compared to WT stability was a) the loss of the WT-F405-K409 interaction and packing of F405, and b) the loss of the strong packing interaction of Y407-Y407 and Y407-T366. See, Figure 29

Consequently, we identified the key residues/sequence motifs responsible for the low stability of AZ8compared to WT. To improve the stability and heterodimer specificity of AZ8the subsequent positive design engineering efforts were therefore specifically focused on stabilizing the loop conformation of positions 399-401 in a more 'closed' - WT like conformation(*See,* Figure 30) and compensating for the overall slightly decreased(looser) packing of the hydrophobic core at positions T366 and L368 (See, Figure 29).

To achieve this stabilization of the loop conformation of positions 399-401 the described computational approach was used to evaluate our different targeted design ideas. Specifically, three different independent options for Heteromultimer AZ8were analyzed to optimize the identified key regions for improving stability. First, the cavity close to position K409 and F405A was evaluated for better hydrophobic packing to both protect the hydrophobic core and stabilize the loop conformation of 399-400 (*See,* Figure 30). Those included, but were not limited to additional point mutations at positions F405 and K392. Second, options for improving the electrostatic interactions of positions 399-409 were evaluated, to stabilize the loop conformation of 399-400 and protect the hydrophobic core. This included, but was not limited to additional point mutations at positions T411 and S400. Third, the cavity at the core packing positions T366, T394W and L368 was evaluated to improve the core hydrophobic packing (*See,* Figure29). Those included, but were not limited to additional point mutations at positions T366 and L368. The different independent positive design ideas were tested in-silico and the best-ranked variants using the computational tools(AZ17-AZ62) were validated experimentally for expression and stability as described in Examples 1-4. See Table 4 for a list of heteromultimers from this design phase with a melting temperature of 70°C or greater.

Heteromultimer AZ33is an example of the development of an Heteromultimer wherein Scaffold 1was modified resulting in Scaffold 1a mutations to improve stability and purity. This Heteromultimer was designed based on AZ8 with the goalimproving the hydrophobic packing at positions 392-394-409 and 366 to both protect the hydrophobic core and stabilize the loop conformation of 399-400. This Heteromultimer AZ33 heterodimer has two additional point mutations different from the core mutations of AZ8, K392M and T366I. The mutations T366I_K392M_T394W/F405A_Y407V are herein referred to as "Scaffold 1a" mutations. The mutation K392M was designed to improve the packing at the cavity close to position K409 and F405A to protect the hydrophobic core and stabilize the loop conformation of 399-400 (See, Figure 31). T366I was designed to improve the core hydrophobic packing and to eliminate the formation of homodimers of the T394W chain (*See,* Figure29). The experimental data for AZ33 showed significantly improved stability over the initial negative design Heteromultimer AZ8 (Tm 68°C) wherein AZ33 has a Tm of 74°C and a heterodimer content of >98%. (*See*, Figure 25C)

### Development of heteromultimers using Scaffold 1 mutations in phase three design of Heteromultimer heterodimers

Although AZ33 provides a significant stability and specificity (or purity) improvement over the initial starting variant AZ8, our analysis indicates that further improvements to the stability of the heterodimer can be made with further amino acid modifications using the experimental data of AZ33 and the above described design methods. The different design ideas have been independently tested for expression and stability, but the independent design ideas are transferable and the most successful heterodimer will contain a combination of the different designs. Specifically, for the optimization of AZ8 packing mutations at the cavity close to K409-F405A-K392 have been evaluated independently from mutations that optimize the core packing at residues L366T-L368. These two regions 366-368 and 409-405-392 are distal from each other and are considered independent. Heteromultimer AZ33 for example has been optimized for packing at 409-405-392, but not at 366-368, because these optimization mutations were separately evaluated. The comparison of the 366-368 mutations suggests that T366L has an improved stability over T366 and also T366I, the point mutation used in the development of Heteromultimer AZ33. Consequently, the presented experimental data immediately suggest further optimization of AZ33 by introducing T366L instead of T366I, for example. Therefore, the amino acid mutations in the CH3 domain T366L_K392M_T394W/F405A_Y407V are herein referred to as "Scaffold 1b" mutations.

In a similar manner the complete experimental data has been analyzed to identify point mutations that can be used to further improve the current Fc variant heterodimer AZ33. These identified mutations were analyzed by the above described computational approach and ranked to yield the list of additional Fc variant heterodimers based on AZ33 as shown in Table 6.

### Example 6: Rational design of heteromultimers using Fc_CH3 engineering - Scaffold 2 (a and b) and, the development of AZ63-101 and AZ2199-AZ2524

To improve the initial negative design phase Heteromultimer AZ15 for stability and purity, the structural and computational strategies described above were employed (*See,* Figure 24). For example, the in depth structure-function analysis of Heteromultimer AZ15 provided a detailed understanding for each of the introduced mutations of AZ15, L351Y_Y407A / E357L_T366A_K409F_T411N compared to wild-type (WT) human IgG1 and indicated that the important core heterodimer mutations were L351Y_Y407A / T366A_K409F, while E357L, T411N were not directly relevant for heterodimer formation and stability. The core mutations (L351Y_Y407A / T366A_K409F) are herein referred to as "Scaffold 2" mutations. The analysis furthermore revealed that the important interface hotspots that are lost with respect to wild-type (WT) homodimer formation are the salt bridge D399-K409, the hydrogen bond Y407-T366 and the packing of Y407-Y407. Our detailed analysis, provided below, describes how we improved the stability of our original Heteromultimer AZ15 and the positions and amino acid modifications made to achieve these heteromultimers with improved stability.

### Development of heteromultimers using Scaffold 2 mutations and the further development of Scaffold 2a mutations.

Our in-silico analysis indicated a non-optimal packing of the Heteromultimer AZ15 mutations K409F_T366A_Y407A and an overall decreased packing of the hydrophobic core due to the loss of the WT-Y407-Y407 interactions. The positive design efforts in the subsequent engineering phase were focused on point mutations to compensate for these packing deficits in the initial Fc variant AZ15. The targeted residues included positions T366, L351, and Y407. Different combinations of these were tested in-silico and the best-ranked Fc variants using the computational tools (AZ63-AZ70) were validated experimentally for expression and stability as described in Examples 1-4.

Fc variant AZ70 is an example of the development of a Heteromultimer wherein Scaffold 2 was modified resulting in Scaffold 2a mutations to improve stability and purity. This Heteromultimer was designed based on AZ15 with the goal of achieving better packing at the hydrophobic core as described above. Heteromultimer AZ70 has the same Scaffold 2 core mutations (L351Y_Y407A / T366A_K409F) as described above except that T366 was mutated to T366V instead of T366A (FIGURE 33). The L351Y mutation improves the 366A_409F/407A variant melting temperature from 71.5°C to 74°C, and the additional change from 366A to 366V improves the Tm to 75.5°C. (*See,* AZ63, AZ64 and AZ70 in Table 4, with a Tm of 71.5°C, 74°C and 75.5°C, respectively) The core mutations (L351Y_Y407A / T366V_K409F) are herein referred to as "Scaffold 2a" mutations. The experimental data for Fc variant AZ70 showed significantly improved stability over the initial negative design Fc variant AZ15 (Tm 71°C) wherein AZ70 has a Tm of 75.5°C and a heterodimer content of >90% (FIGURE 33 and 27).

### Development of heteromultimers using Scaffold 2 mutations and the further development of Scaffold 2b mutations.

The Molecular Dynamics simulation (MD)and packing analysis showed a preferred more 'open' conformation of the loop 399-400, which was likely due to the loss of the WT salt bridge K409-D399. This also results in the unsatisfied D399, which in turn preferred a compensating interaction with K392 and induced a more 'open' conformation of the loop. This more 'open' loop conformation results in an overall decreased packing and higher solvent accessibility of the core CH3 domain interface residues, which in turn significantly destabilized the heterodimer complex. Therefore, one of the targeted positive design efforts was the tethering of this loop in a more 'closed', WT-like conformation by additional point mutations that compensate for the loss of the D399-K409 salt bridge and the packing interactions of K409. The targeted residues included positions T411, D399, S400, F405, N390, K392 and combinations thereof. Different packing, hydrophobic- and electrostatic positive engineering strategies were tested in silico with respect to the above positions and the best-ranked heteromultimers determined using the computational tools (AZ71-AZ101) were validated experimentally for expression and stability as described in Examples 1-4.

Heteromultimer AZ94 is an example of the development of an Fc variant wherein Scaffold 2 is modified resulting in Scaffold 2b mutations along with additional point mutations to improve stability and purity. This Fc variant was designed based on AZ15 with the goal of tethering loop 399-400 in a more 'closed', WT-like conformation and compensating for the loss of the D399-K409 salt bridge as described above. Fc variant AZ94 has four additional point mutations to Scaffold 2 (L351Y_Y407A / T366A_K409F) and returns L351Y to wild-type L351 leaving (Y407A / T366A_K409F) as the core mutations for this Fc variant. The core mutations Y407A / T366A_K409F are herein referred to as "Scaffold 2b" mutations. The four additional point mutations of AZ94 are K392E_T411E / D399R_S400R. The mutations T411E / D399R were engineered to form an additional salt bridge and compensate for the loss of the K409 / D399 interaction (FIGURE 34). Additionally, this salt bridge was designed to prevent homodimer formation by disfavoring charge-charge interactions in both potential homodimers. The additional mutations K392E / S400R were intended to form another salt bridge and hence further tether the 399_400 loop in a more 'closed', WT-like conformation (FIGURE 34). The experimental data for AZ94 showed improved stability and purity over the initial negative design Fc variant AZ15 (Tm 71°C, >90% purity) wherein Fc variant AZ94 has a Tm of 74°C and a heterodimer content or purity of >95% .

### Development of heteromultimers using Scaffold 2 mutations in phase three design of heterodimers

Both Fc variants AZ70 and AZ94 provide a significant improvement in stability and purity over the initial negative design Fc variant AZ15, but our analysis and the comparison of AZ70 and AZ94 directly indicate that further improvements to the stability of the Fc variant heterodimer can be made with further amino acid modifications. For example, Fc variants AZ70 and AZ94 were designed to target two distinct non-optimized regions in the initial variant AZ15, which was accomplished by improving the packing at the hydrophobic core and making mutations outside of the core interface residues resulting in additional salt bridges and hydrogen bonding to stabilize the loop conformation of positions 399-401. The additional point mutations of Fc variants AZ70 and AZ94 are distal from each other and are therefore independent and transferable to other Fc variants designed around the same Scaffold 2 core mutations, including 2a and 2b mutations. Specifically, AZ70 only carries the optimized core mutations L351Y_Y407A / T366A_K409F, but no additional salt bridges, whereas AZ94 comprises four additional electrostatic mutations (K392E_T411E / D399R_S400R), but has one less mutation in the hydrophobic core interface (Y407A / T366A_K409F). These Scaffold 2b mutations are less stable than AZ70 (See, for example AZ63, which has equivalent core mutations as AZ94 and Tm of 72°C), but are compensated for by the addition of K392E_T411E / D399R_S400R mutations. The presented experimental stability and purity data indicates that combining the mutations of AZ70, which optimizes the hydrophobic core, and the electrostatic mutations of AZ94 should further improve stability and purity of the heterodimers that comprise the Fc variants. In a similar manner the complete experimental data for Scaffold 2 Fc variants (AZ63-101) has been analyzed to identify point mutations that can be used to further improve the Fc variant heterodimers AZ70 and AZ94. These identified mutations were further analyzed by the above described computational approach and ranked to yield the list of additional Fc variant heterodimers based on AZ70 and AZ94 as shown in Table 7.

### Example 7:Effect of Heterodimeric CH3 on FcgR binding

As a prototypical example of heterodimeric Fc activity with FcgR, we have tested two variant antibodies with heterodimeric Fc region A:K409D_K392D/B:D399K_D356K (Control 1 (het 1 in Figure 35)) and A:Y349C_T366S_L368A_Y407V/B:S354C_T366W (Control 4 (het 2 in Figure 35)) with Her2 binding Fab arms in an SPR assay described in Example 4 for FcgR binding. As shown in Figure 35, we observe that both the heterodimeric Fc regions bind the different Fcgamma receptors with the same relative strength as the wild type IgG1 Fc region, but overall, the heterodimeric Fc region bound each of the FcgR's slightly better than the wild type antibody. This indicates that mutations at the CH3 interface of Fc can impact the binding strength of the Fc region for Fcgamma receptors across the CH2 domains as observed in our molecular dynamics simulations and analysis.

### Example 8: Effect of Asymetric mutations in CH2 of a heterodimeric Fc on FcgR binding

Mutation of Serine at position 267 in the CH2 domain of the Fc region to an Aspartic acid (S267D) is known to enhance binding to Fcgamma IIbF, IlbY & IIaR receptors when introduced in a homodimeric manner in the two chains of CH2 domain. This mutation can be introduced on only one of the CH2 domains in an heterodimeric Fc molecule to gain roughly half the improvement in binding strength relative to when this mutation is introduced in a homodimeric CH2 Fc as the data presented in Figure 36A indicates. On the other hand, the E269K mutation in a homodimeric CH2 domain of Fc prevents binding of the Fc region to FcgR. We present a scheme for enhanced manipulation of the binding strength of the Fc region for the FcgRecptors by the asymmetric introduction of these favorable and unfavorable mutations on one of the two chains in the CH2 domain of the Fc. The introduction of E269K mutation in an asymmetric manner on one CH2 chain in a heterodimeric Fc acts as a polarity driver by blocking binding of the FcgR at the face where it is present, while letting the other face of the Fc interact with the FcgR in a normal manner. The results from this experimentation are presented in Figure 36A. The opportunity to selectively alter the binding strength via both the chains of Fc in an independent manner provides increased opportunity to manipulate the binding strength and selectivity between Fc and FcgRecptors. Thus, such asymmetric design of mutations in the CH2 domain allows us to introduce positive and negative design strategies to favor or disfavor certain binding models, providing greater opportunity to introduce selectivity.

In a subsequent experiment, we have altered the selectivity profile of the base Fc mutant S239D_D265S_I332E_S298A that shows increased binding strength to the Fcgamma IIIaF and IIIaV receptors while continuing to exhibit weaker binding to the Fcgamma IIaR, IIbF and IIbY receptors. This is shown in the binding profile shown in Figure 36B. By introducing asymmetric mutations E269K in chain A and avoiding the I332E mutation in chain B, we are able to generate a novel FcgR binding profile that further weakens IIa and IIb receptor binding and makes the Fc more specific for the IIIa receptor binding.

In another example shown in Figure 36C, asymmetric mutations are highlighted relative to the homodimeric Fc involving the mutation S239D/K326E/A330L/I332E/S298A in the CH2 domain. Relative to the wild type IgG1 Fc, this variant show increased binding to the IIIa receptor but also binds the IIa and IIb receptors slightly stronger than the wild type Fc. Introduction of these mutations in an asymmetric manner A:S239D/K326E/A330L/I332E and B:S298A while reducing the IIIa binding, also increases the IIa/IIb receptor binding, loosing selectivity in the process. By introducing an asymmetirc E269K mutation in this heterodimeric variant, i.e. A:S239D/K326E/A330L/I332E/E269K and B:S298A, we are able to reduce the IIa/IIb binding back to wild type levels. This highlights the fact that the use of asymmetric mutations in the CH2 domain of Fc is able to provide significant opportunity to design improved FcgammaR selectivity.

The reagents employed in the examples are commercially available or can be prepared using commercially available instrumentation, methods, or reagents known in the art. The foregoing examples illustrate various aspects of the invention and practice of the methods of the invention. The examples are not intended to provide an exhaustive description of the many different embodiments of the invention

### Example 9: FcRn binding determined by SPR.

Binding to FcRn was determined by SPR in two different orientations.
1. Flowing of the heterodimer variant over immobilzed FcRn: In this experiment, high density surfaces aprox 5000 RUs were made using standard NHS/EDC coupling. 100nM of WT and each variant was injected in triplicate at 50 uL min for 120s with 600s dissociation in MES pH6 running buffer.
2. Flowing of FcRn over indirectly captured heterodimer variants: In this SPR experiment, a goat anti-human IgG surface was used to indirectly capture the antibodies (approximately 400RUs each), followed by an injection of a 3-fold FcRn dilution series (6000nM high conc). Running buffer was 10mM MES / 150mM NaCl / 3.4 mM EDTA/0.05 Tween20 at pH6. There was no significant binding of FcRn to the goat polyclonal surface. All variants show similar to WT sensograms. Table 8 below shows the Kd determined by the indirect immobilization with flowing FcRn (2.).

| **Kd [M]** - **pH6.0** | **Kd [M]** - **pH7.5** | **Mutations (Chain-A)** | **Mutations (Chain-B)** |
|---|---|---|---|
| 3.7E-06 | - | Herceptin WT | |
| 4.E-06 | - | L351Y_F405A_Y407V | T366I_K392M_T394W |
| 5.E-06 | - | L351Y_F405A_Y407V | T366L_K392M_T394W |
| 4.3E-06 | - | T350V_L351Y_F405A_Y407V | T350V_T366L_K392M_T394W |
| 4.1E-06 | - | Y349C_T350V_F405A_Y407V | T350V_S354C_T366L_K392M_T394W |
| 5.E-06 | - | T350V_L351Y_S400E_F405A_Y407V | T350V_T366L_N390R_K392M_T394W |
| 3.9E-06 | - | T350V_L351Y_F405A_Y407V | T350V_T366L_K392L_T394W |

### Example 10: Preparation of an exemplary heteromultimers

The following heteromultimer comprising one single domain antigen-binding construct was prepared:
1. v1323, a monovalent anti-EGFR antibody (EG2), where the EGFR binding domain is a camelid VₕH on chain A, and the Fc region is a heterodimer having the mutations T350V_L351Y_F405A_Y407V in Chain A, and T350V_T366L_K392L_T394W in Chain B

This construct was prepared and expressed as follows.

The genes encoding the antibody heavy chains and Fc regions were constructed via gene synthesis using codons optimized for human/mammalian expression. The sdAb sequence encoding the anti-EGFR sdAb was generated from a known EGFR binding antibody EG2 (Bell et al. (2010) Differential tumor-targeting abilities of three single-domain antibody formats. Cancer Letters 289:81).

The final gene products were sub-cloned into the mammalian expression vector pTT5 (NRC-BRI, Canada) and expressed in CHO cells (Durocher, Y., Perret, S. & Kamen, A. High-level and high-throughput recombinant protein production by transient transfection of suspension-growing CHO cells. Nucleic acids research 30, E9 (2002)).

The CHO cells were transfected in exponential growth phase (1.5 to 2 million cells/mL) with aqueous 1mg/mL 25kDa polyethylenimine (PEI, Polysciences) at a PEI:DNA ratio of 2.5:1.(Raymond C. et al. A simplified polyethylenimine-mediated transfection process for large-scale and high-throughput applications. Methods. 55(1):44-51 (2011)). The DNA was transfected in optimal DNA ratios of the chain A (CHA) and chain B (CHB) to allow for heterodimer formation (e.g. CHA/CHB ratio = 50:50) Transfected cells were harvested after 5-6 days with the culture medium collected after centrifugation at 4000rpm and clarified using a 0.45µm filter.

### Example 11: Purification and characterization of an exemplary heteromultimer

The heteromultimer described in Example 1 was expressed and purified by protein A chromatography as described below.

### Protein A purification

The clarified culture medium was loaded onto a MabSelect SuRe (GE Healthcare) protein-A column and washed with 10 column volumes of PBS buffer at pH 7.2. The antibody was eluted with 10 column volumes of citrate buffer at pH 3.6 with the pooled fractions containing the antibody neutralized with TRIS at pH 11.

### UPLC-SEC analysis of heterodimer purity post Protein-A purification

The purity of v1323 was determined using UPLC-SEC under standard conditions described below:
Column: Waters BEH200 SEC, 1.7 µm particles, 4.6 x 150 mm
Solvent: 25 mM NaPO4, 150 mM NaCl, pH 7.00 @ 23.4°C, 20.00 mS/cm @ 23.4°C
Flow rate: 0.4 ml/min, ∼4280 psi
Temperature: 30°C
Samples: v1323 120607-KB
   3 injections (∼2 µg per injection)

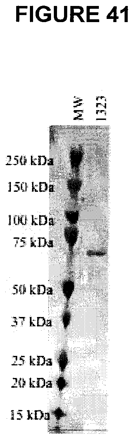

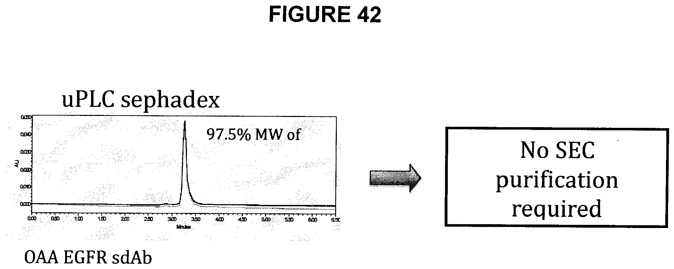

Figures 41 and 42 depict the results of SDS-PAGE and UPLC-SEC analysis, respectively, for the exemplary heteromultimer after Protein-A purification. Figure 41 illustrates the relative purity post Protein-A purification and shows that v1323 contained no detectable contaminant species and did not require additional purification by SEC. Figure 42 contains UPLC-SEC analysis that supports the observations in Figure 41, illustrating that e.g. 1323 is > 97% heterodimer purity post Protein-A purification.

Table 9 provides a summary of the purification procedure and yield for v1323.

**Table 9: Yield and Purification process for v1323**

| **variant** | **purification** | **Conc. mg/ml** | **Total mg per 50ml** |
|---|---|---|---|
| 1323 | protA | 1.3 | 1.95 (1.5 ml) |

### Example 12: Heteromultimers maintain EGFR-ED binding domain properties

The ability of v1323 comprising an EGFR binding domain to bind to the extracellular domain (ED) of EGFR was tested by Surface Plasmon Resonance (SPR) using a ProteOn XPR36 system from BIO-RAD.

Approximately 3000 RU of anti-human IgG 25ug/ml was immobilized on a GLC chip using standard amine coupling. Exemplary SDACs were captured on the anti-human IgG immobilized chip to capture level of approximately 700 RU. Recombinant human EGFR-ED was diluted in running buffer and injected at a flow rate of 50 µl /min for 2 minutes, followed by dissociation for another 4 minutes. Sensograms were fit globally to a 1: 1 Langmuir binding model. All experiments were conducted at room temperature.

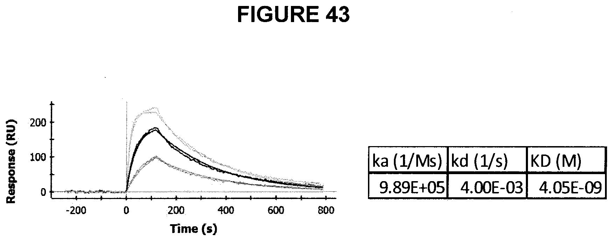

The SPR curves for v1323 are shown in Figure 43. The results show that v1323 binds to EGFR-ED with high affinity in the low nanomolar range between 3-4.5 nM.

### Example 13: Ability of v1323 to bind to A549, BxPc3 and U87 cells

The ability of v1323 to bind to EGFR expressed on the surface of a cell was tested using A549, BxPc3 and U87 cells by a fluorescent cell binding assay as described below.

Binding of the exemplary bi-specific SDACs to the surface of A549, BxPc3 and U87 cells was determined by flow cytometry. Cells were washed with PBS and resuspended in DMEM at 1X105 cells/ 100µl. 100µl cell suspension was added into each microcentrifuge tube, followed by 10µl/ tube of the antibody variants. The tubes were incubated for 2hr 4°C on a rotator. The microcentrifuge tubes were centrifuged for 2min 2000RPM at room temperature and the cell pellets washed with 500µl media. Each cell pellet was resuspended 100µl of fluorochrome-labelled secondary antibody diluted in media to 2µg/ sample. The samples were then incubated for 1hr at 4°C on a rotator. After incubation, the cells were centrifuged for 2 min at 2000 RPM and washed in media. The cells were resuspended in 500 µl media, filtered in tube containing 5µl propidium iodide (PI) and analyzed on a BD LSRII flow cytometer according to the manufacturer's instructions.

The results of this experiment indicated that v1323 was able to bind to the cells tested (data not shown).

## Claims

1. An isolated heteromultimer comprising an immunoglobulin heterodimer Fc region comprising a first monomeric Fc polypeptide and a second monomeric Fc polypeptide and at least one single domain antigen-binding construct attached to one monomeric Fc polypeptide,
wherein the isolated heteromultimer is devoid of immunoglobulin light chains;
wherein the heterodimer Fc region comprises a variant CH3 region comprising amino acid mutations that promote the formation of the heterodimer Fc region as compared to a homodimeric Fc region, the amino acid mutations comprising:
(a) an amino acid mutation at position F405 and the amino acid mutations L351Y and Y407V in the first monomeric Fc polypeptide, and the amino acid mutation T394W in the second monomeric Fc polypeptide, wherein the amino acid mutation at position F405 is F405A, F405I, F405M, F405T, F405S, F405V or F405W, or
(b) amino acid mutations F405A and Y407V in the first monomeric Fc polypeptide, and amino acid mutations T366L or T366I and T394W in the second monomeric Fc polypeptide, or
(c) amino acid mutations L351Y, F405A and Y407V in the first monomeric Fc polypeptide, and an amino acid mutation at position T366 and the amino acid mutation T394W in the second monomeric Fc polypeptide, wherein the amino acid mutation at position T366 is T366A, T366I, T366L, T366M, T366Y, T366S, T366C, T366V or T366W; or
(d) the amino acid mutation Y407A in the first monomeric Fc polypeptide, and the amino acid mutation K409F and one of the amino acid mutations T366A, T366C, T366L, T366M or T366S in the second monomeric Fc polypeptide;
(e) the amino acid mutations L351Y and Y407A in the first monomeric Fc polypeptide, and the amino acid mutation K409F and one of the amino acid mutations T366A or T366V in the second monomeric Fc polypeptide; or
(f) the amino acid mutations L351F and Y407A in the first monomeric Fc polypeptide, and the amino acid mutations T366A and K409F in the second monomeric Fc polypeptide;
wherein the heterodimer Fc region is based on a human type G immunoglobulin (IgG);
wherein the variant CH3 domain has a melting temperature (Tm) of 74°C or greater, and the heterodimer Fc region has a purity greater than about 90%;
wherein the single domain antigen-binding construct is a single domain antibody (sdAb), a camelid immunoglobulin single variable domain (VhH), a cartilaginous fish antibody fragment (V_{NAR}), an SH3-derived fynomer or a fibronectin-derived binding domain, and
wherein the numbering of amino acids is according to the EU index as set forth in Kabat.

2. The isolated heteromultimer according to claim 1(a), wherein the amino acid mutations further comprise an amino acid mutation at position K392 in the second monomeric Fc polypeptide, wherein the amino acid mutation at position K392 is K392L, K392M, K392F, K392V, K392R or K392E.

3. The isolated heteromultimer according to claim 1(a) or 2, wherein the amino acid mutations further comprise an amino acid mutation at position T366 in the second monomeric Fc polypeptide, wherein the amino acid mutation at position T366 is T366A, T366I, T366L, T366M, T366Y, T366S, T366C, T366V or T366W.

4. The isolated heteromultimer according to claim 1(c), wherein the amino acid mutation at position T366 is T366I or T366L.

5. The isolated heteromultimer according to claim 1(b), wherein the amino acid mutations further comprise the amino acid mutation K392M in the second monomeric Fc polypeptide.

6. The isolated heteromultimer according to claim 1, wherein:
a) the first monomeric Fc polypeptide comprises the amino acid mutations L351Y, F405A and Y407V, and the second monomeric Fc polypeptide comprises the amino acid mutations T366L and T394W, and further comprises the amino acid mutation K392M; or
b) the first monomeric Fc polypeptide comprises the amino acid mutations L351Y, F405A and Y407V, and the second monomeric Fc polypeptide comprises the amino acid mutations T366L and T394W, and further comprises the amino acid mutation K392L; or
c) the first monomeric Fc polypeptide comprises the amino acid mutations L351Y, F405A and Y407V, and the second monomeric Fc polypeptide comprises the amino acid mutations T366I and T394W, and further comprises the amino acid mutation K392M; or
d) the first monomeric Fc polypeptide comprises the amino acid mutations F405A and Y407V, and the second monomeric Fc polypeptide comprises the amino acid mutations T366L and T394W; or
e) the first monomeric Fc polypeptide comprises the amino acid mutations F405A and Y407V, and the second monomeric Fc polypeptide comprises the amino acid mutations T366L, K392M and T394W; or
f) the first monomeric Fc polypeptide comprises the amino acid mutations F405A and Y407V, and further comprises the amino acid mutation T350V, and the second monomeric Fc polypeptide comprises the amino acid mutations T366L and T394W, and further comprises the amino acid mutations T350V and K392M; or
g) the first monomeric Fc polypeptide comprises the amino acid mutations L351Y, F405A and Y407V, and further comprises the amino acid mutation T350V, and the second monomeric Fc polypeptide comprises the amino acid mutations T366L and T394W, and further comprises the amino acid mutations T350V and K392M; or
h) the first monomeric Fc polypeptide comprises the amino acid mutations L351Y, F405A and Y407V, and further comprises the amino acid mutation T350V, and the second monomeric Fc polypeptide comprises the amino acid mutations T366L and T394W, and further comprises the amino acid mutations T350V and K392L; or
i) the first monomeric Fc polypeptide comprises the amino acid mutations L351Y, F405A and Y407V, and further comprises the amino acid mutations T350V and S400R, and the second monomeric Fc polypeptide comprises the amino acid mutations T366L and T394W, and further comprises the amino acid mutations T350V and K392M; or
j) the first monomeric Fc polypeptide comprises the amino acid mutations L351Y, F405A and Y407V, and further comprises the amino acid mutations T350V and S400E, and the second monomeric Fc polypeptide comprises the amino acid mutations T366L and T394W, and further comprises the amino acid mutations T350V, N390R and K392M; or
k) the first monomeric Fc polypeptide comprises the amino acid mutations L351Y, F405V and Y407V, and further comprises the amino acid mutations T350V and S400E, and the second monomeric Fc polypeptide comprises the amino acid mutations T366L and T394W, and further comprises the amino acid mutations T350V, N390R and K392M; or
l) the first monomeric Fc polypeptide comprises the amino acid mutations L351Y, F405T and Y407V, and further comprises the amino acid mutations T350V and S400E, and the second monomeric Fc polypeptide comprises the amino acid mutations T366L and T394W, and further comprises the amino acid mutations T350V, N390R and K392M; or
m) the first monomeric Fc polypeptide comprises the amino acid mutations L351Y, F405S and Y407V, and further comprises the amino acid mutations T350V and S400E, and the second monomeric Fc polypeptide comprises the amino acid mutations T366L and T394W, and further comprises the amino acid mutations T350V, N390R and K392M; or
n) the first monomeric Fc polypeptide comprises the amino acid mutations F405A and Y407V, and further comprises the amino acid mutations T350V and S400E, and the second monomeric Fc polypeptide comprises the amino acid mutations T366L and T394W, and further comprises the amino acid mutations T350V, N390R and K392M; or
o) the first monomeric Fc polypeptide comprises the amino acid mutations L351Y, F405A and Y407V, and further comprises the amino acid mutations T350V and S400E, and the second monomeric Fc polypeptide comprises the amino acid mutations T366L and T394W, and further comprises the amino acid mutations T350V, L351Y, N390R and K392M; or
p) the first monomeric Fc polypeptide comprises the amino acid mutations L351Y, F405A and Y407V, and further comprises the amino acid mutations Q347R, T350V and S400E, and the second monomeric Fc polypeptide comprises the amino acid mutations T366L and T394W, and further comprises the amino acid mutations T350V, K360E, N390R and K392M; or
q) the first monomeric Fc polypeptide comprises the amino acid mutations L351Y, F405A and Y407V, and further comprises the amino acid mutations T350V and S400R, and the second monomeric Fc polypeptide comprises the amino acid mutations T366L and T394W, and further comprises the amino acid mutations T350V, N390D and K392M; or
r) the first monomeric Fc polypeptide comprises the amino acid mutations L351Y, F405A and Y407V, and further comprises the amino acid mutations T350V and S400R, and the second monomeric Fc polypeptide comprises the amino acid mutations T366L and T394W, and further comprises the amino acid mutations T350V, N390E and K392M; or
s) the first monomeric Fc polypeptide comprises the amino acid mutations L351Y, F405A and Y407V, and further comprises the amino acid mutations T350V and S400E, the second monomeric Fc polypeptide comprises the amino acid mutations T366L and T394W, and further comprises the amino acid mutations T350V, N390R and K392L; or
t) the first monomeric Fc polypeptide comprises the amino acid mutations L351Y, F405A and Y407V, and further comprises the amino acid mutations T350V and S400E, and the second monomeric Fc polypeptide comprises the amino acid mutations T366L and T394W, and further comprises the amino acid mutations T350V, N390R and K392F; or
u) the first monomeric Fc polypeptide comprises the amino acid mutations F405A and Y407V, and further comprises the amino acid mutation Y349C, and the second monomeric Fc polypeptide comprises the amino acid mutations T366L and T394W, and further comprises the amino acid mutation S354C; or
v) the first monomeric Fc polypeptide comprises the amino acid mutations F405A and Y407V, and further comprises the amino acid mutations Y349C and D399C, and the second monomeric Fc polypeptide comprises the amino acid mutations T366L, and T394W, and further comprises the amino acid mutations S354C and K392C; or
w) the first monomeric Fc polypeptide comprises the amino acid mutations L351Y, F405A and Y407V, and further comprises the amino acid mutations Y349C, T350V and S400E, and the second monomeric Fc polypeptide comprises the amino acid mutations T366L and T394W, and further comprises the amino acid mutations T350V, S354C, N390R and K392M; or
x) the first monomeric Fc polypeptide comprises the amino acid mutations F405A and Y407V, and further comprises the amino acid mutations Y349C, T350V and S400E, and the second monomeric Fc polypeptide comprises the amino acid mutations T366L and T394W, and further comprises the amino acid mutations T350V, S354C, N390R and K392M; or
y) the first monomeric Fc polypeptide comprises the amino acid mutations F405A and Y407V, and further comprises the amino acid mutations Y349C and T350V, and the second monomeric Fc polypeptide comprises the amino acid mutations T366L and T394W, and further comprises the amino acid mutations T350V, S354C and K392M.

7. The isolated heteromultimer according to any one of the preceding claims, wherein the isolated heteromultimer is devoid of immunoglobulin first constant (CH1) regions.

8. The isolated heteromultimer according to any one of the preceding claims, comprising one single domain antigen-binding construct attached to one monomeric Fc polypeptide.

9. The isolated heteromultimer according to any one of claims 1 to 7, comprising a first single domain antigen-binding construct attached to one monomeric Fc polypeptide, and a second single domain antigen-binding construct attached to the other monomeric Fc polypeptide, wherein (a) both single domain antigen-binding constructs bind to the same epitope, or (b) the first single domain antigen-binding construct binds to one epitope, and the second single domain antigen-binding construct binds to a different epitope.

10. The isolated heteromultimer according to any one of the preceding claims, wherein the single-domain antigen-binding construct is a sdAb, VhH or V_{NAR}.

11. The isolated heteromultimer according to any one of the preceding claims, wherein the single domain antigen-binding construct binds to (i) a cytokine or chemokine selected from IL2, IFNa-2a/b, IFN-1a/b, IL-21, IL-17a, TNF, IL23, VEGF, and ANG2; (ii) a tumor associated antigen such as EpCam, EGFR, VEGFR, CEA, or GP100; (iii) an immunoregulatory antigen such as CD16, CD30, CD137, CD22, CD52, CD80, CD23, CD2, CD4, CD40, KIR, CD32b, CD25, LAG3, or B7-H3 or (iv) a bacterial toxin such as Clostridium difficile toxin A, or Clostridium difficile toxin B.

12. The isolated heteromultimer according to any one of the preceding claims, wherein the heterodimer Fc region has a purity greater than about 98%.

13. The isolated heteromultimer according to any one of the preceding claims, wherein the heterodimer Fc region is based on human IgG1.

14. The isolated heteromultimer according to any of one of the preceding claims, wherein at least one single domain antigen-binding construct binds EGFR or EGFRvIII.

15. The isolated heteromultimer according to any one of the preceding claims, wherein the heteromultimer is a bispecific antibody or a multispecific antibody.

16. The isolated heteromultimer according to any one of the preceding claims, wherein the single domain antigen-binding construct competes for binding with a therapeutic antibody, optionally the therapeutic antibody is selected from the group consisting of abagovomab, adalimumab, alemtuzumab, aurograb, bapineuzumab, basiliximab, belimumab, bevacizumab, briakinumab, canakinumab, catumaxomab, certolizumab pegol, cetuximab, daclizumab, denosumab, efalizumab, galiximab, gemtuzumab ozogamicin, golimumab, ibritumomab tiuxetan, infliximab, ipilimumab, lumiliximab, mepolizumab, motavizumab, muromonab, mycograb, natalizumab, nimotuzumab, ocrelizumab, ofatumumab, omalizumab, palivizumab, panitumumab, pertuzumab, ranibizumab, reslizumab, rituximab, teplizumab, tocilizumab/atlizumab, tositumomab, trastuzumab, Proxinium™, Rencarex™, ustekinumab, and zalutumumab.

17. The isolated heteromultimer according to any one of the preceding claims, wherein the heteromultimer is conjugated to a therapeutic agent.

18. A composition comprising the isolated heteromultimer according to any one of claims 1 to 17 and a pharmaceutically acceptable carrier.

19. The isolated heteromultimer according to any one of claims 1 to 17 for use in a method of treating cancer in a patient having a cancer **characterized by** a cancer antigen, optionally wherein the cancer is **characterized by** overexpression of EGFR or EFGRvIII, and/or wherein said cancer is breast cancer, lung cancer, anal cancer or glioblastoma.

20. A nucleic acid encoding the heteromultimer according to any one of claims 1 to 16.

21. A mammalian host cell comprising nucleic acid encoding the isolated heteromultimer according to any one of claims 1 to 16.

22. A method of preparing the heteromultimer according to any one of claims 1 to 16, comprising expressing a polynucleotide containing the nucleic acid of claim 21 in a cell, optionally wherein the cell is a mammalian cell.

## Patentansprüche

1. Isoliertes Heteromultimer, das eine Immunglobulin Heterodimer Fc-Region umfasst, die ein erstes monomeres Fc-Polypeptid und ein zweites monomeres Fc-Polypeptid umfasst und mindestens ein Einzeldomänen-Antigenbindungskonstrukt, das an ein monomeres Fc-Polypeptid gebunden ist,
wobei das isolierte Heteromultimer frei ist von leichten Immunglobulin-Ketten;
wobei die Heterodimer Fc-Region eine abweichende CH3-Region umfasst, die Aminosäuremutationen umfasst, welche die Bildung der Heterodimer Fc-Region im Vergleich zu einer homodimeren Fc-Region fördern, wobei die Aminosäuremutationen folgendes umfassen:
(a) eine Aminosäuremutation an der Position F405 und die Aminosäuremutationen L351Y und Y407V in dem ersten monomeren Fc-Polypeptid und die Aminosäuremutation T394W in dem zweiten monomeren Fc-Polypeptid, wobei die Aminosäuremutation an der Position F405 F405A, F405I, F405M, F405T, F405S, F405V oder F405W ist, oder
(b) die Aminosäuremutationen F405A und Y407V in dem ersten monomeren Fc-Polypeptid und die Aminosäuremutationen T366L oder T366I und T394W in dem zweiten monomeren Fc-Polypeptid, oder
(c) die Aminosäuremutationen L351Y, F405A und Y407V in dem ersten monomeren Fc-Polypeptid und die Aminosäuremutation T394W in dem zweiten monomeren Fc-Polypeptid, wobei die Aminosäuremutation an der Position T366 T366A, T366I, T366L, T366M, T366Y, T366S, T366C, T366V oder T366W ist; oder
(d) die Aminosäuremutation Y407A in dem ersten monomeren Fc-Polypeptid und die Aminosäuremutation K409F und eine der Aminosäuremutationen T366A, T366C, T366L, T366M oder T366S in dem zweiten monomeren Fc-Polypeptid;
(e) die Aminosäuremutationen L351Y und Y407A in dem ersten monomeren Fc-Polypeptid und Aminosäuremutation K409F und eine der Aminosäuremutationen T366A oder T366V in dem zweiten monomeren Fc-Polypeptid; oder
(f) die Aminosäuremutationen L351F und Y407A in dem ersten monomeren Fc-Polypeptid und die Aminosäuremutationen T366A und K409F in dem zweiten monomeren Fc-Polypeptid;
wobei die Heterodimer Fc-Region auf menschlichem Immunglobulin-G (IgG) basiert;
wobei die abweichende CH3-Domäne eine Schmelztemperatur (Tm) von 74 °C oder höher aufweist, und wobei die Heterodimer Fc-Region eine Reinheit von über etwa 90% aufweist;
wobei das Einzeldomänen-Antigenbindungskonstrukt ein Einzeldomänenantikörper (sdAb), ein kamelides Immunglobulin mit einzelner variabler Domäne (VhH), ein Knorpelfisch-Antikörperfragment (V_{NAR}), ein von SH3 abgeleitetes Fynomer oder eine von Fibronektin abgeleitete Bindungsdomäne ist, und
wobei die Nummerierung der Aminosäuren dem in der Kabat-Datenbank ausgeführten EU-Index entspricht.

2. Isoliertes Heteromultimer nach Anspruch 1(a), wobei die Aminosäuremutationen ferner eine Aminosäuremutation an der Position K392 in dem zweiten monomeren Fc-Polypeptid umfassen, wobei die Aminosäuremutation an der Position K392 K392L, K392M, K392F, K392V, K392R oder K392E ist.

3. Isoliertes Heteromultimer nach Anspruch 1(a) oder 2, wobei die Aminosäuremutationen ferner eine Aminosäuremutation an der Position T366 in dem zweiten monomeren Fc-Polypeptid umfassen, wobei die Aminosäuremutation an der Position T366 T366A, T366I, T366L, T366M, T366Y, T366S, T366C, T366V oder T366W ist.

4. Isoliertes Heteromultimer nach Anspruch 1(c), wobei die Aminosäuremutation an der Position T366 T366I oder T366L ist.

5. Isoliertes Heteromultimer nach Anspruch 1(b), wobei die Aminosäuremutationen ferner die Aminosäuremutation K392M in dem zweiten monomeren Fc-Polypeptid umfassen.

6. Isoliertes Heteromultimer nach Anspruch 1, wobei:
a) das erste monomere Fc-Polypeptid die Aminosäuren L351Y, F405A und Y407V umfasst, und wobei das zweite monomere Fc-Polypeptid die Aminosäuremutationen T366L und T394W umfasst, und wobei es ferner die Aminosäuremutation K392M umfasst; oder
b) das erste monomere Fc-Polypeptid die Aminosäuren L351Y, F405A und Y407V umfasst, und wobei das zweite monomere Fc-Polypeptid die Aminosäuremutationen T366L und T394W umfasst, und wobei es ferner die Aminosäuremutation K392L umfasst; oder
c) das erste monomere Fc-Polypeptid die Aminosäuren L351Y, F405A und Y407V umfasst, und wobei das zweite monomere Fc-Polypeptid die Aminosäuremutationen T366I und T394W, und wobei es ferner die Aminosäuremutation K392M umfasst; oder
d) das erste monomere Fc-Polypeptid die Aminosäuren F405A und Y407V umfasst, und wobei das zweite monomere Fc-Polypeptid die Aminosäuremutationen T366L und T394W umfasst; oder
e) das erste monomere Fc-Polypeptid die Aminosäuren F405A und Y407V umfasst, und wobei das zweite monomere Fc-Polypeptid die Aminosäuremutationen T366L, K392M und T394W umfasst; oder
f) das erste monomere Fc-Polypeptid die Aminosäuren F405A und Y407V umfasst, und wobei es ferner die Aminosäuremutation T350V umfasst, und wobei das zweite monomere Fc-Polypeptid die Aminosäuremutationen T366L und T394W umfasst, und wobei es ferner die Aminosäuremutationen T350V und K392M umfasst; oder
g) das erste monomere Fc-Polypeptid die Aminosäuren L351Y, F405A und Y407V umfasst, und wobei es ferner die Aminosäuremutation T350V umfasst, und wobei das zweite monomere Fc-Polypeptid die Aminosäuremutationen T366L und T394W umfasst, und wobei es ferner die Aminosäuremutationen T350V und K392M umfasst; oder
h) das erste monomere Fc-Polypeptid die Aminosäuren L351Y, F405A und Y407V umfasst, und wobei es ferner die Aminosäuremutation T350V umfasst, und wobei das zweite monomere Fc-Polypeptid die Aminosäuremutationen T366L und T394W umfasst, und wobei es ferner die Aminosäuremutationen T350V und K392L umfasst; oder
i) das erste monomere Fc-Polypeptid die Aminosäuren L351Y, F405A und Y407V umfasst, und wobei es ferner die Aminosäuremutationen T350V und S400R umfasst, und wobei das zweite monomere Fc-Polypeptid die Aminosäuremutationen T366L und T394W umfasst, und wobei es ferner die Aminosäuremutationen T350V und K392M umfasst; oder
j) das erste monomere Fc-Polypeptid die Aminosäuren L351Y, F405A und Y407V umfasst, und wobei es ferner die Aminosäuremutationen T350V und S400E umfasst, und wobei das zweite monomere Fc-Polypeptid die Aminosäuremutationen T366L und T394W umfasst, und wobei es ferner die Aminosäuremutationen T350V, N390R und K392M umfasst; oder
k) das erste monomere Fc-Polypeptid die Aminosäuren L351Y, F405V und Y407V umfasst, und wobei es ferner die Aminosäuremutationen T350V und S400E umfasst, und wobei das zweite monomere Fc-Polypeptid die Aminosäuremutationen T366L und T394W umfasst, und wobei es ferner die Aminosäuremutationen T350V, N390R und K392M umfasst; oder
l) das erste monomere Fc-Polypeptid die Aminosäuren L351Y, F405T und Y407V umfasst, und wobei es ferner die Aminosäuremutationen T350V und S400E umfasst, und wobei das zweite monomere Fc-Polypeptid die Aminosäuremutationen T366L und T394W umfasst, und wobei es ferner die Aminosäuremutationen T350V, N390R und K392M umfasst; oder
m) das erste monomere Fc-Polypeptid die Aminosäuren L351Y, F405S und Y407V umfasst, und wobei es ferner die Aminosäuremutationen T350V und S400E umfasst, und wobei das zweite monomere Fc-Polypeptid die Aminosäuremutationen T366L und T394W umfasst, und wobei es ferner die Aminosäuremutationen T350V, N390R und K392M umfasst; oder
n) das erste monomere Fc-Polypeptid die Aminosäuren F405A und Y407V umfasst, und wobei es ferner die Aminosäuremutationen T350V und S400E umfasst, und wobei das zweite monomere Fc-Polypeptid die Aminosäuremutationen T366L und T394W umfasst, und wobei es ferner die Aminosäuremutationen T350V, N390R und K392M umfasst; oder
o) das erste monomere Fc-Polypeptid die Aminosäuren L351Y, F405A und Y407V umfasst, und wobei es ferner die Aminosäuremutationen T350V und S400E umfasst, und wobei das zweite monomere Fc-Polypeptid die Aminosäuremutationen T366L und T394W umfasst, und wobei es ferner die Aminosäuremutationen T350V, L351Y, N390R und K392M umfasst; oder
p) das erste monomere Fc-Polypeptid die Aminosäuren L351Y, F405A und Y407V umfasst, und wobei es ferner die Aminosäuremutationen Q347R, T350V und S400E umfasst, und wobei das zweite monomere Fc-Polypeptid die Aminosäuremutationen T366L und T394W umfasst, und wobei es ferner die Aminosäuremutationen T350V, K360E, N390R und K392M umfasst; oder
q) das erste monomere Fc-Polypeptid die Aminosäuren L351Y, F405A und Y407V umfasst, und wobei es ferner die Aminosäuremutationen T350V und S400R umfasst, und wobei das zweite monomere Fc-Polypeptid die Aminosäuremutationen T366L und T394W, und wobei es ferner die Aminosäuremutationen T350V, N390D und K392M umfasst; oder
r) das erste monomere Fc-Polypeptid die Aminosäuren L351Y, F405A und Y407V umfasst, und wobei es ferner die Aminosäuremutationen T350V und S400R umfasst, und wobei das zweite monomere Fc-Polypeptid die Aminosäuremutationen T366L und T394W umfasst, und wobei es ferner die Aminosäuremutationen T350V, N390E und K392M umfasst; oder
s) das erste monomere Fc-Polypeptid die Aminosäuren L351Y, F405A und Y407V umfasst, und wobei es ferner die Aminosäuremutationen T350V und S400E umfasst, und wobei das zweite monomere Fc-Polypeptid die Aminosäuremutationen T366L und T394W umfasst, und wobei es ferner die Aminosäuremutationen T350V, N390R und K392L umfasst; oder
t) das erste monomere Fc-Polypeptid die Aminosäuren L351Y, F405A und Y407V umfasst, und wobei es ferner die Aminosäuremutationen T350V und S400E umfasst, und wobei das zweite monomere Fc-Polypeptid die Aminosäuremutationen T366L und T394W umfasst, und wobei es ferner die Aminosäuremutationen T350V, N390R und K392F umfasst; oder
u) das erste monomere Fc-Polypeptid die Aminosäuren F405A und Y407V umfasst, und wobei es ferner die Aminosäuremutation Y349C umfasst, und wobei das zweite monomere Fc-Polypeptid die Aminosäuremutationen T366L und T394W umfasst, und wobei es ferner die Aminosäuremutation S354C umfasst; oder
v) das erste monomere Fc-Polypeptid die Aminosäuren F405A und Y407V umfasst, und wobei es ferner die Aminosäuremutationen Y349C und D399C umfasst, und wobei das zweite monomere Fc-Polypeptid die Aminosäuremutationen T366L und T394W umfasst, und wobei es ferner die Aminosäuremutationen S354C und K392C umfasst; oder
w) das erste monomere Fc-Polypeptid die Aminosäuren L351Y, F405A und Y407V umfasst, und wobei es ferner die Aminosäuremutationen Y349C, T350V und S400E umfasst, und wobei das zweite monomere Fc-Polypeptid die Aminosäuremutationen T366L und T394W umfasst, und wobei es ferner die Aminosäuremutationen T350V, S354C, N390R und K392M umfasst; oder
x) das erste monomere Fc-Polypeptid die Aminosäuren F405A und Y407V umfasst, und wobei es ferner die Aminosäuremutationen Y349C, T350V und S400E umfasst, und wobei das zweite monomere Fc-Polypeptid die Aminosäuremutationen T366L und T394W umfasst, und wobei es ferner die Aminosäuremutationen T350V, S354C, N390R und K392M umfasst; oder
y) das erste monomere Fc-Polypeptid die Aminosäuren F405A und Y407V umfasst, und wobei es ferner die Aminosäuremutationen Y349C und T350V umfasst, und wobei das zweite monomere Fc-Polypeptid die Aminosäuremutationen T366L und T394W umfasst, und wobei es ferner die Aminosäuremutationen T350V, S354C und K392M umfasst.

7. Isoliertes Heteromultimer nach einem der vorstehenden Ansprüche, wobei das isolierte Heteromultimer frei ist von ersten konstanten Immunglobulin-Regionen (CH1).

8. Isoliertes Heteromultimer nach einem der vorstehenden Ansprüche, das ein Einzeldomänen-Antigenbindungskonstrukt umfasst, das an ein monomeres Fc-Polypeptid gebunden ist.

9. Isoliertes Heteromultimer nach einem der Ansprüche 1 bis 7, das ein erstes Einzeldomänen-Antigenbindungskonstrukt umfasst, das an ein monomeres Fc-Polypeptid gebunden ist und ein zweites Einzeldomänen-Antigenbindungskonstrukt, das an das andere monomere Fc-Polypeptid gebunden ist, wobei (a) beide Einzeldomänen-Antigenbindungskonstrukte das gleiche Epitop binden, oder (b) das erste Einzeldomänen-Antigenbindungskonstrukt an ein Epitop bindet und das zweite Einzeldomänen-Antigenbindungskonstrukt an ein anderes Epitop bindet.

10. Isoliertes Heteromultimer nach einem der vorstehenden Ansprüche, wobei das Einzeldomänen-Antigenbindungskonstrukt sdAb, VhH oder V_{NAR} ist.

11. Isoliertes Heteromultimer nach einem der vorstehenden Ansprüche, wobei das Einzeldomänen-Antigenbindungskonstrukt an folgendes bindet: (i) ein Zytokin oder ein Chemokin, ausgewählt aus IL2, IFNa-2a/b, IFN-1a/b, IL-21, IL-17a, TNF, IL23, VEGF und ANG2; (ii) ein Tumor assoziiertes Antigen, wie etwa EpCam, EGFR, VEGFR, CEA oder GP100; (iii) ein immunregulatorisches Antigen, wie etwa CD16, CD30, CD137, CD22, CD52, CD80, CD23, CD2, CD4, CD40, KIR, CD32b, CD25, LAG3 oder B7-H3, oder (iv) ein bakterielles Toxin, wie etwa Clostridium difficile Toxin A oder Clostridium difficile Toxin B.

12. Isoliertes Heteromultimer nach einem der vorstehenden Ansprüche, wobei die Heterodimer Fc-Region eine Reinheit von über etwa 98% aufweist.

13. Isoliertes Heteromultimer nach einem der vorstehenden Ansprüche, wobei die Heterodimer Fc-Region auf menschlichem IgG1 basiert.

14. Isoliertes Heteromultimer nach einem der vorstehenden Ansprüche, wobei mindestens ein Einzeldomänen-Antigenbindungskonstrukt an EGFR oder EGFRvIII bindet.

15. Isoliertes Heteromultimer nach einem der vorstehenden Ansprüche, wobei das Heteromultimer ein bispezifischer Antikörper oder ein multispezifischer Antikörper ist.

16. Isoliertes Heteromultimer nach einem der vorstehenden Ansprüche, wobei das Einzeldomänen-Antigenbindungskonstrukt zur Bindung mit einem therapeutischen Antikörper konkurriert, wobei der therapeutische Antikörper optional ausgewählt ist aus der Gruppe bestehend aus Abagovomab, Adalimumab, Alemtuzumab, Aurograb, Bapineuzumab, Basiliximab, Belimumab, Bevacizumab, Briakinumab, Canakinumab, Catumaxomab, Certolizumab pegol, Cetuximab, Daclizumab, Denosumab, Efalizumab, Galiximab, Gemtuzumab Ozogamicin, Golimumab, Ibritumomab-Tiuxetan, Infliximab, Ipilimumab, Lumiliximab, Mepolizumab, Motavizumab, Muromonab, Mycograb, Natalizumab, Nimotuzumab, Ocrelizumab, Ofatumumab, Omalizumab, Palivizumab, Panitumumab, Pertuzumab, Ranibizumab, Reslizumab, Rituximab, Teplizumab, Tocilizumab/Atlizumab, Tositumomab, Trastuzumab, Proxinium™, Rencarex™, Ustekinumab und Zalutumumab.

17. Isoliertes Heteromultimer nach einem der vorstehenden Ansprüche, wobei das Heteromultimer an ein Therapeutikum konjugiert ist.

18. Isoliertes Heteromultimer nach einem der Ansprüche 1 bis 17 und ein pharmazeutisch akzeptabler Träger.

19. Isoliertes Heteromultimer nach einem der Ansprüche 1 bis 17 zur Verwendung in einem Verfahren zur Krebsbehandlung in einem Patienten mit einer Krebserkrankung, **gekennzeichnet durch** ein Krebsantigen, wobei die Krebserkrankung optional **gekennzeichnet ist durch** eine Überexprimierung von EGFR oder EFGRvIII, und/oder wobei die Krebserkrankung Brustkrebs, Lungenkrebs, Anuskrebs oder ein Glioblastom ist.

20. Nukleinsäure, die für das Heteromultimer nach einem der Ansprüche 1 bis 16 codiert.

21. Säugetier-Wirtszelle, die Nukleinsäure umfasst, die für das isolierte Heteromultimer nach einem der Ansprüche 1 bis 16 codiert.

22. Verfahren zur Herstellung des Heteromultimers nach einem der Ansprüche 1 bis 16, umfassend das Exprimieren eines Polynukleotids, das die Nukleinsäure nach Anspruch 21 in einer Zelle enthält, wobei die Zelle optional eine Säugetierzelle ist.

## Revendications

1. Hétéromultimère isolé comprenant une région Fc d'hétérodimère d'immunoglobuline comprenant un premier polypeptide Fc monomère et un deuxième polypeptide Fc monomère et au moins une construction de liaison à l'antigène à domaine unique attachée à un polypeptide Fc monomère,
dans lequel l'hétéromultimère isolé est exempt de chaînes légères d'immunoglobuline ;
dans lequel la région Fc d'hétérodimère comprend une région CH3 variante comprenant des mutations d'acides aminés qui favorisent la formation de la région Fc d'hétérodimère en comparaison d'une région Fc homodimère, les mutations d'acides aminés comprenant :
(a) une mutation d'acide aminé à la position F405 et les mutations d'acides aminés L351Y et Y407V dans le première polypeptide Fc monomère, et la mutation d'acide aminé T394W dans le deuxième polypeptide Fc monomère, la mutation d'acide aminé à la position F405 étant F405A, F405I, F405M, F405T, F405S, F405V ou F405W, ou
(b) les mutations d'acides aminés F405A et Y407V dans le premier polypeptide Fc monomère, et les mutations d'acides aminés T366L ou T366I et T394W dans le deuxième polypeptide Fc monomère, ou
(c) les mutations d'acides aminés L351Y, F405A et Y407V dans le premier polypeptide Fc monomère, et une mutation d'acide aminé à la position T366 et la mutation d'acide aminé T394W dans le deuxième polypeptide Fc monomère, la mutation d'acide aminé à la position T366 étant T366A, T366I, T366L, T366M, T366Y, T366S, T366C, T366V ou T366W ; ou
(d) la mutation d'acide aminé Y407A dans le premier polypeptide Fc monomère et la mutation d'acide aminé K409F et une des mutations d'acides aminés T366A, T366C, T366L, T366M ou T366S dans le deuxième polypeptide Fc monomère ;
(e) les mutations d'acides aminés L351Y et Y407A dans le premier polypeptide Fc monomère, et la mutation d'acide aminé K409F et une des mutations d'acides aminés T366A ou T366V dans le deuxième polypeptide Fc monomère ; ou
(f) les mutations d'acides aminés L351F et Y407A dans le premier polypeptide Fc monomère, et les mutations d'acides aminés T366A et K409F dans le deuxième polypeptide Fc monomère ;
dans lequel la région Fc d'hétérodimère est basée sur une immunoglobuline humaine de type G (IgG);
dans lequel le domaine CH3 variant a une température de fusion (Tm) de 74 °C ou plus, et la région Fc d'hétérodimère a une pureté supérieure à environ 90 % ;
dans lequel la construction de liaison à l'antigène à domaine unique est un anticorps à domaine unique (sdAb), un domaine variable unique d'immunoglobuline de camélidé (VhH), un fragment d'anticorps de poisson cartilagineux (V_{NAR}), un fynomère dérivé de SH3 ou un domaine de liaison dérivé de fibronectine, et
dans lequel la numérotation des acides aminés est selon l'indice de l'UE tel qu'indiqué dans la numérotation de Kabat.

2. Hétéromultimère isolé selon la revendication 1(a), dans lequel les mutations d'acides aminés comprennent en outre une mutation d'acide aminé à la position K392 dans le deuxième polypeptide Fc monomère, la mutation d'acide aminé à la position K392 étant K392L, K392M, K392F, K392V, K392R ou K392E.

3. Hétéromultimère isolé selon la revendication 1(a) ou 2, dans lequel les mutations d'acides aminés comprennent en outre une mutation d'acide aminé à la position T366 dans le deuxième polypeptide Fc monomère, la mutation d'acide aminés à la position T366 étant T366A, T366I, T366L, T366M, T366Y, T366S, T366C, T366V ou T366W.

4. Hétéromultimère isolé selon la revendication 1(c), dans lequel la mutation d'acide aminé à la position T366 est T366I ou T366L.

5. Hétéromultimère isolé selon la revendication 1(b), dans lequel les mutations d'acides aminés comprennent en outre la mutation d'acide aminé K392M dans le deuxième polypeptide Fc monomère.

6. Hétéromultimère isolé selon la revendication 1, dans lequel :
a) le premier polypeptide Fc monomère comprend les mutations d'acides aminés L351Y, F405A et Y407V, et le deuxième polypeptide Fc monomère comprend les mutations d'acides aminés T366L et T394W, et comprend en outre la mutation d'acide aminé K392M ; ou
b) le premier polypeptide Fc monomère comprend les mutations d'acides aminés L351Y, F405A et Y407V, et le deuxième polypeptide Fc monomère comprend les mutations d'acides aminés T366L et T394W, et comprend en outre la mutation d'acide aminé K392L ; ou
c) le premier polypeptide Fc monomère comprend les mutations d'acides aminés L351Y, F405A et Y407V, et le deuxième polypeptide Fc monomère comprend les mutations d'acides aminés T366I et T394W, et comprend en outre la mutation d'acide aminé K392M ; ou
d) le premier polypeptide Fc monomère comprend les mutations d'acides aminés F405A et Y407V, et le deuxième polypeptide Fc monomère comprend les mutations d'acides aminés T366L et T394W ; ou
e) le premier polypeptide Fc monomère comprend les mutations d'acide aminé F405A et Y407V, et le deuxième polypeptide Fc monomère comprend les mutations d'acides aminés T366L, K392M et T394W ; ou
f) le premier polypeptide Fc monomère comprend les mutations d'acides aminés F405A et Y407V, et comprend en outre la mutation d'acide aminé T350V, et le deuxième polypeptide Fc monomère comprend les mutations d'acides aminés T366L et T394W, et comprend en outre les mutations d'acides aminés T350V et K392M ; ou
g) le premier polypeptide Fc monomère comprend les mutations d'acides aminés L351Y, F405A et Y407V, et comprend en outre la mutation d'acide aminé T350V, et le deuxième polypeptide Fc monomère comprend les mutations d'acides aminés T366L et T394W, et comprend en outre les mutations d'acides aminés T350V et K392M ; ou
h) le premier polypeptide Fc monomère comprend les mutations d'acides aminés L351Y, F405A and Y407V, et comprend en outre la mutation d'acide aminé T350V, et le deuxième polypeptide Fc monomère comprend les mutations d'acides aminés T366L et T394W, et comprend en outre les mutations d'acides aminés T350V et K392L ; ou
i) le premier polypeptide Fc monomère comprend les mutations d'acides aminés L351Y, F405A et Y407V, et comprend en outre les mutations d'acides aminés T350V et S400R, et le deuxième polypeptide Fc monomère comprend les mutations d'acides aminés T366L et T394W, et comprend en outre les mutations d'acides aminés T350V et K392M ; ou
j) le premier polypeptide Fc monomère comprend les mutations d'acides aminés L351Y, F405A et Y407V, et comprend en outre les mutations d'acides aminés T350V et S400E, et le deuxième polypeptide Fc monomère comprend les mutations d'acides aminés T366L et T394W, et comprend en outre les mutations d'acides aminés T350V, N390R et K392M ; ou
k) le premier polypeptide Fc monomère comprend les mutations d'acides aminés L351Y, F405V et Y407V, et comprend en outre les mutations d'acides aminés T350V et S400E, et le deuxième polypeptide Fc monomère comprend les mutations d'acides aminés T366L et T394W, et comprend en outre les mutations d'acides aminés T350V, N390R et K392M ; ou
l) le premier polypeptide Fc monomère comprend les mutations d'acides aminés L351Y, F405T et Y407V, et comprend en outre les mutations d'acides aminés T350V et S400E, et le deuxième polypeptide Fc monomère comprend les mutations d'acides aminés T366L et T394W, et comprend en outre les mutations d'acides aminés T350V, N390R et K392M ; ou
m) le premier polypeptide Fc monomère comprend les mutations d'acides aminés L351Y, F405S et Y407V, et comprend en outre les mutations d'acides aminés T350V et S400E, et le deuxième polypeptide Fc monomère comprend les mutations d'acides aminés T366L et T394W, et comprend en outre les mutations d'acides aminés T350V, N390R et K392M ; ou
n) le premier polypeptide Fc monomère comprend les mutations d'acides aminés F405A et Y407V, et comprend en outre les mutations d'acides aminés T350V et S400E, et le deuxième polypeptide Fc monomère comprend les mutations d'acides aminés T366L et T394W, et comprend en outre les mutations d'acides aminés T350V, N390R et K392M ; ou
o) le premier polypeptide Fc monomère comprend les mutations d'acides aminés L351Y, F405A et Y407V, et comprend en outre les mutations d'acides aminés T350V et S400E, et le deuxième polypeptide Fc monomère comprend les mutations d'acides aminés T366L et T394W, et comprend en outre les mutations d'acides aminés T350V, L351Y, N390R et K392M ; ou
p) le premier polypeptide Fc monomère comprend les mutations d'acides aminés L351Y, F405A et Y407V, et comprend en outre les mutations d'acides aminés Q347R, T350V et S400E, et le deuxième polypeptide Fc monomère comprend les mutations d'acides aminés T366L et T394W, et comprend en outre les mutations d'acides aminés T350V, K360E, N390R et K392M ; ou
q) le premier polypeptide Fc monomère comprend les mutations d'acides aminés L351Y, F405A et Y407V, et comprend en outre les mutations d'acides aminés T350V et S400R, et le deuxième polypeptide Fc monomère comprend les mutations d'acides aminés T366L et T394W, et comprend en outre les mutations d'acides aminés T350V, N390D et K392M ; ou
r) le premier polypeptide Fc monomère comprend les mutations d'acides aminés L351Y, F405A et Y407V, et comprend en outre les mutations d'acides aminés T350V et S400R, et le deuxième polypeptide Fc monomère comprend les mutations d'acides aminés T366L et T394W, et comprend en outre les mutations d'acides aminés T350V, N390E et K392M ; ou
s) le premier polypeptide Fc monomère comprend les mutations d'acides aminés L351Y, F405A et Y407V, et comprend en outre les mutations d'acides aminés T350V et S400E, le deuxième polypeptide Fc monomère comprend les mutations d'acides aminés T366L et T394W, et comprend en outre les mutations d'acides aminés T350V, N390R et K392L ; ou
t) le premier polypeptide Fc monomère comprend les mutations d'acides aminés L351Y, F405A et Y407V, et comprend en outre les mutations d'acides aminés T350V et S400E, et le deuxième polypeptide Fc monomère comprend les mutations d'acides aminés T366L et T394W, et comprend en outre les mutations d'acides aminés T350V, N390R et K392F ; ou
u) le premier polypeptide Fc monomère comprend les mutations d'acides aminés F405A et Y407V, et comprend en outre la mutation d'acide aminé Y349C, et le deuxième polypeptide Fc monomère comprend les mutations d'acides aminés T366L et T394W, et comprend en outre la mutation d'acide aminé S354C ; ou
v) le premier polypeptide Fc monomère comprend les mutations d'acides aminés F405A et Y407V, et comprend en outre les mutations d'acides aminés Y349C et D399C, et le deuxième polypeptide Fc monomère comprend les mutations d'acides aminés T366L etT394W, et comprend en outre les mutations d'acides aminés S354C et K392C ; ou
w) le premier polypeptide Fc monomère comprend les mutations d'acides aminés L351Y, F405A et Y407V, et comprend en outre les mutations d'acides aminés Y349C, T350V et S400E, et le deuxième polypeptide Fc monomère comprend les mutations d'acides aminés T366L et T394W, et comprend en outre les mutations d'acides aminés T350V, S354C, N390R et K392M ; ou
x) le premier polypeptide Fc monomère comprend les mutations d'acides aminés F405A et Y407V, et comprend en outre les mutations d'acides aminés Y349C, T350V et S400E, et le deuxième polypeptide Fc monomère comprend les mutations d'acides aminés T366L et T394W, et comprend en outre les mutations d'acides aminés T350V, S354C, N390R et K392M ; ou
y) le premier polypeptide Fc monomère comprend les mutations d'acides aminés F405A et Y407V, et comprend en outre les mutations d'acides aminés Y349C et T350V, et le deuxième polypeptide Fc monomère comprend les mutations d'acides aminés T366L et T394W, et comprend en outre les mutations d'acides aminés T350V, S354C et K392M.

7. Hétéromultimère isolé selon l'une quelconque des revendications précédentes, dans lequel l'hétéromultimère isolé est exempt de premières régions constantes (CH1) d'immunoglobuline.

8. Hétéromultimère isolé selon l'une quelconque des revendications précédentes, comprenant une construction de liaison à l'antigène à domaine unique attachée à un polypeptide Fc monomère.

9. Hétéromultimère isolé selon l'une quelconque des revendications 1 à 7, comprenant une première construction de liaison à l'antigène à domaine unique attachée à un polypeptide Fc monomère, et une deuxième construction de liaison à l'antigène à domaine unique attachée à l'autre polypeptide Fc monomère, dans lequel (a) les deux constructions de liaison à l'antigène à domaine unique se lient au même épitope, ou (b) la première construction de liaison à l'antigène à domaine unique se lie à un épitope, et la deuxième construction de liaison à l'antigène à domaine unique se lie à un épitope différent.

10. Hétéromultimère isolé selon l'une quelconque des revendications précédentes, dans lequel la construction de liaison à l'antigène à domaine unique est un sdAb, VhH ou V_{NAR}.

11. Hétéromultimère isolé selon l'une quelconque des revendications précédentes, dans lequel la construction de liaison à l'antigène à domaine unique se lie à (i) une cytokine ou une chimiokine choisie parmi IL2, IFNa-2a/b, IFN-1a/b, IL-21, IL-17a, TNF, IL23, VEGF et ANG2 ; (ii) un antigène associé à une tumeur tel qu'EpCam, EGFR, VEGFR, CEA ou GP100 ; (iii) un antigène immunorégulateur tel que CD16, CD30, CD137, CD22, CD52, CD80, CD23, CD2, CD4, CD40, KIR, CD32b, CD25, LAG3 ou B7-H3, ou (iv) une toxine bactérienne telle que la toxine A de Clostridium difficile ou la toxine B de Clostridium difficile.

12. Hétéromultimère isolé selon l'une quelconque des revendications précédentes, dans lequel la région Fc d'hétérodimère a une pureté supérieure à environ 98 %.

13. Hétéromultimère isolé selon l'une quelconque des revendications précédentes, dans lequel la région Fc d'hétérodimère est base sur l'IgG1 humaine.

14. Hétéromultimère isolé selon l'une quelconque des revendications précédentes, dans lequel au moins une construction de liaison à l'antigène à domaine unique se lie à EGFR ou EGFRvIII.

15. Hétéromultimère isolé selon l'une quelconque des revendications précédentes, dans lequel l'hétéromultimère est un anticorps bispécifique ou un anticorps multispécifique.

16. Hétéromultimère isolé selon l'une quelconque des revendications précédentes, dans lequel la construction de liaison à l'antigène à domaine unique rivalise pour se lier avec un anticorps thérapeutique, l'anticorps thérapeutique étant éventuellement choisi dans le groupe constitué par abagovomab, adalimumab, alemtuzumab, aurograb, bapineuzumab, basiliximab, belimumab, bévacizumab, briakinumab, canakinumab, catumaxomab, certolizumab pegol, cétuximab, daclizumab, denosumab, efalizumab, galiximab, gemtuzumab ozogamicine, golimumab, ibritumomab tiuxetan, infliximab, ipilimumab, lumiliximab, mepolizumab, motavizumab, muromonab, mycograb, natalizumab, nimotuzumab, ocrelizumab, ofatumumab, omalizumab, palivizumab, panitumumab, pertuzumab, ranibizumab, reslizumab, rituximab, teplizumab, tocilizumab/atlizumab, tositumomab, trastuzumab, Proxinium™, Rencarex™, ustekinumab et zalutumumab.

17. Hétéromultimère isolé selon l'une quelconque des revendications précédentes, dans lequel l'hétéromultimère est conjugué à un agent thérapeutique.

18. Composition comprenant l'hétéromultimère isolé selon l'une quelconque des revendications 1 à 17 et un véhicule pharmaceutiquement acceptable.

19. Hétéromultimère isolé selon l'une quelconque des revendications 1 à 17 pour une utilisation dans un procédé de traitement d'un cancer chez un patient ayant un cancer **caractérisé par** un antigène cancéreux, éventuellement dans lequel le cancer est **caractérisé par** une surexpression d'EGFR ou d'EFGRvIII, et/ou dans lequel ledit cancer est un cancer du sein, un cancer du poumon, un cancer anal ou un glioblastome.

20. Acide nucléique codant l'hétéromultimère selon l'une quelconque des revendications 1 à 16.

21. Cellule hôte mammalienne comprenant un acide nucléique codant l'hétéromultimère isolé selon l'une quelconque des revendications 1 à 16.

22. Procédé de préparation de l'hétéromultimère selon l'une quelconque des revendications 1 à 16, comprenant l'expression d'un polynucléotide contenant l'acide nucléique de la revendication 21 dans une cellule, éventuellement dans lequel la cellule est une cellule mammalienne.
